# EUROPEAN PATENT APPLICATION

(11) **EP 4 549 554 A2**
(43) Date of publication of application: **07.05.2025**
(21) Application number: 25151557.3
(22) Date of filing: 28.07.2018
(51) Int. Cl.: C12N 5/0783

(54) **REAGENTS FOR EXPANDING CELLS EXPRESSING RECOMBINANT RECEPTORS**

(30) Priority: 29.07.2017 US 201762538671 P; 08.12.2017 US 201762596742 P; 09.02.2018 US 201862628889 P; 01.05.2018 US 201862665468 P
(62) Divisional of application: 18759009.6
(71) Applicant: Juno Therapeutics, Inc., Seattle, WA 98109 (US)
(72) Inventor: HAUSKINS, Collin, Seattle 98109 (US); HUSSELL, Scott, Seattle 98109 (US); SIERRA, Catherine, Seattle 98109 (US); WORKS, Melissa, Seattle 98109 (US)
(74) Representative: Carpmaels & Ransford LLP

(57) **Abstract**

The present disclosure provides compositions and methods for stimulating, enriching, expanding, and/or activating engineered cells that express a recombinant receptor, e.g., a chimeric antigen receptor. In some embodiments, the provided methods include ex vivo or in vitro stimulation, enrichment, expansion, and/or activation of cells by incubation with a particle, e.g., a bead particle, attached to a binding molecule, such as a polypeptide antigen or anti-idiotype antibody, that recognizes or binds to the recombinant receptor. Also provided herein are methods for transfecting or transducing cells that have not been previously incubated with an activating or stimulating agent, such as have not been incubated with anti-CD3/anti-CD28 antibodies and/or one or more recombinant cytokines, by transducing or transfecting the cells in the presence of the particles with attached binding molecules. In some embodiments, the provided compositions can be used in methods to prepare cells, e.g., genetically engineered T cells, for of adoptive immunotherapy.

## Description

### Cross-Reference to Related Applications

The application claims the benefit of priority to U.S. provisional patent application 62/538,671, entitled "REAGENTS FOR EXPANDING CELLS EXPRESSING RECOMBINANT RECEPTORS" filed July 29, 2017; U.S. provisional patent application 62/596,742, entitled "REAGENTS FOR EXPANDING CELLS EXPRESSING RECOMBINANT RECEPTORS" filed December 8, 2017; U.S. provisional patent application 62/628,889, entitled "REAGENTS FOR EXPANDING CELLS EXPRESSING RECOMBINANT RECEPTORS" filed February 9, 2018; and U.S. provisional patent application 62/665,468, entitled "REAGENTS FOR EXPANDING CELLS EXPRESSING RECOMBINANT RECEPTORS" filed May 1, 2018; the contents of which are hereby incorporated by reference in their entirety for all purposes.

### Incorporation by Reference of Sequence Listing

The present application is being filed along with a Sequence Listing in electronic format. The Sequence Listing is provided as a file entitled 735042007340SeqList.TXT, created July 27, 2018, which is 123,336 bytes in size. The information in the electronic format of the Sequence Listing is incorporated by reference in its entirety.

### Field

The present disclosure provides compositions and methods for stimulating, enriching, expanding, and/or activating engineered cells that express a recombinant receptor, e.g., a chimeric antigen receptor. In some embodiments, the provided methods include the *ex vivo* or *in vitro* stimulation, enrichment, expansion, and/or activation of cells by incubation with a particle, e.g., a bead particle, with an attached binding molecule that recognizes or binds to the recombinant receptor. In some embodiments, the attached binding molecule is a polypeptide, e.g., a polypeptide antigen or an anti-idiotype antibody that binds to the recombinant receptor. In some embodiments, the provided compositions can be used in methods to prepare cells, e.g., genetically engineered T cells, for of adoptive immunotherapy.

### Background

Various strategies are available for stimulating or expanding cell populations *in vitro* or *ex vivo,* including for expanding antigen-specific T cells in vitro for use in adoptive cellular immunotherapy or cancer therapy. Improved strategies are needed for stimulating or expanding cell populations, including for research, diagnostic and therapeutic purposes. Provided are reagents, methods, and articles of manufacture and kits that meet such needs.

### Summary

Provided herein is a method of expanding cells, including incubating an input composition containing cells expressing a recombinant antigen receptor containing an extracellular antigen-binding domain that specifically binds or recognizes an antigen with a plurality of particles, each of the plurality of particles containing a binding molecule that specifically binds to the antigen-binding domain, wherein binding of the binding molecule to the antigen-binding domain induces expansion of the cells containing the recombinant antigen receptor, thereby producing an output composition containing expanded cells. In some embodiments, the recombinant antigen receptor is a chimeric antigen receptor (CAR). In some embodiments, the antigen-binding domain contains an antibody or antigen-binding fragment thereof. In some cases, the antigen-binding fragment is or contains a single chain antibody fragment. In some embodiments, the antigen-binding fragment thereof contains antibody variable regions joined by a flexible linker. In some of any such embodiments, the antigen-binding fragment thereof is or contains an scFv.

In some of any such embodiments, the antigen is selected from among αvβ6 integrin (avb6 integrin), B cell maturation antigen (BCMA), B7-H3, B7-H6, carbonic anhydrase 9 (CA9, also known as CAIX or G250), a cancer-testis antigen, cancer/testis antigen 1B (CTAG, also known as NY-ESO-1 and LAGE-2), carcinoembryonic antigen (CEA), a cyclin, cyclin A2, C-C Motif Chemokine Ligand 1 (CCL-1), CD19, CD20, CD22, CD23, CD24, CD30, CD33, CD38, CD44, CD44v6, CD44v7/8, CD123, CD138, CD171, chondroitin sulfate proteoglycan 4 (CSPG4), epidermal growth factor protein (EGFR), type III epidermal growth factor receptor mutation (EGFR vIII), epithelial glycoprotein 2 (EPG-2), epithelial glycoprotein 40 (EPG-40), ephrinB2, ephrin receptor A2 (EPHa2), estrogen receptor, Fc receptor like 5 (FCRL5; also known as Fc receptor homolog 5 or FCRH5), fetal acetylcholine receptor (fetal AchR), a folate binding protein (FBP), folate receptor alpha, ganglioside GD2, O-acetylated GD2 (OGD2), ganglioside GD3, glycoprotein 100 (gp100), G Protein Coupled Receptor 5D (GPRC5D), Her2/neu (receptor tyrosine kinase erb-B2), Her3 (erb-B3), Her4 (erb-B4), erbB dimers, Human high molecular weight-melanoma-associated antigen (HMW-MAA), hepatitis B surface antigen, Human leukocyte antigen A1 (HLA-A1), Human leukocyte antigen A2 (HLA-A2), IL-22 receptor alpha(IL-22Ra), IL-13 receptor alpha 2 (IL-13Ra2), kinase insert domain receptor (kdr), kappa light chain, L1 cell adhesion molecule (L1-CAM), CE7 epitope of L1-CAM, Leucine Rich Repeat Containing 8 Family Member A (LRRC8A), Lewis Y, Melanoma-associated antigen (MAGE)-A1, MAGE-A3, MAGE-A6, mesothelin, c-Met, murine cytomegalovirus (CMV), mucin 1 (MUC1), MUC16, natural killer group 2 member D (NKG2D) ligands, melan A (MART-1), neural cell adhesion molecule (NCAM), oncofetal antigen, Preferentially expressed antigen of melanoma (PRAME), progesterone receptor, a prostate specific antigen, prostate stem cell antigen (PSCA), prostate specific membrane antigen (PSMA), Receptor Tyrosine Kinase Like Orphan Receptor 1 (ROR1), survivin, Trophoblast glycoprotein (TPBG also known as 5T4), tumor-associated glycoprotein 72 (TAG72), vascular endothelial growth factor receptor (VEGFR), vascular endothelial growth factor receptor 2 (VEGFR2), Wilms Tumor 1 (WT-1), a pathogen-specific or pathogen-expressed antigen, or an antigen associated with a universal tag, and/or biotinylated molecules, and/or molecules expressed by HIV, HCV, HBV or other pathogens.

In some of any such embodiments, the antigen is selected from among ROR1, B cell maturation antigen (BCMA), carbonic anhydrase 9 (CAIX), Her2/neu (receptor tyrosine kinase erbB2), Ll-CAM, CD19, CD20, CD22, mesothelin, CEA, and hepatitis B surface antigen, anti-folate receptor, CD23, CD24, CD30, CD33, CD38, CD44, EGFR, epithelial glycoprotein 2 (EPG-2), epithelial glycoprotein 40 (EPG-40), EPHa2, erb-B2, erb-B3, erb-B4, erbB dimers, EGFR vIII, folate binding protein (FBP), FCRL5, FCRH5, fetal acetylcholine receptor, GD2, GD3, HMW-MAA, IL-22R-alpha, IL-13R-alpha2, kinase insert domain receptor (kdr), kappa light chain, Lewis Y, L1-cell adhesion molecule, (L1-CAM), Melanoma-associated antigen (MAGE)-A1, MAGE-A3, MAGE-A6, Preferentially expressed antigen of melanoma (PRAME), survivin, TAG72, B7-H6, IL-13 receptor alpha 2 (IL-13Ra2), CA9, GD3, HMW-MAA, CD171, G250/CAIX, HLA-AI MAGE Al, HLA-A2 NY-ESO-1, PSCA, folate receptor-a, CD44v6, CD44v7/8, avb6 integrin, 8H9, NCAM, VEGF receptors, 5T4, Foetal AchR, NKG2D ligands, CD44v6, dual antigen, a cancer-testes antigen, mesothelin, murine CMV, mucin 1 (MUC1), MUC16, PSCA, NKG2D, NY-ESO-1, MART-1, gp100, oncofetal antigen, ROR1, TAG72, VEGF-R2, carcinoembryonic antigen (CEA), prostate specific antigen, PSMA, Her2/neu, estrogen receptor, progesterone receptor, ephrinB2, CD123, c-Met, GD-2, O-acetylated GD2 (OGD2), CE7, Wilms Tumor 1 (WT-1), a cyclin, cyclin A2, CCL-1, CD138, a pathogen-specific antigen and an antigen associated with a universal tag.

In some of any such embodiments, the binding molecule does not bind or recognize a linker or spacer region of the recombinant antigen receptor, said linker or spacer region connecting the antigen-binding domain to the transmembrane domain of the antigen receptor. In some of any such embodiments, the binding molecule is an anti-idiotypic antibody or antigen-binding fragment thereof that specifically binds to the antigen-binding domain.

Provided herein is a method of expanding cells, including incubating an input composition containing cells expressing a chimeric antigen receptor (CAR) containing an antigen-binding domain that specifically binds or recognizes an antigen with a plurality of particles, each of the plurality of particles, e.g., beads, containing a binding molecule that is an anti-idiotypic antibody or antigen-binding fragment thereof that specifically binds to the antigen-binding domain, wherein binding of the anti-idiotypic antibody or antigen-binding fragment thereof to the antigen-binding domain induces expansion of the cells containing the chimeric antigen receptor, thereby producing an output composition containing expanded cells. In some of any such embodiments, the binding molecule contains a recombinant antigen or a portion thereof recognized by the antigen-binding domain.

Provided herein is a method of expanding cells, including incubating an input composition containing cells expressing a chimeric antigen receptor (CAR) containing an antigen-binding domain that specifically binds or recognizes an antigen with a plurality of particles, e.g., beads, each of the plurality of particles containing a binding molecule containing a recombinant antigen or a portion thereof recognized by the antigen-binding domain, wherein binding of the recombinant antigen or portion thereof to the antigen-binding domain induces expansion of the cells containing the chimeric antigen receptor, thereby producing an output composition containing expanded cells.

In some of any such embodiments, the antigen is selected from among αvβ6 integrin (avb6 integrin), B cell maturation antigen (BCMA), B7-H3, B7-H6, carbonic anhydrase 9 (CA9, also known as CAIX or G250), a cancer-testis antigen, cancer/testis antigen 1B (CTAG, also known as NY-ESO-1 and LAGE-2), carcinoembryonic antigen (CEA), a cyclin, cyclin A2, C-C Motif Chemokine Ligand 1 (CCL-1), CD19, CD20, CD22, CD23, CD24, CD30, CD33, CD38, CD44, CD44v6, CD44v7/8, CD123, CD133, CD138, CD171, chondroitin sulfate proteoglycan 4 (CSPG4), epidermal growth factor protein (EGFR), type III epidermal growth factor receptor mutation (EGFR vIII), epithelial glycoprotein 2 (EPG-2), epithelial glycoprotein 40 (EPG-40), ephrinB2, ephrin receptor A2 (EPHa2), estrogen receptor, Fc receptor like 5 (FCRL5; also known as Fc receptor homolog 5 or FCRH5), fetal acetylcholine receptor (fetal AchR), a folate binding protein (FBP), folate receptor alpha, ganglioside GD2, O-acetylated GD2 (OGD2), ganglioside GD3, glycoprotein 100 (gp100), glypican-3 (GPC3), G Protein Coupled Receptor 5D (GPRC5D), Her2/neu (receptor tyrosine kinase erb-B2), Her3 (erb-B3), Her4 (erb-B4), erbB dimers, Human high molecular weight-melanoma-associated antigen (HMW-MAA), hepatitis B surface antigen, Human leukocyte antigen A1 (HLA-A1), Human leukocyte antigen A2 (HLA-A2), IL-22 receptor alpha(IL-22Rα), IL-13 receptor alpha 2 (IL-13Rα2), kinase insert domain receptor (kdr), kappa light chain, L1 cell adhesion molecule (L1-CAM), CE7 epitope of L1-CAM, Leucine Rich Repeat Containing 8 Family Member A (LRRC8A), Lewis Y, Melanoma-associated antigen (MAGE)-A1, MAGE-A3, MAGE-A6, MAGE-A10, mesothelin (MSLN), c-Met, murine cytomegalovirus (CMV), mucin 1 (MUC1), MUC16, natural killer group 2 member D (NKG2D) ligands, melan A (MART-1), neural cell adhesion molecule (NCAM), oncofetal antigen, Preferentially expressed antigen of melanoma (PRAME), progesterone receptor, a prostate specific antigen, prostate stem cell antigen (PSCA), prostate specific membrane antigen (PSMA), Receptor Tyrosine Kinase Like Orphan Receptor 1 (ROR1), survivin, Trophoblast glycoprotein (TPBG also known as 5T4), tumor-associated glycoprotein 72 (TAG72), Tyrosinase related protein 1 (TRP1, also known as TYRP1 or gp75), Tyrosinase related protein 2 (TRP2, also known as dopachrome tautomerase, dopachrome delta-isomerase or DCT), vascular endothelial growth factor receptor (VEGFR), vascular endothelial growth factor receptor 2 (VEGFR2), Wilms Tumor 1 (WT-1), a pathogen-specific or pathogen-expressed antigen, or an antigen associated with a universal tag, and/or biotinylated molecules, and/or molecules expressed by HIV, HCV, HBV or other pathogens.

In some embodiments, the recombinant antigen is selected from among ROR1, B cell maturation antigen (BCMA), carbonic anhydrase 9 (CAIX), Her2/neu (receptor tyrosine kinase erbB2), Ll-CAM, CD19, CD20, CD22, mesothelin, CEA, and hepatitis B surface antigen, anti-folate receptor, CD23, CD24, CD30, CD33, CD38, CD44, EGFR, epithelial glycoprotein 2 (EPG-2), epithelial glycoprotein 40 (EPG-40), EPHa2, erb-B2, erb-B3, erb-B4, erbB dimers, EGFR vIII, folate binding protein (FBP), FCRL5, FCRH5, fetal acetylcholine receptor, GD2, GD3, HMW-MAA, IL-22R-alpha, IL-13R-alpha2, kinase insert domain receptor (kdr), kappa light chain, Lewis Y, L1-cell adhesion molecule, (L1-CAM), Melanoma-associated antigen (MAGE)-A1, MAGE-A3, MAGE-A6, Preferentially expressed antigen of melanoma (PRAME), survivin, TAG72, B7-H6, IL-13 receptor alpha 2 (IL-13Ra2), CA9, GD3, HMW-MAA, CD171, G250/CAIX, HLA-AI MAGE Al, HLA-A2 NY-ESO-1, PSCA, folate receptor-a, CD44v6, CD44v7/8, avb6 integrin, 8H9, NCAM, VEGF receptors, 5T4, Foetal AchR, NKG2D ligands, CD44v6, dual antigen, a cancer-testes antigen, mesothelin, murine CMV, mucin 1 (MUC1), MUC16, PSCA, NKG2D, NY-ESO-1, MART-1, gp100, oncofetal antigen, ROR1, TAG72, VEGF-R2, carcinoembryonic antigen (CEA), prostate specific antigen, PSMA, Her2/neu, estrogen receptor, progesterone receptor, ephrinB2, CD123, c-Met, GD-2, O-acetylated GD2 (OGD2), CE7, Wilms Tumor 1 (WT-1), a cyclin, cyclin A2, CCL-1, CD138 and a pathogen-specific antigen or a portion of any of the foregoing recognized by the antigen-binding domain. In some cases, the recombinant antigen is BCMA, CD22 or ROR1, or is a portion thereof recognized by the antigen-binding domain.

In some of any such embodiments, the portion of the recombinant antigen recognized by the antigen-binding domain contains the extracellular domain or a portion of the extracellular domain of the antigen. In some of any such embodiments, the portion of the recombinant antigen recognized by the antigen-binding domain contains essentially of the extracellular domain or a portion of the extracellular domain of the antigen.

In some embodiments, provided herein is a method of expanding cells, comprising incubating an input composition, said input composition comprising cells expressing a chimeric antigen receptor comprising an extracellular antigen-binding domain that specifically binds or recognizes an antigen, with a plurality of particles that are or comprise beads having attached a binding molecule that specifically binds to or recognizes the antigen-binding domain, wherein (i) the plurality of particles are from a composition having a concentration of the binding molecule of between or between about 0.5 µg/mL and 500 µg/mL, inclusive, and, during the incubating, the ratio of total cells present in the input composition to the plurality of particles is from or from about 5:1 to 1:5, inclusive; and (ii) binding of the binding molecule to the antigen-binding domain induces expansion of the cells comprising the chimeric antigen receptor, thereby producing an output composition comprising expanded cells.

Also provided herein is a method of expanding cells, including incubating an input composition containing cells expressing a chimeric antigen receptor (CAR) containing an antigen-binding domain that specifically binds or recognizes B cell maturation antigen (BCMA) with a plurality of particles, e.g., beads, each of the plurality of particles, e.g., beads, containing a binding molecule containing the extracellular domain of BCMA or a portion of the extracellular domain recognized by the antigen-binding domain, wherein binding of the extracellular domain of BCMA or portion thereof to the antigen-binding domain induces expansion of the cells containing the chimeric antigen receptor, thereby producing an output composition containing expanded cells. In some examples, the portion of BCMA consists essentially of the extracellular domain or a portion of the extracellular domain.

In some of any such embodiments, the binding molecule is a fusion polypeptide containing the recombinant antigen or the portion thereof linked to a moiety, optionally wherein the moiety facilitates attachment to the particle. In some cases, the moiety is linked to the C-terminus of the recombinant antigen. In some instances, the moiety is hydrophobic or is enriched in hydrophobic amino acids. In some embodiments, the moiety is or contains an Fc domain. In some examples, the Fc region is derived from human IgG. In some of any such embodiments, the antigen is CD19.

Also provided herein is a method of expanding cells, including incubating an input composition containing cells expressing a chimeric antigen receptor (CAR) containing an antigen-binding domain that specifically binds or recognizes CD19 with a plurality of particles, e.g., beads, each of the plurality of particles containing a binding molecule that is an anti-idiotypic antibody or antigen-binding fragment thereof that specifically binds to the antigen-binding domain, wherein binding of the anti-idiotypic antibody or antigen-binding fragment thereof to the antigen-binding domain induces expansion of the cells containing the chimeric antigen receptor, thereby producing an output composition containing expanded cells. In some of any such embodiments, the antigen-binding domain of the antigen receptor is or contains antibody SJ25C1 or an antigen-binding fragment thereof. In some of any such embodiments, the antigen-binding domain of the antigen receptor is or contains antibody FMC63 or an antigen-binding fragment thereof.

In some of any such embodiments, the antigen-binding fragment is or contains an scFv. In some of any such embodiments, the antigen or recombinant antigen is human. In some of any such embodiments, the anti-idiotypic antibody or antigen-binding fragment thereof contains at least a portion of an immunoglobulin constant region. In some examples, the at least a portion of an immunoglobulin constant region contains an Fc region or a portion of the Fc containing the CH2 and CH3 domains. In some embodiments, the constant region or Fc region is derived from human IgG.

In some of any such embodiments, the anti-idiotypic antibody or antigen-binding fragment thereof is an intact antibody or full-length antibody. In some of any such embodiments, the binding molecule is covalently or non-covalently attached to the particles, e.g., beads. In some of any such embodiments, the binding molecule is attached to each of the plurality of particles, e.g., beads, at or near the C-terminal amino acid residue of the binding molecule and/or attachment of the binding molecule to each of the plurality of particles, e.g., beads, is carried out such that the region or epitope of the binding molecule recognized by the antigen-binding domain of the antigen receptor is oriented so that it is capable of being recognized by the antigen receptor.

In some of any such embodiments, the particles, e.g., beads, are synthetic particles, insoluble particles, solid particles or are non-cellular particles. In some of any such embodiments disclosed herein, the particles are beads. In some of any such embodiments, the plurality of particles contains beads. In some of any such embodiments, the particles are or comprise one or more polymers or oligomers and/or are polymeric and/or oligomeric. In some of any such embodiments, the plurality of particles contains a mean diameter of between or between about 1 µm and 10 µm or between or between about 2 µm and 5 µm. In some of any such embodiments, the plurality of particles, e.g., beads, includes a mean diameter of about 2.8 µm. In some of any such embodiments, the plurality of particles, e.g., beads, includes a mean diameter of about 4.5 µm. In some of any such embodiments, the plurality of particles, e.g., beads, includes a mean density of between about 0.5 g/cm³ and 5.0 g/cm³ or between or between about 1 g/cm³ and about 2 g/cm³. In some of any such embodiments, the plurality of particles, e.g., beads, includes a mean density of about 1.3 g/cm³. In some of any such embodiments, the plurality of particles, e.g., beads, includes a mean density of about 1.5 g/cm³.

In some of any such embodiments, the plurality of particles, e.g., beads, is monodisperse. In some of any such embodiments, the binding molecule is covalently attached to the particles. In some of any such embodiments, the particle contains a surface exposed functional group for attachment of the binding molecule and/or wherein the binding molecule is covalently attached to the particle via a surface exposed functional group.

In some of any such embodiments, the surface exposed functional group is an amino group, a carboxyl group, a thiol group, an aldehyde group, a chloromethyl group, an epoxy group, a hydroxyl group, a tosyl group or a hydrazine group. In some embodiments, the surface exposed functional group is a tosyl group.

In some of any such embodiments, the plurality of particles, e.g., beads, are biocompatible or non-toxic to cells. In some of any such embodiments, the plurality of particles, e.g., beads, contains particles including glass, silica, polyesters of hydroxy carboxylic acids, polyanhydrides of dicarboxylic acids, copolymers of hydroxy carboxylic acids, copolymers dicarboxylic acids, or metal. In some of any such embodiments, the particles, e.g., beads, contain a surface including a polymer, a polysaccharide, a silica, a fatty acid, a carbon or a combination thereof. In some examples, the polymer is polyethylene glycol, poly(lactic-co-glycolic acid), polyglutaraldehyde, polyurethane, polystyrene, and polyvinyl alcohol or combinations thereof. In some of any such embodiments, the plurality of particles contain particles including a hydrophobic surface. In some of any such embodiments, the plurality of particles, e.g., beads, contain particles including a polystyrene surface.

In some of any such embodiments, the plurality of particles, e.g., beads, contains particles that are magnetic and/or include a magnetic core, a paramagnetic core or a superparamagnetic core.

In some of any such embodiments, the particles are from a composition having a concentration of the binding molecule is between or between about 0.5 µg/mL and 500 µg/mL, 1 µg/mL and 200 µg/mL or 5 µg/mL and 100 µg/mL, inclusive. In some of any such embodiments, the particles are from a composition having a concentration of the binding molecule is at least or at least about 1 µg/mL, 5 µg/mL, 10 µg/mL, 25 µg/mL, 50 µg/mL, 100 µg/mL or 200 µg/mL. In some of any such embodiments, each of the plurality of particles, e.g., beads, contains at least or about at least 10 copies, 10² copies, 10³ copies, 10⁴ copies, 10⁵ copies or 10⁶ copies of the binding molecule.

In some of any such embodiments, at least a portion of the incubation is performed in the presence of an agent that specifically binds to an additional molecule on the cell to provide an accessory signal and/or to block an inhibitory signal. In some of any such embodiments, the agent is provided together with the particles, e.g., beads, optionally the agent is contained by each of the plurality of particles or a subset thereof. In some of any such embodiments, the agent is provided separately from the plurality of particles, e.g., beads.

In some of any such embodiments, the particles, e.g., beads, further include an agent that specifically binds to an additional molecule on the cell to provide an accessory signal and/or to block an inhibitory signal. In some of any such embodiments, the agent is a ligand or is an antibody or antigen-binding fragment thereof. In some of any such embodiments, the molecule is a costimulatory molecule or is an activating co-receptor. In some of any such embodiments, the costimulatory molecule or activating co-receptor is OX-40, ICOS, DAP10, CD28 or 4-1BB. In some embodiments, the molecule is a ligand of an activating receptor or co-receptor, such as OX-40L, ICOSL, B7-1, B7-2 or 4-1BBL. In some of any such embodiments, the molecule is an inhibitory receptor. In some examples, the inhibitory receptor is CTLA-4, PD-1, LAG-3, Tim-3, BTLA or TIGIT. In some of any such embodiments, the agent is covalently attached to the particles, e.g., beads. In some embodiments, the molecule is a ligand of an inhibitory receptor, such as is PD-L1, PD-L2, CD155, CD112 or LIGHT.

In some of any such embodiments, the ratio, optionally molar or weight ratio, of the binding molecule and the agent contained by the particles, e.g., beads, is or is about 1:1. In some of any such embodiments, the ratio of total cells present in the input composition to particles, e.g., beads, is from or from about 5:1 to 1:5, 3:1 to 1:3 or 2:1 to 1:2. In some of any such embodiments, the ratio of total cells present in the input composition to particles, e.g., beads, is from or from about 1:0.1 to 1:5. In some of any such embodiments, the ratio of total cells present in the input composition to particles, e.g., beads, is or is about 1:1.

In some of any such embodiments, the incubation is carried out for at least or greater than or greater than about 2 hours, 4 hours, 8 hours, 12 hours, 24 hours, 2 days, 3 days, 4 days, 5 days, 6 days, 7 days, 8 days, 9 days, 10 days, 11 days, 12 days, 13 days, or 14 days. In some of any such embodiments, the incubation is carried out at a temperature between or between about 30 °C and 39 °C, inclusive. In some of any such embodiments, the incubation is carried out at a temperature of 37 ° ± 2.0 °C.

In some of any such embodiments, the cells include immune cells or induced pluripotent stem cells (iPSC). In some of any such embodiments, the immune cell is a T cell or an NK cell. In some of any such embodiments, the cells contain CD4+ and/or CD8+ T cells. In some of any such embodiments, the ratio of the CD4+ cells to the CD8+ cells is or is about 1:1, 1:2, 2:1, 1:3 or 3:1. In some of any such embodiments, the cells are primary cells obtained from a subject, optionally a human subject. In some of any such embodiments, the cells are human.

In some of any such embodiments, the input composition is produced by a method including contacting a composition of cells with a nucleic acid molecule encoding the recombinant antigen receptor under conditions to introduce the nucleic acid molecule into one or more cells in the composition.

Also provided herein is a method of genetically engineering a cell, including contacting a composition of cells with a nucleic acid molecule encoding a recombinant antigen receptor under conditions to introduce the nucleic acid molecule into one or more cells in the composition, thereby producing an input composition; and incubating cells of the input composition according to the methods described herein. In some embodiments, at least a portion of the contacting and incubating are carried out simultaneously.

In some of any such embodiments, the nucleic acid molecule is included in a viral vector, an episomal vector or a transposon. In some of any such embodiments, the contacting is carried out by transposon/transposase gene transfer. In some of any such embodiments, the contacting is carried out by transduction with a viral vector. In some embodiments, the viral vector is a retrovirus, which optionally is a gamma-retroviral vector or a lentiviral vector.

In some instances, the contacting includes a step of spinoculating the viral vector with the composition of cells. In some cases, spinoculating includes rotating, in an internal cavity of a centrifugal chamber, the viral vector particles and composition of cells, wherein the rotation is at a relative centrifugal force at an internal surface of the side wall of the cavity that is between or between about 500 g and 2500 g, 500 g and 2000 g, 500 g and 1600 g, 500 g an 1000 g, 600 g and 1600 g, 600 g and 1000 g, 1000 g and 2000 g or 1000 g and 1600 g, each inclusive; or at least or at least about 600 g, 800 g, 1000 g, 1200 g, 1600 g, or 2000 g. In some embodiments, spinoculating is for a time that is greater than or about 5 minutes, greater than or about 10 minutes, greater than or about 15 minutes, greater than or about 20 minutes, greater than or about 30 minutes, greater than or about 45 minutes, greater than or about 60 minutes, greater than or about 90 minutes or greater than or about 120 minutes; or between or between about 5 minutes and 60 minutes, 10 minutes and 60 minutes, 15 minutes and 60 minutes, 15 minutes and 45 minutes, 30 minutes and 60 minutes or 45 minutes and 60 minutes, each inclusive.

In some of any such embodiments, the contacting is carried out in the presence of a transduction adjuvant. In some of any such embodiments, the composition of cells contains a plurality of T cells and, prior to the contacting, the method does not include stimulating or activating the T cells.

In some of any such embodiments, the composition of cells contains a plurality of T cells and, prior to the contacting, the method does not include incubating the composition in the presence of an agent or agents capable of inducing a signal through a TCR complex and/or incubation in the presence of an agent or agents capable of inducing proliferation of T cells, CD4+ T cells, and/or CD8+ T cells; and/or CD3-binding molecules, CD28-binding molecules, recombinant IL-2, recombinant IL-15, and recombinant IL-7 or a combination thereof. In some embodiments, prior to the contacting, the method does not include stimulating the T cells in the presence of an anti-CD3 antibody and/or an anti-CD28 antibody.

In some of any such embodiments, the composition of cells contains a plurality of T cells, said plurality of cells having been obtained from a sample from a subject, wherein the contacting is initiated no more than 24 hours after obtaining the sample from the subject; and/or prior to the contacting, the T cells have not been subjected to a temperature greater than or greater than about 15° C, about 18 ° C, about 22 ° C or about 25 ° C for a duration of more than 1 hour, 2 hours, 4 hours, 6 hours, 8 hours, 12 hours, or 24 hours after obtaining the sample from the subject; and/or prior to the contacting, the T cells have not been subjected to a temperature of, of about, greater than, or greater than about 37 ° ± 2.0 °C for a duration of more than 15 minutes, 30 minutes, 1 hour or 2 hours after obtaining the sample from the subject. In some of any such embodiments, prior to said contacting, no more than 5 %, 10 %, 20 %, 30 %, or 40 % of the T cells are activated cells, express a surface marker selected from the group consisting of HLA-DR, CD25, CD69, CD71, CD40L and 4-1BB; include intracellular expression of a cytokine selected from the group consisting of IL-2, IFN-gamma, TNF-alpha, are in the G1 or later phase of the cell cycle and/or are capable of proliferating.

In some of any such embodiments, the method further includes, prior to the incubation or the contacting, obtaining a biological sample from the subject containing the cells and, optionally, selecting or enriching the cells, optionally T cells, from the sample. In some of any such embodiments, the percent of cells expressing the recombinant antigen receptor in the input composition is less than or less than about 75%, 70%, 60%, 50%, 40%, 30%, 20%, 15%, 10% or less. In some of any such embodiments, the composition of cells or the input composition contains at least or at least about 1 x 10² cells, 1 x 10³ cells, 1 x 10⁴ cells, 1 x 10⁵ cells, 1 x 10⁶ cells or 1 x 10⁷ cells.

In some of any such embodiments, the surface expression of an activation marker or exhaustion marker of cells present in the output composition is less than surface expression of the marker in a composition of cells produced after a similar incubation but in the presence of polyclonal stimulatory molecule capable of activating one or more intracellular signaling domains of one or more components of a TCR complex. In some cases, the exhaustion marker is an inhibitory receptor. In some instances, the exhaustion marker is PD-1, CTLA-4, TIM-3, LAG-3, BTLA or TIGIT. In some examples, the activation marker is HLA-DR, CD25, CD69, CD71, CD40L or 4-1BB. In some of any such embodiments, the surface expression is at least or at least about 1.2-fold, 1.5-fold, 2.0-fold, 3.0-fold, 4.0-fold, 5.0-fold, 10.0-fold or more.

In some of any such embodiments, the number of cells in the output composition is substantially the same or is greater than the number of cells in a composition of cells produced by a similar incubation but in the presence of a polyclonal stimulatory molecule capable of activating one or more intracellular signaling domains of one or more components of a TCR complex. In some of any such embodiments, the polyclonal stimulatory molecule contains an anti-CD3 antibody or fragment and/or an anti-CD28 antibody or fragment.

In some of any such embodiments, the number of the cells in the output composition is greater than the number of the cells in the input composition by greater than or greater than about 1.2-fold, 1.5-fold, 2.0-fold, 3.0-fold, 4.0-fold, 5.0-fold, 10.0-fold, 25-fold, 50-fold, 100-fold or more. In some of any such embodiments, the percent of cells containing the recombinant antigen receptor in the output composition is greater than or greater than about 50%, 60%, 70%, 80%, 90%, 95% or more. In some of any such embodiments, the number of cells in the output composition containing the recombinant antigen receptor is increased or enriched by 1.2-fold, 2.0-fold, 3.0-fold, 4.0-fold, 5.0-fold, 6.0-fold, 7.0-fold, 8.0-fold, 9.0-fold, 10-fold or more compared to the number of the cells containing the antigen receptor in the input composition.

In some of any such embodiments, at least a portion of the incubation is carried out in the presence of one or more additional agents that modulate cell expansion or activity. In some cases, the one or more additional agent is lenalidomide.

In some of any such embodiments, the method is performed in vitro or ex vivo. In some of any such embodiments, the antigen receptor is a CAR and the CAR further an intracellular signaling domain containing an ITAM. In some cases, the intracellular signaling domain contains an intracellular domain of a CD3-zeta (CD3ζ) chain. In some embodiments, the CAR further contains a costimulatory signaling region. In some aspects, the costimulatory signaling region contains a signaling domain of CD28 or 4-1BB. In some examples, the costimulatory domain is CD28. In some of any such embodiments, the method further includes removing the plurality of particles, e.g., beads, from the output composition.

Provided herein is a composition of cells produced by any of the methods provided herein. Also provided herein is a surface modified particle containing a particle and a binding molecule bound to the surface of the particle, wherein the binding molecule specifically binds to an extracellular antigen-binding domain of an antigen receptor. In some embodiments, the antigen receptor is a chimeric antigen receptor (CAR). In some embodiments, the binding molecule does not bind or recognize a linker or spacer region of the recombinant antigen receptor, said linker or spacer region connecting the antigen-binding domain to the transmembrane domain of the antigen receptor.

In some of any such embodiments, the recombinant antigen is selected from among αvβ6 integrin (avb6 integrin), B cell maturation antigen (BCMA), B7-H3, B7-H6, carbonic anhydrase 9 (CA9, also known as CAIX or G250), a cancer-testis antigen, cancer/testis antigen 1B (CTAG, also known as NY-ESO-1 and LAGE-2), carcinoembryonic antigen (CEA), a cyclin, cyclin A2, C-C Motif Chemokine Ligand 1 (CCL-1), CD19, CD20, CD22, CD23, CD24, CD30, CD33, CD38, CD44, CD44v6, CD44v7/8, CD123, CD133, CD138, CD171, chondroitin sulfate proteoglycan 4 (CSPG4), epidermal growth factor protein (EGFR), type III epidermal growth factor receptor mutation (EGFR vIII), epithelial glycoprotein 2 (EPG-2), epithelial glycoprotein 40 (EPG-40), ephrinB2, ephrin receptor A2 (EPHa2), estrogen receptor, Fc receptor like 5 (FCRL5; also known as Fc receptor homolog 5 or FCRH5), fetal acetylcholine receptor (fetal AchR), a folate binding protein (FBP), folate receptor alpha, ganglioside GD2, O-acetylated GD2 (OGD2), ganglioside GD3, glycoprotein 100 (gp100), glypican-3 (GPC3), G Protein Coupled Receptor 5D (GPRC5D), Her2/neu (receptor tyrosine kinase erb-B2), Her3 (erb-B3), Her4 (erb-B4), erbB dimers, Human high molecular weight-melanoma-associated antigen (HMW-MAA), hepatitis B surface antigen, Human leukocyte antigen A1 (HLA-A1), Human leukocyte antigen A2 (HLA-A2), IL-22 receptor alpha(IL-22Rα), IL-13 receptor alpha 2 (IL-13Rα2), kinase insert domain receptor (kdr), kappa light chain, L1 cell adhesion molecule (L1-CAM), CE7 epitope of L1-CAM, Leucine Rich Repeat Containing 8 Family Member A (LRRC8A), Lewis Y, Melanoma-associated antigen (MAGE)-A1, MAGE-A3, MAGE-A6, MAGE-A10, mesothelin (MSLN), c-Met, murine cytomegalovirus (CMV), mucin 1 (MUC1), MUC16, natural killer group 2 member D (NKG2D) ligands, melan A (MART-1), neural cell adhesion molecule (NCAM), oncofetal antigen, Preferentially expressed antigen of melanoma (PRAME), progesterone receptor, a prostate specific antigen, prostate stem cell antigen (PSCA), prostate specific membrane antigen (PSMA), Receptor Tyrosine Kinase Like Orphan Receptor 1 (ROR1), survivin, Trophoblast glycoprotein (TPBG also known as 5T4), tumor-associated glycoprotein 72 (TAG72), Tyrosinase related protein 1 (TRP1, also known as TYRP1 or gp75), Tyrosinase related protein 2 (TRP2, also known as dopachrome tautomerase, dopachrome delta-isomerase or DCT), vascular endothelial growth factor receptor (VEGFR), vascular endothelial growth factor receptor 2 (VEGFR2), Wilms Tumor 1 (WT-1), a pathogen-specific or pathogen-expressed antigen, or an antigen associated with a universal tag, and/or biotinylated molecules, and/or molecules expressed by HIV, HCV, HBV or other pathogens.

In some of any such embodiments, the binding molecule contains a recombinant antigen or a portion thereof recognized by the antigen-binding domain. In some aspects, the recombinant antigen is selected from among ROR1, B cell maturation antigen (BCMA), carbonic anhydrase 9 (CAIX), Her2/neu (receptor tyrosine kinase erbB2), Ll-CAM, CD19, CD20, CD22, mesothelin, CEA, and hepatitis B surface antigen, anti-folate receptor, CD23, CD24, CD30, CD33, CD38, CD44, EGFR, epithelial glycoprotein 2 (EPG-2), epithelial glycoprotein 40 (EPG-40), EPHa2, erb-B2, erb-B3, erb-B4, erbB dimers, EGFR vIII, folate binding protein (FBP), FCRL5, FCRH5, fetal acetylcholine receptor, GD2, GD3, HMW-MAA, IL-22R-alpha, IL-13R-alpha2, kinase insert domain receptor (kdr), kappa light chain, Lewis Y, L1-cell adhesion molecule, (L1-CAM), Melanoma-associated antigen (MAGE)-A1, MAGE-A3, MAGE-A6, Preferentially expressed antigen of melanoma (PRAME), survivin, TAG72, B7-H6, IL-13 receptor alpha 2 (IL-13Ra2), CA9, GD3, HMW-MAA, CD171, G250/CAIX, HLA-AI MAGE Al, HLA-A2 NY-ESO-1, PSCA, folate receptor-a, CD44v6, CD44v7/8, avb6 integrin, 8H9, NCAM, VEGF receptors, 5T4, Foetal AchR, NKG2D ligands, CD44v6, dual antigen, a cancer-testes antigen, mesothelin, murine CMV, mucin 1 (MUC1), MUC16, PSCA, NKG2D, NY-ESO-1, MART-1, gp100, oncofetal antigen, ROR1, TAG72, VEGF-R2, carcinoembryonic antigen (CEA), prostate specific antigen, PSMA, Her2/neu, estrogen receptor, progesterone receptor, ephrinB2, CD123, c-Met, GD-2, O-acetylated GD2 (OGD2), CE7, Wilms Tumor 1 (WT-1), a cyclin, cyclin A2, CCL-1, CD138 and a pathogen-specific antigen or a portion of any of the foregoing recognized by the antigen-binding domain.

In some embodiments, the recombinant antigen is CD19, BCMA, CD22 or ROR1, or is a portion thereof recognized by the antigen-binding domain. In some of any such embodiments, the portion of the recombinant antigen recognized by the antigen-binding domain contains the extracellular domain or a portion of the extracellular domain of the antigen. In some of any such embodiments, the portion of the recombinant antigen recognized by the antigen-binding domain consists essentially of the extracellular domain or a portion of the extracellular domain of the antigen.

Provided herein is a surface modified particle, containing a particle and a binding molecule bound to the surface of the particle, wherein the binding molecule contains an extracellular domain or a portion thereof of B cell maturation antigen (BCMA). In some of any such embodiments, the binding molecule is a fusion polypeptide containing the recombinant antigen or the portion thereof linked to a moiety, optionally wherein the moiety facilitates attachment to the particle. In some cases, the moiety is linked to the C-terminus of the recombinant antigen. In some embodiments, the moiety is hydrophobic or is enriched in hydrophobic amino acids.

In some of any such embodiments, the moiety is or contains an Fc domain. In some instances, the Fc region is derived from human IgG. In some of any such embodiments, the recombinant antigen is human.

In some of any such embodiments, the binding molecule contains an anti-idiotypic antibody or antigen-binding fragment thereof. In some of any such embodiments, the antigen recognized by the antigen-binding domain is CD19. In some of any such embodiments, the antigen-binding domain of the antigen receptor is or contains antibody SJ25C1 or an antigen-binding fragment thereof. In some embodiments, the antigen-binding domain of the antigen receptor is or contains antibody FMC63 or an antigen-binding fragment thereof. In some embodiments, the antigen-binding fragment is or contains an scFv.

In some of any such embodiments, the anti-idiotypic antibody or antigen-binding fragment thereof contains at least a portion of an immunoglobulin constant region. In some instances, the at least a portion of an immunoglobulin constant region contains an Fc region or a portion of the Fc containing the CH2 and CH3 domains. In some embodiments, the constant region or Fc region is derived from human IgG. In some of any such embodiments, the anti-idiotypic antibody or antigen-binding fragment thereof is an intact antibody or full-length antibody.

In some of any such embodiments, the binding molecule is covalently or non-covalently attached to the particles, e.g., beads. In some of any such embodiments, the binding molecule is attached to each of the plurality of particles, e.g., beads, at or near the C-terminal amino acid residue of the binding molecule and/or attachment of the binding molecule to each of the plurality of particles, e.g., beads, is carried out such that the region or epitope of the binding molecule recognized by the antigen-binding domain of the antigen receptor is oriented so that it is capable of being recognized by the antigen receptor. In some of any such embodiments, the particles, e.g., beads, are synthetic particles, insoluble particles, solid particles or are non-cellular particles.

In some of any such embodiments, the plurality of particles, e.g., beads, contains beads. In some of any such embodiments, the particle has a diameter of between or between about 1 µm and 10 µm or between or between about 2 µm and 5 µm, each inclusive. In some of any such embodiments, the particle has a diameter of about 2.8 µm. In some of any such embodiments, the particle has a diameter of about 4.5 µm. In some of any such embodiments, the binding molecule is covalently attached to the particles, e.g., beads.

In some of any such embodiments, the particle contains a surface exposed functional group for attachment of the binding molecule and/or wherein the binding molecule is covalently attached to the particle via a surface exposed functional group. In some embodiments, the surface exposed functional group is an amino group, a carboxyl group, a thiol group, an aldehyde group, a chloromethyl group, an epoxy group, a hydroxyl group, a tosyl group or a hydrazine group. In some cases, the surface exposed functional group is a tosyl group.

In some of any such embodiments, the particle is biocompatible or non-toxic to cells. In some of any such embodiments, the plurality of particles, e.g., beads, include particles, e.g., beads, containing glass, silica, polyesters of hydroxy carboxylic acids, polyanhydrides of dicarboxylic acids, copolymers of hydroxy carboxylic acids, copolymers dicarboxylic acids, or metal. In some of any such embodiments, the particles, e.g., beads, contain a surface including a polymer, a polysaccharide, a silica, a fatty acid, a carbon or a combination thereof. In some instances, the polymer is polyethylene glycol, poly(lactic-co-glycolic acid), polyglutaraldehyde, polyurethane, polystyrene, and polyvinyl alcohol or combinations thereof.

In some of any such embodiments, the particle contains a hydrophobic surface. In some of any such embodiments, the particles, e.g., beads, include a polystyrene surface. In some of any such embodiments, the particle is magnetic and/or contains a magnetic core, a paramagnetic core or a superparamagnetic core. In some of any such embodiments, the particle contains at least or about at least 10 copies, 10² copies, 10³ copies, 10⁴ copies, 10⁵ copies or 10⁶ copies of the binding molecule.

In some of any such embodiments, the particle further contains an agent that specifically binds to an additional molecule on the cell to provide an accessory signal and/or to block an inhibitory signal, thereby modulating expansion of the cells. In some of any such embodiments, the agent is a ligand or is an antibody or antigen-binding fragment thereof. In some embodiments, the molecule is a costimulatory molecule or is an activating co-receptor. In some examples, the costimulatory molecule or activating co-receptor is OX-40, ICOS, DAP10, CD28 or 4-1BB. In some embodiments, the molecule is a ligand of an activating receptor or co-receptor, such as OX-40L, ICOSL, B7-1, B7-2 or 4-1BBL. In some cases, the molecule is an inhibitory receptor. In some examples, the inhibitory receptor is CTLA-4, PD-1, LAG-3, Tim-3, BTLA or TIGIT. In some embodiments, the molecule is a ligand of an inhibitory receptor, such as is PD-L1, PD-L2, CD155, CD112 or LIGHT.

In some of any such embodiments, the agent is covalently attached to the particles, e.g., beads. In some of any such embodiments, the ratio, optionally molar or weight ratio, of the binding molecule and the agent contained by the particle is or is about 1:1.

Provided herein is a composition containing a plurality of the surface modified particles, e.g., beads, described herein. In some embodiments, the particles are from a composition having a concentration of the binding molecule is between or between about 0.5 µg/mL and 500 µg/mL, 1 µg/mL and 200 µg/mL or 5 µg/mL and 100 µg/mL, each inclusive. In some embodiments, the particles are from a composition having a concentration of the binding molecule that is at least or at least about 1 µg/mL, 5 µg/mL, 10 µg/mL, 25 µg/mL, 50 µg/mL, 100 µg/mL or 200 µg/mL. In some of any such embodiments, the composition is monodisperse.

Also provided herein is a kit containing any of the particles, e.g., beads, described herein or the any of the composition described herein and instructions for use. In some cases, instructions are for selecting or enriching, from a population of cells, cells expressing an antigen receptor containing an antigen-binding domain specifically recognized by the binding molecule. In some instances, instructions are for expanding, from a population of cells, cells expressing an antigen receptor containing an antigen-binding domain specifically recognized by the binding molecule. In some embodiments, the percent of cells expressing a recombinant antigen receptor in the population of cells in less than or less than about 75%, 70%, 60%, 50%, 40%, 30%, 20%, 15%, 10% or less.

Provided here is a method for expanding cells including incubating a population of cells with any of the particles, e.g., beads, described herein or any of the compositions described herein. Also provided is a method of selecting or enriching cells including contacting a population of cells with any of the particles, e.g., beads, described herein or any of the compositions described herein.

Provided herein is a long-term stimulation method for assessing a cell composition including incubating, for a period of time of at least 10 days, an input composition under conditions to stimulate a CAR-dependent activity in cells in the input composition, said input composition containing T cells expressing a chimeric antigen receptor (CAR) containing an extracellular antigen-binding domain that specifically binds or recognizes an antigen, thereby producing an output composition; and assessing one or more phenotype or activity of one or more cells of the output composition.

In some of any of the embodiments of a long-term stimulation method, the conditions to stimulate a CAR-dependent activity includes the presence of a binding molecule that specifically binds to the antigen-binding domain of the CAR. In some embodiments, the binding molecule is attached to a support. In some embodiments, the support is a solid support. In some embodiments, the solid support is the surface of a well of a microplate or a bead. In some embodiments, the solid support is a microplate having the binding molecule attached to the microplate, and the incubation is carried out in the microplate. In some embodiments, the solid support is a bead having attached the binding molecule, and the incubation is carried out in the presence of a plurality of the beads.

In some of any of the embodiments of a long-term stimulation method, the binding molecule is or comprises a recombinant antigen or a portion thereof recognized by the antigen-binding domain. In some embodiments, the recombinant antigen or portion thereof is BCMA, or is a portion thereof recognized by the antigen-binding domain. In some embodiments, the binding molecule is or includes an anti-iditopytic antibody or antigen-binding fragment thereof that specifically binds to the antigen-binding domain. In some embodiments, the antigen-binding domain of the antigen receptor is or comprises antibody SJ25C1 or an antigen-binding fragment thereof. In some embodiments, the antigen-binding domain of the antigen receptor is or includes antibody FMC63 or an antigen-binding fragment thereof.

In some of any of the embodiments of a long-term stimulation method, the method is carried out in vitro or ex vivo.

In some of any of the embodiments of a long-term stimulation method, the input composition is incubated in the presence of a media that does not comprise recombinant cytokines. In some embodiments, the incubation is carried out continuously or is not interrupted for the period of time. In some embodiments, during the incubation, cells are not replated, media is not changed and binding molecule is not added.

In some of any of the embodiments of a long-term stimulation method, the method includes assessing one or more phenotypes of activation, exhaustion or differentiation state of the one or more cells of the output composition. In some embodiments, the phenotype is exhaustion and the assessing includes measuring the expression, optionally surface expression, of one or more markers selected from CTLA-4, FOXP3, PD-1, TIGIT, LAB-3, 2B4, BTLA, TIM3, VISTA, or CD96. In some embodiments, the phenotype is activation and the assessing includes measuring the expression, optionally surface expression, of one or more markers selected from CD25, CD26, CD27, CD28, CD30, CD71, CD154, CD40L, CD127, LAG3, or Ki67. In some embodiments, the phenotype is differentiation state and the assessing includes measuring one or more markers selected from (i) one or more of CD25, CD45RO, CD56, KLRG1, CD95 and/or (ii) one or of CD45RA, CD27, CD28, CD62L, and CCR7, optionally wherein the one or more markers are markers are positively or inversely associated with naive-like T cells.

In some of any of the embodiments of a long-term stimulation method, the method includes assessing one or more activities of the one or more cells of the output composition. In some embodiments, the one or more activities comprises a CAR-dependent activity, optionally an antigen-stimulated activity. In some of any of the embodiments of a long-term stimulation method, the one or more activities comprises cytolytic activity or cytokine production.

In some embodiments of any of the long-term stimulation method, the period of time is at least or at least about 11 days, 12 days, 13 days, 14 days, or 15 days. In some embodiments, the period of time is or is about 11 days, 12 days, 13 days, 14 days or 15 days.

In some of any of the embodiments of a long-term stimulation method, the input composition contains cells that have been exposed or contacted with a test agent or compound prior to the incubation, optionally wherein the exposing or contacting is carried out during one or more steps of a process for producing the input composition comprising the T cells expressing the CAR. In some embodiments, the method is carried out on a plurality of input compositions, each of said input compositions of the plurality being produced by a different process.

In some of any of the embodiments of a long-term stimulation method, the method further includes comparing the phenotype or activity of the output composition to the phenotype or activity of a control composition, optionally wherein the control composition is a composition of T cells that have been incubated for the at least 10 days under the same conditions to stimulate the CAR-dependent activity, said composition of T cells having not been produced in the presence of the test agent or compound or having been produced by an alternative process compared to the input composition. In some embodiments, the method further includes identifying an output composition that exhibits reduced exhaustion, reduced activation or decreased differentiation, such as compared to the control composition. In some embodiments, the decreased differentiation comprises increased expression of one more naive-like T cell markers.

### Brief Description of the Drawings

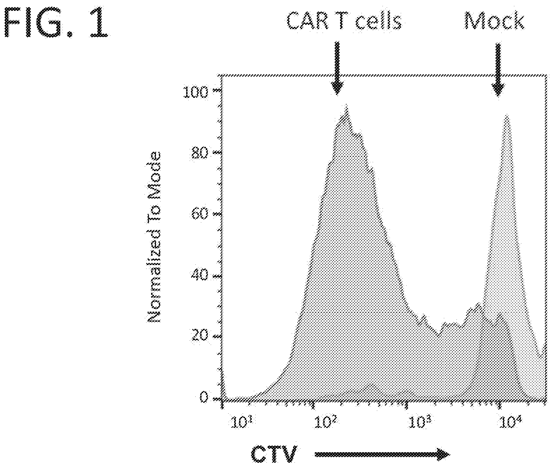

**FIG. 1** shows a histogram of Cell Trace Violet (CTV) staining in CD3⁺ T cells expressing anti-BCMA CAR (CAR T cells) and CD3⁺ cells that do not express a CAR (Mock) following incubation with BCMA conjugated-beads.

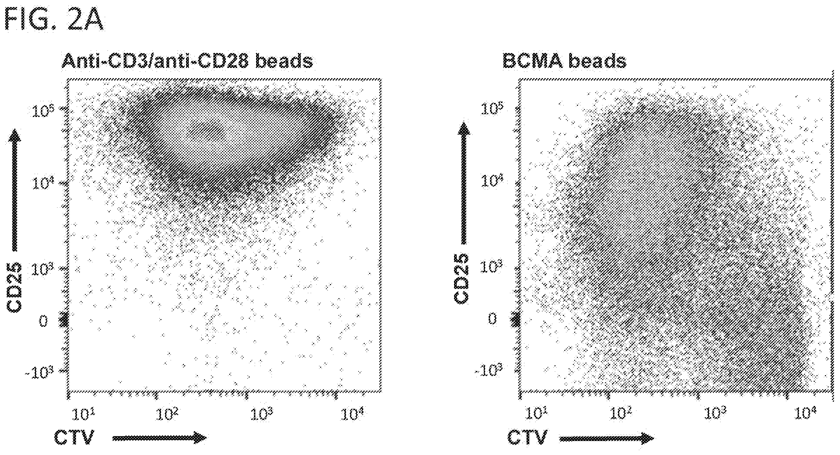

**FIG. 2A** shows dot plots for CD25 surface expression (y-axis) and CTV staining intensity (x-axis) in cells following incubation with anti-CD3/ anti-CD28 antibody-conjugated beads (left) or BCMA-conjugated beads (right).

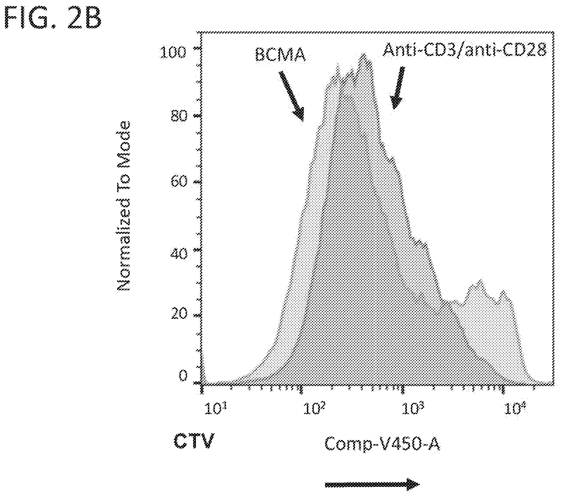

**FIG. 2B** shows a histogram of CTV staining of cells following treatment with anti-CD3/ anti-CD28 antibody-conjugated beads or BCMA-conjugated beads.

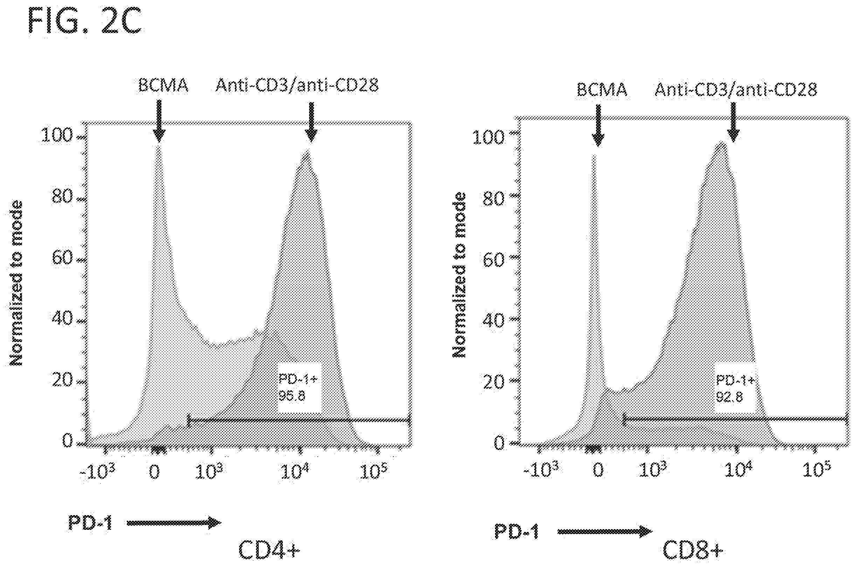

**FIG. 2C** shows histograms of PD-1 expression in CD4+ cells (left) or CD8+ cells (right) following incubation cells with anti-CD3/ anti-CD28 antibody-conjugated beads or BCMA-conjugated beads.

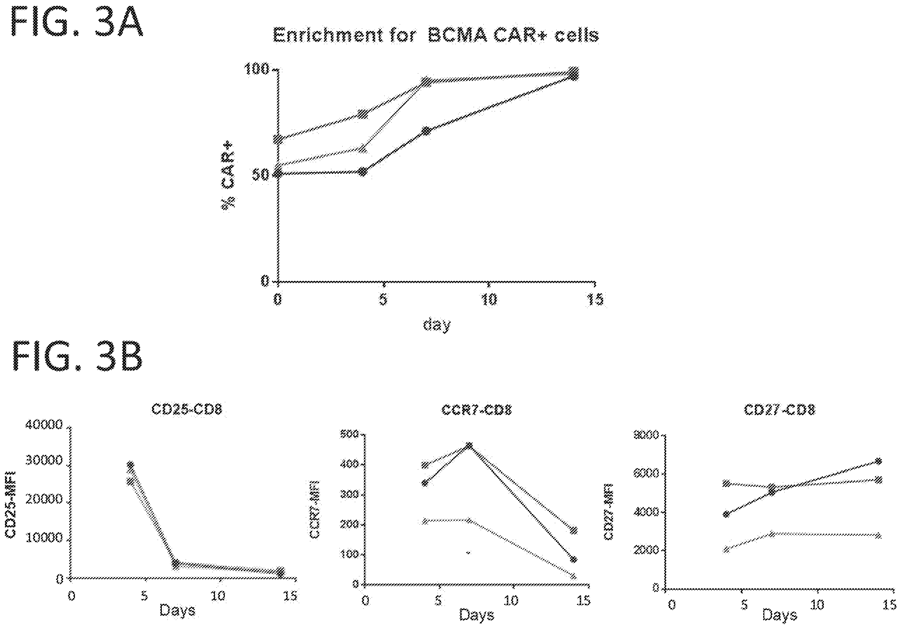

**FIG. 3A** shows the percentage of anti-BCMA CAR+ T cells following incubation of each of three different T cell compositions containing the CAR-expressing T cells (each generated from a different donor) with BCMA-conjugated beads for up to 14 days. Results from each cell composition are separately plotted with triangles, squares, and circles.
**FIG. 3B** shows the surface expression as determined by mean florescence intensity (MFI) for detection of CD25 (left), CCR7 (center), and CD27 (right) in CD8+ cells following four, seven, or fourteen days of incubation with BCMA conjugated beads for up to 14 days. Results are shown for each of the three different cell compositions containing anti-BCMA CAR expressing cells described in **FIG. 3A** and are separately plotted with triangles, squares, and circles.

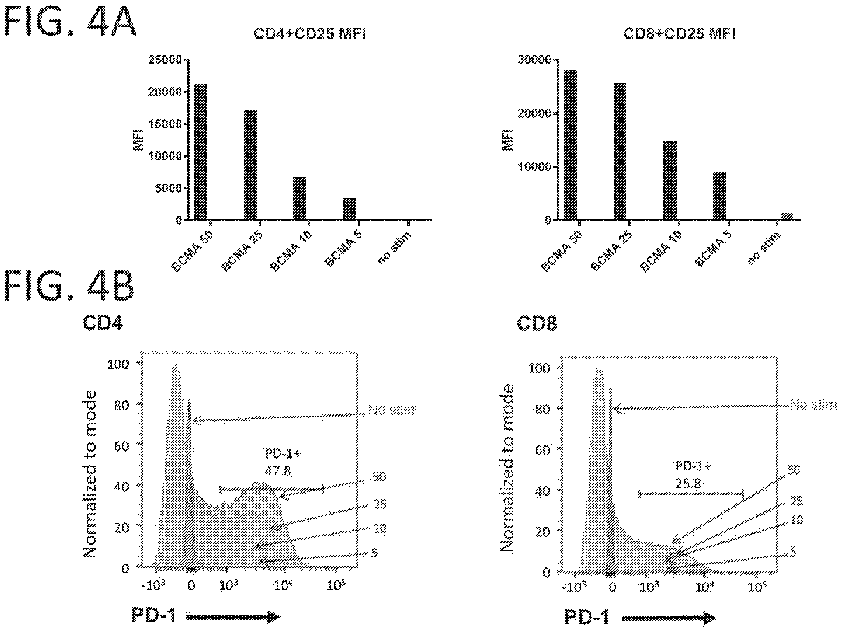

**FIG. 4A** show graphs for surface expression of CD25 in CD4+ T cells (left panels) or CD8+ T cells (right panels) present in an anti-BCMA CAR+ T cell composition after incubation with different amounts of BCMA-conjugated beads. **FIG. 4B** show histogram plots for surface expression of PD-1 in CD4+ T cells (left panels) or CD8+ T cells (right panels) present an anti-BCMA CAR+ T cell composition after incubation with different amounts of BCMA-conjugated beads. BCMA 50, BCMA 25, BCMA 10, and BCMA 5 indicate BCMA-conjugated beads generated by incubating BCMA with the beads in an amount of 50, 25, 10, and 5 µg of BCMA per approximately 4×10⁸ beads, respectively.

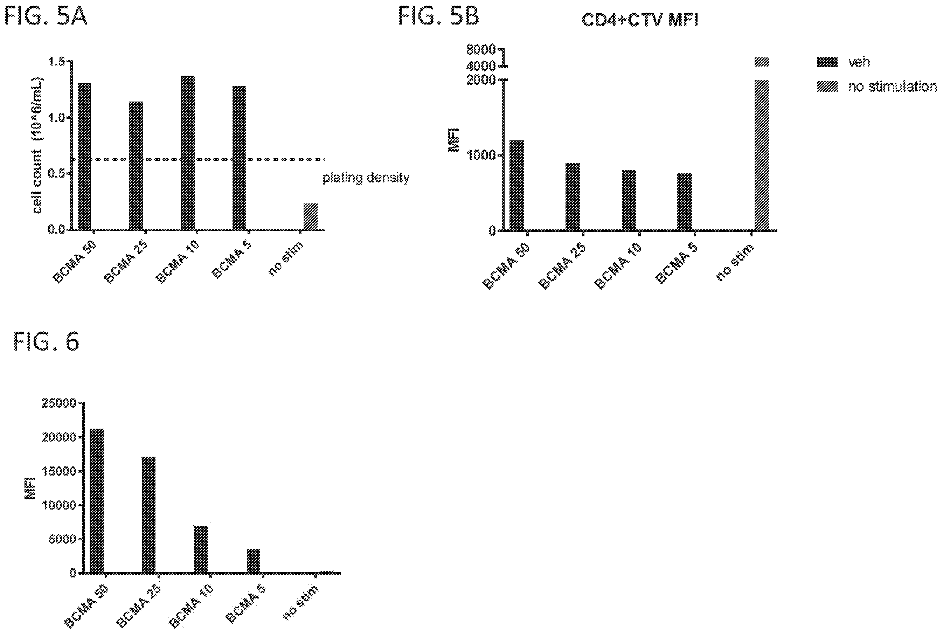

**FIG. 5A-B** show graphs displaying the total CD3⁺ T cell number **(****FIG. 5A****)** or levels of CTV proliferation marker dye on CD4+ T cells **(****FIG. 5B****)** present an anti-BCMA CAR+ T cell composition after incubation with different amounts of BCMA-conjugated beads. BCMA 50, BCMA 25, BCMA 10, and BCMA 5 indicate BCMA-conjugated beads generated by incubating BCMA with the beads in an amount of 50, 25, 10, and 5 µg of BCMA per approximately 4×10⁸ beads, respectively.
**FIG. 6** shows a graph displaying CD25 surface expression on CD4+ T cells present an anti-BCMA CAR+ T cell composition after incubation with different amounts of BCMA-conjugated beads. BCMA 50, BCMA 25, BCMA 10, and BCMA 5 indicate BCMA-conjugated beads generated by incubating BCMA with the beads in an amount of 50, 25, 10, and 5 µg of BCMA per approximately 4×10⁸ beads, respectively.

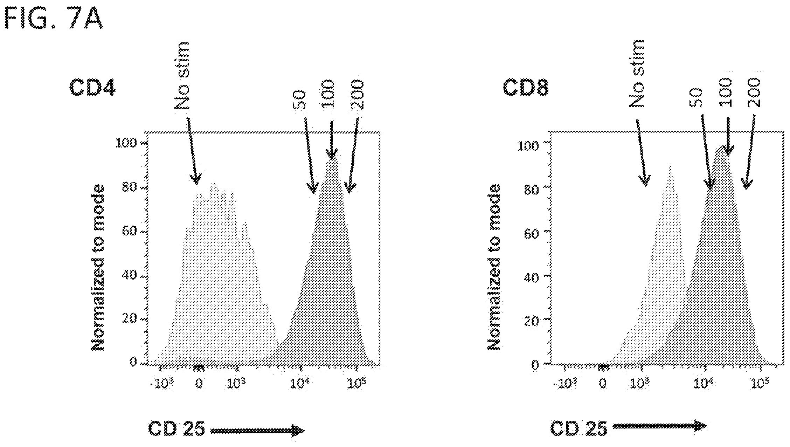

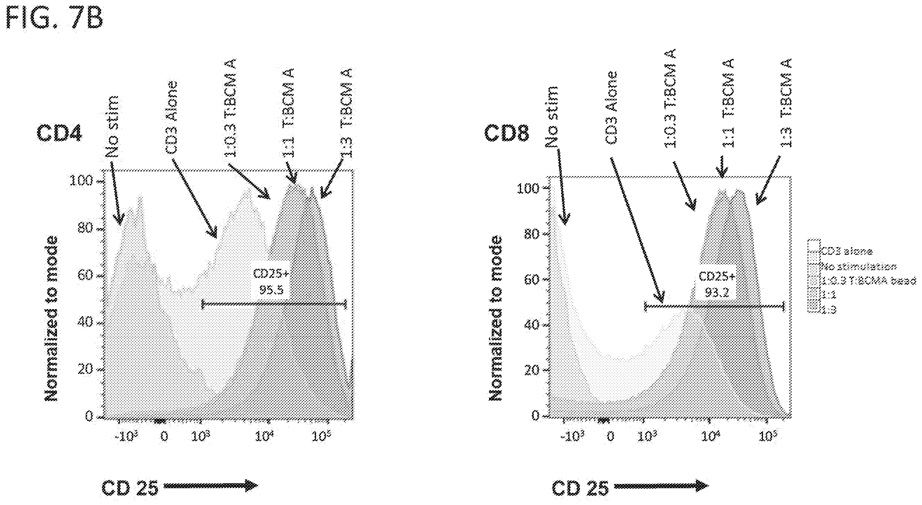

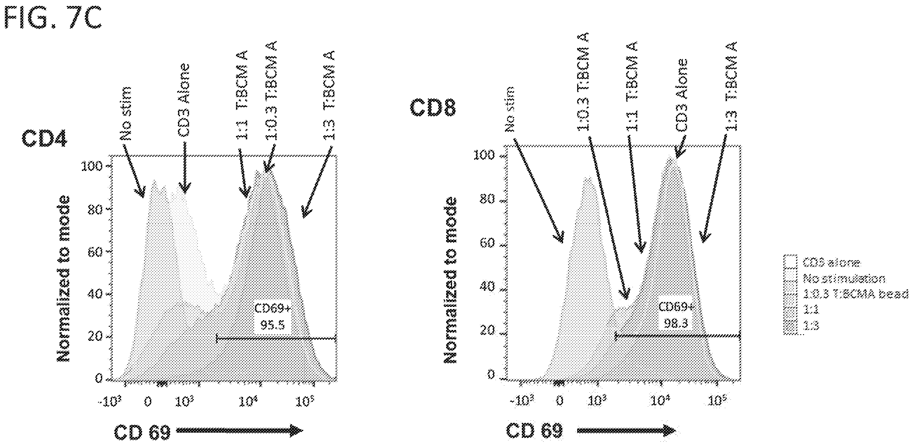

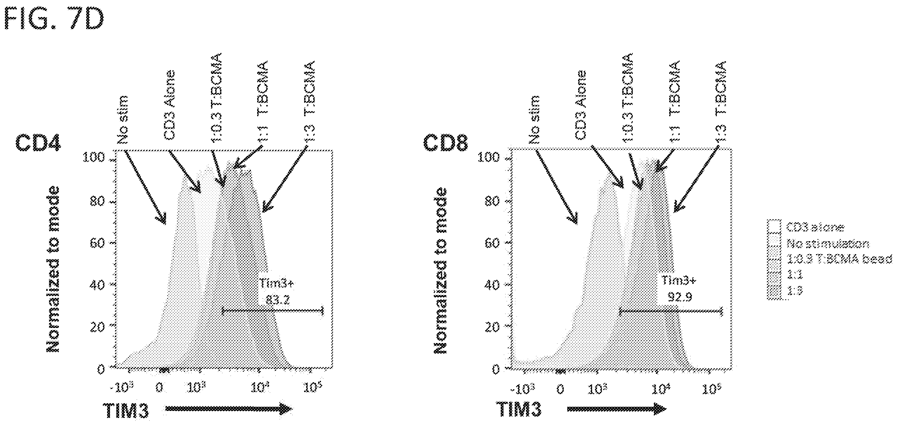

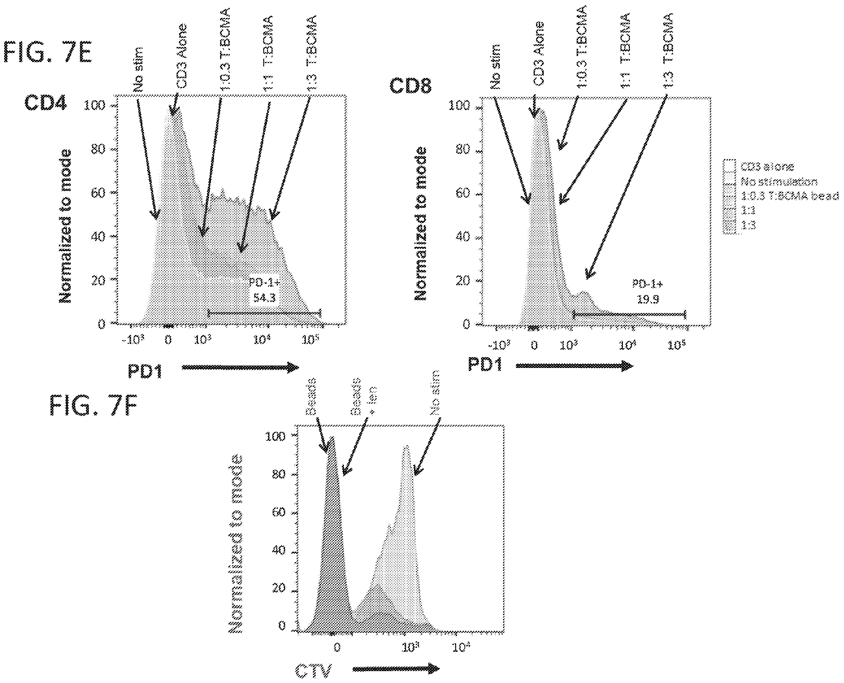

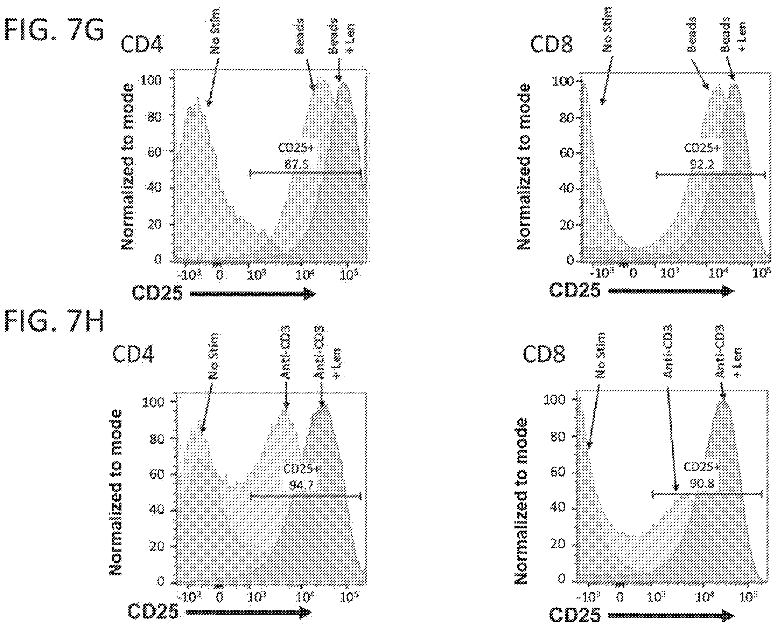

**FIGS. 7A-7H** shows staining for various markers or level of proliferation in anti-BCMA CAR+ T cell compositions incubated with BCMA-conjugated beads. **FIG. 7A** show flow cytometry histograms for surface expression of CD25 in CD4+ T cells (left panels) or CD8+ T cells (right panels) present in an anti-BCMA CAR+ T cell composition after incubation with different amounts of BCMA-conjugated beads. **FIG. 7B** show flow cytometry histograms for surface expression of CD25 in CD4+ T cells (left panels) or CD8+ T cells (right panels) present in an anti-BCMA CAR+ T cell composition after incubation with different ratios of T cells to beads (200 µg/mL BCMA-conjugated bead composition) or immobilized anti-CD3. **FIG. 7C** shows flow cytometry histograms for surface expression of CD69 in CD4+ T cells (left panels) or CD8+ T cells (right panels) present in an anti-BCMA CAR+ T cell composition after incubation with different ratios of T cells to beads (200 µg/mL BCMA-conjugated bead composition) or immobilized anti-CD3. **FIG. 7D** shows flow cytometry histograms for surface expression of TIM3 in CD4+ T cells (left panels) or CD8+ T cells (right panels) present in an anti-BCMA CAR+ T cell composition after incubation with different ratios of T cells to beads (200 µg/mL BCMA-conjugated bead composition) or immobilized anti-CD3. **FIG. 7E** shows flow cytometry histograms for surface expression of PD-1 in CD4+ T cells (left panels) or CD8+ T cells (right panels) present in an anti-BCMA CAR+ T cell composition after incubation with different ratios of T cells to beads (200 µg/mL BCMA-conjugated bead composition) or immobilized anti-CD3. **FIG. 7F** shows a histogram plot of CTV staining (measure of proliferation) of total cells in an anti-BCMA CAR+ T cell composition after incubation with beads (200 µg/mL BCMA-conjugated bead composition) at a ratio of 1:1 T cells to beads and in the presence or absence of 5µM lenalidomide. **FIG. 7G and FIG. 7H** show flow cytometry histograms for CD25 in CD4+ T cells (left panels) or CD8+ T cells (right panels) present in an anti-BCMA CAR+ T cell composition after incubation with beads (200 µg/mL BCMA-conjugated bead composition) at a ratio of 1:1 T cells to beads or immobilized anti-CD3, respectively, in the presence or absence of lenalidomide. In the above figures, "50," "100," and "200" indicate BCMA-conjugated beads generated by incubating BCMA with the beads in an amount of 50, 100, and 200 µg of BCMA per approximately 4×10⁸ beads, respectively.

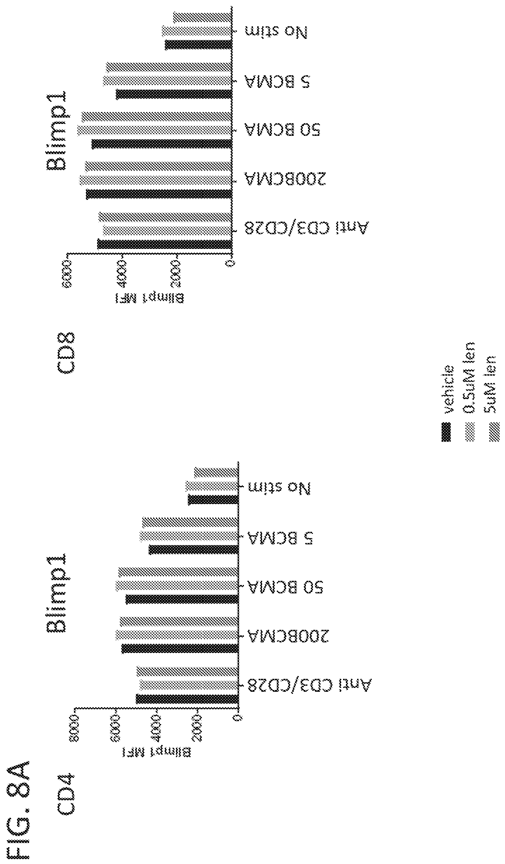

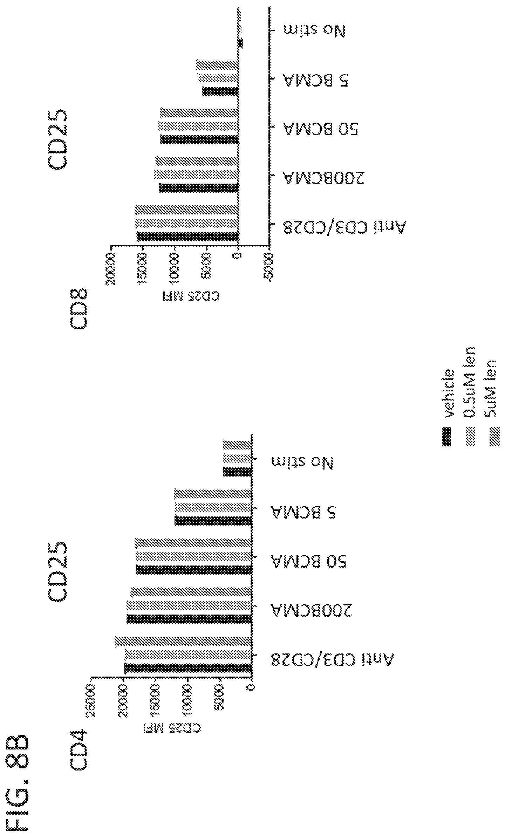

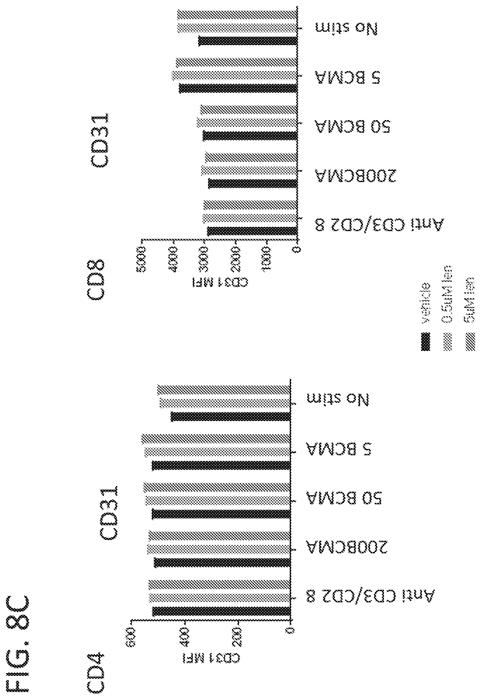

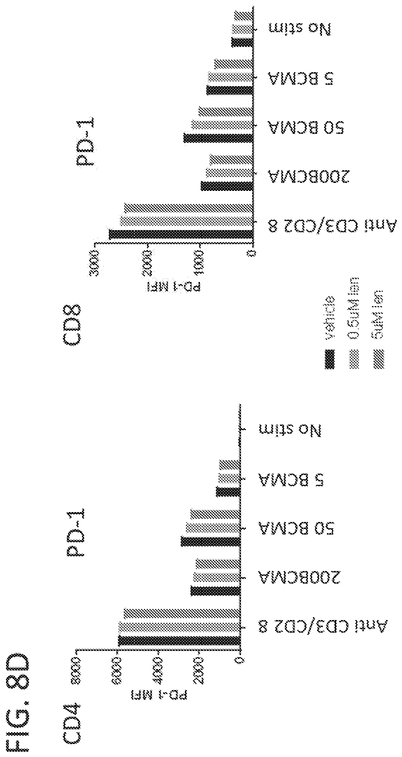

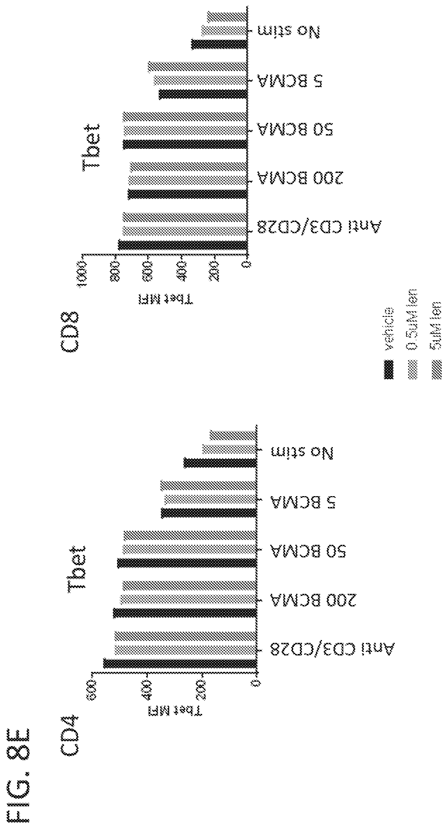

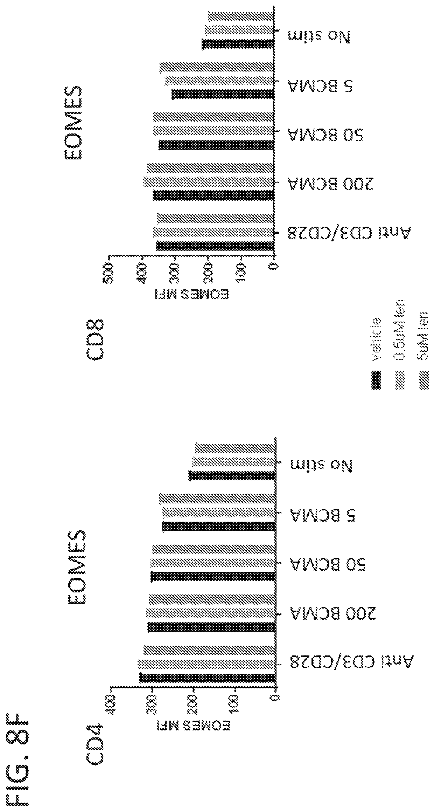

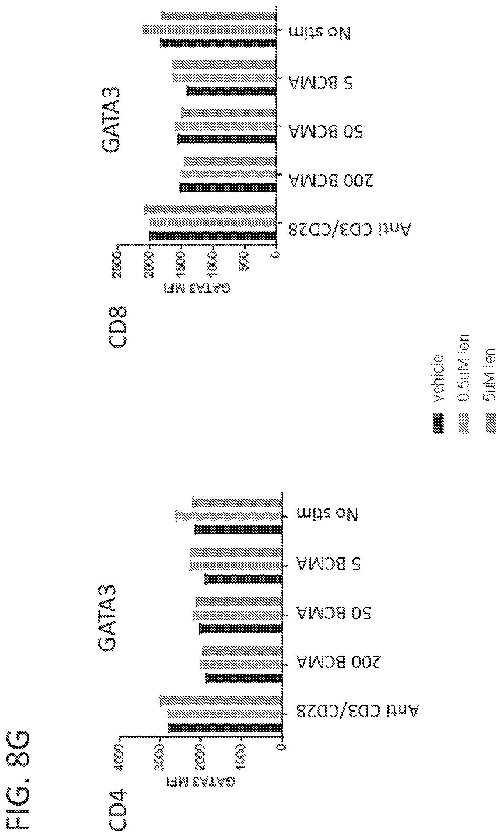

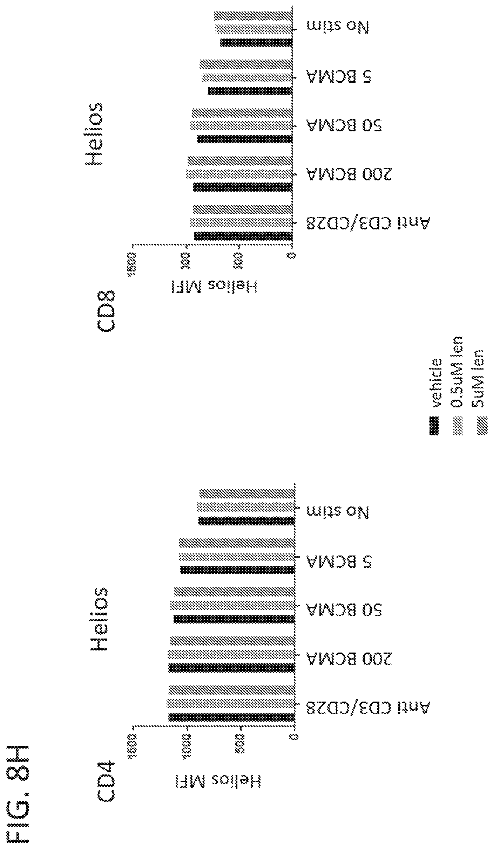

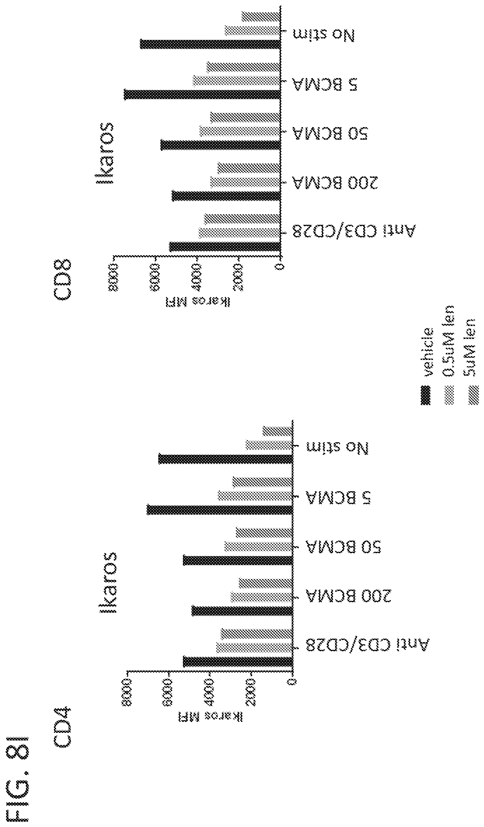

**FIGS. 8A-8I** show graphs displaying the levels of transcription factors and activation markers in or on CD4+ T cells (left panels) or CD8+ T cells (right panels) present in an anti-BCMA CAR+ T cell composition after incubation without stimulation or with different amounts of BCMA-conjugated bead or anti-CD3 and anti-CD28 conjugated beads and in the presence of 0 µM, 0.5 µM, or 50 µM lenalidomide. Levels of Blimp1 **(****FIG. 8A****),** CD25 **(****FIG. 8B****),** CD31 **(****FIG. 8C****),** PD-1 **(****FIG. 8D****),** Tbet **(****FIG. 8E****),** EOMES **(****FIG. 8F****),** GATA3 **(****FIG. 8G****),** Helios **(****FIG. 8H****),** and Ikaros **(****FIG. 8I****)** are shown. 200 BCMA, 50 BCMA, and 5 BCMA indicate BCMA-conjugated beads generated by incubating BCMA with the beads in an amount of 200, 50, and 5 µg of BCMA per approximately 4×10⁸ beads, respectively.

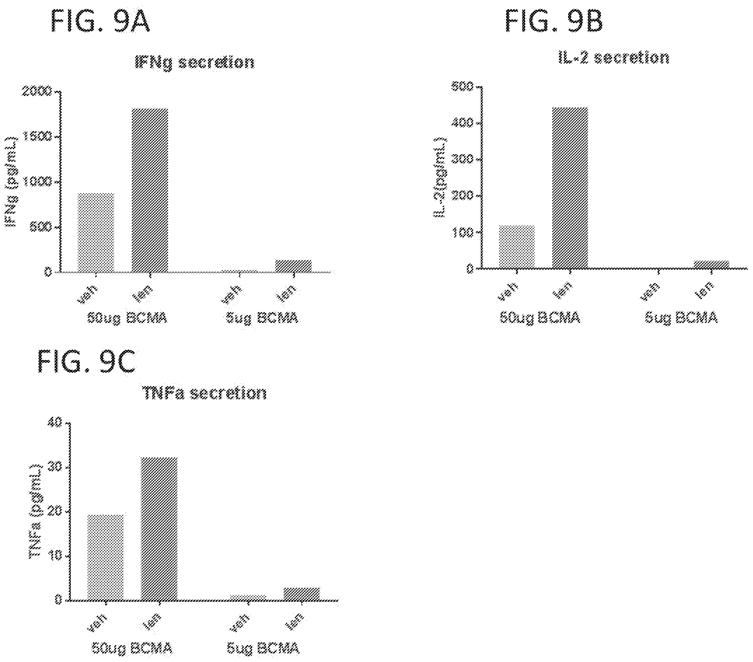

**FIG. 9A-9C** shows graphs displaying the levels of extracellular IFN-gamma **(****FIG. 9A****),** IL-2 **(****FIG. 9B****),** and TNF alpha **(****FIG. 9C****)** from cultures following incubation of an anti-BCMA CAR+ T cell composition with two different amounts of BCMA-conjugated beads in the presence or absence of 5µM lenalidomide. 50 µg BCMA and 5 µg BCMA indicate BCMA-conjugated beads generated by incubating BCMA with the beads in an amount of 50 and 5 µg of BCMA per approximately 4×10⁸ beads, respectively.

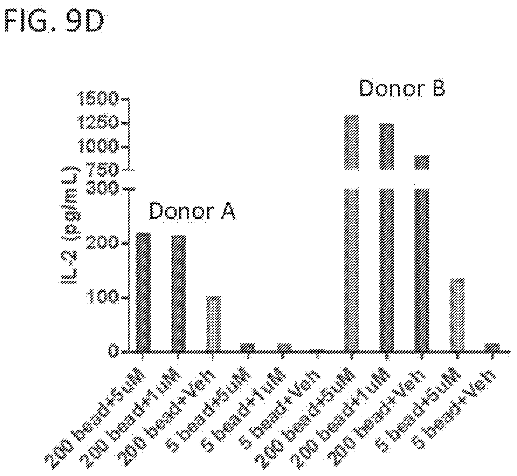

**FIG. 9D** shows a graph displaying the levels of extracellular IL-2 from cultures following incubation of an anti-BCMA CAR+ T cell composition from cells from two different donors (donor A and donor B) with different amounts of BCMA-conjugated beads in the presence of 0 µM, 1 µM, or 5 µM lenalidomide. 200 BCMA and 5 BCMA indicate BCMA-conjugated beads generated by incubating BCMA with the beads in an amount of 200, and 5 µg of BCMA per approximately 4×10⁸ beads, respectively.

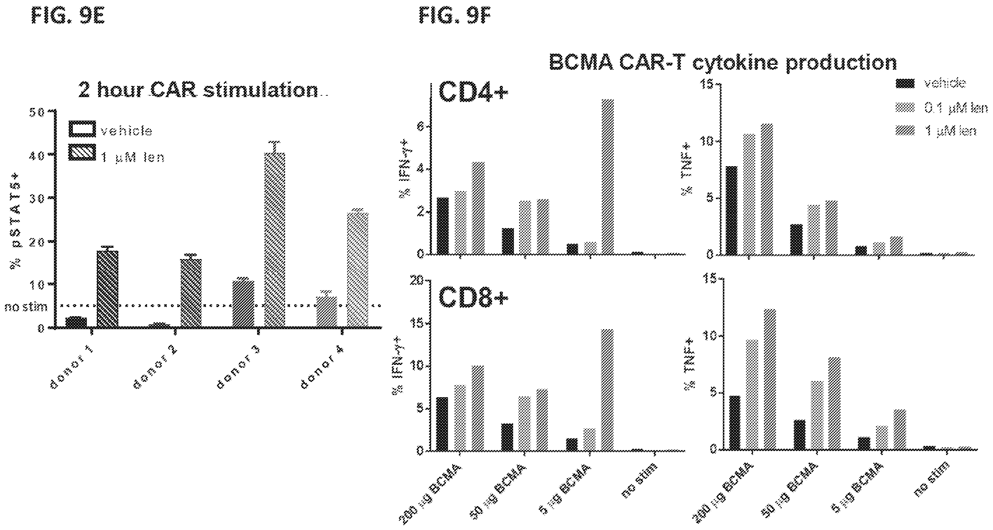

**FIG. 9E** shows the flow cytometric analysis of phosphorylated STAT5 (pSTAT5) after 2 hours of CAR stimulation (stim) with 50 µg BCMA beads. No stimulation control shown with dotted line. **FIG. 9F** shows the flow cytometric analysis of intracellular cytokine levels on a representative normal CAR T donor after 24 hours of BCMA bead stimulation (gated on transduced, live CD3+).

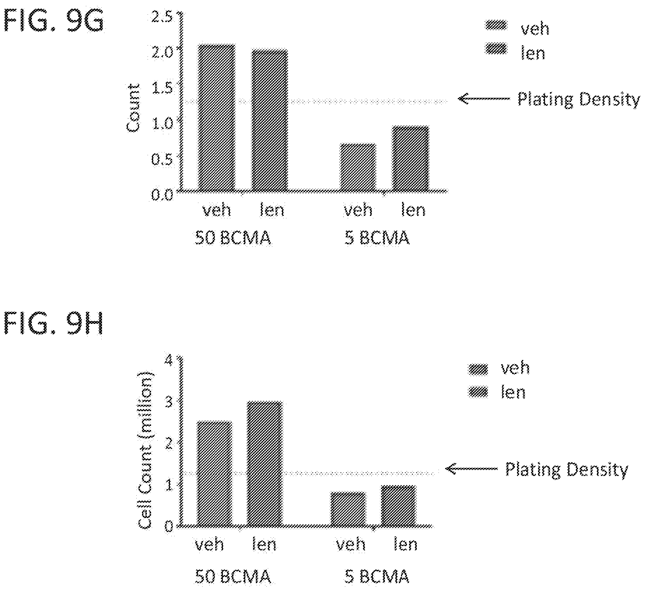

**FIG. 9G and FIG. 9H** show total cell count following culture of an anti-BCMA CAR+ T cell composition after incubation for 4 days **(****FIG. 9G****)** or 7 days **(****FIG. 9H****)** with different amounts of BCMA-conjugated beads in the presence of 5 µM lenalidomide. 50 BCMA and 5 BCMA indicate BCMA-conjugated beads generated by incubating BCMA with the beads in an amount of 50 and 5 µg of BCMA per approximately 4×10⁸ beads, respectively.

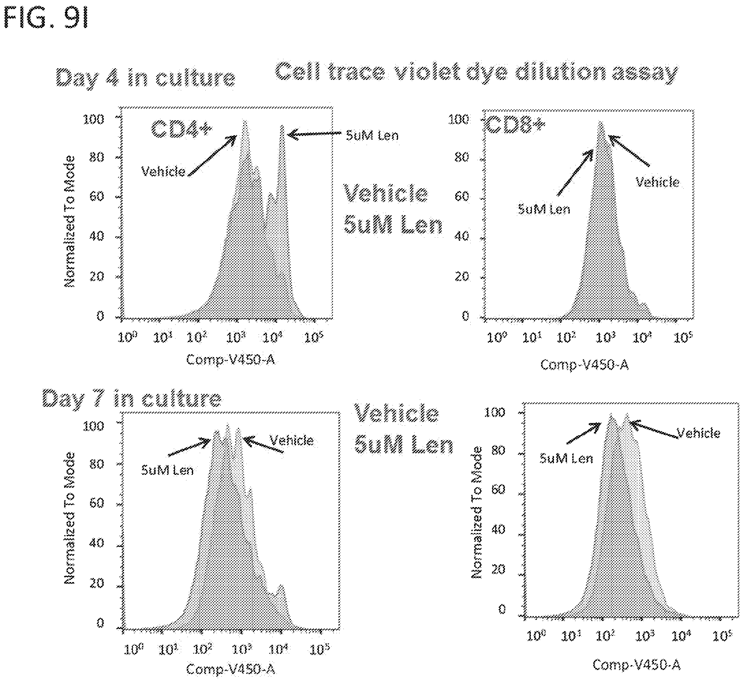

**FIG. 9I** shows histogram plots of CTV staining (measure of proliferation) of CD4+ T cells or CD8+ T cells in an anti-BCMA CAR+ T cell composition after incubation for 4 or 7 days with BCMA-conjugated beads in the presence of 5 µM lenalidomide (5uM Len) or absence of lenalidomide (vehicle).

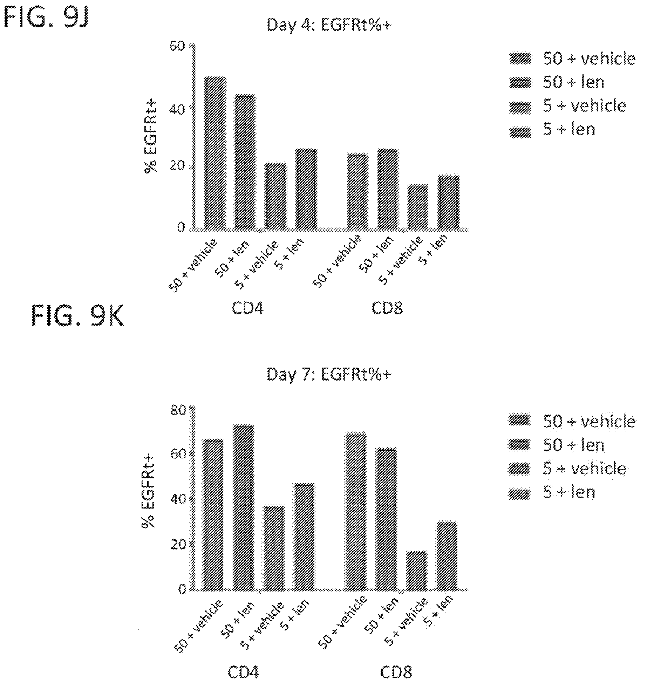

**FIG. 9J and 9K** show graphs displaying the percentage of cells positive for the surrogate marker EGFRt as determined with an anti-EGFR antibody following incubation of an anti-BCMA CAR+ T cell composition for 4 days **(****FIG. 9J****)** or 7 days **(****FIG. 9K****)** with different amounts of BCMA-conjugated beads in the presence of 5µM lenalidomide or absence of lenalidomide (vehicle). "50" and "5" indicate BCMA-conjugated beads generated by incubating BCMA with the beads in an amount of 50 and 5 µg of BCMA per approximately 4×10⁸ beads, respectively.

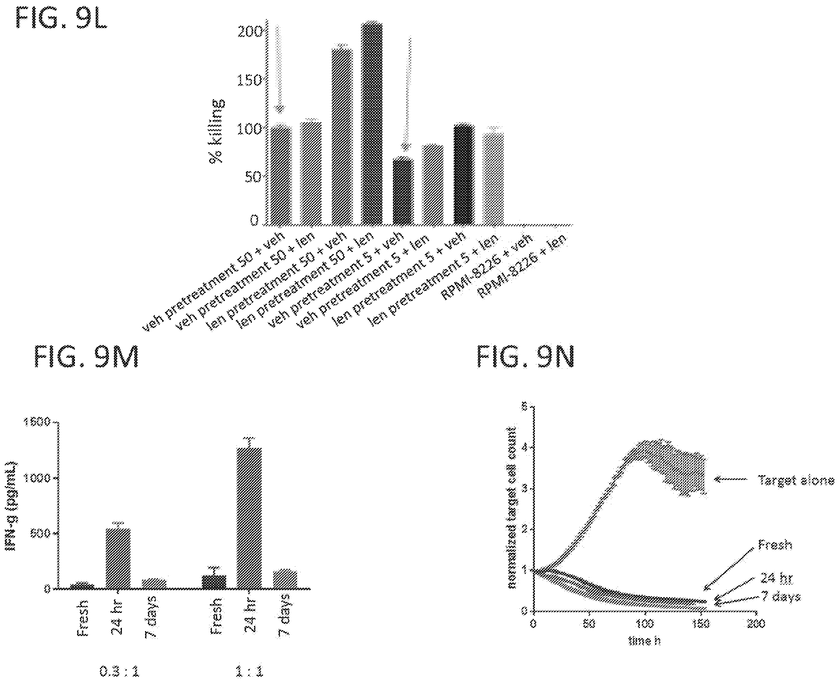

**FIG. 9L** shows the percent cell killing of RPMI-8226 target cells by anti-BCMA CAR+ T cells effector cells that had been incubated with different amounts of BCMA-conjugated beads in the presence of 5µM lenalidomide or absence of lenalidomide (vehicle). Cytolytic activity of compositions containing a ratio of effector cells to target cells of 3: 1 or 1: 1 and in the further presence or absence of lenalidomide are shown. "50" and "5" indicate BCMA-conjugated beads generated by incubating BCMA with the beads in an amount of 50 and 5 µg of BCMA per approximately 4×10⁸ beads, respectively.
**FIG. 9M** shows a graph displaying the levels of extracellular IFN-gamma produced during a killing assay in a culture containing RPMI-8226 target cells and fresh anti-BCMA CAR+ T cells effector cells or anti-BCMA CAR+ T cells effector cells that had been incubated for 24 hours or for seven days with BCMA-conjugated beads (50 µg/mL composition generated by incubating BCMA with the beads in an amount of 50 µg of BCMA per approximately 4×10⁸ beads). Results are shown from cultures containing an 0.3:1 or 1:1 effector cell to target cell ratio.
**FIG. 9N** depicts cell killing normalized to target cell count in a culture containing RPMI-8226 target cells and fresh anti-BCMA CAR+ T cells effector cells or anti-BCMA CAR+ T cells effector cells that had been incubated for 24 hours or for seven days with BCMA-conjugated beads (50 µg/mL composition generated by incubating BCMA with the beads in an amount of 50 µg of BCMA per approximately 4×10⁸ beads). Results are shown from cultures containing an 0.3:1 or 1:1 effector cell to target cell ratio.

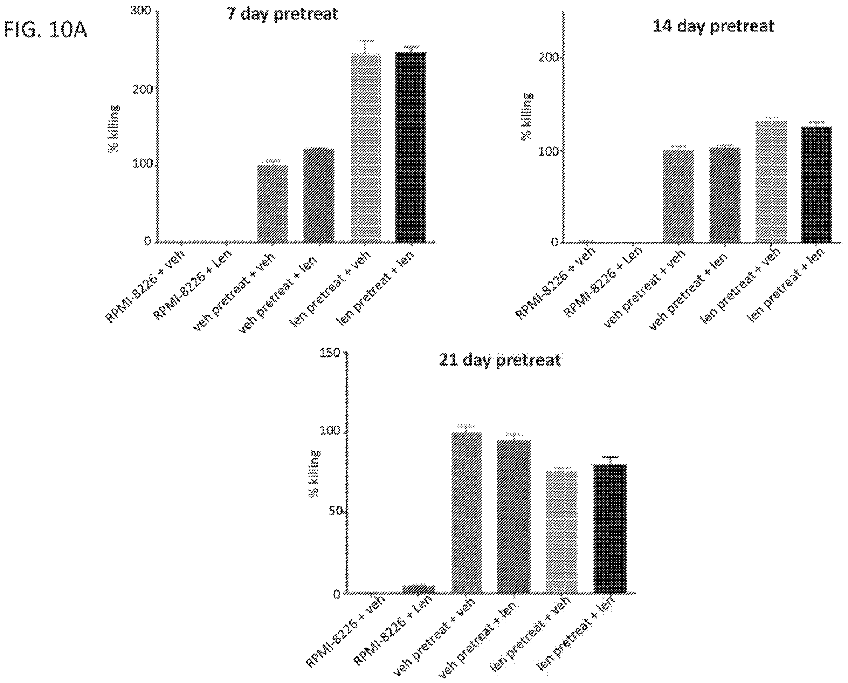

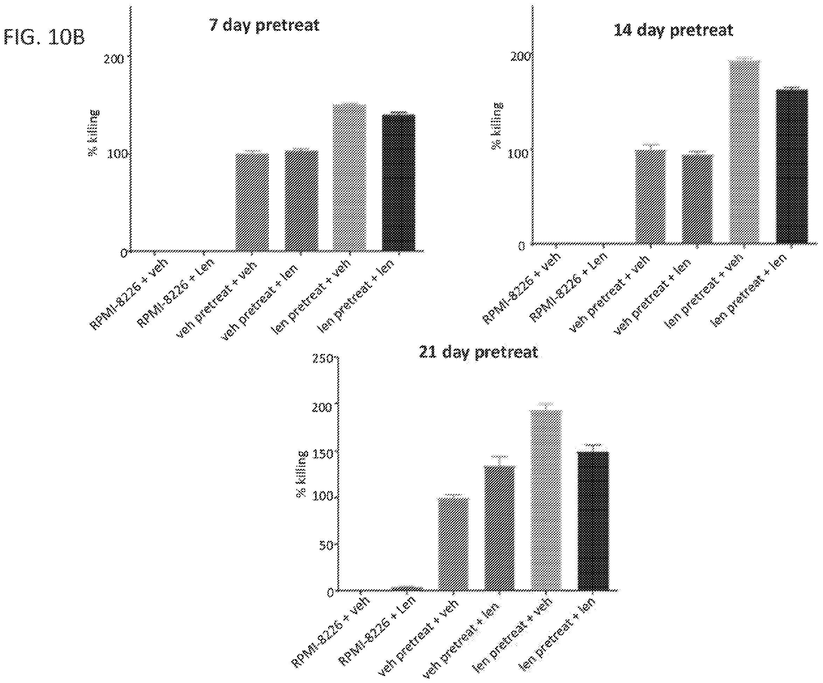

**FIG. 10A-10B** depicts results of a serial restimulation assay of anti-BCMA CAR T cell compositions that had been incubated for seven days with BCMA-conjugated beads (50 µg/mL generated by incubating BCMA with the beads in an amount of 50 µg of BCMA per approximately 4×10⁸ beads). Results from three different donor compositions are shown. **FIG. 10A** and **FIG. 10B** show the cytolytic activity of the anti-BCMA CAR+ T cells at each of the time points for two different donors.

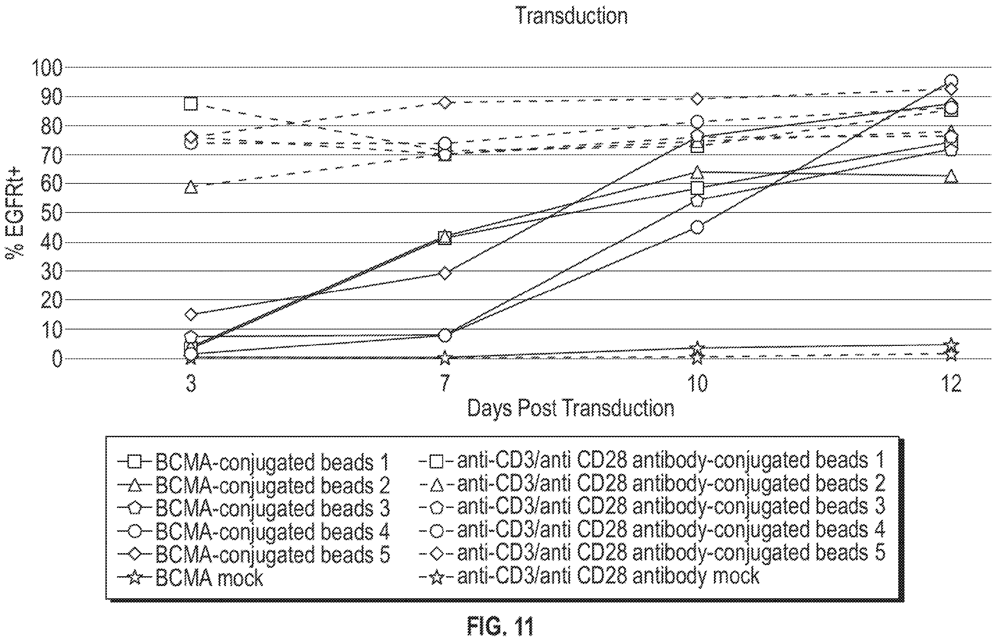

**FIG. 11** shows a graph displaying the percentage of CAR+ T cells over time in five different anti-BCMA CAR+ cell compositions that were incubated in the presence of BCMA - conjugated beads (solid lines) or with anti-CD3/anti-CD28 antibody conjugated beads (dotted lines) immediately after transduction with a lentiviral vector encoding the anti-BCMA CAR.

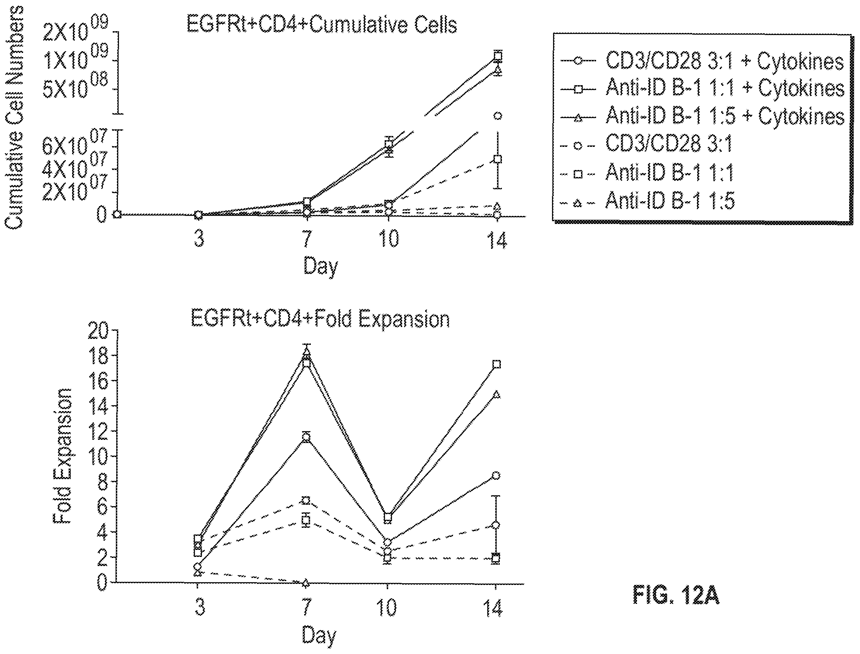

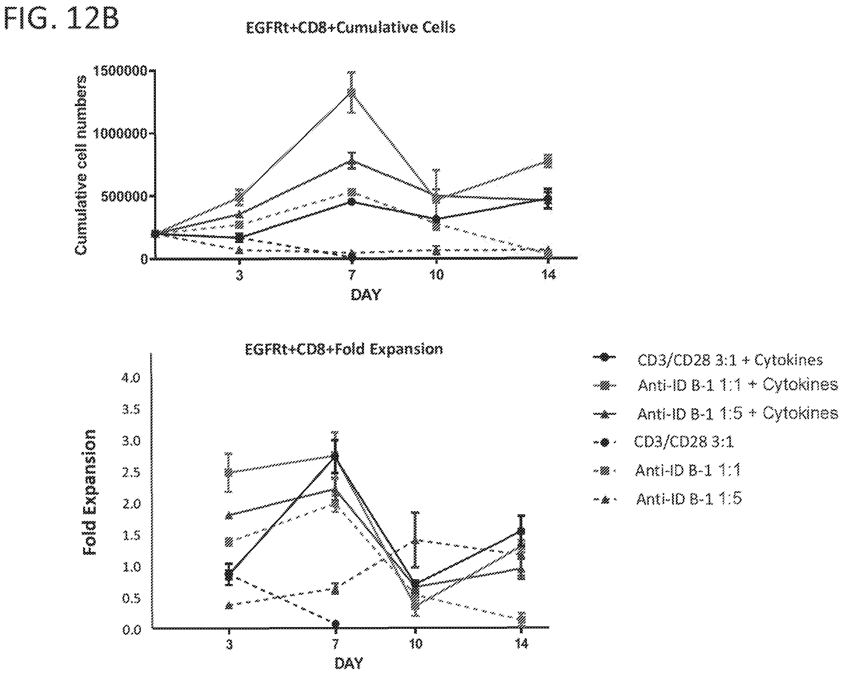

**FIG. 12A** and **FIG. 12B** shows fold expansion and cumulative cell numbers of EGFRt+/CD4+ T cells or EGFRt+/CD4+ T cells, respectively, stimulated with the indicated ratio of beads coated with anti-idiotype antibody (anti-ID B-1) or control anti-CD3/anti-CD28 antibody coated beads in the presence (solid lines) or absence (dashed lines) of cytokines. Results of stimulation with 3:1 anti-CD3/anti-CD28 antibody coated beads to cells (circles), 1:1 anti-ID B-1 coated beads to cells (squares), and 1:5 anti-ID B-1 coated beads to cells (triangles) are shown.

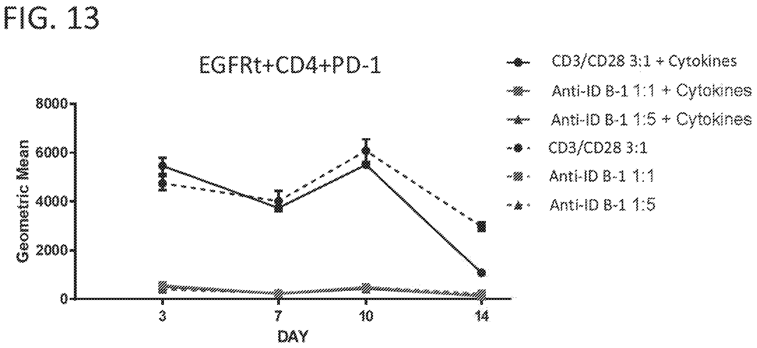

**FIG. 13** shows PD-1 expression levels of CD4+ T cells positive for an anti-EGFR antibody after stimulation with the indicated ratio of beads coated with anti-idiotype antibody (anti-ID B-1) or control anti-CD3/anti-CD28 antibody coated beads in the presence (solid lines) or absence (dashed lines) of cytokines as assessed by flow cytometry at days 3, 7, 10 and 14 of culture. Results of stimulation with 3:1 anti-CD3/anti-CD28 antibody coated beads to cells (circles), 1:1 anti-ID B-1 coated beads to cells (squares), and 1:5 anti-ID B-1 coated beads to cells (triangles) are shown.

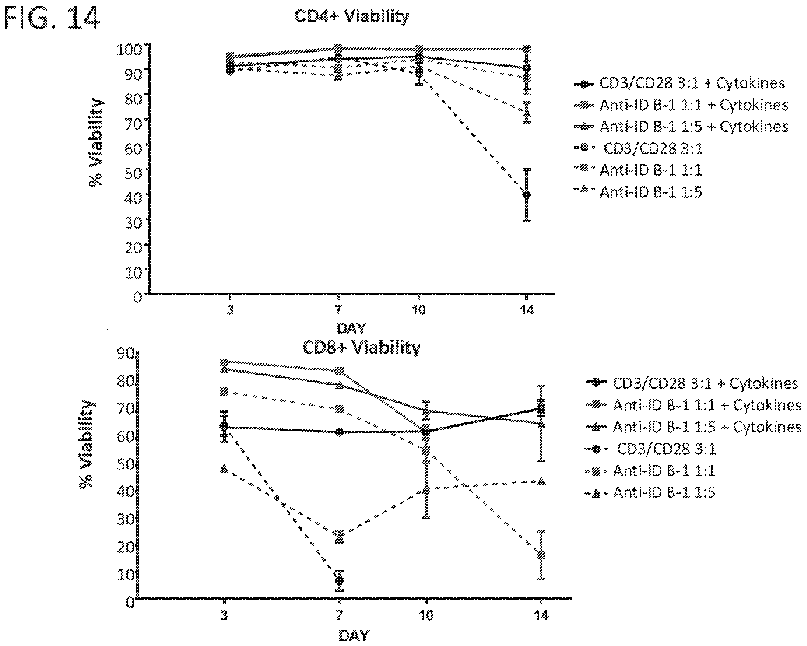

**FIG. 14** shows the viability of CD4⁺ or CD8⁺ T cells expressing an FMC63-derived CAR as assessed by flow cytometry following stimulation with the indicated ratio of beads coated with anti-idiotype antibody (anti-ID B-1) or control anti-CD3/anti-CD28 antibody coated beads in the presence (solid lines) or absence(dashed lines) of cytokines as assessed by flow cytometry at days 3, 7, 10 and 14 of culture. Results of stimulation with 1:3 anti-CD3/anti-CD28 antibody coated beads to cells (circles), 1:1 anti-ID B-1 coated beads to cells (squares), and 1:5 anti-ID B-1 coated beads to cells (triangles) are shown.

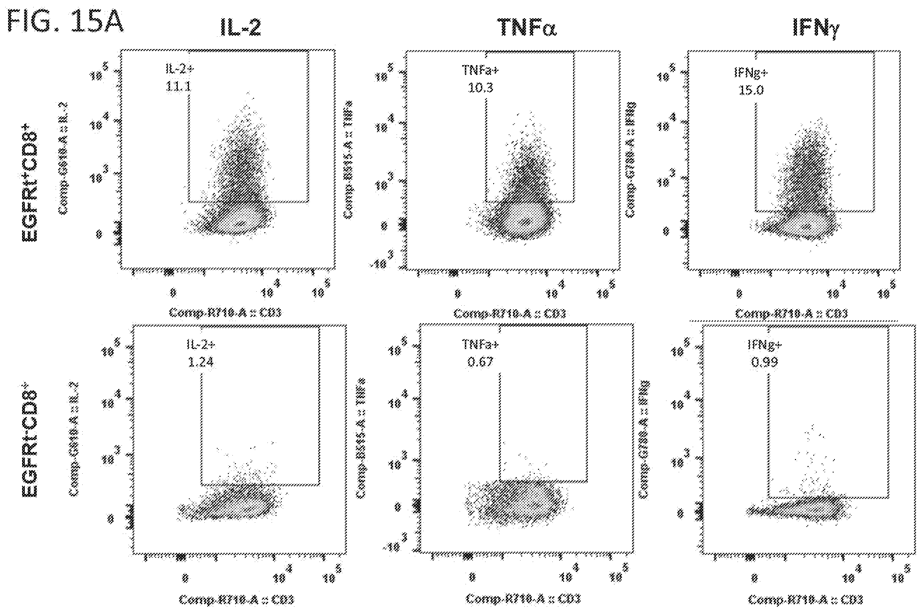

**FIG. 15A** shows intracellular cytokine staining for IL-2, TNFα, and IFNγ of T cells expressing an FMC63-derived CAR following stimulation with FMC63- derived scFv-specific anti-idiotype antibody (anti-ID B-1) coated beads. Shown are results for CD8+ T cells positive or negative for the EGFRt surrogate marker (EGFRt+ or EGFRt-).

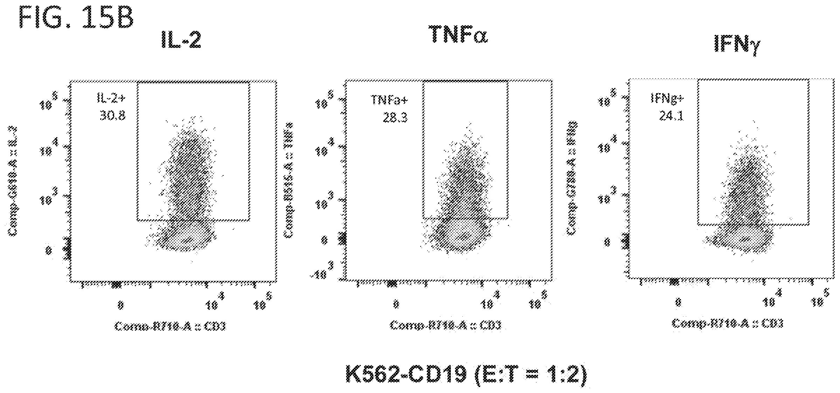

**FIG. 15B** shows intracellular cytokine staining for IL-2, TNFα, and IFNγ of T cells expressing an FMC63-derived CAR following stimulation with antigen-expressing K562-CD19 cells. Shown are results of CD8+ T cells positive for EGFRt surrogate marker.

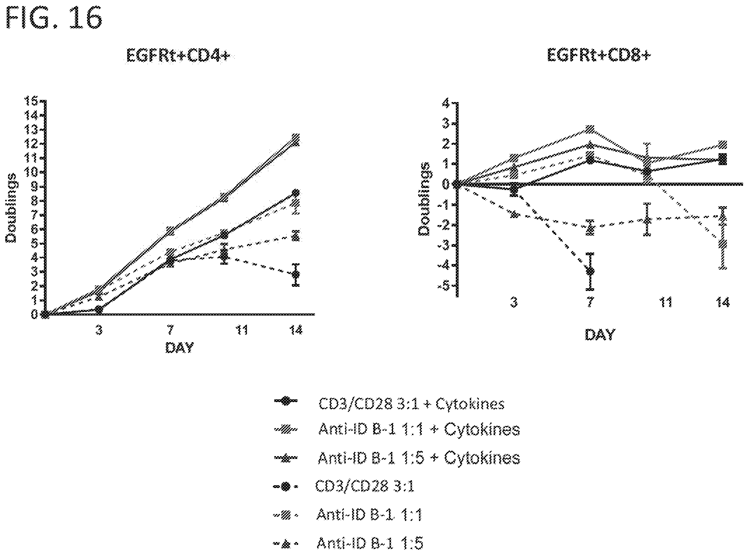

**FIG. 16** shows the number of population doublings in a serial stimulation assay over a 14 day culture period of T cells expressing an FMC63-derived CAR following stimulation with the indicated ratio of beads coated with anti-idiotype antibody (anti-ID B-1) or control anti-CD3/anti-CD28 antibody coated beads in the presence (solid lines) or absence (dashed lines) of cytokines. Shown are results for CD4+ T cells positive for EFGRt surrogate marker (EGFRt+) or CD8+ T cells positive for the EGFRt surrogate marker (EGFRt +). Results of stimulation with 1:3 anti-CD3/anti-CD28 antibody coated beads to cells (circles), 1:1 anti-ID B-1 coated beads to cells (squares), and 1:5 anti-ID B-1 coated beads to cells (triangles) are shown.

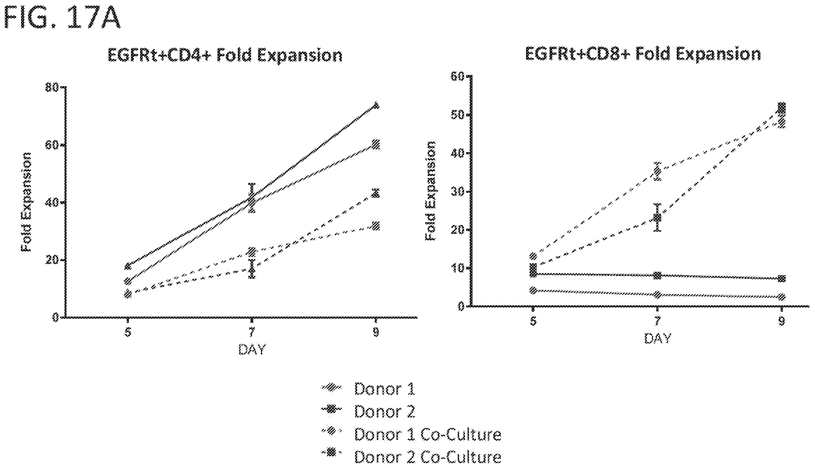

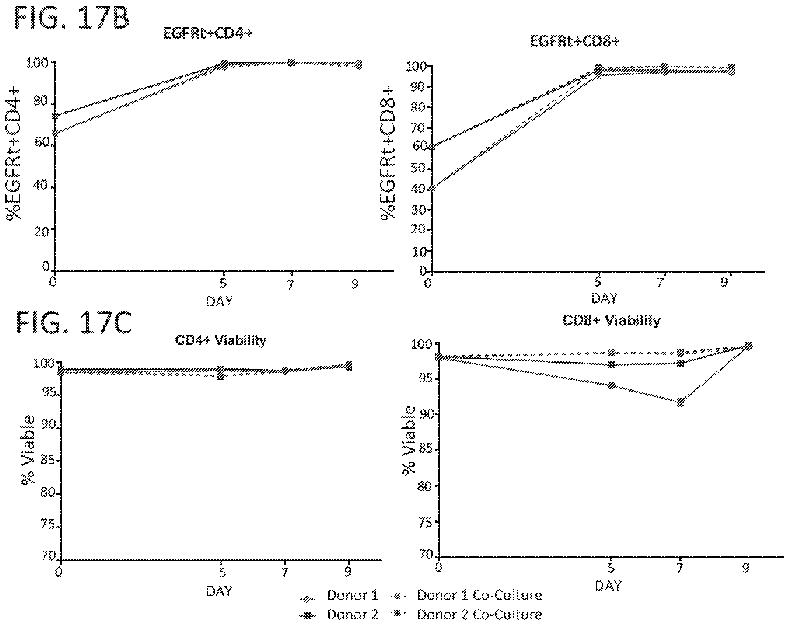

**FIG. 17A-17C** show results following stimulation of CD4+ or CD8+ T cells expressing an FMC63-derived CAR, cultured alone (solid lines) or as a co-culture (dashed lines), with FMC63- derived scFv-specific anti-idiotype antibody (anti-ID B-1) coated beads. Results are shown for two different donors (depicted as circles and squares). **FIG. 17A** depicts the fold-expansion of CD4+ T cells or CD8+ T cells in the cultures that were positive for the EGFRt surrogate marker (EGFRt+/CD4+ or EGFRt+/CD8+). **FIG. 17B** shows the frequency of CD4+ T cells or CD8+ T cells in the cultures that were positive for the EGFRt surrogate marker (EGFRt +/CD4+ or EGFRt +/CD8+). **FIG. 17C** shows the viability of CD4+ T cells or CD8+ T cells in the cultures.

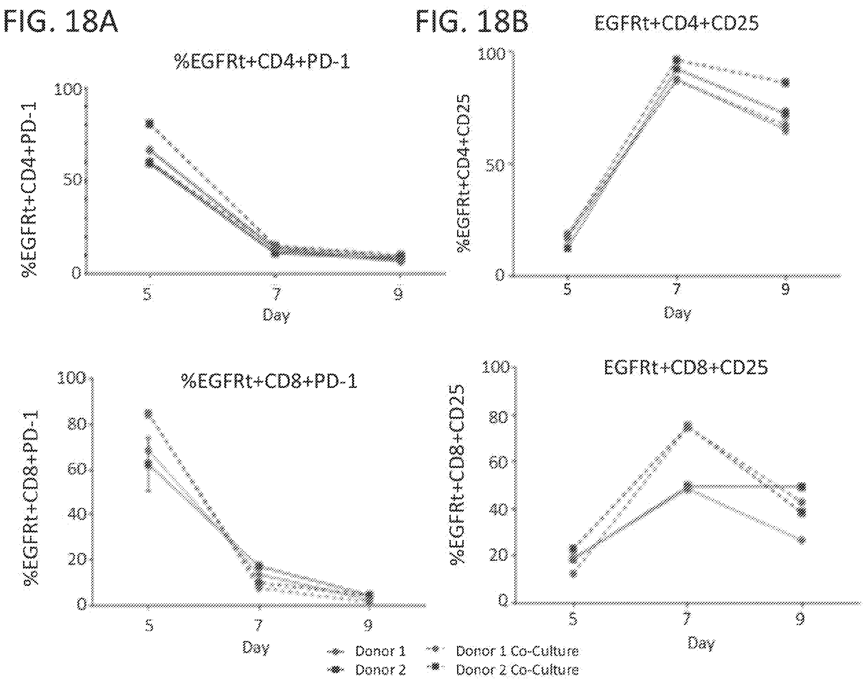

**FIG. 18A** and **18B** show flow cytometry results for T cell surface markers at days 5, 7 and 9 of culture following stimulation of CD4+ or CD8+ T cells expressing an FMC63-derived CAR, cultured alone or as a co-culture, with FMC63-derived scFv-specific anti-idiotype antibody (anti-ID B-1) coated beads. **FIG. 18A** shows surface expression of PD-1 on CD4+ T cells or CD8+ T cells in the cultures that were positive for the EGFRt surrogate marker (EGFRt+/CD4+ or EGFRt+/CD8+). **FIG. 18B** shows surface expression of CD25 on CD4+ T cells or CD8+ T cells in the cultures that were positive for the EGFRt surrogate marker (EGFRt+/CD4+ or EGFRt+/CD8+).

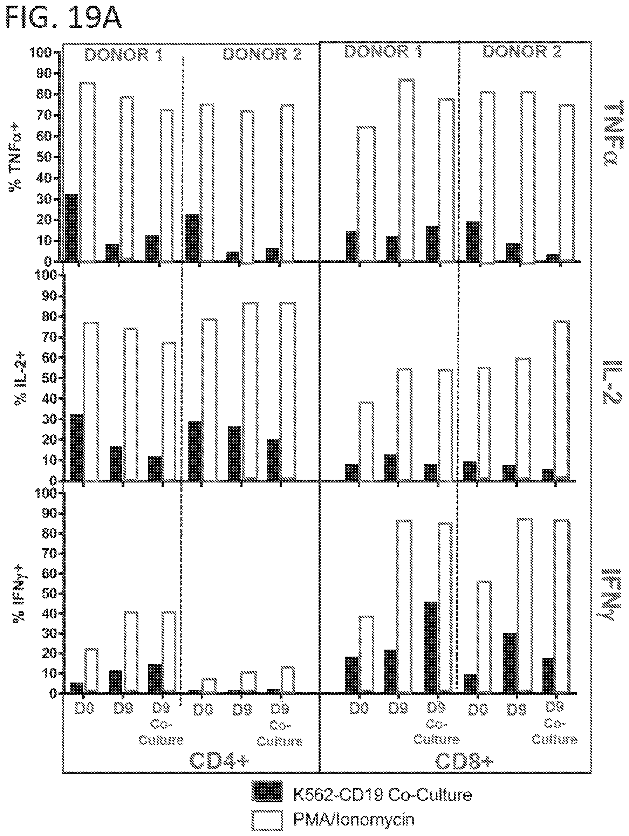

**FIG. 19A** shows intracellular cytokine levels of TNFα, IFNγ, and IL-2 as assessed by flow cytometry of CD4+ or CD8+ T cells present in a thawed composition containing T cells expressing an FMC63-derived CAR that had been expanded in culture either with CD19 expressing K562 cells or with PMA/Ionomycin. Shown are the level of the cytokines in CD4+ and CD8+ T cells, alone or as a co-culture, at thaw (d=0) or after a further culture for an additional 9 days in the presence of anti-ID B-1 conjugated beads.

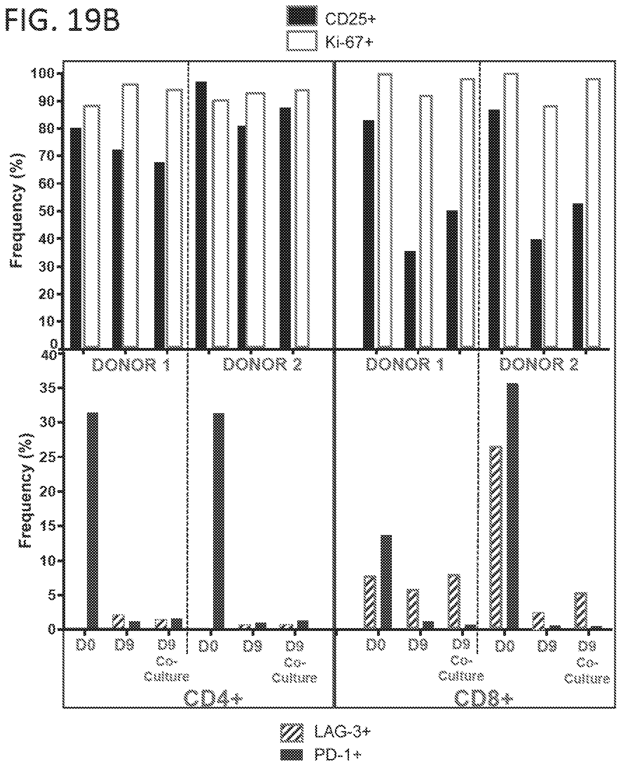

**FIG. 19B** shows the frequency of cells positive for CD25 or Ki67 as assessed by flow cytometry of CD4+ or CD8+ T cells present in a thawed composition containing T cells expressing an FMC63-derived CAR that had been expanded in culture either with CD19 expressing K562 cells or with PMA/Ionomycin. Shown are the level of the markers in CD4+ and CD8+ T cells, alone or as a co-culture, at thaw (d=0) or after a further culture for an additional 9 days in the presence of anti-ID B-1 conjugated beads.

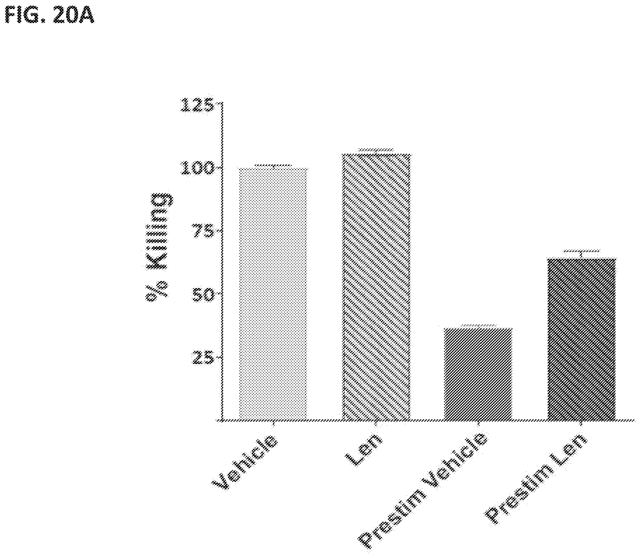

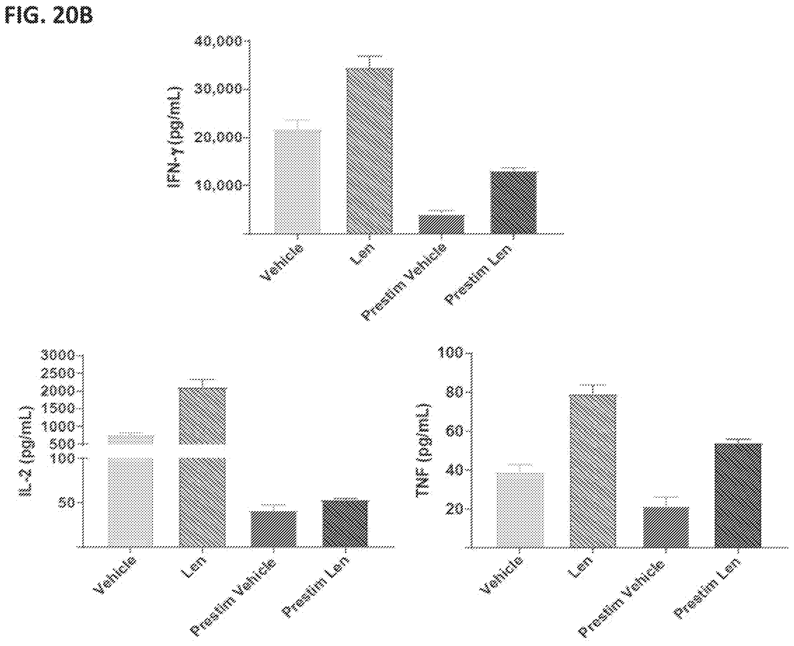

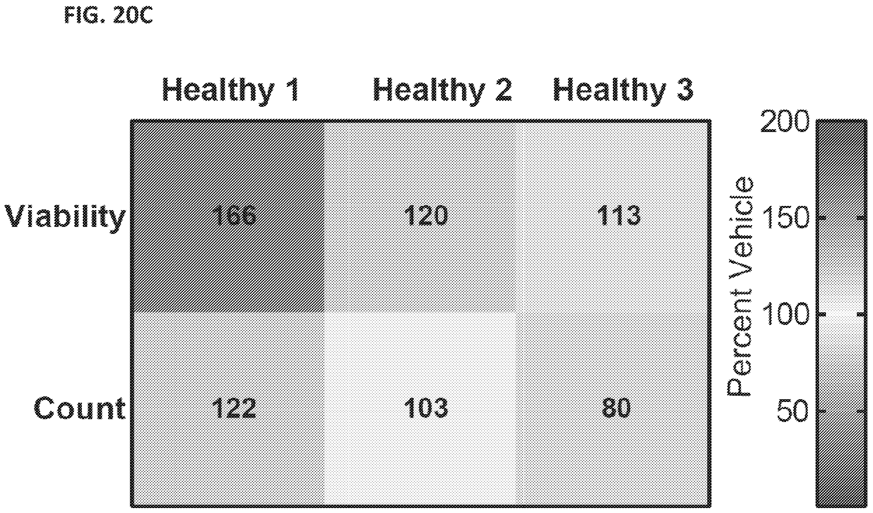

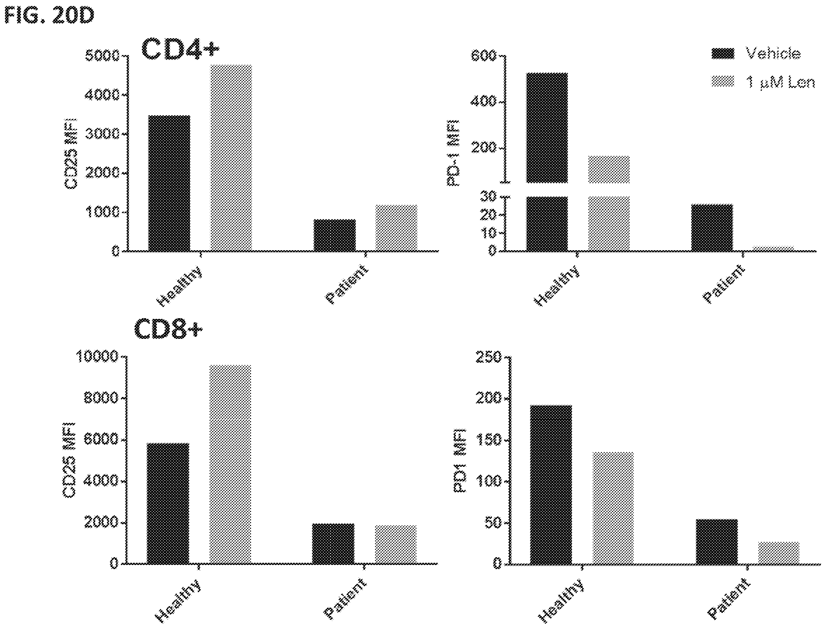

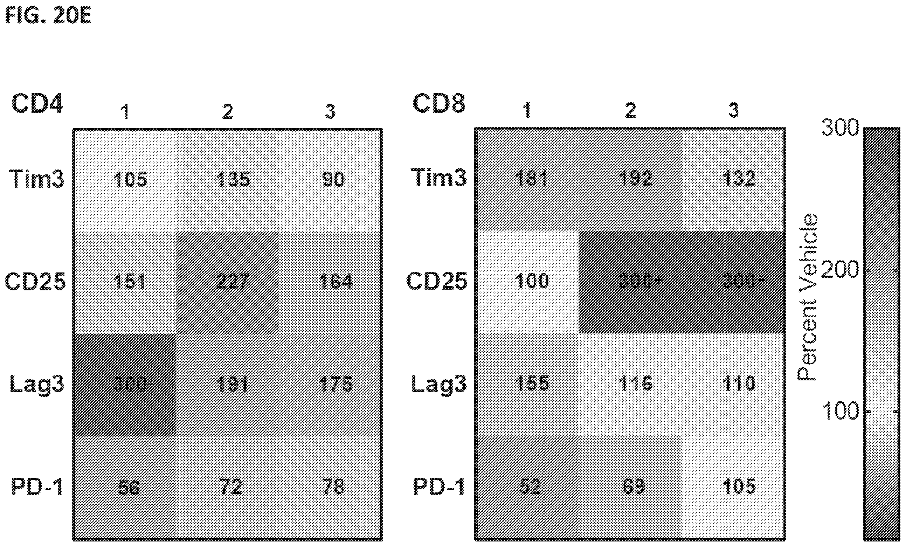

**FIG. 20A** shows results for CAR antigen-specific cytolytic activity and **FIG. 20B** shows results for cytokine production for anti-BCMA CAR-T cells that had been prestimulated with BCMA beads (compared to freshly-thawed (non-prestimulated) anti-BCMA CAR-T cells) in the co-cultures, comparing cells cultured in the presence versus absence of lenalidomide. **FIG. 20C** shows the overall viability and cell count assessed for three anti-BCMA CAR T donors. **FIG. 20D** shows results of flow cytometric analysis of surface CD25 and PD-1 expression (mean fluorescent intensity (MFI), for CD4+ and CD8+ anti-BCMA CAR T-cells after stimulation (pretreatment) with BCMA beads for 7 days, in the presence or absence of 1 µM lenalidomide. **FIG. 20E** shows the flow cytometric analysis across CAR T donors for median fluorescence intensity (MFI; CD25 and Tim3) or percentage positive PD-1 and Lag3 on the surface of T-cell markers in CD4+ CAR+ and CD8+ CAR+ subsets (gated on live CD3+ cells). Values shown are percentage baseline (Veh) MFI, viability, or count.

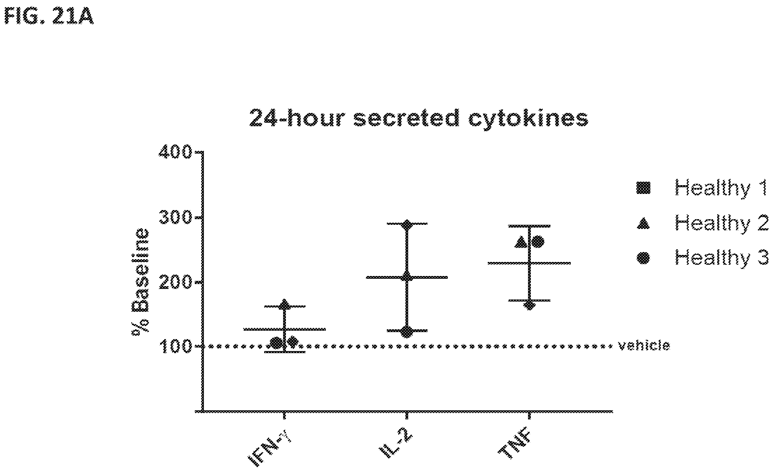

**FIG. 21A** shows the analysis of effector cytokine production following CAR-specific stimulation on 50 µg BCMA beads for 24 hours in the presence of 1 µM lenalidomide compared with baseline (vehicle) response for each of three donors.

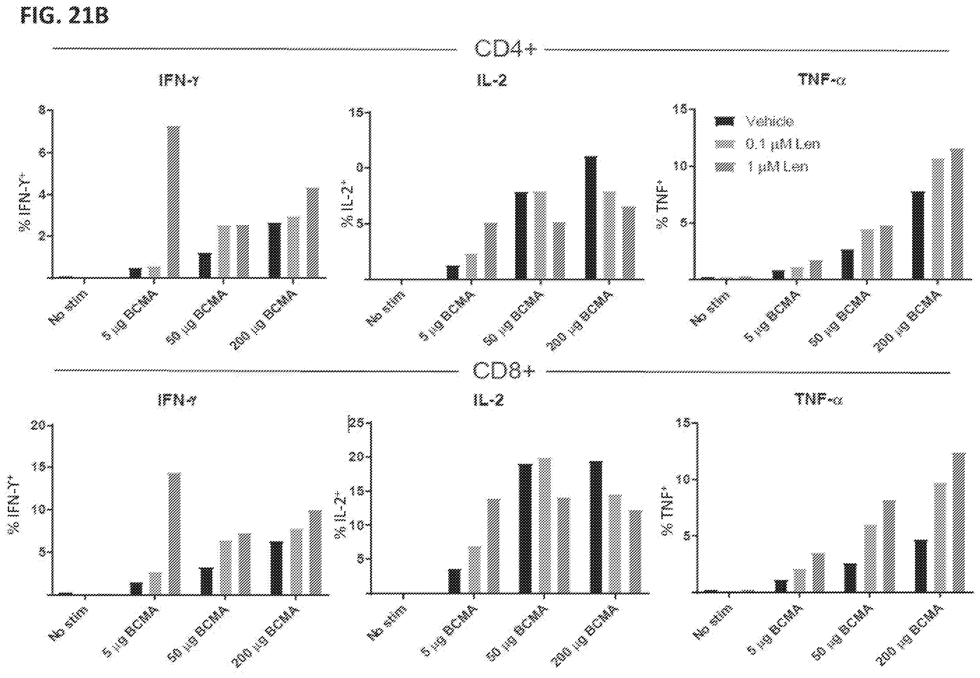

**FIG. 21B** shows the effects of anti-BCMA CAR T cells activated on different concentrations of BCMA beads (i.e., 5 µg, 50 µg, and 200 µg) in the absence (left bars) or presence of 0.1 µM (middle bar) or 1 µM (right bars) of lenalidomide on CAR T effector cytokine production.

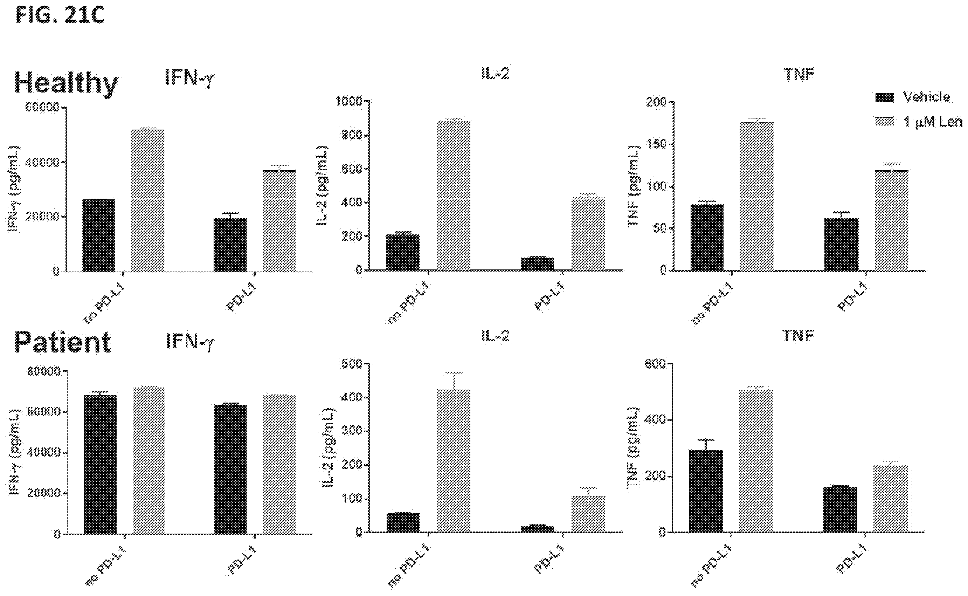

**FIG. 21C** shows the cytokine production of anti-BCMA CAR T cells derived from representative healthy donors and multiple myeloma patients stimulated on BCMA beads with or without addition of PD-L1 on the beads, in the presence or absence of 1 µM lenalidomide.

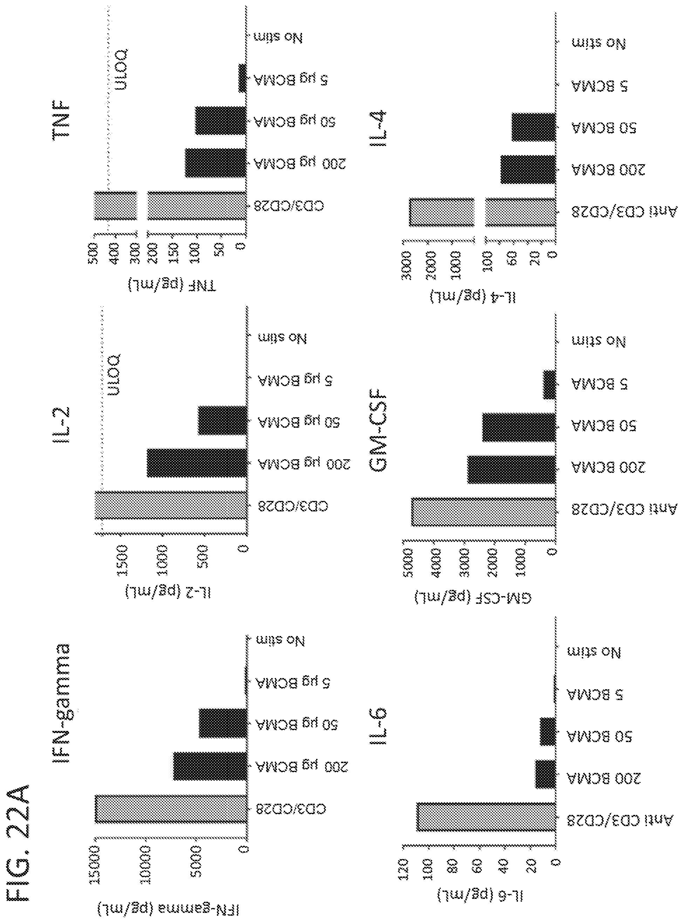

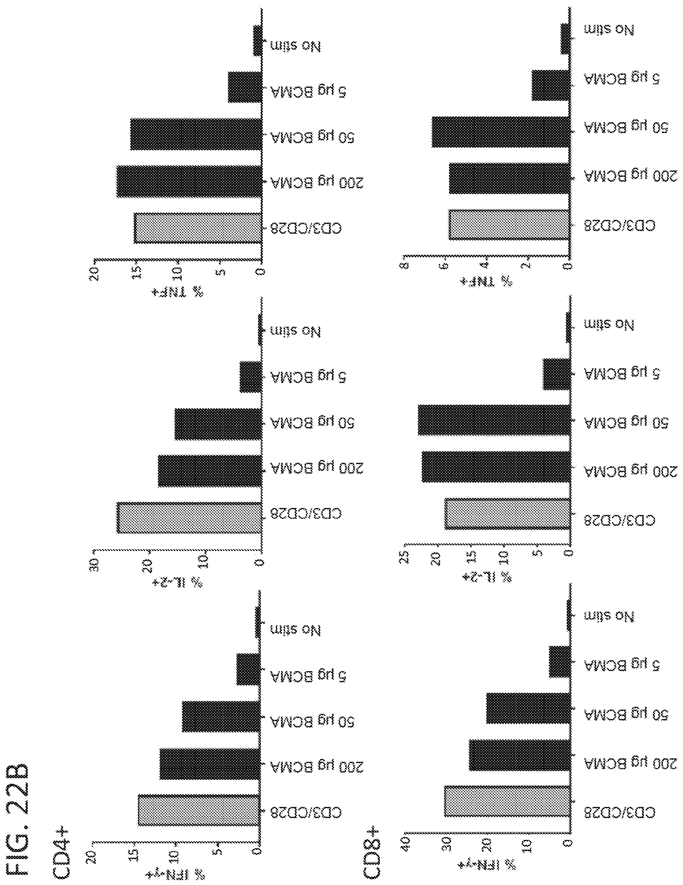

**FIGS. 22A** **and** **22B** present graphs showing cytokine production in anti-BCMA CAR expressing T cells. **FIG. 22A** depicts concentration (pg/mL) of IFN-gamma, IL-2, TNF-alpha, IL-6, GM-CSF, and IL-4 in supernatant collected from anti-BCMA CAR expressing T cells following a 24 hour incubation with anti-CD3/anti-CD28 antibody-conjugated beads (CD3/CD28), BCMA-conjugated beads with a concentration of 200 µg/ml, 50 µg/ml, or 5 µg/ml conjugated BCMA per approximately 4×10⁸ beads (200 µg, 50 µg, or 5 µg BCMA, respectively), or cells incubated without beads (no stim). Dashed horizontal lines indicate the upper limit of quantification (ULOQ). **FIG. 22B** depicts the percentage of CD4+CAR+ (top row) or CD8+CAR+ cells (bottom row) positive for intracellular staining of IFN-gamma, IL-2, or TNF-alpha following incubation with CD3/CD28 beads, 200 µg, 50 µg, or 5 µg BCMA-conjugated beads or no stim.

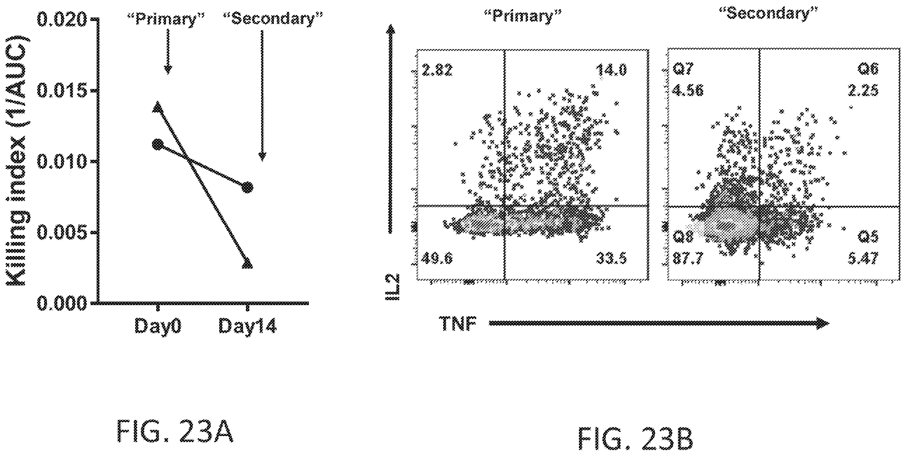

**FIGS. 23A and 23B** present graphs showing activity of T cell compositions containing anti-CD19 CAR+ T cells. Cells were either incubated with anti-CD19 antibody anti-ID conjugated beads for 14 days (Day 14; secondary) or were not incubated prior to assessing activity. Results from a cytotoxicity assay **(****FIG. 23A****)** and an intracellular cytokine staining (ICS) assay following exposure to CD19 expressing cells **(****FIG. 23B****)** are shown.

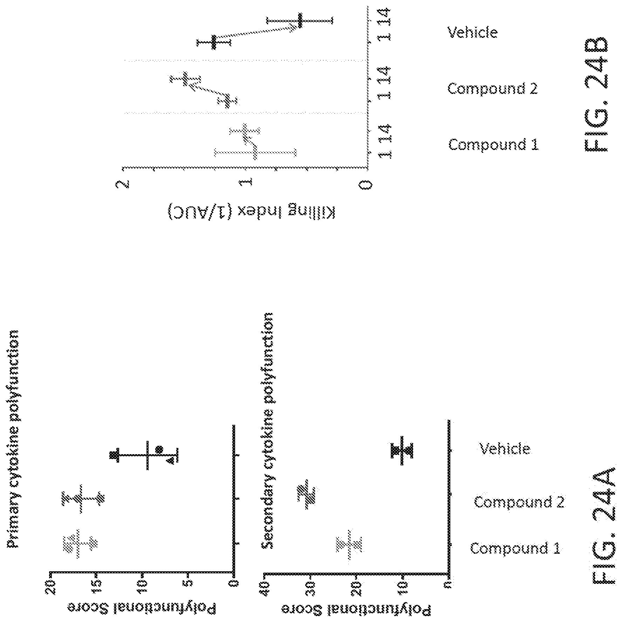

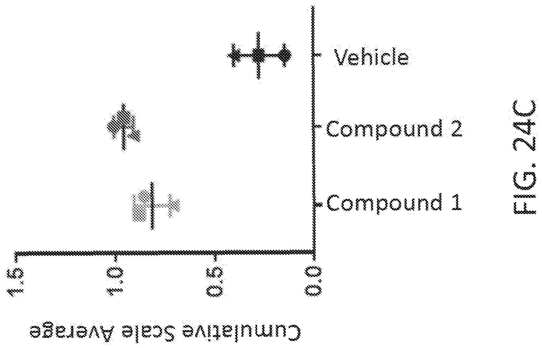

**FIGS. 24A-24C** present graphs showing characteristics of T cell compositions containing anti-CD19 CAR+ T cells during or following incubation with anti-CD19 antibody anti-ID conjugated beads for 14 days. Results from T cell compositions that were generated in the presence of different test compounds or a vehicle are shown. **FIGS. 24A and 24B** show activity in response to exposure to CD19 cells of T cell compositions that were not incubated (primary) or incubated for 14 days (secondary). Results of polyfunctional staining by ICS **(****FIG. 24A****)** and a cytolytic activity **(****FIG. 24B****)** following exposure to CD19 expressing cells are shown. **FIG. 24C** depicts levels of secreted cytokine from supernatant of cell compositions containing anti-CD19 CAR expressing cells that were incubated at a ratio of 1:1 with CD19 expressing cells for 20 hours. Amounts of IL2, TNF, and IFN-gamma were measured and the average of the scaled scores for all three cytokines is shown.

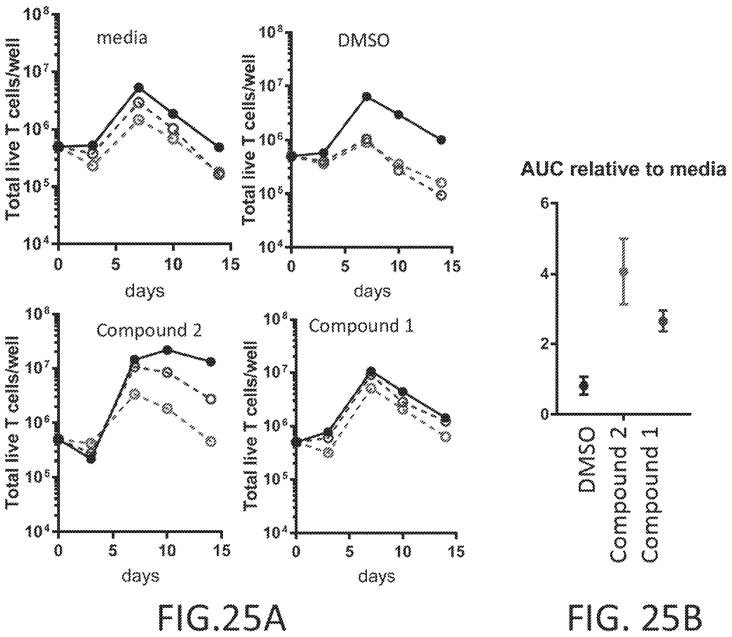

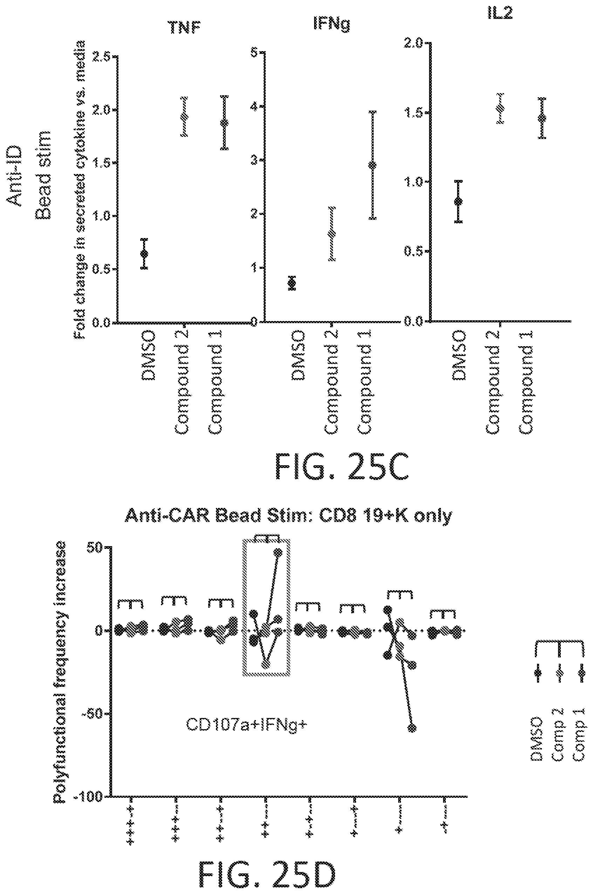

**FIGS. 25A-25D** show graphs displaying activity of T cells from generated anti-CD19 CAR-T cell compositions that were expanded in the presence of media only, DMSO vehicle control, Compound 1 or Compound 2 following stimulation with beads surface conjugated with anti-idiotype antibody specific to the anti-CD19 CAR. **FIG. 25A** shows the total live T cell counts per well of T cells from generated anti-CD19 CAR-T cell compositions co-cultured with beads surface conjugated with the anti-idiotype antibody. **FIG. 25B** displays the area under the curve (AUC) calculated for the live T cell counts relative to media only controls. **FIG. 25C** shows a graph displaying the production of TNF-alpha (TNF), IFN-gamma (IFNg), and IL-2 by T cells from generated anti-CD19 CAR-T cell compositions following stimulation with a 16 hour co-culture with irradiated K562-CD19 target cells that followed a 15 day incubation with beads surface conjugated to the anti-idiotype antibody. The fold change of extracellular TNF-alpha (TNF), IFN-gamma (IFNg), and IL-2 as compared to the media only condition is shown. **FIG. 25D** shows graphs depicting the polyfunctional cytokine profiles of CD8+ T cells from generated anti-CD19 CAR-T cell compositions that followed a 15 day incubation with beads conjugated with the anti-idiotype antibody.

### Detailed Description

Provided herein are compositions and methods for enriching, expanding, and/or activating genetically engineered cells that express a recombinant receptor, e.g., a chimeric antigen receptor (CAR). In some embodiments, the provided methods include the *ex vivo* or *in vitro* enrichment, expansion, and/or activation of cells by incubation with a particle, e.g., a bead particle, with an attached binding molecule that recognizes or binds to the recombinant receptor. In some embodiments, the attached binding molecule is a polypeptide, e.g., a polypeptide antigen or an anti-idiotype antibody that binds to the recombinant receptor.

In some embodiments, the compositions and methods provided herein possess one or more advantages over the existing means of expanding, enriching, or activating cells that express a recombinant receptor, e.g., a CAR. Many existing protocols for *ex vivo* or *in vitro* expansion rely on incubating the cells, e.g., T cells, with one or more polyclonal stimulatory molecules capable of activating components of the TCR complex. For example, a common protocol for expanding cells is to incubate the cells with anti-CD3 and anti-CD28 antibodies, such as by incubating the cells with anti-CD3 and anti-CD28 antibodies that are attached to paramagnetic beads. One drawback of this method is that all or most of the cells of a given composition, e.g., cultured T cells, will be contacted and stimulated by the antibodies. Thus, in some embodiments, for a given composition of cells contacted with anti-CD3 and anti-CD28 antibodies, all or most of the cells become activated regardless of whether or not they express the recombinant receptor. In contrast, in particular embodiments, when cells are incubated with the particles, e.g., beads, provided herein, the binding molecules of the particles, e.g., beads, directly bind to the recombinant receptor, thus resulting in a greater stimulation, activation, proliferation, and/or expansion in the cells expressing the recombinant receptor as compared to the cells that lack the receptor. In some embodiments, incubating the cells that include cells that express a recombinant receptor with the particles, e.g., beads, described herein increases the portion, fraction, or subset of the cells that express the recombinant receptor.

In particular embodiments, one advantage of expanding, enriching, and/or activing the cells with the particles, e.g., beads, described herein as compared to other methods, e.g., stimulation with anti-CD3 and anti-CD28 antibodies, is that incubation with the particles provided herein results in less activation and/or exhaustion as opposed to cells that are expanded, enriched, and/or activated by other methods. For example, in some embodiments, cells incubated with the particles provided herein express lower levels or markers associated with activation, e.g., surface expression of CD25, or exhaustion, e.g., expression of PD1, as compared to cells that are incubated with polyclonal stimulatory molecules capable of activating components of the TCR complex, e.g., anti-CD3 and anti-CD28 antibodies.

In certain embodiments, provided are methods for transducing or transfecting cells with a viral or non-viral vector to deliver a nucleic acid that encodes a recombinant receptor or CAR to cells while they are contacted, treated, or incubated with the particles, e.g., beads, described herein at least for the portion of the transduction or transfection process. In some embodiments, one advantage of transducing or transfecting the cells in the presence of the particles, e.g., beads, allows for the transfection or transduction of cells that have not been previously activated with polyclonal stimulatory molecules, e.g., anti-CD3 and anti-CD28 antibodies. Particular embodiments contemplate that cells that are transduced or transfected without prior activation with polyclonal molecules will exhibit increased persistence and/or reduced exhaustion as compared to cells that were activated prior to the transfection or transducing.

Particular embodiments contemplate that particular recombinant antigens, or fragments thereof, attached to particles, e.g., beads, as described may be particularly suited for uses to specifically stimulate a CAR containing an antigen-binding domain that recognizes the antigen. In some cases, certain recombinant antigens, e.g. BCMA, may have few or no non-specific interactions, such as non-specific protein-protein interactions or non-specific interactions with cells that prevents or minimizes the antigen non-specifically sticking to the cells or getting soaked up by the cells. In some cases, this can improve the quality of stimulation of the CAR-expressing cells. In some embodiments, attachment to beads or particles results in an increased, enhanced, consistent, and/or more reliable stimulation, activation, or expansion of cells as compared to when the recombinant antigen is unbound or bound to a different solid support, such as the surface of a plate or dish. In certain embodiments, the binding molecule is or includes recombinant BCMA or a fragment thereof.

In particular embodiments, particles or beads containing attached recombinant BCMA (such as a recombinant BCMA-FC fusion) are used in conjunction with the provided methods to activate, stimulate, or expand cells of a cell composition. In some embodiments, the particles or beads containing the recombinant BCMA selectively activate, stimulate, or expand the cells expressing a recombinant receptor containing an antigen-binding domain that binds to or recognizes BCMA, e.g., an anti-BCMA CAR. In certain embodiments, the activation, stimulation, or expansion of the cells, e.g., the anti-BCMA CAR expressing cells, is greater or increased as compared to activation, stimulation, or expansion by other reagents, such as for example anti-CD3/anti-CD28 antibody conjugated bead reagents. In certain embodiments, the activation, stimulation, or expansion of the cells, e.g., the anti-BCMA CAR expressing cells, is more indicative of activation, stimulation, or expansion of the cells in vivo and/or in response to endogenous antigen as compared to activation, stimulation, or expansion by other reagents, such as for example anti-CD3/anti-CD28 antibody conjugated bead reagents. In some aspects, the degree to which the cells are activated, stimulated, or expanded by the particles or beads particles or beads containing attached recombinant BCMA can be adjusted, modified or controlled by altering the amount of recombinant BCMA attached to the particles or beads or by altering the ratio of particles or beads to cells. In some aspects, activation, stimulation, or expansion of cells, e.g., anti-BCMA CAR expressing cells, by recombinant BCMA is more effective when the recombinant BCMA is attached to particles or beads than when the recombinant BCMA is free floating or unattached, or than when the recombinant BCMA is attached to a different surface, e.g., a culture dish or plate.

In some embodiments, the binding molecule is an anti-idiotypic antibody (anti-ID) that binds to or recognizes a recombinant receptor, e.g., a CAR. In particular embodiments, particles or beads containing attached recombinant anti-IDs are used in conjunction with the provided methods to activate, stimulate, or expand cells of a cell composition. In various embodiments, the particles or beads containing anti-IDs selectively activate, stimulate, or expand the cells of the composition, such as cells expressing the recombinant receptor, e.g., a CAR. In certain embodiments, the activation, stimulation, or expansion of the cells, e.g., CAR expressing cells, is greater or increased as compared to activation, stimulation, or expansion by other reagents, such as for example anti-CD3/anti-CD28 antibody conjugated bead reagents. In certain aspects, the degree to which the cells are activated, stimulated, or expanded by the particles or beads particles or beads containing anti-IDs can be adjusted, modified or controlled by altering the amount of anti-IDs attached to the particles or beads or by altering the ratio of particles or beads to cells. In particular embodiments, the anti-ID is an anti-CD19 antibody anti-ID. In particular embodiments, the activation, stimulation, or expansion of anti-CD 19 CAR expressing cells by particles or beads containing anti-CD19 antibody anti-IDs is greater or increased as compared to activation, stimulation, or expansion by other reagents, such as for example, anti-CD3/anti-CD28 antibody conjugated bead reagents.

In some embodiments, the provided compositions and methods expand and activate genetically engineered T cells that express recombinant receptors or CARs, that, when administered to a subject, exhibit increased persistence and/or reduced exhaustion as compared to genetically engineered T cells that are expanded by other techniques. In some embodiments, genetically engineered T cells with increased persistence and/or reduced exhaustion exhibit better potency in a subject to which they are administered.

All publications, including patent documents, scientific articles and databases, referred to in this application are incorporated by reference in their entirety for all purposes to the same extent as if each individual publication were individually incorporated by reference. If a definition set forth herein is contrary to or otherwise inconsistent with a definition set forth in the patents, applications, published applications and other publications that are herein incorporated by reference, the definition set forth herein prevails over the definition that is incorporated herein by reference.

The section headings used herein are for organizational purposes only and are not to be construed as limiting the subject matter described.

### I. PARTICLE CONJUGATES

Provided herein are particles, such as beads, that are conjugated or otherwise attached to a binding molecule that binds or is recognized by an antigen-binding domain of a recombinant receptor, such as a chimeric antigen receptor (CAR) and methods for use thereof. In some embodiments, the particles, e.g., beads, are non-cell particles.

### A. Particles

In some embodiments, a binding molecule that binds to or is recognized by an antigen-binding domain of a recombinant receptor, such as a chimeric antigen receptor (CAR), is bound to or otherwise attached to a particle (e.g., bead particles), for example to the surface of the particle. In certain embodiments, the particle is a non-cell particle. In particular embodiments, the particle may include a colloidal particle, a microsphere, nanoparticle, a bead, such as a magnetic bead, or the like. In some embodiments, the particles or beads are biocompatible, i.e. non-toxic. In certain embodiments the particles or beads are non-toxic to cultured cells, e.g., cultured T cells. In particular embodiments, the particles are monodisperse. In certain embodiments, "monodisperse" encompasses particles (e.g., bead particles) with size dispersions having a standard deviation of less than 5%, e.g., having less than a 5% standard deviation in diameter.

In some embodiments, a particle described herein (e.g., bead particle) provides a solid support or matrix to which a binding molecule, such as a binding molecule described herein (e.g., an antigen or an antibody), can be bound or attached in a manner that permits an interaction between the binding molecule and a cell, in particular binding between the binding molecule and a recombinant receptor, e.g., a CAR, expressed on the surface of the cell. In particular embodiments, the interaction between the conjugated or attached binding molecule and the cell can be used in methods to facilitate enrichment, activation, stimulation and/or expansion of one or more cell types in a cell population based on expression or expression level of one or more recombinant receptors on the surface of a cell. In certain embodiments, the particle (e.g., a bead particle) comprises one or more binding molecules (e.g., an antibody or an antigen) that bind to an antigen binding region of a recombinant receptor, e.g., a CAR that is expressed on the surface of the cell.

In some embodiments, the particle or bead is biocompatible, i.e., composed of a material that is suitable for biological use. In some embodiments, the particles, e.g., beads, are non-toxic to cultured cells, e.g., cultured T cells. In some embodiments, the particles, e.g., beads, may be any particles which are capable of attaching binding molecules in a manner that permits an interaction between the binding molecule and a cell. In certain embodiments, the particles, e.g., beads, may be any particles that can be modified, e.g., surface functionalized, to allow for the attachment of a binding molecule at the surface of the particle. In some embodiments, the particles, e.g., beads, are composed of glass, silica, polyesters of hydroxy carboxylic acids, polyanhydrides of dicarboxylic acids, or copolymers of hydroxy carboxylic acids and dicarboxylic acids. In some embodiments, the particles, e.g., beads, may be composed of or at least partially composed of polyesters of straight chain or branched, substituted or unsubstituted, saturated or unsaturated, linear or cross-linked, alkanyl, haloalkyl, thioalkyl, aminoalkyl, aryl, aralkyl, alkenyl, aralkenyl, heteroaryl, or alkoxy hydroxy acids, or polyanhydrides of straight chain or branched, substituted or unsubstituted, saturated or unsaturated, linear or cross-linked, alkanyl, haloalkyl, thioalkyl, aminoalkyl, aryl, aralkyl, alkenyl, aralkenyl, heteroaryl, or alkoxy dicarboxylic acids. Additionally, particles, e.g., beads, can be quantum dots, or composed of quantum dots, such as quantum dot polystyrene particles, e.g., beads. Particles, e.g., beads, including mixtures of ester and anhydride bonds (e.g., copolymers of glycolic and sebacic acid) may also be employed. For example, particles, e.g., beads, may comprise materials including polyglycolic acid polymers (PGA), polylactic acid polymers (PLA), polysebacic acid polymers (PSA), poly(lactic-co-glycolic) acid copolymers (PLGA), [rho]poly(lactic-co-sebacic) acid copolymers (PLSA), poly(glycolic-co-sebacic) acid copolymers (PGSA), etc. Other polymers that particles, e.g., beads, may be composed of include polymers or copolymers of caprolactones, carbonates, amides, amino acids, orthoesters, acetals, cyanoacrylates and degradable urethanes, as well as copolymers of these with straight chain or branched, substituted or unsubstituted, alkanyl, haloalkyl, thioalkyl, aminoalkyl, alkenyl, or aromatic hydroxy- or di-carboxylic acids. In addition, the biologically important amino acids with reactive side chain groups, such as lysine, arginine, aspartic acid, glutamic acid, serine, threonine, tyrosine and cysteine, or their enantiomers, may be included in copolymers with any of the aforementioned materials to provide reactive groups for conjugating to binding molecules such as polypeptide antigen or antibodies.

In some embodiments, the particles or beads have a diameter of greater than 0.001 µm, greater than 0.01 µm, greater than 0.05 µm, greater than 0.1 µm, greater than 0.2 µm, greater than 0.3 µm, greater than 0.4 µm, greater than 0.5 µm, greater than 0.6 µm, greater than 0.7 µm, greater than 0.8 µm, greater than 0.9 µm, greater than 1 µm, greater than 2 µm, greater than 3 µm, greater than 4 µm, greater than 5 µm, greater than 6 µm, greater than 7 µm, greater than 8 µm, greater than 9 µm, greater than 10 µm, greater than 20 µm, greater than 30 µm, greater than 40 µm, greater than 50 µm, greater than 100 µm, greater than 500 µm, and/or greater than 1,000 µm. In some embodiments, the particles or beads have a diameter of between or between about 0.001 µm and 1,000 µm, 0.01 µm and 100 µm, 0.1 µm and 10, µm, 0.1 µm and 100 µm, 0.1 µm and 10 µm, 0.001 µm and 0.01 µm, 0.01 µm and 0.1 µm, 0.1 µm and 1 µm, 1 µm and 10 µm, 1 µm and 2 µm, 2 µm and 3 µm, 3 µm and 4 µm, 4 µm and 5 µm, 1 µm and 5 µm, and/or 5 µm and 10 µm, each inclusive. In certain embodiments, the particles or beads have a mean diameter of 1 µm and 10 µm, each inclusive. In certain embodiments, the particles, e.g., beads, have a diameter of or of about 1 µm. In particular embodiments, the particles, e.g., beads, have a mean diameter of or of about 2.8 µm. In some embodiments, the particles, e.g., beads, have a diameter of or of about 4.8 µm.

In certain embodiments, a plurality of the particles, e.g., beads, has a uniform particle size. In some embodiments, a uniform particle size comprises a diameter standard deviation of less than 10%, less than 5%, or less than 1% of the mean diameter of the plurality. In particular embodiments, the plurality of the particles, e.g., beads, has a diameter standard deviation of less than 10%, less than 5%, or less than 1% of the mean diameter of the plurality.

In particular embodiments, the particles (e.g., bead particles) are uniformly shaped. In some embodiments, the particles, e.g., beads, are spherical. In certain embodiments, the particles, e.g., beads, are non-spherical.

In some embodiments, the particles, e.g., beads, have a density of greater than 0.001 g/cm³, greater than 0.01 g/cm³, greater than 0.05 g/cm³, greater than 0.1 g/cm³, greater than 0.5 g/cm³, greater than 0.6 g/cm³, greater than 0.7 g/cm³, greater than 0.8 g/cm³, greater than 0.9 g/cm³, greater than 1 g/cm³, greater than 1.1 g/cm³, greater than 1.2 g/cm³, greater than 1.3 g/cm³, greater than 1.4 g/cm³, greater than 1.5 g/cm³, greater than 2 g/cm³, greater than 3 g/cm³, greater than 4 g/cm³, or greater than 5g/cm³. In some embodiments, the particles or beads have a density of between or between about 0.001 g/cm³ and 100 g/cm³, 0.01 g/cm³ and 50 g/cm³, 0.1 g/cm³ and 10 g/cm³, 0.1 g/cm³ and 0.5 g/cm³, 0.5 g/cm³ and 1 g/cm³, 0.5 g/cm³ and 1.5 g/cm³, 1 g/cm³ and 1.5 g/cm³, 1 g/cm³ and 2 g/cm³, or 1 g/cm³ and 5 g/cm³. In some embodiments, the particles or beads have a density of, of at least, or of about 0.5 g/cm³, 0.5 g/cm³, t 0.6 g/cm³, 0.7 g/cm³, a0.8 g/cm³, 0.9 g/cm³, 1.0 g/cm³, 1.1 g/cm³, 1.2 g/cm³, 1.3 g/cm³, 1.4 g/cm³, 1.5 g/cm³, 1.6 g/cm³, 1.7 g/cm³, 1.8 g/cm³, 1.9 g/cm³, or 2.0 g/cm³, each inclusive. In certain embodiments, the beads or particles have a density of or of about 1.6 g/cm³. In particular embodiments, the beads or particles have a density of or of about 1.5 g/cm³. In certain embodiments, the particles, e.g., beads, have a density of or of about 1.3 g/cm³.

In certain embodiments, a plurality of the particles or beads has a uniform density. In certain embodiments, a uniform density comprises a density standard deviation of less than 10%, less than 5%, or less than 1% of the mean particle density.

In some embodiments, the particles or beads have a surface area of between or between about 0.001 m² per each gram of particles, e.g., beads, (m²/g) and 1,000 m²/g,0.010 m²/g and 100 m²/g, 0.1 m²/g and 10 m²/g, 0.1 m²/g and 1 m²/g, 1 m²/g and 10 m²/g, 10 m²/g and 100 m²/g, 0.5 m²/g and 20 m²/g, 0.5 m²/g and 5 m²/g, or 1 m²/g and 4 m²/g, each inclusive. In some embodiments, the particles or beads have a surface area of or of about 1 m²/g to 4 m²/g.

In particular embodiments, the particles (e.g., bead particles) have a specific gravity, i.e. the ratio of the density of the particles, e.g., beads, to the density of water, of between or between about 0.01 and 100, 0.1 and 10, 0.5 and 5, 1 and 10, 1 and 2, 1.1 and 1.8, or 1.2 and 1.5, each inclusive. In certain embodiments, the particles, e.g., beads, have a specific gravity of 1.2 and 1.5. In certain embodiments, the particles, e.g., beads, are monodisperse and the specific gravity is uniform. In particular embodiments, particles, e.g., beads, with a uniform specific gravity have a specific gravity standard deviation of less than 10%, less than 5%, or less than 1%. In various embodiments, the particles are monodisperse and have a specific gravity with a standard deviation of less than 10%, less than 5%, or less than 1%.

In certain embodiments, the particle surface comprises attached biomolecules that can bind or attach binding molecules. In particular embodiments, the biomolecules are polypeptides. In some embodiments, the particles, e.g., beads, comprise surface exposed protein A, protein G, or biotin.

In some of the embodiments, the particle contains one or more coats or coatings such as one or more coats or coatings on the surface of the particle (e.g., a surface coating). In some embodiments, the one or more coats or coatings provide a material for conjugation or coupling to a binding molecule, e.g., a coat that is or is capable of being surface functionalized. In certain embodiments, the coat comprises, or is capable of attaching, surface exposed functional groups. In particular embodiments, the coating comprises, or is capable of attaching, surface-exposed carboxyl groups, amino groups, hydroxyl groups, tosyl groups, epoxy groups, chloromethyl groups, or combinations thereof. In some embodiments, the coat or coating is hydrophobic. In particular embodiments the coat or coating is non-hydrophobic.

### 1. Magnetic Particles

In some embodiments, the particle (e.g., bead particle) reacts in a magnetic field. In some embodiments, the particle is a magnetic particle (e.g., magnetic bead particle). In some embodiments, the magnetic particle is paramagnetic. In particular embodiments, the magnetic particle is superparamagnetic. In certain embodiments, the particles, e.g., beads, do not display any magnetic properties unless they are exposed to a magnetic field.

In particular embodiments, the particle can be a composite particle containing an inner core. In some embodiments, the inner core is a magnetic core, a paramagnetic core or a superparamagnetic core. In some embodiments, the inner core (e.g., magnetic core) is or contains a metal. In some embodiments, the metal can be, but is not limited to, iron, nickel, copper, cobalt, gadolinium, manganese, tantalum, zinc, zirconium or any combinations thereof. Suitable substances that may be included in an inner core described herein (e.g., a magnetic core) includes, but is not limited to, metal oxides (e.g., iron oxides), ferrites (e.g., manganese ferrites, cobalt ferrites, nickel ferrites, etc.), hematite and metal alloys (e.g., CoTaZn). In some embodiments, the inner core comprises one or more of a ferrite, a metal, a metal alloy, an iron oxide, or chromium dioxide. In some embodiments, the inner core comprises elemental iron or a compound thereof. In some embodiments, the inner core comprises one or more of magnetite (Fe3O4), maghemite (γFe2O3), or greigite (Fe3S4). In some embodiments, the inner core comprises an iron oxide (e.g., Fe₃O₄).

In certain embodiments, the particle contains a magnetic, paramagnetic, and/or superparamagnetic core that is covered by a surface functionalized coat or coating. In some embodiments, the particle comprises surface exposed tosyl groups.

In some embodiments, the coat can contain a material that can include, but is not limited to, a polymer, a polysaccharide, a silica, a fatty acid, a protein, a carbon, or a combination thereof. In some embodiments, the polymer can be a polyethylene glycol, poly (lactic-co-glycolic acid), polyglutaraldehyde, polyurethane, polystyrene, or a polyvinyl alcohol. In certain embodiments, the outer coat or coating comprises polystyrene. In some embodiments, the coat contains or includes a material that is or includes a protein that is an albumin (e.g., human serum albumin), Protein A, and Protein G. In some embodiments, the carbon is an acrylamide or maleic acid. In some embodiments, the material is coupled, linked or conjugated to a binding molecule described herein.

In some embodiments, a particle described herein (e.g., a bead particle) can have an inner core and a coat (e.g., protective coat) wherein the coat contains one or more material described herein. In some embodiments, the coat is hydrophobic. In certain embodiments, the coat is hydrophilic. In some embodiments, a particle described herein (e.g., a bead particle) has a metal oxide core (e.g., an iron oxide inner core) and a coat (e.g., a protective coat), wherein the coat comprises polystyrene.

The particles (e.g., bead particles) used in the methods described herein can be produced or obtained commercially. Particles, e.g., beads, including methods of producing particles, e.g., beads, are well known in the art. See, for example, U.S. Pat. Nos. 6,074,884; 5,834,121; 5,395,688; 5,356,713; 5,318,797; 5,283,079; 5,232,782; 5,091,206; 4,774,265; 4,654,267; 4,554,088; 4,490,436; 4,452,773; U.S. Patent Application Publication No. 20100207051; and Sharpe, Pau T., Methods of Cell Separation, Elsevier, 1988. Commercially available particles, e.g., beads, (e.g., bead particles) include, but are not limited to, ProMagTM (PolySciences, Inc.); COMPELTM (PolySciences, Inc.); BioMag^{®} (PolySciences, Inc.), including BioMag^{®} Plus (PolySciences, Inc.) and BioMag^{®} Maxi (Bang Laboratories, Inc.); M-PVA (Cehmagen Biopolymer Technologie AG); SiMAG (Chemicell GmbH); beadMAG (Chemicell GmbH); MagaPhase^{®} (Cortex Biochem); Dynabeads^{®} (Invitrogen), including Dynabeads^{®} M-280 Sheep Anti-rabbit IgG (Invitrogen), Dynabeads^{®} FlowCompTM (e.g., Dynabeads^{®} FlowCompTMHuman CD3, Invitrogen), Dynabeads^{®} M-450 (e.g., Dynabeads^{®} M-450 Tosylactivated, Invitrogen), Dynabeads^{®} UntouchedTM (e.g., Dynabeads^{®} UntouchedTM Human CD8 T Cells, Invitrogen), and Dynabeads^{®} that bind, expand and/or activate T cells (e.g., Dynabeads^{®} Human T-Activator CD3/CD28 for T Cell Expansion and Activation, Invitrogen); Estapor^{®} M (Merk Chimie SAS); Estapor^{®} EM (Merk Chimie SAS); MACSiBeadsTM Particles (e.g., anti-biotin MACSiBead Particles, Miltenyi Biotec, catalog #130-091-147); Streptamer^{®} Magnetic Beads (IBA BioTAGnology); Strep-Tactin^{®} Magnetic Beads (IBA BioTAGnology); Sicastar^{®}-M (Micormod Partikeltechnologie GmbH) Micromer^{®}-M (Micromod Partikeltechnologie); MagneSilTM (Promega GmbH); MGP (Roche Applied Science Inc.); Pierce^{™} Protein G Magnetic Beads (Thermo Fisher Scientific Inc.); Pierce^{™} Protein A Magnetic Beads (Thermo Fisher Scientific Inc.); Pierce^{™} Protein A/G Magnetic Beads (Thermo Fisher Scientific Inc.); Pierce^{™} NHS-Activated Magnetic Beads (Thermo Fisher Scientific Inc.); Pierce^{™} Protein L Magnetic Beads (Thermo Fisher Scientific Inc.); Pierce^{™} Anti-HA Magnetic Beads (Thermo Fisher Scientific Inc.); Pierce^{™} Anti-c-Myc Magnetic Beads (Thermo Fisher Scientific Inc.); Pierce^{™} Glutathione Magnetic Beads (Thermo Fisher Scientific Inc.); Pierce^{™} Streptavidin Magnetic Beads (Thermo Fisher Scientific Inc.); MagnaBindTM Magnetic Beads (Thermo Fisher Scientific Inc.); Sera-MagTM Magnetic Beads (Thermo Fisher Scientific Inc.); Anti-FLAG^{®} M2 Magnetic Beads (Sigma-Aldrich); SPHEROTM Magnetic Particles (Spherotech Inc.); and HisPurTM Ni-NTA Magnetic Beads (Thermo Fisher Scientific Inc.).

In certain embodiments, the particle is monodisperse, superparamagnetic bead particles comprising a superparamagnetic iron core, e.g., a magnetite (Fe₃O₄) or maghemite (γFe₂O₃) core, a polystyrene coat or coating, and a functionalized surface comprising exposed tosyl groups. In certain embodiments, the particles, e.g., beads, have a density of about 1.5 g/cm³ and a surface area of about 1 m²/g to about 4 m²/g. In particular embodiments, the particles, e.g., beads, are monodisperse superparamagnetic particles, e.g., beads, that have a diameter of about 4.5 µm and a density of about 1.5 g/cm³. In some embodiments, the particles, e.g., beads, the particles are monodisperse superparamagnetic particles that have a mean diameter of about 2.8 µm and a density of about 1.3 g/cm³.

### 2. Oligomer Particles

In some embodiments, the particle is an oligomer or polymer. In some embodiments, the particle is an oligomer or a polymer composed of proteins, e.g., streptavidin. In some embodiments, the particle is an oligomer or polymer that can be generated by linking directly or indirectly individual molecules of a protein, e.g., streptavidin or a variant thereof, as it exists naturally, either by linking directly or indirectly individual molecules of a monomer or a complex of subunits that make up an individual molecule (e.g., linking directly or indirectly dimers, trimers, tetramers, etc. of a protein as it exists naturally). For example, a tetrameric homodimer or heterodimer of streptavidin or avidin may be referred to as an individual molecule or smallest building block of a respective oligomer or polymer. In some embodiments, the oligomer or polymer can contain linkage of at least 2 individual molecules of the protein (e.g., is a 2-mer), or can be at least a 3-mer, 4-mer, 5-mer, 6-mer, 7-mer, 8-mer, 9-mer, 10-mer, 11-mer, 12-mer, 13-mer, 14-mer, 15-mer, 16-mer, 17-mer, 18-mer, 19-mer, 20-mer, 25-mer, 30-mer, 35-mer, 40-mer, 45-mer or 50-mer of individual molecules of the protein (e.g., monomers, tetramers). In some embodiments, the reagent is a multimer, or the oligomeric reagent is a multimeric reagent. In particular embodiments, the particle is an oligomer or polymer that comprises streptavidin or mutein of streptavidin (e.g., STREP-TACTIN^{®} or STREP-TACTIN^{®} XT streptavidin muteins)

Oligomers can be generated using any methods known in the art, such as any described in published U.S. Patent Application No. US2004/0082012. In some embodiments, the oligomer or polymer comprises two or more individual molecules that may be crosslinked, such as by a polysaccharide or a bifunctional linker.

In some embodiments, the particle is an oligomer or polymer that is obtained by crosslinking individual molecules or a complex of subunits that make up an individual molecule in the presence of a polysaccharide. In some embodiments, the particles are oligomers or polymers that can be prepared by the introduction of carboxyl residues into a polysaccharide, e.g., dextran. In some aspects, individual molecules of the particle (e.g., monomers, tetramers) can be coupled via primary amino groups of internal lysine residues and/or the free N-terminus to the carboxyl groups in the dextran backbone using conventional carbodiimide chemistry. In some embodiments, the coupling reaction is performed at a molar ratio of about 60 moles of individual molecules of the reagent (e.g., monomers, tetramers) per mole of dextran.

In some embodiments the particle is an oligomer or a polymer of one or more streptavidin or avidin or of any analog or mutein of streptavidin (e.g., Strep-Tactin^{®} or Strep-Tactin^{®} XT) or analog or mutein of avidin (e.g., neutravidin). In some embodiments, the avidin or streptavidin comprises a binding site Z that is a natural biotin binding site of avidin or streptavidin for which there can be up to four binding sites in an individual molecule (e.g., a tetramer contains four binding sites Z), whereby a homo-tetramer can contain up to 4 binding sites that are the same, i.e. Z1, whereas a hetero-tetramer can contain up to 4 binding sites that may be different, e.g., containing Z1 and Z2. In some embodiments, the oligomer is generated or produced from a plurality of individual molecules (e.g., a plurality of homo-tetramers) of the same streptavidin, streptavidin mutein, avidin or avidin mutein, in which case each binding site Z, e.g., Z1, of the oligomer is the same. For example, in some cases, an oligomer can contain a plurality of binding sites Z1, such as at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 40, 45, 50 or more binding sites Z1. In some embodiments, the oligomer is generated or produced from a plurality of individual molecules that can be hetero-tetramers of a streptavidin, streptavidin mutein, avidin or avidin mutein and/or from a plurality of two or more different individual molecules (e.g., different homo-tetramers) of streptavidin, streptavidin mutein, avidin or avidin mutein that differ in their binding sites Z, e.g., Z1 and Z2, in which case a plurality of different binding sites Z, e.g., Z1 and Z2, may be present in the oligomer. For example, in some cases, an oligomer can contain a plurality of binding sites Z1 and a plurality of binding sites Z, which, in combination, can include at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 40, 45, 50 or more combined binding sites Z1 and Z2.

In some embodiments, the particle is an oligomer or polymer is obtained by crosslinking individual molecules or a complex of subunits that make up an individual molecule using a bifunctional linker or other chemical linker, such as avidin or by other methods known in the art. In some aspects, cross-linked oligomers or polymers of streptavidin or avidin or of any mutein or analog of streptavidin or avidin may be obtained by crosslinking individual streptavidin or avidin molecules via bifunctional molecules, serving as a linker, such as glutaraldehyde or by other methods described in the art. It is, for example, possible to generate oligomers of streptavidin muteins by introducing thiol groups into the streptavidin mutein (this can, for example, be done by reacting the streptavidin mutein with 2-iminothiolan (Traut's reagent) and by activating, for example in a separate reaction, amino groups available in the streptavidin mutein. In some embodiments, this activation of amino groups can be achieved by reaction of the streptavidin mutein with a commercially available heterobifunctional crosslinker such as sulfosuccinimidyl 4-(N-maleimidomethyl)cyclohexane-1-carboxylate (sulfo SMCC) or Succinimidyl-6-[(β-maleimidopropionamido)hexanoate (SMPH). In some such embodiments, the two reaction products so obtained are mixed together, typically leading to the reaction of the thiol groups contained in the one batch of modified streptavidin mutein with the activated (such as by maleimide functions) amino acids of the other batch of modified streptavidin mutein. In some cases, by this reaction, multimers/oligomers of the streptavidin mutein are formed. These oligomers can have any suitable number of individual molecules, such as at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 40, 45, 50 or more, and the oligomerization degree can be varied according to the reaction condition.

In some embodiments, the oligomeric or polymeric reagent can be isolated via size exclusion chromatography and any desired fraction can be used as the particle. In some embodiments, after reacting the modified streptavidin mutein in the presence of 2-iminothiolan and a heterobifunctional crosslinker such as sulfo SMCC, the oligomeric or polymeric reagent can be isolated via size exclusion chromatography and any desired fraction can be used as the particle. In some embodiments, the oligomers do not have (and do not need to have) a single molecular weight but they may observe a statistical weight distribution such as Gaussian distribution. In some cases, any oligomer with more than three streptavidin or mutein tetramers, e.g., homotetramers or heterotetramers, can be used as a soluble particle, such as generally 3 to 50 tetramers, e.g., homotetramers or heterotetramers, 10 to 40 tetramers, e.g., homotetramers or heterotetramers, or 25 to 35 tetramers, e.g., homotetramers or heterotetramers. The oligomers might have, for example, from 3 to 25 streptavidin mutein tetramers, e.g., homotetramers or heterotetramers. In some aspects, with a molecular weight of about 50 kDa for streptavidin muteins, the soluble oligomers can have a molecular weight from or from about 150 kDa to 2000 kDa, 150 kDa to 1500 kDa, 150 kDa to 1250 kDa, 150 kDa to 1000 kDa, 150 kDa to 500 kDa or 150 kDa to 300 kDa, 300 kDa to 2000 kDa, 300 kDa to 1500 kDa, 300 kDa to 1250 kDa, 300 kDa to 1000 kDa, 300 kDa to 500 kDa, 500 kDa to 2000 kDa, 500 kDa to 1500 kDa, 500 kDa to 1250 kDa, 500 kDa to 1000 kDa, 1000 kDa to 2000 kDa, 1000 kDa to 1500 kDa, 1000 kDa to 1250 kDa, 1250 kDa to 2000 kDa or 1500 kDa to 2000 kDa. Generally, because each streptavidin molecule/mutein has four biotin binding sites, such a reagent can provide 12 to 160 binding sites Z, such as 12 to 100 binding sites Z.

### B. Binding Molecules

Provided herein are binding molecules, e.g., polypeptide antigens or antibodies, that bind or are recognized by an antigen-binding domain of a recombinant receptor, such as a CAR, that are conjugated or attached to a particle, e.g., a bead particle. In some embodiments, the binding molecule is a polypeptide. In certain embodiments, the binding molecule includes any polypeptide for which a recombinant receptor, such as antigen receptor, e.g. CAR, can be designed to bind or recognize. In particular embodiments, the binding molecule is an anti-idiotype antibody that binds to or recognizes the antigen binding domain of a recombinant receptor, such as an antigen receptor, e.g. a CAR. In particular embodiments, the binding molecule is a polypeptide that is bound by or is recognized by the antigen binding domain of a recombinant receptor, such an antigen receptor, e.g. a CAR. In certain embodiments, the binding molecule is a polypeptide that is bound by or is recognized by the antigen binding domain of a CAR.

In some embodiments, the binding molecule binds to or is recognized by a CAR that does not comprise a transmembrane domain or an intracellular signaling domain of a killer cell immunoglobulin-like receptor (KIR). In certain embodiments, the binding molecule binds to or is recognized by a CAR that does not comprise a transmembrane domain or an intracellular domain from any of KIR2DS2, KIR2DL3, KIR2DL1, KIR2DL2, KIR2DL4, KIR2DL5A, KIR2DL5B, KIR2DS1, KIR2DS3, KIR2DS4, KIR2DS5, KIR3DL1, KIR3DS1, KIR3DL2, KIR3DL3, KIR2DP1 and KIR3DP1.

### 1. Antigens

In some embodiments, the binding molecule is an antigen, e.g., a recombinant antigen or fragment thereof. In certain embodiments, the antigen is a polypeptide, or a portion of a polypeptide, that is associated with a disease, e.g., a cancer. In some embodiments, the antigen is a polypeptide, or a variant or fragment of a polypeptide that is expressed on the surface of a cell that is associated with a disease, for example, a cancer cell and/or a tumor cell.

In some embodiments, the antigen is or includes αvβ6 integrin (avb6 integrin), B cell maturation antigen (BCMA), B7-H3, B7-H6, carbonic anhydrase 9 (CA9, also known as CAIX or G250), a cancer-testis antigen, cancer/testis antigen 1B (CTAG, also known as NY-ESO-1 and LAGE-2), carcinoembryonic antigen (CEA), a cyclin, cyclin A2, C-C Motif Chemokine Ligand 1 (CCL-1), CD19, CD20, CD22, CD23, CD24, CD30, CD33, CD38, CD44, CD44v6, CD44v7/8, CD123, CD133, CD138, CD171, chondroitin sulfate proteoglycan 4 (CSPG4), epidermal growth factor protein (EGFR), type III epidermal growth factor receptor mutation (EGFR vIII), epithelial glycoprotein 2 (EPG-2), epithelial glycoprotein 40 (EPG-40), ephrinB2, ephrin receptor A2 (EPHa2), estrogen receptor, Fc receptor like 5 (FCRL5; also known as Fc receptor homolog 5 or FCRH5), fetal acetylcholine receptor (fetal AchR), a folate binding protein (FBP), folate receptor alpha, ganglioside GD2, O-acetylated GD2 (OGD2), ganglioside GD3, glycoprotein 100 (gp100), glypican-3 (GPC3), G Protein Coupled Receptor 5D (GPRC5D), Her2/neu (receptor tyrosine kinase erb-B2), Her3 (erb-B3), Her4 (erb-B4), erbB dimers, Human high molecular weight-melanoma-associated antigen (HMW-MAA), hepatitis B surface antigen, Human leukocyte antigen A1 (HLA-A1), Human leukocyte antigen A2 (HLA-A2), IL-22 receptor alpha(IL-22Rα), IL-13 receptor alpha 2 (IL-13Rα2), kinase insert domain receptor (kdr), kappa light chain, L1 cell adhesion molecule (L1-CAM), CE7 epitope of L1-CAM, Leucine Rich Repeat Containing 8 Family Member A (LRRC8A), Lewis Y, Melanoma-associated antigen (MAGE)-A1, MAGE-A3, MAGE-A6, MAGE-A10, mesothelin (MSLN), c-Met, murine cytomegalovirus (CMV), mucin 1 (MUC1), MUC16, natural killer group 2 member D (NKG2D) ligands, melan A (MART-1), neural cell adhesion molecule (NCAM), oncofetal antigen, Preferentially expressed antigen of melanoma (PRAME), progesterone receptor, a prostate specific antigen, prostate stem cell antigen (PSCA), prostate specific membrane antigen (PSMA), Receptor Tyrosine Kinase Like Orphan Receptor 1 (ROR1), survivin, Trophoblast glycoprotein (TPBG also known as 5T4), tumor-associated glycoprotein 72 (TAG72), Tyrosinase related protein 1 (TRP1, also known as TYRP1 or gp75), Tyrosinase related protein 2 (TRP2, also known as dopachrome tautomerase, dopachrome delta-isomerase or DCT), vascular endothelial growth factor receptor (VEGFR), vascular endothelial growth factor receptor 2 (VEGFR2), Wilms Tumor 1 (WT-1), a pathogen-specific or pathogen-expressed antigen, or an antigen associated with a universal tag, and/or biotinylated molecules, and/or molecules expressed by HIV, HCV, HBV or other pathogens. Antigens targeted by the receptors in some embodiments include antigens associated with a B cell malignancy, such as any of a number of known B cell marker. In some embodiments, the antigen is or includes CD20, CD19, CD22, ROR1, CD45, CD21, CD5, CD33, Igkappa, Iglambda, CD79a, CD79b or CD30.

In some embodiments, the binding molecule comprises a portion of a polypeptide antigen that is recognized by or bound by a recombinant receptor and/or a CAR. In particular embodiments, the binding molecule comprises an epitope that is recognized by or bound by a recombinant receptor and/or a CAR. In certain embodiments, the portion of the polypeptide antigen contains, about, or contains at least 10, 15, 20, 25, 30, 35, 40, 50, 55, 60, 65, 70, 75, 80, 85, 90, 95, 100, 110, 120, 130, 140, 150, 160, 170, 180, 190, 200, 250, 300, 400, or 500 amino acids, in some cases contiguous amino acids, of the polypeptide that is recognized by or bound by a recombinant receptor and or a CAR. In certain embodiments, the polypeptide portion comprises an amino acid sequence of the epitope that is recognized by the recombinant receptor and/or CAR.

In certain embodiments, the binding molecule is a polypeptide variant that contains, contains about, or contains at least 50%, 55%, 60%, 65%, 70%, 75%, 80%, 85%, 90%, 95%, 97%, 98%, 99%, or 99.5% amino acid sequence identity to a polypeptide that is bound by and/or recognized by recombinant receptor and/or CAR.

In certain embodiments, a particle is bound to a binding molecule comprising a portion of the polypeptide antigen, wherein the portion is a portion of an antigen that is bound by and/or recognized by, or can potentially be bound by or recognized by a recombinant receptor, i.e. a CAR. In some embodiments, the binding molecule comprises a portion of the antigen containing the epitope that is recognized by the recombinant receptor. In certain embodiments, the portion of the antigen is a portion of In some embodiments, the antigen is or includes αvβ6 integrin (avb6 integrin), B cell maturation antigen (BCMA), B7-H3, B7-H6, carbonic anhydrase 9 (CA9, also known as CAIX or G250), a cancer-testis antigen, cancer/testis antigen 1B (CTAG, also known as NY-ESO-1 and LAGE-2), carcinoembryonic antigen (CEA), a cyclin, cyclin A2, C-C Motif Chemokine Ligand 1 (CCL-1), CD19, CD20, CD22, CD23, CD24, CD30, CD33, CD38, CD44, CD44v6, CD44v7/8, CD123, CD133, CD138, CD171, chondroitin sulfate proteoglycan 4 (CSPG4), epidermal growth factor protein (EGFR), type III epidermal growth factor receptor mutation (EGFR vIII), epithelial glycoprotein 2 (EPG-2), epithelial glycoprotein 40 (EPG-40), ephrinB2, ephrin receptor A2 (EPHa2), estrogen receptor, Fc receptor like 5 (FCRL5; also known as Fc receptor homolog 5 or FCRH5), fetal acetylcholine receptor (fetal AchR), a folate binding protein (FBP), folate receptor alpha, ganglioside GD2, O-acetylated GD2 (OGD2), ganglioside GD3, glycoprotein 100 (gp100), glypican-3 (GPC3), G Protein Coupled Receptor 5D (GPRC5D), Her2/neu (receptor tyrosine kinase erb-B2), Her3 (erb-B3), Her4 (erb-B4), erbB dimers, Human high molecular weight-melanoma-associated antigen (HMW-MAA), hepatitis B surface antigen, Human leukocyte antigen A1 (HLA-A1), Human leukocyte antigen A2 (HLA-A2), IL-22 receptor alpha(IL-22Rα), IL-13 receptor alpha 2 (IL-13Rα2), kinase insert domain receptor (kdr), kappa light chain, L1 cell adhesion molecule (L1-CAM), CE7 epitope of L1-CAM, Leucine Rich Repeat Containing 8 Family Member A (LRRC8A), Lewis Y, Melanoma-associated antigen (MAGE)-A1, MAGE-A3, MAGE-A6, MAGE-A10, mesothelin (MSLN), c-Met, murine cytomegalovirus (CMV), mucin 1 (MUC1), MUC16, natural killer group 2 member D (NKG2D) ligands, melan A (MART-1), neural cell adhesion molecule (NCAM), oncofetal antigen, Preferentially expressed antigen of melanoma (PRAME), progesterone receptor, a prostate specific antigen, prostate stem cell antigen (PSCA), prostate specific membrane antigen (PSMA), Receptor Tyrosine Kinase Like Orphan Receptor 1 (ROR1), survivin, Trophoblast glycoprotein (TPBG also known as 5T4), tumor-associated glycoprotein 72 (TAG72), Tyrosinase related protein 1 (TRP1, also known as TYRP1 or gp75), Tyrosinase related protein 2 (TRP2, also known as dopachrome tautomerase, dopachrome delta-isomerase or DCT), vascular endothelial growth factor receptor (VEGFR), vascular endothelial growth factor receptor 2 (VEGFR2), Wilms Tumor 1 (WT-1), a pathogen-specific or pathogen-expressed antigen, or an antigen associated with a universal tag, and/or biotinylated molecules, and/or molecules expressed by HIV, HCV, HBV or other pathogens. Antigens targeted by the receptors in some embodiments include antigens associated with a B cell malignancy, such as any of a number of known B cell marker. In some embodiments, the antigen is or includes CD20, CD19, CD22, ROR1, CD45, CD21, CD5, CD33, Igkappa, Iglambda, CD79a, CD79b or CD30.

In certain embodiments, the particle is bound to a binding molecule comprising a portion of a BCMA polypeptide. In some embodiments, the particle is bound to a binding molecule comprising a portion of a CD22 polypeptide. In certain embodiments, the particle is bound to a binding molecule comprising a portion of a ROR1 polypeptide.

In certain embodiments, the portion of the polypeptide antigen is a portion of a BCMA polypeptide. In certain embodiments, the polypeptide antigen contains, contains about, or contains at least 50%, 55%, 60%, 65%, 70%, 75%, 80%, 85%, 90%, 95%, 97%, 98%, 99%, or 99.5% amino acid sequence identity to at least 10, at least 15, at least 20, at least 25, at least 30, at least 35, at least 40, at least 50, at least 55, at least 60, at least 65, at least 70, at least 75, at least 80, at least 85, at least 90, at least 95, at least 100, at least 110, at least 120, at least 130, at least 140, at least 150, at least 160, at least 170, or at least 180 contiguous amino acids of a BCMA polypeptide. In particular embodiments, the BCMA polypeptide variant comprises an amino acid sequence that is, is about, or is at least 70%, 75%, 80%, 85%, 90%, 95%, 97%, or 98% identical to the amino acid sequence of a BCMA epitope that is bound by and/or recognized by a recombinant receptor and/or a CAR.

In particular embodiments, the antigen is BCMA, or a portion or variant thereof. In some embodiments, the BCMA polypeptide is a mammalian BCMA polypeptide. In particular embodiments, the BCMA polypeptide is a human BCMA polypeptide. In some embodiments, the BCMA antigen is or comprises an extracellular domain of BCMA or a portion thereof comprising an epitope recognized by an antigen receptor, e.g. CAR. In certain embodiments, the BCMA antigen is or comprises a polypeptide with an amino acid sequence with at least 70%, 75%, 80%, 85%, 86%, 87%, 88%, 89%, 90%, 91%, 92%, 93%, 94%, 95%, 96%, 97%, 98%, 99% or more sequence identity to SEQ ID NO: 1 or a fragment thereof containing at least 50, at least 55, at least 60, at least 65, at least 70, at least 75, at least 80, at least 85, at least 90, at least 95, at least 100, at least 110, at least 120, at least 130, at least 140, at least 150, at least 160, at least 170, or at least 180 contiguous amino acids of SEQ ID NO: 1. In some embodiments, the BCMA antigen is or includes the sequence set forth in SEQ ID NO:1 or a portion thereof that is or contains an epitope recognized by an antigen receptor, e.g. CAR.

In some embodiments, the antigen is ROR1, or a portion or variant thereof. In certain embodiments, the ROR1 polypeptide is mammalian. In particular embodiments, the ROR1 polypeptide is human. In some embodiments, the ROR1 antigen is or comprises an extracellular domain of ROR1 or a portion thereof comprising an epitope recognized by an antigen receptor, e.g. CAR. In some embodiments, the ROR1 antigen is a polypeptide with an amino acid sequence with at least 70%, 75%, 80%, 85%, 86%, 87%, 88%, 89%, 90%, 91%, 92%, 93%, 94%, 95%, 96%, 97%, 98%, 99% or more sequence identity to SEQ ID NO: 31 or a fragment thereof containing at least 50, at least 55, at least 60, at least 65, at least 70, at least 75, at least 80, at least 85, at least 90, at least 95, at least 100, at least 110, at least 120, at least 130, at least 140, at least 150, at least 160, at least 170, or at least 180 contiguous amino acids of SEQ ID NO: 31. In some embodiments, the ROR antigen comprises the sequence set forth in SEQ ID NO :31 or a portion thereof comprising an epitope recognized by an antigen receptor, e.g. CAR.

In some embodiments, the antigen is CD22, or a portion or variant thereof. In certain embodiments, the CD22 polypeptide is mammalian. In particular embodiments, the CD22 polypeptide is human. In some embodiments, the antigen is an extracellular domain of CD22 or a portion thereof comprising an epitope recognized by an antigen receptor, e.g. CAR. In some embodiments, the antigen is a polypeptide with an amino acid sequence with at least 70%, 75%, 80%, 85%, 86%, 87%, 88%, 89%, 90%, 91%, 92%, 93%, 94%, 95%, 96%, 97%, 98%, 99% or more sequence identity to SEQ ID NO: 29 or a fragment thereof containing at least 50, at least 55, at least 60, at least 65, at least 70, at least 75, at least 80, at least 85, at least 90, at least 95, at least 100, at least 110, at least 120, at least 130, at least 140, at least 150, at least 160, at least 170, or at least 180 contiguous amino acids of SEQ ID NO: 29. In some embodiments, the CD22 antigen comprises the sequence set forth in SEQ ID NO :29 or a portion thereof comprising an epitope recognized by an antigen receptor, e.g. CAR.

In some embodiments, the portion of the polypeptide antigen is a portion of a BCMA polypeptide. In certain embodiments, the portion of the polypeptide antigen is a portion of a CD22 polypeptide. In particular embodiments, the portion of the polypeptide antigen is a portion of a ROR1 polypeptide. In certain embodiments, the portion of the polypeptide contains at least 10, at least 15, at least 20, at least 25, at least 30, at least 35, at least 40, at least 50, at least 55, at least 60, at least 65, at least 70, at least 75, at least 80, at least 85, at least 90, at least 95, at least 100, at least 110, at least 120, at least 130, at least 140, at least 150, at least 160, at least 170, or at least 180 contiguous amino acids of the amino acid sequence set forth in SEQ ID NOS: 1, 29, or 31.

In some embodiments, the cell expresses a CAR that binds to or recognizes a universal tag that can be fused to an antibody or a fragment or variant thereof. In particular embodiments, cells expressing such CARs are able to specifically recognize and kill target cells, for example tumor cells, that have been bound by antibodies that have been fused with the universal tag. One example includes, but is not limited to, anti-FITC CAR expressing T cells can bind to and/or recognize various human cancer cells when those cells are bound by cancer-reactive FITC-labeled antibodies. Thus, in some embodiments, the same CAR that binds to the universal Tag is useful for the treatment of different cancers, provided there are available antibodies that recognize antigens associated with the cancers that contain the universal tag. In particular embodiments, a particle (e.g., a bead particle) comprises a surface exposed binding molecule that comprises an antigen, or a portion thereof, that binds or is recognized by an antigen receptor, e.g. CAR, that binds to a universal tag. In certain embodiments, the binding molecule is a universal tag or a portion thereof bound or recognized by the antigen receptor, e.g. CAR.

Particular embodiments contemplate that any polypeptide domain that can be fused to an antibody, or an antigen binding fragment or variant thereof, that does not prevent the antibody from binding to its respective target is suitable for use as a universal tag. In some embodiments, a particle is bound to a binding molecule that comprises a universal tag, or a portion thereof, selected from the group consisting of: FITC, streptavidin, biotin, histidine, dinitrophenol, peridinin chlorophyll protein complex, green fluorescent protein, PE, HRP, palmitoylation, nitrosylation, alkalanine phosphatase, glucose oxidase, and maltose binding protein.

In some embodiments, the binding molecule is a multimer, e.g. a dimer, comprising two or more polypeptide antigens, or portion or variant thereof, that is recognized and/or bound by a recombinant receptor, such as an antigen receptor (e.g. a CAR). In some embodiments, the polypeptide antigen, or portion thereof, are identical. In certain embodiments, the polypeptide antigen is linked, directly or indirectly, to a region or domain, e.g. a multimerization domain, that promotes or stabilizes interaction between two or more polypeptide antigens via complementary interactions between the domains or regions. In some embodiments, providing the polypeptide antigen as a multimer, e.g. dimer, provides for a multivalent interaction between the binding molecule and the antigen-binding domain of the antigen receptor, e.g. CAR, which, in some aspects, can increase the avidity of the interaction. In some embodiment, an increased avidity may favor stimulatory or agonist activity of antigen receptor, e.g. CAR, by the binding molecule conjugated to the bead.

In some embodiments, a polypeptide is joined directly or indirectly to a multimerization domain. Exemplary multimerization domains include the immunoglobulin sequences or portions thereof, leucine zippers, hydrophobic regions, hydrophilic regions, and compatible protein-protein interaction domains. The multimerization domain, for example, can be an immunoglobulin constant region or domain, such as, for example, the Fc domain or portions thereof from IgG, including IgG1, IgG2, IgG3 or IgG4 subtypes, IgA, IgE, IgD and IgM and modified forms thereof. In particular embodiments, the polypeptide antigen is linked, directly or indirectly, to an Fc domain. In some embodiments, the polypeptide is a fusion polypeptide comprising the polypeptide antigen or portion thereof and the Fc domain.

In particular embodiments, a binding molecule is a fusion polypeptide that comprises an Fc domain. In some embodiments, the Fc domain is composed of the second and third constant domains *(i.e.,* CH2 and CH3 domains) of the heavy chain of a IgG, IgA or IgD isotype, e.g. CH2 or CH3 of IgG, IgA and IgD isotypes. In some embodiments, the Fc domain is composed of three heavy chain constant domain (i.e., CH2, CH3, and CH4 domains) of an IgM or IgE isotype. In some embodiments, the Fc domain may further include a hinge sequence or portion thereof. In certain aspects, the Fc domain contains part or all of a hinge domain of an immunoglobulin molecule plus a CH2 and a CH3 domain. In some cases, the Fc domain can form a dimer of two polypeptide chains joined by one or more disulfide bonds. In some embodiments, the Fc domain is derived from an immunoglobulin (e.g., IgG, IgA, IgM, or IgE) of a suitable mammal (e.g., human, mouse, rat, goat, sheep, or monkey). In some embodiments, the Fc domain comprises C_{H}2 and C_{H}3 domains of IgG. In certain embodiments, the Fc domain is fused to the C-terminal of the polypeptide antigen. In particular embodiments, the Fc domain is fused to the N-terminal of the polypeptide antigen.

In some embodiments, the Fc domain is an IgG Fc domain, or a portion or variant thereof. In some embodiments, the Fc domain is a human IgG Fc domain, or a portion or a variant thereof, that comprises an amino acid sequence set forth in SEQ ID NO: 2 or an amino acid sequence that is at least 70%, 75%, 80%, 85%, 86%, 87%, 88%, 89%, 90%, 91%, 92%, 93%, 94%, 95%, 96%, 97%, 98%, 99% sequence identity to the sequence set forth in SEQ ID NO: 2. In particualr embodiments, the Fc domain is a wild-type human IgG Fc domain, or a portion or variant thereof. In particular embodiments, the Fc domain is a variant of the wild-type human IgG1 Fc domain.

In some embodiments, the fusion polypeptide comprises a variant Fc domain. In certain embodiments, the variant human IgG Fc domain contains a mutation, e.g., a substitution, deletion, or insertion, that reduces, decreases, and/or diminishes pairing between the Fc domain and a light chain. In some embodiments, the variant human IgG Fc domain contains a mutation that reduces the binding affinity between the Fc domain and an Fc Receptor. In particular embodiments, the variant human IgG Fc domain contains a mutation that reduces, decreases, and/or diminishes the interactions, or the probabiltity or likelihood of an interaction, between the Fc domain and an Fc Receptor. In some embodiments, the variant human IgG Fc domain contains a mutation that reduces the binding affinity between the Fc domain and a protein of the complement system. In particular embodiments, the variant human IgG Fc domain contains a mutation that reduces, decreases, and/or diminishes the interactions, or the probabiltity or likelihood of an interaction, between the Fc domain and a protein of the complement system.

In some embodiments, the binding molecule comprises a variant human IgG1 Fc domain. In some embodiments, the variant human IgG Fc domain contains a cystine to serine substitution in the hinge region of the Fc domain. In some embodiments, the variant human IgG Fc domain contains a leucine to alanine substitution in the hinge region of the Fc domain. In particular embodiments, the variant human IgG Fc domain contains a glycine to alanine substitution in the hinge region. In certain embodiments, the variant human IgG Fc domain contains an alanine to a serine substitution in the CH2 region of the Fc domain. In some embodiments, the variant human IgG Fc domain comprises a proline to serine substitution in the CH2 region of the Fc domain. In some embodiments, the variant human IgG Fc domain comprises an amino acid sequence as set forth by SEQ ID NO: 28.

In some embodiments, the binding molecule comprises a fusion polypeptide comprising an Fc domain, wherein the Fc domain is present at the C-terminus of the fusion polypeptide.

In some embodiments, the binding molecule is a fusion polypeptide comprising a BCMA polypeptide, or a portion thereof, and an Fc domain. In some embodiments, the BCMA polypeptide or portion thereof comprises an amino acid sequence set forth in SEQ ID NO: 1 or a sequence of amino acids that exhibits at least 70%, 75%, 80%, 85%, 86%, 87%, 88%, 89%, 90%, 91%, 92%, 93%, 94%, 95%, 96%, 97%, 98%, 99% to SEQ ID NO:1 and contains an epitope recognized by the antigen receptor, e.g. the CAR. In some embodiments, the Fc domain comprises an amino acid sequence set forth in SEQ ID NO: 2 or a sequence of amino acids that exhibits at least 70%, 75%, 80%, 85%, 86%, 87%, 88%, 89%, 90%, 91%, 92%, 93%, 94%, 95%, 96%, 97%, 98%, 99% sequence identity to SEQ ID NO:2. In some embodiments, the Fc domain comprises an amino acid sequence set forth in SEQ ID NO: 28 or a sequence of amino acids that exhibits at least 70%, 75%, 80%, 85%, 86%, 87%, 88%, 89%, 90%, 91%, 92%, 93%, 94%, 95%, 96%, 97%, 98%, 99% to SEQ ID NO:28. In some embodiments, the BCMA antigen includes BCMA, or a portion or variant thereof, and a tag or a fusion domain, e.g., an Fc domain. In particular embodiments, the BCMA antigen contains all or a portion of the amino acid sequence set forth in SEQ ID NO: 35 or a sequence of amino acids that exhibits at least 70%, 75%, 80%, 85%, 86%, 87%, 88%, 89%, 90%, 91%, 92%, 93%, 94%, 95%, 96%, 97%, 98%, 99% to SEQ ID NO:35, and that comprises an epitope recognize by an antigen receptor, e.g. CAR.

In some embodiments, the binding molecule is a fusion polypeptide comprising a ROR1 polypeptide or portion thereof and an Fc domain. In some embodiments, the ROR1 polypeptide or portion thereof comprises an amino acid sequence set forth in SEQ ID NO: 31 or a sequence of amino acids that exhibits at least 70%, 75%, 80%, 85%, 86%, 87%, 88%, 89%, 90%, 91%, 92%, 93%, 94%, 95%, 96%, 97%, 98%, 99% to SEQ ID NO:31 and comprising an epitope recognized by the antigen receptor, e.g. CAR, and an Fc domain. In some embodiments, the Fc domain comprises an amino acid sequence set forth in SEQ ID NO: 2 or a sequence of amino acids that exhibits at least 70%, 75%, 80%, 85%, 86%, 87%, 88%, 89%, 90%, 91%, 92%, 93%, 94%, 95%, 96%, 97%, 98%, 99% sequence identity to SEQ ID NO:2. In some embodiments the Fc domain comprises an amino acid sequence set forth in SEQ ID NO: 28 or a sequence of amino acids that exhibits at least 70%, 75%, 80%, 85%, 86%, 87%, 88%, 89%, 90%, 91%, 92%, 93%, 94%, 95%, 96%, 97%, 98%, 99% to SEQ ID NO:28. In certain embodiments, the binding molecule is a fusion polypeptide comprising an amino acid sequence set forth in SEQ ID NO: 33 or a sequence of amino acids that exhibits at least 70%, 75%, 80%, 85%, 86%, 87%, 88%, 89%, 90%, 91%, 92%, 93%, 94%, 95%, 96%, 97%, 98%, 99% to SEQ ID NO:33 and that comprises an epitope recognize by an antigen receptor, e.g. CAR.

In particular embodiments, the binding molecule is a fusion polypeptide comprising a CD22 polypeptide set forth in SEQ ID NO:29 or portion thereof or a sequence of amino acids that exhibits at least 70%, 75%, 80%, 85%, 86%, 87%, 88%, 89%, 90%, 91%, 92%, 93%, 94%, 95%, 96%, 97%, 98%, 99% to SEQ ID NO:29 and comprising an epitope recognized by the antigen receptor, e.g. CAR, and an Fc domain. In some embodiments, the Fc domain comprises an amino acid sequence set forth in SEQ ID NO: 2 or a sequence of amino acids that exhibits at least 70%, 75%, 80%, 85%, 86%, 87%, 88%, 89%, 90%, 91%, 92%, 93%, 94%, 95%, 96%, 97%, 98%, 99% sequence identity to SEQ ID NO:2. In some embodiments the Fc domain comprises an amino acid sequence set forth in SEQ ID NO: 28 or a sequence of amino acids that exhibits at least 70%, 75%, 80%, 85%, 86%, 87%, 88%, 89%, 90%, 91%, 92%, 93%, 94%, 95%, 96%, 97%, 98%, 99% to SEQ ID NO:28. In certain embodiments, the fusion polypeptide comprises an amino acid sequence set forth in SEQ ID NO: 34 or a sequence of amino acids that exhibits at least 70%, 75%, 80%, 85%, 86%, 87%, 88%, 89%, 90%, 91%, 92%, 93%, 94%, 95%, 96%, 97%, 98%, 99% to SEQ ID NO:34 and that comprises an epitope recognize by an antigen receptor, e.g. CAR.

In some embodiments, the antigen and the multimerization domain, such as Fc domain, are connected by a linker, such as an amino acid linker. In certain embodiments, the antigen is fused to the N-terminus of an amino acid linker, and the multimerization domain, such as Fc domain, is fused to the C-terminus of the linker. Although amino acid linkers can be any length and contain any combination of amino acids, the linker length may be relatively short (e.g., ten or fewer amino acids) to reduce interactions between the linked domains. The amino acid composition of the linker also may be adjusted to reduce the number of amino acids with bulky side chains or amino acids likely to introduce secondary structure. Suitable amino acid linkers include, but are not limited to, those up to 3, 4, 5, 6, 7, 10, 15, 20, or 25 amino acids in length. Representative amino acid linker sequences include GGGGS (SEQ ID NO: 27), and linkers comprising 2, 3, 4, or 5 copies of GGGGS (SEQ ID NO: 27).

In some embodiments, the binding molecule is a dimer formed by two Fc fusion polypeptides containing a polypeptide antigen or portion thereof an Fc domain, e.g. BCMA-Fc, ROR1-Fc or CD22-Fc. Also provided are nucleic acid molecules encoding any of the Fc fusion polypeptides. Also provided are vectors, including expression vectors, encoding the nucleic acid molecules. In some embodiments, the binding molecule can be produced in cells by expression in a suitable host cell. In some embodiments, the host cell is a mammalian cell line. Exemplary of mammalian cells for recombinant expression of proteins include HEK293 cells or CHO cells or derivatives thereof. In some aspects, the binding molecule further includes a signal peptide for secretion from the cell. In an exemplary embodiment, the signal peptide is CD33 (e.g. set forth in SEQ ID NO: 30). In some embodiments, the resulting polypeptide antigen-Fc fusion protein, e.g. BCMA-Fc, ROR1-Fc or CD22-Fc, can be expressed in host cells, e.g. transformed with the expression vectors, whereby assembly between Fc domains can occurs by interchain disulfide bonds formed between the Fc moieties to yield a dimeric, such as divalent, polypeptide antigen fusion protein.

In particular embodiments, the binding molecule is or contains human BCMA or a portion thereof. In some embodiments, the binding molecule comprises the extracellular domain of human BCMA. In particular embodiments, the binding molecule comprises a portion of human BCMA with a polypeptide sequence set forth in SEQ ID NO: 1. In particular embodiments, the binding molecule is a fusion polypeptide that comprises an extracellular domain of human BCMA and an Fc domain. In certain embodiments, the Fc domain is a variant of a human IgG1 Fc domain. In certain embodiments, the Fc domain is positioned at the C-terminus of the fusion polypeptide. In particular embodiments, the fusion polypeptide comprises a portion of human BCMA comprising an amino acid sequence set forth in SEQ ID NO: 1 and a variant human IgG1 Fc domain comprising an amino acid set forth in SEQ ID NO: 29. In some embodiments, fusion polypeptide comprises a linker that joins the BCMA polypeptide to the Fc domain. In particular embodiments, the linker has an amino acid sequence set forth in SEQ ID NO: 27. In certain embodiments, the binding molecule is a fusion polypeptide comprising a human BCMA polypeptide (or a portion or a variant thereof) and a C-terminal Fc domain and is surface conjugated or otherwise attached to a particle (e.g., bead particle). In particular embodiments, the fusion polypeptide is attached to the particle at a site within in the Fc domain. In some embodiments, the particle further comprises a surface conjugated antibody or a fragment or variant thereof, that binds to or recognizes CD28.

### 2. Anti-Idiotype Antibodies

In some embodiments, the binding molecule conjugated to a particle, e.g., bead, as provided herein is an anti-idiotype antibody, or an antigen binding fragment thereof, ("anti-ID") that binds to a target antigen receptor, e.g. CAR, or an active fragment thereof. In certain embodiments, the biding molecule is an anti-ID that binds to the antigen binding domain of the CAR. In particular embodiments, the antigen binding domain of the CAR comprises an scFv domain. In some embodiments, the binding molecule is an anti-ID that binds to a CAR at the antigen binding domain, and does not bind to the linker or spacer region of the CAR that connects the antigen binding domain, e.g., an scFv, with a transmembrane domain of the CAR.

In some embodiments, the anti-ID is an anti-idiotype antibody or antigen-binding fragments that specifically recognizes an antibody that binds to a target antigen, such as a target antigen associated with or expressed on a cell or tissue of a disease or condition, e.g. cancer. In some embodiments, the anti-ID is an anti-idiotype antibody or antigen-binding fragment thereof that specifically recognizes a target antibody that binds the antigen that is or includes In some embodiments, the antigen is or includes αvβ6 integrin (avb6 integrin), B cell maturation antigen (BCMA), B7-H3, B7-H6, carbonic anhydrase 9 (CA9, also known as CAIX or G250), a cancer-testis antigen, cancer/testis antigen 1B (CTAG, also known as NY-ESO-1 and LAGE-2), carcinoembryonic antigen (CEA), a cyclin, cyclin A2, C-C Motif Chemokine Ligand 1 (CCL-1), CD19, CD20, CD22, CD23, CD24, CD30, CD33, CD38, CD44, CD44v6, CD44v7/8, CD123, CD133, CD138, CD171, chondroitin sulfate proteoglycan 4 (CSPG4), epidermal growth factor protein (EGFR), type III epidermal growth factor receptor mutation (EGFR vIII), epithelial glycoprotein 2 (EPG-2), epithelial glycoprotein 40 (EPG-40), ephrinB2, ephrin receptor A2 (EPHa2), estrogen receptor, Fc receptor like 5 (FCRL5; also known as Fc receptor homolog 5 or FCRH5), fetal acetylcholine receptor (fetal AchR), a folate binding protein (FBP), folate receptor alpha, ganglioside GD2, O-acetylated GD2 (OGD2), ganglioside GD3, glycoprotein 100 (gp100), glypican-3 (GPC3), G Protein Coupled Receptor 5D (GPRC5D), Her2/neu (receptor tyrosine kinase erb-B2), Her3 (erb-B3), Her4 (erb-B4), erbB dimers, Human high molecular weight-melanoma-associated antigen (HMW-MAA), hepatitis B surface antigen, Human leukocyte antigen A1 (HLA-A1), Human leukocyte antigen A2 (HLA-A2), IL-22 receptor alpha(IL-22Rα), IL-13 receptor alpha 2 (IL-13Rα2), kinase insert domain receptor (kdr), kappa light chain, L1 cell adhesion molecule (L1-CAM), CE7 epitope of L 1-CAM, Leucine Rich Repeat Containing 8 Family Member A (LRRC8A), Lewis Y, Melanoma-associated antigen (MAGE)-A1, MAGE-A3, MAGE-A6, MAGE-A10, mesothelin (MSLN), c-Met, murine cytomegalovirus (CMV), mucin 1 (MUC1), MUC16, natural killer group 2 member D (NKG2D) ligands, melan A (MART-1), neural cell adhesion molecule (NCAM), oncofetal antigen, Preferentially expressed antigen of melanoma (PRAME), progesterone receptor, a prostate specific antigen, prostate stem cell antigen (PSCA), prostate specific membrane antigen (PSMA), Receptor Tyrosine Kinase Like Orphan Receptor 1 (ROR1), survivin, Trophoblast glycoprotein (TPBG also known as 5T4), tumor-associated glycoprotein 72 (TAG72), Tyrosinase related protein 1 (TRP1, also known as TYRP1 or gp75), Tyrosinase related protein 2 (TRP2, also known as dopachrome tautomerase, dopachrome delta-isomerase or DCT), vascular endothelial growth factor receptor (VEGFR), vascular endothelial growth factor receptor 2 (VEGFR2), Wilms Tumor 1 (WT-1), a pathogen-specific or pathogen-expressed antigen, or an antigen associated with a universal tag, and/or biotinylated molecules, and/or molecules expressed by HIV, HCV, HBV or other pathogens. Antigens targeted by the receptors in some embodiments include antigens associated with a B cell malignancy, such as any of a number of known B cell marker. In some embodiments, the antigen is or includes CD20, CD19, CD22, ROR1, CD45, CD21, CD5, CD33, Igkappa, Iglambda, CD79a, CD79b or CD30.

In some embodiments, the anti-ID is an anti-idiotype antibody or antigen-binding fragments that specifically recognizes a target antibody that binds ROR1, B cell maturation antigen (BCMA), carbonic anhydrase 9 (CAIX), Her2/neu (receptor tyrosine kinase erbB2), Ll-CAM, CD19, CD20, CD22, mesothelin, CEA, and hepatitis B surface antigen, anti-folate receptor, CD23, CD24, CD30, CD33, CD38, CD44, EGFR, epithelial glycoprotein 2 (EPG-2), epithelial glycoprotein 40 (EPG-40), EPHa2, erb-B2, erb-B3, erb-B4, erbB dimers, EGFR vIII, folate binding protein (FBP), FCRL5, FCRH5, fetal acetylcholine receptor, GD2, GD3, HMW-MAA, IL-22R-alpha, IL-13R-alpha2, kinase insert domain receptor (kdr), kappa light chain, Lewis Y, L1-cell adhesion molecule, (L1-CAM), Melanoma-associated antigen (MAGE)-A1, MAGE-A3, MAGE-A6, Preferentially expressed antigen of melanoma (PRAME), survivin, TAG72, B7-H6, IL-13 receptor alpha 2 (IL-13Ra2), CA9, GD3, HMW-MAA, CD171, G250/CAIX, HLA-AI MAGE Al, HLA-A2 NY-ESO-1, PSCA, folate receptor-a, CD44v6, CD44v7/8, avb6 integrin, 8H9, NCAM, VEGF receptors, 5T4, Foetal AchR, NKG2D ligands, CD44v6, dual antigen, a cancer-testes antigen, mesothelin, murine CMV, mucin 1 (MUC1), MUC16, PSCA, NKG2D, NY-ESO-1, MART-1, gp100, oncofetal antigen, ROR1, TAG72, VEGF-R2, carcinoembryonic antigen (CEA), prostate specific antigen, PSMA, Her2/neu, estrogen receptor, progesterone receptor, ephrinB2, CD123, c-Met, GD-2, O-acetylated GD2 (OGD2), CE7, Wilms Tumor 1 (WT-1), a cyclin, cyclin A2, CCL-1, CD138, or a pathogen-specific antigen.

In some embodiments, the anti-ID is an anti-idiotype antibody or antigen-binding fragments ("anti-IDs") that specifically recognizes a target anti-CD19 antibody moiety. In some embodiments, the provided antibodies recognize a target anti-CD19 antibody that is SJ25C1 or an antigen-binding fragment thereof or is an antibody or antigen-binding fragment derived from SJ25C1. In some embodiments, the provided antibodies recognize a target anti-CD19 antibody that is FMC63 or an antigen-binding fragment thereof is an antibody or antigen-binding fragment derived from FMC63.

SJ25C1 is a mouse monoclonal IgG1 antibody raised against Nalm-1 and -16 cells expressing CD19 of human origin (Ling, N. R., et al. (1987). Leucocyte typing III. 302). The SJ25C1 antibody comprises CDRH1, H2 and H3 set forth in SEQ ID NOS: 40-42, respectively, and CDRL1, L2 and L3 sequences set forth in SEQ ID NOS: 43-45, respectively. The SJ25C1 antibody comprises the heavy chain variable region (V_{H}) comprising the amino acid sequence of SEQ ID NO: 36 and the light chain variable region (V_{L}) comprising the amino acid sequence of SEQ ID NO: 37.

In some embodiments, the target antibody is SJ25C1 or an antibody-derived from SJ25C1.In some embodiments, the antibody derived from SJ25C1"is an antibody or antigen-binding fragment that comprises the V_{H} and/or V_{L} of SJ25C1, the idiotype of SJ25C1, the paratope of SJ25C1, or one or more complementarity determining regions (CDRs) of SJ25C1. In some embodiments, the target antibody that is SJ25C1 or an antibody-derived from SJ25C1 is an antibody or antigen-binding fragment comprises the V_{H} of SJ25C1 set forth in SEQ ID NO: 36, or a variant thereof having at least 90% sequence identity set forth in SEQ ID NO:36, and/or the V_{L} of SJ25C1 set forth in SEQ ID NO:37, or a variant thereof having at least 90% sequence identity to SEQ ID NO:37. In some embodiments, the antibody or antigen-binding fragment comprises the V_{H} of SJ25C1 set forth in SEQ ID NO:36, or a variant thereof having at least 90% sequence identity to SEQ ID NO:36, and the V_{L} of SJ25C1 set forth in SEQ ID NO:37, or a variant thereof having at least 90% sequence identity to SEQ ID NO:37. In some embodiments, the antibody or antigen-binding fragment comprises the V_{H} and V_{L} of SJ25C1 set forth in SEQ ID NO:36 and SEQ ID NO:37, respectively. In some embodiments, the variant has at least 91%, 92%, 93%, 94%, 95%, 96%, 97%, 98%, 99% or more sequence identity to SEQ ID NO:36 and/or SEQ ID NO:37.

In some embodiments, the target antibody that is SJ25C1 or an antibody-derived from SJ25C1 is an antibody or antigen-binding fragment comprises one or more heavy chain CDRs (CDR-H) of SJ25C1 V_{H} set forth in SEQ ID NO:36, such as set forth in SEQ ID NOS: 40-42 and/or one or more light chain CDRs (CDR-Ls) of SJ25C1 V_{L} set forth in SEQ ID NO:37, such as set forth in SEQ ID NOS: 43-45. In some embodiments, the antibody or antigen-binding fragment comprises CDR-H3 of SJ25C1 (e.g. set forth in SEQ ID NO: 42) and/or CDR-L3 of SJ25C1 (e.g. set forth in SEQ ID NO: 45). In some embodiments, the antibody or antigen-binding fragment comprises CDR-H3 and CDR-L3 of SJ25C1 (e.g. set forth in SEQ ID NO:42 and SEQ ID NO:43, respectively). In some embodiments, the antibody or antigen-binding fragment comprises one or more of CDR-H1, CDR-H2, and CDR-H3 of SJ25C1 (e.g. set forth in SEQ ID NOS: 40, 41, 42, respectively) and/or one or more of CDR-L1, CDR-L2, and CDR-L3 of SJ25C1 (e.g. set forth in SEQ ID NOS: 43, 44, 45, respectively). In some embodiments, the antibody or antigen-binding fragment comprises CDR-H1, CDR-H2, and CDR-H3 of SJ25C1 (e.g. set forth in SEQ ID NOS: 40, 41, 42, respectively) and/or CDR-L1, CDR-L2, and CDR-L3 of SJ25C1 (e.g. set forth in SEQ ID NOS: 43, 44, 45, respectively). In some embodiments, the antibody or antigen-binding fragment comprises CDR-H1, CDR-H2, and CDR-H3 of SJ25C1 (e.g. set forth in SEQ ID NOS: 40, 41, 42, respectively) and CDR-L1, CDR-L2, and CDR-L3 of SJ25C1 (e.g. set forth in SEQ ID NOS: 43, 44, 45, respectively). In some embodiments, the antibody or antigen-binding fragment comprises an antigen-binding fragment, such as a fragment antigen-binding (Fab), a F(ab')2, a Fab', a fragment variable (Fv), or a single chain Fv (scFv). *See* for example Bejcek, B. E., et al. (1995). Cancer research. 55(11): 2346-2351.

FMC63 is a mouse monoclonal IgG1 antibody raised against JVM3 cells expressing CD19 of human origin (Nicholson., et al. (1997). Molecular Immunology. 34(16-17):1157-1165). The FMC63 antibody comprises CDRH1, H2 and H3 set forth in SEQ ID NOS: 46-48, respectively, and CDRLI1, L2 and L3 sequences set forth in SEQ ID NOS: 49-51, respectively. The FMC63 antibody comprises the heavy chain variable region (V_{H}) comprising the amino acid sequence of SEQ ID NO: 38 and the light chain variable region (V_{L}) comprising the amino acid sequence of SEQ ID NO: 39.

In some embodiments, the target antibody is FMC63 or an antibody-derived from FMC63. In some embodiments, the antibody derived from FMC63, is an antibody or antigen-binding fragment that comprises the V_{H} and/or V_{L} of FMC63, the idiotype of FMC63, the paratope of FMC63, or one or more complementarity determining regions (CDRs) of FMC63. In some embodiments, the target antibody that is FMC63 or an antibody-derived from FMC63 is an antibody or antigen-binding fragment comprises the V_{H} of FMC63 set forth in SEQ ID NO:38, or a variant thereof having at least 90% sequence identity to SEQ ID NO:38, and/or the V_{L} of FMC63 set forth in SEQ ID NO:39, or a variant thereof having at least 90% sequence identity to SEQ ID NO:39. In some embodiments, the antibody or antigen-binding fragment comprises the V_{H} of FMC63 set forth in SEQ ID NO:38, or a variant thereof having at least 90% sequence identity to SEQ ID NO:38, and the V_{L} of FMC63 set forth in SEQ ID NO:39, or a variant thereof having at least 90% sequence identity to SEQ ID NO:39. In some embodiments, the antibody or antigen-binding fragment comprises the V_{H} and V_{L} of FMC63 set forth in SEQ ID NO:38 and 39, respectively. In some embodiments, the variant has at least 91%, 92%, 93%, 94%, 95%, 96%, 97%, 98%, 99% or more sequence identity to SEQ ID NO:38 and/or SEQ ID NO:39

In some embodiments, the target antibody that is FMC63 or an antibody-derived from FMC63 is an antibody or antigen-binding fragment comprises one or more heavy chain CDRs (CDR-H) of FMC63 V_{H} set forth in SEQ ID NO:38, such as set forth in SEQ ID NOS: 46-48 and/or one or more light chain CDRs (CDR-Ls) of FMC63V_{L} set forth in SEQ ID NO:39, such as set forth in SEQ ID NOS: 49-51. In some embodiments, the target antibody that is FMC63 or an antibody-derived from FMC63 is an antibody or antigen-binding fragment comprises CDR-H3 of FMC63 (e.g. set forth in SEQ ID NO: 48) and/or CDR-L3 of FMC63 (e.g. set forth in SEQ ID NO: 51). In some embodiments, the antibody or antigen-binding fragment comprises CDR-H3 (e.g. set forth in SEQ ID NO: 48) and CDR-L3 of FMC63 (e.g. set forth in SEQ ID NO: 51). In some embodiments, the target antibody that is FMC63 or an antibody-derived from FMC63 is an antibody or antigen-binding fragment comprises one or more of CDR-H1, CDR-H2, and CDR-H3 of FMC63 (e.g. set forth in SEQ ID NOS: 46, 47, 48, respectively) and/or one or more of CDR-L1, CDR-L2, and CDR-L3 of FMC63 (e.g. set forth in SEQ ID NOS: 49, 50, 51, respectively). In some embodiments, the target antibody that is FMC63 or an antibody-derived from FMC63 is an antibody or antigen-binding fragment comprises CDR-H1, CDR-H2, and CDR-H3 of FMC63 (e.g. set forth in SEQ ID NOS: 46, 47, 48, respectively) and/or CDR-L1, CDR-L2, and CDR-L3 of FMC63 (e.g. set forth in SEQ ID NOS: 49, 50, 51, respectively). In some embodiments, the target antibody that is FMC63 or an antibody-derived from FMC63 is an antibody or antigen-binding fragment comprises CDR-H1, CDR-H2, and CDR-H3 of FMC63 (e.g. set forth in SEQ ID NOS: 46, 47, 48, respectively) and CDR-L1, CDR-L2, and CDR-L3 of FMC63 (e.g. set forth in SEQ ID NOS: 49, 50, 51, respectively). In some embodiments, the antibody or antigen-binding fragment comprises an antigen-binding fragment, such as a fragment antigen-binding (Fab), a F(ab')2, a Fab', a fragment variable (Fv), or a single chain Fv (scFv).

In some embodiments, the provided anti-idiotype antibodies include antibodies that specifically bind to a variable domain (Fv), such as a single chain Fv (scFv), derived from SJ25C1 or FMC63. In some embodiments, the anti-idiotype antibodies specifically bind to a particular epitope or region of an Fv, generally an epitope or region comprising one or more complementarity determining regions. In some embodiments, the anti-idiotype antibodies specifically bind to an epitope or region overlapping an Fv paratope.

In some embodiments, the provided anti-idiotype antibodies include those that specifically bind to an anti-CD19 moiety derived from SJ25C1 or FMC63 that is contained as part of the extracellular domain of a target chimeric antigen receptor (CAR). In some embodiments, the target CAR contains an antigen-binding portion that contains the SJ25C1 or FMC63 antibody molecule or antigen-binding fragment or portion of the SJ25C1 or FMC63 antibody. In some embodiments, the target CAR includes an antigen-binding domain that is an scFv derived from the VH and VL chains of the antibody SJ25C1 or FMC63. In some embodiments, there is provided an anti-idiotype antibody that specifically binds to an anti-CD19 CAR that contains an scFv derived from antibody SJ25C1 or FMC63. Exemplary features of CARs are described further below.

The term "antibody" herein is used in the broadest sense and includes polyclonal and monoclonal antibodies, including intact antibodies and functional (antigen-binding) antibody fragments, including fragment antigen binding (Fab) fragments, F(ab')₂ fragments, Fab' fragments, Fv fragments, recombinant IgG (rIgG) fragments, single chain antibody fragments, including single chain variable fragments (scFv), and single domain antibodies (e.g., sdAb, sdFv, nanobody) fragments. The term encompasses genetically engineered and/or otherwise modified forms of immunoglobulins, such as intrabodies, peptibodies, chimeric antibodies, fully human antibodies, humanized antibodies, and heteroconjugate antibodies, multispecific, e.g., bispecific, antibodies, diabodies, triabodies, and tetrabodies, tandem di-scFv, tandem tri-scFv. Unless otherwise stated, the term "antibody" should be understood to encompass functional antibody fragments thereof. The term also encompasses intact or full-length antibodies, including antibodies of any class or sub-class, including IgG and sub-classes thereof, IgM, IgE, IgA, and IgD.

The term "anti-idiotype antibody" refers to an antibody, including antigen-binding fragments thereof, that specifically recognizes, is specifically targeted to, and/or specifically binds to an idiotope of an antibody, such as an antigen-binding fragment. The idiotopes of an antibody may include, but are not necessarily limited to, residues within one or more of complementarity determining region(s) (CDRs) of the antibody, variable regions of the antibody, and/or partial portions or portions of such variable regions and/or of such CDRs, and/or any combination of the foregoing. The CDR may be one or more selected from the group consisting of CDR-H1, CDR-H2, CDR-H3, CDR-L1, CDR-L2, and CDR-L3. The variable regions of the antibody may be heavy chain variable regions, light chain variable regions, or a combination of the heavy chain variable regions and the light chain variable regions. The partial fragments or portions of the heavy chain variable regions and/or the light chain variable regions of the antibody may be fragments including 2 or more, 5 or more, or 10 or more contiguous amino acids, for example, from or from about 2 to 100, 5 to 100, 10 to 100, 2 to 50, 5 to 50, or 10 to 50 contiguous amino acids within the heavy chain variable regions or the light chain variable regions of the antibody; the idiotope may include multiple non-contiguous stretches of amino acids. The partial fragments of the heavy chain variable regions and the light chain variable regions of the antibody may be fragments including 2 or more, 5 or more, or 10 or more contiguous amino acids, for example, from or from 2 to 00, 5 to 100, 10 to 100, 2 to 50, 5 to 50, or 10 to 50 contiguous amino acids within the variable regions, and in some embodiments contain one or more CDRs or CDR fragments. The CDR fragments may be consecutive or nonconsecutive 2 or more, or 5 or more amino acids within the CDR. Therefore, the idiotopes of the antibody may be from or from about 2 to 100, 5 to 100, 10 to 100, 2 to 50, 5 to 50, or 10 to 50 contiguous amino acids containing one or more CDR or one or more CDR fragments within the heavy chain variable regions or the light chain variable regions of the antibody. In another embodiment, the idiotopes may be a single amino acid which is located at the variable regions of the antibody, for example, CDR sites.

In some embodiments, the idiotope is any single antigenic determinant or epitope within the variable portion of an antibody. In some cases it can overlap the actual antigen-binding site of the antibody, and in some cases it may comprise variable region sequences outside of the antigen-binding site of the antibody. The set of individual idiotopes of an antibody is in some embodiments referred to as the "idiotype" of such antibody.

The terms "complementarity determining region," and "CDR," synonymous with "hypervariable region" or "HVR," are known in the art to refer to non-contiguous sequences of amino acids within antibody variable regions, which confer antigen specificity and/or binding affinity. In general, there are three CDRs in each heavy chain variable region (CDR-H1, CDR-H2, CDR-H3) and three CDRs in each light chain variable region (CDR-L1, CDR-L2, CDR-L3). "Framework regions" and "FR" are known in the art to refer to the non-CDR portions of the variable regions of the heavy and light chains. In general, there are four FRs in each full-length heavy chain variable region (FR-H1, FR-H2, FR-H3, and FR-H4), and four FRs in each full-length light chain variable region (FR-L1, FR-L2, FR-L3, and FR-L4).

The precise amino acid sequence boundaries of a given CDR or FR can be readily determined using any of a number of well-known schemes, including those described by Kabat et al. (1991), "Sequences of Proteins of Immunological Interest," 5th Ed. Public Health Service, National Institutes of Health, Bethesda, MD ("Kabat" numbering scheme), Al-Lazikani et al., (1997) JMB 273,927-948 ("Chothia" numbering scheme), MacCallum et al., J. Mol. Biol. 262: 732-745 (1996), "Antibody-antigen interactions: Contact analysis and binding site topography," J. Mol. Biol. 262, 732-745." ("Contact" numbering scheme), Lefranc MP et al., "IMGT unique numbering for immunoglobulin and T cell receptor variable domains and Ig superfamily V-like domains," Dev Comp Immunol, 2003 Jan;27(1): 55-77 ("IMGT" numbering scheme), and Honegger A and Plückthun A, "Yet another numbering scheme for immunoglobulin variable domains: an automatic modeling and analysis tool," J Mol Biol, 2001 Jun 8;309(3): 657-70, ("Aho" numbering scheme).

The boundaries of a given CDR or FR may vary depending on the scheme used for identification. For example, the Kabat scheme is based structural alignments, while the Chothia scheme is based on structural information. Numbering for both the Kabat and Chothia schemes is based upon the most common antibody region sequence lengths, with insertions accommodated by insertion letters, for example, "30a," and deletions appearing in some antibodies. The two schemes place certain insertions and deletions ("indels") at different positions, resulting in differential numbering. The Contact scheme is based on analysis of complex crystal structures and is similar in many respects to the Chothia numbering scheme.

Table 1, below, lists exemplary position boundaries of CDR-L1, CDR-L2, CDR-L3 and CDR-H1, CDR-H2, CDR-H3 as identified by Kabat, Chothia, and Contact schemes, respectively. For CDR-H1, residue numbering is listed using both the Kabat and Chothia numbering schemes. FRs are located between CDRs, for example, with FR-L1 located between CDR-L1 and CDR-L2, and so forth. It is noted that because the shown Kabat numbering scheme places insertions at H35A and H35B, the end of the Chothia CDR-H1 loop when numbered using the shown Kabat numbering convention varies between H32 and H34, depending on the length of the loop.

**Table 1**

| **CDR** | **Kabat** | **Chothia** | **Contact** |
|---|---|---|---|
| CDR-L1 | L24--L34 | L24--L34 | L30--L36 |
| CDR-L2 | L50--L56 | L50--L56 | L46--L55 |
| CDR-L3 | L89--L97 | L89--L97 | L89--L96 |
| CDR-H1 (Kabat Numbering¹) | H31--H35B | H26--H32..34 | H30--H35B |
| CDR-H1 (Chothia Numbering²) | H31--H35 | H26--H32 | H30--H35 |
| CDR-H2 | H50--H65 | H52--H56 | H47--H58 |
| CDR-H3 | H95--H102 | H95--H102 | H93--H101 |

| | | | |
|---|---|---|---|
| 1 - Kabat et al. (1991), "Sequences of Proteins of Immunological Interest," 5th Ed. Public Health Service, National Institutes of Health, Bethesda, MD 2 - Al-Lazikani et al., (1997) JMB 273,927-948 | | | |

Thus, unless otherwise specified, a "CDR" or "complementary determining region," or individual specified CDRs (e.g., "CDR-H1, CDR-H2), of a given antibody or region thereof, such as a variable region thereof, should be understood to encompass a (or the specific) complementary determining region as defined by any of the aforementioned schemes. For example, where it is stated that a particular CDR (e.g., a CDR-H3) contains the amino acid sequence of a corresponding CDR in a given V_{H} or V_{L} amino acid sequence, it is understood that such a CDR has a sequence of the corresponding CDR (e.g., CDR-H3) within the variable region, as defined by any of the aforementioned schemes. In some embodiments, specified CDR sequences are specified.

Likewise, unless otherwise specified, a FR or individual specified FR(s) (e.g., FR-H1, FR-H2), of a given antibody or region thereof, such as a variable region thereof, should be understood to encompass a (or the specific) framework region as defined by any of the known schemes. In some instances, the scheme for identification of a particular CDR, FR, or FRs or CDRs is specified, such as the CDR as defined by the Kabat, Chothia, or Contact method. In other cases, the particular amino acid sequence of a CDR or FR is given.

The term "variable region" or "variable domain" refers to the domain of an antibody heavy or light chain that is involved in binding the antibody to antigen. The variable domains of the heavy chain and light chain (V_{H} and V_{L}, respectively) of a native antibody generally have similar structures, with each domain comprising four conserved framework regions (FRs) and three CDRs. (See, e.g., Kindt et al. Kuby Immunology, 6th ed., W.H. Freeman and Co., page 91 (2007). A single V_{H} or V_{L} domain may be sufficient to confer antigen-binding specificity. Furthermore, antibodies that bind a particular antigen may be isolated using a V_{H} or V_{L} domain from an antibody that binds the antigen to screen a library of complementary V_{L} or V_{H} domains, respectively. See, e.g., Portolano et al., J. Immunol. 150: 880-887 (1993); Clarkson et al., Nature 352: 624-628 (1991).

Among the provided antibodies are antibody fragments. An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')₂; diabodies; linear antibodies; single-chain antibody molecules (*e.g*. scFv); and multispecific antibodies formed from antibody fragments. In particular embodiments, the antibodies are single-chain antibody fragments comprising a variable heavy chain region and/or a variable light chain region, such as scFvs.

Single-domain antibodies are antibody fragments comprising all or a portion of the heavy chain variable domain or all or a portion of the light chain variable domain of an antibody. In certain embodiments, a single-domain antibody is a human single-domain antibody.

Antibody fragments can be made by various techniques, including but not limited to proteolytic digestion of an intact antibody as well as production by recombinant host cells. In some embodiments, the antibodies are recombinantly produced fragments, such as fragments comprising arrangements that do not occur naturally, such as those with two or more antibody regions or chains joined by synthetic linkers, e.g., peptide linkers, and/or that are may not be produced by enzyme digestion of a naturally-occurring intact antibody. In some aspects, the antibody fragments are scFvs.

A "humanized" antibody is an antibody in which all or substantially all CDR amino acid residues are derived from non-human CDRs and all or substantially all FR amino acid residues are derived from human FRs. A humanized antibody optionally may include at least a portion of an antibody constant region derived from a human antibody. A "humanized form" of a non-human antibody, refers to a variant of the non-human antibody that has undergone humanization, typically to reduce immunogenicity to humans, while retaining the specificity and affinity of the parental non-human antibody. In some embodiments, some FR residues in a humanized antibody are substituted with corresponding residues from a non-human antibody (e.g., the antibody from which the CDR residues are derived), e.g., to restore or improve antibody specificity or affinity.

Among the provided antibodies are human antibodies. A "human antibody" is an antibody with an amino acid sequence corresponding to that of an antibody produced by a human or a human cell, or non-human source that utilizes human antibody repertoires or other human antibody-encoding sequences, including human antibody libraries. The term excludes humanized forms of non-human antibodies comprising non-human antigen-binding regions, such as those in which all or substantially all CDRs are non-human.

Human antibodies may be prepared by administering an immunogen to a transgenic animal that has been modified to produce intact human antibodies or intact antibodies with human variable regions in response to antigenic challenge. Such animals typically contain all or a portion of the human immunoglobulin loci, which replace the endogenous immunoglobulin loci, or which are present extrachromosomally or integrated randomly into the animal's chromosomes. In such transgenic animals, the endogenous immunoglobulin loci have generally been inactivated. Human antibodies also may be derived from human antibody libraries, including phage display and cell-free libraries, containing antibody-encoding sequences derived from a human repertoire.

Among the provided antibodies are monoclonal antibodies, including monoclonal antibody fragments. The term "monoclonal antibody" as used herein refers to an antibody obtained from or within a population of substantially homogeneous antibodies, i.e., the individual antibodies comprising the population are identical, except for possible variants containing naturally occurring mutations or arising during production of a monoclonal antibody preparation, such variants generally being present in minor amounts. In contrast to polyclonal antibody preparations, which typically include different antibodies directed against different epitopes, each monoclonal antibody of a monoclonal antibody preparation is directed against a single epitope on an antigen. The term is not to be construed as requiring production of the antibody by any particular method. A monoclonal antibody may be made by a variety of techniques, including but not limited to generation from a hybridoma, recombinant DNA methods, phage-display and other antibody display methods.

### a. SJ25C1-derived Antibodies

In some embodiments, the anti-idiotype antibody is specific to a target anti-CD19 antibody that is or is derived from antibody SJ25C1 or an antigen-binding fragment thereof.

In some embodiments, the anti-idiotype antibodies or antigen-binding fragments thereof includes a heavy chain variable (V_{H}) region comprising at least 90% sequence identity to the V_{H} region amino acid sequence set forth in SEQ ID NO: 52, such as at least 91%, 92%, 93%, 94%, 95%, 96%, 97%, 98%, or 99% sequence identity thereto.

In some embodiments, the anti-idiotype antibody or antigen-binding fragments thereof includes a heavy chain variable (VH) region containing a heavy chain complementarity determining region 3 (CDR-H3) having the amino acid sequence set forth in SEQ ID NO: 53 or 54 and/or a CDR-H3 contained within the heavy chain variable (V_{H}) sequence set forth in SEQ ID NO: 52.

In some of any such embodiments, the V_{H} region includes a heavy chain complementarity determining region 1 (CDR-H1) comprising the amino acid sequence set forth in SEQ ID NO: 55, 56, 57, or 58, and/or a CDR-H1 contained within the V_{H} sequence set forth in SEQ ID NO: 52; and/or a heavy chain complementarity determining region 2 (CDR-H2) comprising the amino acid sequence set forth in SEQ ID NO: 59, 60, 61, or 62 and/or a CDR-H2 contained within the V_{H} sequence set forth in SEQ ID NO: 52.

In some embodiments, the anti-idiotype antibody or antigen-binding fragment thereof includes a heavy chain variable (VH) region comprising a heavy chain complementarity determining region 1 (CDR-H1), CDR-H2, and CDR-H3, wherein the CDR-H1 comprises the amino acid sequence set forth in SEQ ID NO: 55, 56, 57, or 58; the CDR-H2 comprises the amino acid sequence set forth in SEQ ID NO: 59, 60, 61, or 62; and/or the CDR-H3 comprises the amino acid sequence set forth in SEQ ID NO: 53 or 54. In some embodiments, provided are antibodies or antigen-binding fragments thereof that include a CDR-H1 having the amino acid sequence set forth in SEQ ID NO: 55, 56, 57, or 58; a CDR-H2 having the amino acid sequence set forth in SEQ ID NO: 59, 60, 61, or 62; and a CDR-H3 having the amino acid sequence set forth in SEQ ID NO: 53 or 54.

In some embodiments, the anti-idiotype antibody or antigen-binding fragment thereof that includes a heavy chain complementarity determining region 1 (CDR-H1), a CDR-H2, and a CDR-H3, respectively, comprising the amino acid sequences of a CDR-H1, a CDR-H2, and a CDR-H3 contained within the V_{H} region amino acid sequence set forth in SEQ ID NO: 52.

In some of any such embodiments, the V_{H} region contains a framework region 1 (FR1), a FR2, a FR3, and/or a FR4 sequence having at least 90% sequence identity, respectively, to a FR1, FR2, FR3, and/or FR4 of the amino acid sequence set forth in SEQ ID NO: 52. In some embodiments, the V_{H} region contains a framework region 1 (FR1), a FR2, a FR3, and/or a FR4 sequence having at least 91%, 92%, 93%, 94%, 95%, 96%, 97%, 98%, 98%, 99% sequence identity, respectively, to a FR1, FR2, FR3, and/or FR4 of the amino acid sequence set forth in SEQ ID NO: 52. In some embodiments, the V_{H} region contains a framework region 1 (FR1), a FR2, a FR3, and a FR4 sequence having at least 90%, 91%, 92%, 93%, 94%, 95%, 96%, 97%, 98%, 98%, 99% sequence identity, respectively, to a FR1, FR2, FR3, and FR4 of the amino acid sequence set forth in SEQ ID NO: 52.

In some of any of such embodiments, the V_{H} region has the sequence of amino acids set forth in SEQ ID NO: 52.

In some of any such embodiments, the anti-idiotype antibody or antigen-binding fragment is a heavy chain only, a VH-only, and/or does not include a VL or antigen-binding portion thereof and/or the antigen-binding site of the anti-idiotype antibody or fragment includes residues from the heavy chain only and/or does not include residues from a light chain.

In some of any such embodiments, the anti-idiotype antibody or fragment does not contain a light chain variable (V_{L}) region, does not contain a CDR-L1, CDR-L2, and/or CDR-L3, and/or is a single-domain antibody (sdAb) containing only the V_{H} region. In some embodiments, the antibody or fragment is a sdAb that only contains a VH region from any as described.

In some embodiments of any of the anti-idiotype antibodies or fragments containing any of the above VH region sequences, the anti-idiotype antibody or fragment further comprises a light chain variable (V_{L}) region. In some such embodiments, the V_{L} region has at least 90% sequence identity to the V_{L} region amino acid sequence set forth in SEQ ID NO: 63, such as at least 91%, 92%, 93%, 94%, 95%, 96%, 97%, 98%, or 99% sequence identity to the V_{L} region amino acid sequence set forth in SEQ ID NO: 63.

In some of any such embodiments, the V_{L} region comprises a light chain complementarity determining region 3 (CDR-L3) comprising the amino acid sequence set forth in SEQ ID NO: 64 or 65. In some of any such embodiments, the V_{L} region comprises a light chain complementarity determining region 3 (CDR-L3) having the amino acid sequence set forth in SEQ ID NO: 64 or 65.

In some of any such embodiments, the V_{L} region comprises a light chain complementarity determining region 1 (CDR-L1) comprising the amino acid sequence set forth in SEQ ID NO: 66 or 67, and/or a CDR-L1 contained within the V_{L} sequence set forth in SEQ ID NO: 63; and/or a light chain complementarity determining region 2 (CDR-L2) comprising the amino acid sequence set forth in SEQ ID NO: 68 or 69, and/or a CDR-L2 contained within the V_{L} sequence set forth in SEQ ID NO: 63. In some of any such embodiments, the V_{L} region comprises a light chain complementarity determining region 1 (CDR-L1) having the amino acid sequence set forth in SEQ ID NO: 66 or 67, and/or a CDR-L1 contained within the V_{L} sequence set forth in SEQ ID NO: 63; and/or a light chain complementarity determining region 2 (CDR-L2) having the amino acid sequence set forth in SEQ ID NO: 68 or 69, and/or a CDR-L2 contained within the V_{L} sequence set forth in SEQ ID NO: 63.

In some of any such embodiments, the V_{L} region comprises a CDR-L1 containing the amino acid sequence set forth in SEQ ID NO: 66 or 67; a CDR-L2 containing the amino acid sequence set forth in SEQ ID NO: 68 or 69; and a CDR-L3 containing the amino acid sequence set forth in SEQ ID NO: 64 or 65.

In some of any such embodiments, the V_{L} region comprises the CDR-L1, CDR-L2, and CDR-L3, respectively, comprising the amino acid sequences of a CDR-L1, a CDR-L2, and a CDR-L3 contained within the V_{L} region amino acid sequence set forth in SEQ ID NO: 63.

In some of any such embodiments, the V_{L} region comprises a framework region 1 (FR1), a FR2, a FR3, and/or a FR4 having at least 90% sequence identity, respectively, to the FR1, FR2, FR3, and/or FR4 of the amino acid sequence set forth in SEQ ID NO: 63. In some embodiments, the V_{L} region comprises a framework region 1 (FR1), a FR2, a FR3, and/or a FR4 sequence having at least 91%, 92%, 93%, 94%, 95%, 96%, 97%, 98%, 98%, 99% sequence identity, respectively, to a FR1, FR2, FR3, and/or FR4 of the amino acid sequence set forth in SEQ ID NO: 63. In some embodiments, the V_{L} region comprises a framework region 1 (FR1), a FR2, a FR3, and a FR4 sequence having at least 90%, 91%, 92%, 93%, 94%, 95%, 96%, 97%, 98%, 98%, 99% sequence identity, respectively, to a FR1, FR2, FR3, and FR4 of the amino acid sequence set forth in SEQ ID NO: 63.

In some of any such embodiments, the V_{L} region has the amino acid sequence set forth in SEQ ID NO: 63.

In some embodiments, the anti-idiotype antibody or antigen-binding fragment thereof comprises the amino acid sequences of CDR-H1, CDR-H2, and CDR-H3 sequences contained within the V_{H} region amino acid sequence set forth in SEQ ID NO: 52; and/or comprise the amino acid sequences of CDR-L1, CDR-L2, and CDR-L3 sequences contained within the light chain variable (VL) region amino acid sequence set forth in SEQ ID NO: 63.

In some embodiments, the anti-idiotype antibody or antigen-binding fragment thereof includes the V_{H} and V_{L} regions having amino acid sequences having at least 90 %, 91%, 92%, 93%, 94%, 95%, 96%, 97%, 98% or 99% identity to SEQ ID NOS: 52 and 63, respectively.

In some embodiments, provided are anti-idiotype antibodies or antigen-binding fragments thereof that include the V_{H} and V_{L} regions having amino acid sequences set forth in SEQ ID NOS: 52 and 63, respectively.

In some of any such embodiments, the V_{H} and V_{L} regions include the amino acid sequences of SEQ ID NOS: 52 and 63, respectively.

In some embodiments, the anti-idiotype antibody specific to antibody SJ25C1 or an antigen-binding fragment thereof is a single-chain antibody fragment, such as an scFv or diabody. In some embodiments, the single-chain antibody includes one or more linkers joining two antibody domains or regions, such as a variable heavy chain (V_{H}) region and a variable light chain (V_{L}). The linker typically is a peptide linker, e.g., a flexible and/or soluble peptide linker. Among the linkers are those rich in glycine and serine and/or in some cases threonine. In some embodiments, the linkers further include charged residues such as lysine and/or glutamate, which can improve solubility. In some embodiments, the linkers further include one or more proline.

In some embodiments, the anti-idiotype antibody is an intact antibody or full-length antibody. In some embodiments, the anti-ID may contain at least a portion of an immunoglobulin constant region, such as one or more constant region domains. In some embodiments, the constant regions include a light chain constant region (CL) and/or a heavy chain constant region 1 (CH1). In some embodiments, the anti-ID includes a CH2 and/or CH3 domain, such as an Fc region. In some embodiments, the Fc region is an Fc region of a human IgG, such as IgG1 or IgG4. In some embodiments, the anti-idiotype antibody contains the CH domain set forth in SEQ ID NO:115 or a sequence of amino acids that exhibits at least 85%, 86%, 87%, 88%, 89%, 90%, 91%, 92%, 93%, 94%, 95%, 96%, 97%, 98%, or 99% sequence identity to SEQ ID NO:115. In some embodiments, the anti-idiotype antibody contains the CL domain set forth in SEQ ID NO:118 or a portion thereof or a sequence of amino acids that exhibits at least 85%, 86%, 87%, 88%, 89%, 90%, 91%, 92%, 93%, 94%, 95%, 96%, 97%, 98%, or 99% sequence identity to SEQ ID NO:118 or a portion thereof.

In some embodiments, the anti-idiotype antibody specific for SJ25C1 comprises the heavy chain sequence set forth in SEQ ID NO:116 or a sequence that exhibits at least 85%, 86%, 87%, 88%, 89%, 90%, 91%, 92%, 93%, 94%, 95%, 96%, 97%, 98%, or 99% sequence identity to SEQ ID NO:116 and/or comprises the light chain sequence set forth in SEQ ID NO:119 or a sequence that exhibits at least 85%, 86%, 87%, 88%, 89%, 90%, 91%, 92%, 93%, 94%, 95%, 96%, 97%, 98%, or 99% sequence identity to SEQ ID NO:119. In some embodiments, the anti-idiotype antibody specific for SJ25C1 comprises the heavy chain sequence set forth in SEQ ID NO:116 and/or the light chain sequence set forth in SEQ ID NO:119. In some embodiments, the heavy chain and/or light chain of the anti-idiotype antibody further comprises a signal peptide. In some cases, the signal peptide has the sequence set forth in SEQ ID NO:117 or SEQ ID NO:120.

In some embodiments, the anti-idiotype antibody is an antigen-binding fragment. In some embodiments, the antigen-binding fragment is selected from the group consisting of fragment antigen binding (Fab) fragments, F(ab')₂ fragments, Fab' fragments, Fv fragments, a single chain variable fragment (scFv) or a single domain antibody.

Accordingly, provided are single-chain antibody fragments, such as scFvs and diabodies, particularly human single-chain fragments, typically comprising linker(s) joining two anti-idiotype antibody domains or regions, such V_{H} and V_{L} domains. The linker typically is a peptide linker, e.g., a flexible and/or soluble peptide linker, such as one rich in glycine and serine.

In some aspects, the linkers rich in glycine and serine (and/or threonine) include at least 80, 85, 90, 91, 92, 93, 94, 95, 96, 97, 98, or 99% such amino acid(s). In some embodiments, they include at least at or about 50%, 55%, 60%, 70%, or 75%, glycine, serine, and/or threonine. In some embodiments, the linker is comprised substantially entirely of glycine, serine, and/or threonine. The linkers generally are between or between about 5 and50 amino acids in length, typically between at or about 10 and at or about 30, e.g., 10, 11, 12, 13, 14, 15, 16, 17, 18, 19, 20, 21, 22, 23, 24, 25, 26, 27, 28, 29, or 30, and in some examples between 10 and 25 amino acids in length. Exemplary linkers include linkers having various numbers of repeats of the sequence GGGS (3GS; SEQ ID NO: 70) or GGGGS (4GS; SEQ ID NO: 27), such as between 2, 3, 4, and 5 repeats of such a sequence. Exemplary linkers include those having or consisting of a sequence set forth in SEQ ID NO: 71 (GGGGSGGGGSGGGGS). Exemplary linkers further include those having or consisting of the sequence set forth in SEQ ID NO: 72 (GSTSGSGKPGSGEGSTKG).

In some embodiments, the anti-idiotype antibodies include isolated antibodies. In some embodiments, the anti-ID is humanized, recombinant, and/or monoclonal. In some embodiments, the anti-ID is human.

### b. FMC63-derived Antibodies

In some embodiments, the anti-idiotype antibody is specific to a target anti-CD19 antibody that is or is derived from antibody FMC63 or an antigen-binding fragment thereof.

In some embodiments, the anti-idiotype antibody or antigen-binding fragment thereof includes a heavy chain variable (V_{H}) region comprising at least 90% sequence identity to the V_{H} region amino acid sequence set forth in SEQ ID NO: 73 or 74, such as at least 91%, 92%, 93%, 94%, 95%, 96%, 97%, 98%, or 99% sequence identity thereto.

In some embodiments, the antibody or antigen-binding fragment thereof includes a VH region having a heavy chain complementarity determining region 1 (CDR-H1) containing the amino acid sequence of GYX₃FX₅X₆YX₈MX₁₀ (SEQ ID NO: 95), wherein X₃ is T or S, X₅ is T or S, X₆ is D or R, X₈ is Y or W, and X₁₀ is K or N; and/or a heavy chain complementarity determining region 2 (CDR-H2) containing the amino acid sequence of WIGX₄IX₆PX₈X₉X₁₀X₁₁TX₁₃X₁₄NQX₁₇FKX₂₀ (SEQ ID NO: 96), wherein X₄ is D or M, X₆ is N or H, X₈ is N or S, X₉ is N or D, X₁₀ is G or S, X₁₁ is G or E, X₁₃ is D or R, X₁₄ is Y or L, X₁₇ is N or K, and X₂₀ is G or D; and/or a heavy chain complementarity determining region 3 (CDR-H3) containing the amino acid sequence of AX₂X₃X₄X₅X₆X₇X₈X₉X₁₀X₁₁X₁₂X₁₃X₁₄X₁₅ (SEQ ID NO: 97), wherein X₂ is R or S, X₃ is E or I, X₄ is G or Y, X₅ is N or Y, X₆ is N or E, X₇ is Y or null, X₈ is G or null, X₉ is S or null, X₁₀ is R or null, X₁₁ is D or null, X₁₂ is A or null, X₁₃ is M or null, X₁₄ is D or E, and X₁₅ is Y or A.

In some embodiments, the anti-idiotype antibody or antigen-binding fragment thereof includes a heavy chain variable (VH) region containing a heavy chain complementarity determining region 3 (CDR-H3) having the amino acid sequence set forth in SEQ ID NO: 75, 76, 77, or 78 and/or a CDR-H3 contained within the heavy chain variable (V_{H}) sequence set forth in SEQ ID NO: 73 or 74.

In some of any such embodiments, the V_{H} region includes a heavy chain complementarity determining region 1 (CDR-H1) comprising the amino acid sequence set forth in SEQ ID NO: 79, 80, 81, 82, 83, 84, 85, or 86, and/or a CDR-H1 contained within the V_{H} sequence set forth in SEQ ID NO: 73 or 74; and/or a heavy chain complementarity determining region 2 (CDR-H2) comprising the amino acid sequence set forth in SEQ ID NO: 87, 88, 89, 90, 91, 92, 93, or 94, and/or a CDR-H2 contained within the V_{H} sequence set forth in SEQ ID NO: 73 or 74.

In some embodiments, the anti-idiotype antibody or antigen-binding fragment thereof includes a heavy chain variable (VH) region comprising a heavy chain complementarity determining region 1 (CDR-H1), CDR-H2, and CDR-H3, wherein the CDR-H1 comprises the amino acid sequence set forth in SEQ ID NO: 79, 80, 81, 82, 83, 84, 85, or 86; the CDR-H2 comprises the amino acid sequence set forth in SEQ ID NO: 87, 88, 89, 90, 91, 92, 93, or 94; and/or the CDR-H3 comprises the amino acid sequence set forth in SEQ ID NO: 75, 76, 77, or 78. In some embodiments, provided are antibodies or antigen-binding fragments thereof that include a CDR-H1 having the amino acid sequence set forth in SEQ ID NO: 79, 80, 81, 82, 83, 84, 85, or 86; a CDR-H2 having the amino acid sequence set forth in SEQ ID NO: 87, 88, 89, 90, 91, 92, 93, or 94; and a CDR-H3 having the amino acid sequence set forth in SEQ ID NO: 75, 76, 77, or 78.

In some embodiments, the anti-idiotype antibody or antigen-binding fragments thereof includes a heavy chain complementarity determining region 1 (CDR-H1), a CDR-H2, and a CDR-H3, respectively, comprising the amino acid sequences of a CDR-H1, a CDR-H2, and a CDR-H3 contained within the V_{H} region amino acid sequence set forth in SEQ ID NO: 73 or 74.

In some embodiments, the anti-idiotype antibody or antigen-binding fragment thereof includes a CDR-H1 set forth in SEQ ID NOS: 79, 81, 82, 83; a CDR-H2 set forth in SEQ ID NOS: 87, 89, 90, 91; and/or a CDR-H3 set forth in SEQ ID NO: 71 or 77. In some embodiments, the anti-idiotype antibody or antigen-binding fragment thereof includes a CDR-H1 set forth in SEQ ID NO: 80, 84, 85, 86; a CDR-H2 set forth in SEQ ID NO: 88, 92, 93, 94; and/or a CDR-H3 set forth in SEQ ID NO: 76, 78, respectively.

In some of any such embodiments, the V_{H} region contains a framework region 1 (FR1), a FR2, a FR3, and/or a FR4 sequence having at least 90% sequence identity, respectively, to a FR1, FR2, FR3, and/or FR4 of the amino acid sequence set forth in SEQ ID NO: 73 or 74. In some embodiments, the V_{H} region contains a framework region 1 (FR1), a FR2, a FR3, and/or a FR4 sequence having at least 91%, 92%, 93%, 94%, 95%, 96%, 97%, 98%, 98%, 99% sequence identity, respectively, to a FR1, FR2, FR3, and/or FR4 of the amino acid sequence set forth in SEQ ID NO: 73 or 74. In some embodiments, the V_{H} region contains a framework region 1 (FR1), a FR2, a FR3, and a FR4 sequence having at least 90%, 91%, 92%, 93%, 94%, 95%, 96%, 97%, 98%, 98%, 99% sequence identity, respectively, to a FR1, FR2, FR3, and FR4 of the amino acid sequence set forth in SEQ ID NO: 73 or 74.

In some of any of such embodiments, the V_{H} region has the sequence of amino acids set forth in SEQ ID NO: 73 or 74.

In some of any such embodiments, the anti-idiotype antibody or antigen-binding fragment is a heavy chain only, a VH-only, and/or does not include a VL or antigen-binding portion thereof and/or the antigen-binding site of the anti-idiotype antibody or fragment includes residues from the heavy chain only and/or does not include residues from a light chain.

In some of any such embodiments, the anti-idiotype antibody or fragment does not contain a light chain variable (V_{L}) region, does not contain a CDR-L1, CDR-L2, and/or CDR-L3, and/or is a single-domain antibody (sdAb) containing only the V_{H} region. In some embodiments, the antibody or fragment is a sdAb that only contains a VH region from any as described.

In some embodiments of any of the anti-idiotype antibodies or fragments containing any of the above VH region sequences, the anti-idiotype antibody or fragment further comprises a light chain variable (V_{L}) region. In some such embodiments, the V_{L} region has at least 90% sequence identity to the V_{L} region amino acid sequence set forth in SEQ ID NO: 98 or 99, such as at least 91%, 92%, 93%, 94%, 95%, 96%, 97%, 98%, or 99% sequence identity to the V_{L} region amino acid sequence set forth in SEQ ID NO: 98 or 99.

In some embodiments, provided are antibodies or antigen-binding fragments thereof that include a VH region having a light chain complementarity determining region 1 (CDR-L1) containing the amino acid sequence of X₁AX₃X₄X₅X₆X₇X₈YX₁₀X₁₁WY (SEQ ID NO: 112), wherein X₁ is S or R, X₃ is S or R, X₄ is S or G, X₅ is G or N, X₆ is V or I, X₇ is I or H, X₈ is N or null, X₁₀ is M or L, and X₁₁ is Y or A; and/or a light chain complementarity determining region 2 (CDR-L2) containing the amino acid sequence of X₁X₂X₃YX₅X₆X₇X₈LAX₁₁ (SEQ ID NO: 113), wherein X₁ is P or L, X₂ is W or L, X₃ is I or V, X₅ is L or N, X₆ is T or A, X₇ is S or K, X₈ is N or T, and X₁₁ is S or D; and/or a light chain complementarity determining region 3 (CDR-L3) containing the amino acid sequence of QX₂X₃X₄X₅X₆PX₈T (SEQ ID NO: 114), wherein X₂ is Q or H, X₃ is W or F, X₄ is S or W, X₅ is S or W, X₆ is N or T, and X₈ is or Y.

In some of any such embodiments, the V_{L} region comprises a light chain complementarity determining region 3 (CDR-L3) comprising the amino acid sequence set forth in SEQ ID NO: 100, 101, 102, or 103. In some of any such embodiments, the V_{L} region comprises a light chain complementarity determining region 3 (CDR-L3) having the amino acid sequence set forth in SEQ ID NO: 100, 101, 102, or 103.

In some of any such embodiments, the V_{L} region comprises a light chain complementarity determining region 1 (CDR-L1) comprising the amino acid sequence set forth in SEQ ID NO: 104, 105, 106, or 107, and/or a CDR-L1 contained within the V_{L} sequence set forth in SEQ ID NO: 98 or 99; and/or a light chain complementarity determining region 2 (CDR-L2) comprising the amino acid sequence set forth in SEQ ID NO: 108, 109, 110, or 111, and/or a CDR-L2 contained within the V_{L} sequence set forth in SEQ ID NO: 98 or 99. In some of any such embodiments, the V_{L} region comprises a light chain complementarity determining region 1 (CDR-L1) having the amino acid sequence set forth in SEQ ID NO: 104, 105, 106, or 107, and/or a CDR-L1 contained within the V_{L} sequence set forth in SEQ ID NO: 98 or 99; and/or a light chain complementarity determining region 2 (CDR-L2) having the amino acid sequence set forth in SEQ ID NO: 108, 109, 110, or 111, and/or a CDR-L2 contained within the V_{L} sequence set forth in SEQ ID NO: 98 or 99.

In some of any such embodiments, the V_{L} region comprises a CDR-L1 containing the amino acid sequence set forth in SEQ ID NO: 104, 105, 106, or 107; a CDR-L2 containing the amino acid sequence set forth in SEQ ID NO: 108, 109, 110, or 111; and a CDR-L3 containing the amino acid sequence set forth in SEQ ID NO: 100, 101, 102, or 103.

In some of any such embodiments, the V_{L} region comprises the CDR-L1, CDR-L2, and CDR-L3, respectively, comprising the amino acid sequences of a CDR-L1, a CDR-L2, and a CDR-L3 contained within the V_{L} region amino acid sequence set forth in SEQ ID NO: 98 or 99.

In some embodiments, the anti-idiotype antibody or antigen-binding fragment thereof includes a CDR-L1 set forth in SEQ ID NOS: 104 or 106; a CDR-L2 set forth in SEQ ID NOS: 108 or 110; and/or a CDR-L3 set forth in SEQ ID NO: 100 or 101. In some embodiments, the anti-idiotype antibody or antigen-binding fragment thereof includes a CDR-L1 set forth in SEQ ID NO: 105 or 07; a CDR-L2 set forth in SEQ ID NO: 109 or 111; and/or a CDR-L3 set forth in SEQ ID NO: 102 or 103, respectively.

In some of any such embodiments, the V_{L} region comprises a framework region 1 (FR1), a FR2, a FR3, and/or a FR4 having at least 90% sequence identity, respectively, to the FR1, FR2, FR3, and/or FR4 of the amino acid sequence set forth in SEQ ID NO: 98 or 99. In some embodiments, the V_{L} region comprises a framework region 1 (FR1), a FR2, a FR3, and/or a FR4 sequence having at least 91%, 92%, 93%, 94%, 95%, 96%, 97%, 98%, 98%, 99% sequence identity, respectively, to a FR1, FR2, FR3, and/or FR4 of the amino acid sequence set forth in SEQ ID NO: 98 or 99. In some embodiments, the V_{L} region comprises a framework region 1 (FR1), a FR2, a FR3, and a FR4 sequence having at least 90%, 91%, 92%, 93%, 94%, 95%, 96%, 97%, 98%, 98%, 99% sequence identity, respectively, to a FR1, FR2, FR3, and FR4 of the amino acid sequence set forth in SEQ ID NO: 98 or 99.

In some of any such embodiments, the V_{L} region has the amino acid sequence set forth in SEQ ID NO: 98 or 99.

In some embodiments, the anti-idiotype antibody or antigen-binding fragment thereof comprises the amino acid sequences of CDR-H1, CDR-H2, and CDR-H3 sequences contained within the V_{H} region amino acid sequence set forth in SEQ ID NO: 73 or 74; and/or comprise the amino acid sequences of CDR-L1, CDR-L2, and CDR-L3 sequences contained within the light chain variable (VL) region amino acid sequence set forth in SEQ ID NO: 98 or 99.

In some embodiments, the anti-idiotype antibody or antigen-binding fragments thereof includes the V_{H} region having amino acid sequences having at least 90 %, 91%, 92%, 93%, 94%, 95%, 96%, 97%, 98% or 99% identity to SEQ ID NOS: 73 or 74 and V_{L} region having amino acid sequences having at least 90 %, 91%, 92%, 93%, 94%, 95%, 96%, 97%, 98% or 99% identity to SEQ ID NOS: 98 or 99.

In some embodiments, provided are anti-idiotype antibodies or antigen-binding fragments thereof that include the V_{H} region having amino acid sequences set forth in SEQ ID NOS: 73 or 74 and V_{L} region having amino acid sequences set forth in SEQ ID NOS: 98 or 99. In some of embodiments, the provided antibody contains the V_{H} region set forth in SEQ ID NO: 73 and the V_{L} region set forth in SEQ ID NO: 98. In some of embodiments, the provided antibody contains the V_{H} region set forth in SEQ ID NO: 74 and the V_{L} region set forth in SEQ ID NOS: 99.

In some embodiments, the anti-idiotype antibody is a single-chain antibody fragment, such as an scFv or diabody. In some embodiments, the single-chain antibody includes one or more linkers joining two antibody domains or regions, such as a variable heavy chain (V_{H}) region and a variable light chain (V_{L}). The linker typically is a peptide linker, e.g., a flexible and/or soluble peptide linker. Among the linkers are those rich in glycine and serine and/or in some cases threonine. In some embodiments, the linkers further include charged residues such as lysine and/or glutamate, which can improve solubility. In some embodiments, the linkers further include one or more proline.

In some embodiments, the anti-idiotype antibody is an intact antibody or full-length antibody. In some embodiments, the anti-ID may contain at least a portion of an immunoglobulin constant region, such as one or more constant region domains. In some embodiments, the constant regions include a light chain constant region (CL) and/or a heavy chain constant region 1 (CH1). In some embodiments, the anti-ID includes a CH2 and/or CH3 domain, such as an Fc region. In some embodiments, the Fc region is an Fc region of a human IgG, such as IgG1 or IgG4. In some embodiments, the anti-idiotype antibody contains the CH domain set forth in SEQ ID NO:121 or 127 or a portion thereof or a sequence of amino acids that exhibits at least 85%, 86%, 87%, 88%, 89%, 90%, 91%, 92%, 93%, 94%, 95%, 96%, 97%, 98%, or 99% sequence identity to SEQ ID NO:121 or 127 or a portion thereof. In some embodiments, the anti-idiotype antibody contains the CL domain set forth in SEQ ID NO:124 or 130 or a portion thereof or a sequence of amino acids that exhibits at least 85%, 86%, 87%, 88%, 89%, 90%, 91%, 92%, 93%, 94%, 95%, 96%, 97%, 98%, or 99% sequence identity to SEQ ID NO:124 or 130 or a portion thereof.

In some embodiments, the anti-idiotype antibody specific for SJ25C1 comprises the heavy chain sequence set forth in SEQ ID NO:122 or 128 or a sequence that exhibits at least 85%, 86%, 87%, 88%, 89%, 90%, 91%, 92%, 93%, 94%, 95%, 96%, 97%, 98%, or 99% sequence identity to SEQ ID NO:122 or 128 and/or comprises the light chain sequence set forth in SEQ ID NO:125 or 131 or a sequence that exhibits at least 85%, 86%, 87%, 88%, 89%, 90%, 91%, 92%, 93%, 94%, 95%, 96%, 97%, 98%, or 99% sequence identity to SEQ ID NO:125 o 131. In some embodiments, the anti-idiotype antibody specific for SJ25C1 comprises the heavy chain sequence set forth in SEQ ID NO:122 and/or the light chain sequence set forth in SEQ ID NO:131. In some embodiments, the anti-idiotype antibody specific for SJ25C1 comprises the heavy chain sequence set forth in SEQ ID NO:128 and/or the light chain sequence set forth in SEQ ID NO:131. In some embodiments, the heavy chain and/or light chain of the anti-idiotype antibody further comprises a signal peptide. In some cases, the signal peptide has the sequence set forth in SEQ ID NO:123, 126, 129 or 132.

In some embodiments, the anti-idiotype antibody is an antigen-binding fragment. In some embodiments, the antigen-binding fragment is selected from the group consisting of fragment antigen binding (Fab) fragments, F(ab')₂ fragments, Fab' fragments, Fv fragments, a single chain variable fragment (scFv) or a single domain antibody.

Accordingly, provided are single-chain antibody fragments, such as scFvs and diabodies, particularly human single-chain fragments, typically comprising linker(s) joining two anti-idiotype antibody domains or regions, such V_{H} and V_{L} domains. The linker typically is a peptide linker, e.g., a flexible and/or soluble peptide linker, such as one rich in glycine and serine.

In some aspects, the linkers rich in glycine and serine (and/or threonine) include at least 80, 85, 90, 91, 92, 93, 94, 95, 96, 97, 98, or 99% such amino acid(s). In some embodiments, they include at least at or about 50%, 55%, 60%, 70%, or 75%, glycine, serine, and/or threonine. In some embodiments, the linker is comprised substantially entirely of glycine, serine, and/or threonine. The linkers generally are between or between about 5 and 50 amino acids in length, typically between at or about 10 and at or about 30, e.g., 10, 11, 12, 13, 14, 15, 16, 17, 18, 19, 20, 21, 22, 23, 24, 25, 26, 27, 28, 29, or 30, and in some examples between 10 and 25 amino acids in length. Exemplary linkers include linkers having various numbers of repeats of the sequence GGGS (3GS; SEQ ID NO: 70) or GGGGS (4GS; SEQ ID NO: 27), such as between 2, 3, 4, and 5 repeats of such a sequence. Exemplary linkers include those having or consisting of a sequence set forth in SEQ ID NO: 71 (GGGGSGGGGSGGGGS). Exemplary linkers further include those having or consisting of the sequence set forth in SEQ ID NO: 72 (GSTSGSGKPGSGEGSTKG).

In some embodiments, the anti-idiotype antibodies include isolated antibodies. In some embodiments, the anti-ID is humanized, recombinant, and/or monoclonal. In some embodiments, the anti-ID is human.

### 3. Additional Components

In certain embodiments, particles, e.g., bead particles, comprise a surface conjugated or otherwise attached binding molecule that binds or is recognized by an antigen-binding domain of a recombinant receptor, e.g., a CAR, and one or more additional agents that is capable of binding to and/or recognizing to an additional molecule on the cell. In some embodiments, the one or more additional agent is an additional binding molecule. In some embodiments, the one or more additional binding molecules are antibodies (or fragments or variants thereof) that bind to polypeptides (e.g., glycoproteins) that present on the surface of cells that express the recombinant receptor. In particular embodiments, the one or more additional binding molecules are polypeptides or portions thereof that bind to a cell surface molecule, such as a receptor, e.g. activating, costimulatory or co-receptor, expressed on the surface of a cell, such as a cell that expresses the recombinant receptor. In particular embodiments, the one or more additional binding molecules are ligands or portions thereof that bind to polypeptides that are present on the surface of cells, such as cells that express the recombinant receptor. In certain embodiments, the one or more additional agents are exposed on the particle surface.

In particular embodiments, the one or more additional agent can modulate a function of the cells, e.g., expansion, by binding to the additional molecule on the surface of the cell. In some embodiments, the agent binds to the additional molecule on the cell and activates an accessory signal on the cell, i.e., the binding of the additional agent to the additional molecule on the cell surface has the same or similar effects on one or more cell functions, e.g., expansion, as the binding of an accessory molecule to the additional molecule on the surface of the cell. In some embodiments, the additional agent is an antibody or a fragment thereof that binds to or recognizes the additional molecule on the cell surface. In particular embodiments, the agent is a ligand or a portion thereof that binds to or recognizes the additional molecule on the surface of the cell.

In some embodiments, the molecule recognized by or bound by the one or more additional agent is CD2, CD3, CD4, CD5, CD8, CD25, CD27, CD28, CD29, CD31, CD44, CD45RA, CD45RO, CD54 (ICAM-1), CD127, MHCI, MHCII, CTLA-4, ICOS, ICOSL, PD-1 (CD279), PD-L1 (CD274, B7-H1), PDL2 (CD273, B7-DC), OX40 (CD134, TNFRSF4), OX-40L, DAP10, CD27L (CD70), 4-1BB (CD137), 4-1BBL, CD30L, LIGHT, IL-2R, IL-12R, IL-1R, IL-15R; IFN-gammaR, TNF-alphaR, IL-4R, IL- 10R, CD18/CDI la (LFA-1), CD62L (L-selectin), CD29/CD49d (VLA-4), Notch ligand (e.g., Delta-like 1/4, Jagged 1/2, etc.), CCR1, CCR2, CCR3, CCR4, CCR5, CCR7, CXCR3, CTLA-4, LAG-3 (CD223), TIM-3, 4-1BB (CD137), GITR (TNFRSF18, AITR), CD40, CXCR2, tumor associated antigens (TAA), B7-H3, B7-H4, BTLA, HVEM, GAL9, B7H3, B7H4, VISTA, KIR, 2B4 (belongs to the CD2 family of molecules and is expressed on all NK, γδ, and memory CD8+ (αβ) T cells), CD160 (also referred to as BY55), CGEN-15049, CEACAM (e.g., CEACAM-1, CEACAM-3 and/or CEACAM-5), TIGIT, CD155, CD155, LAIR1, CD160, 2B4, CD80, CD86, B7-H3 (CD276), B7-H4 (VTCN1), HVEM (TNFRSF14 or CD270), LIGHT, KIR, A2aR, MHC class I, MHC class II, GAL9, adenosine, or a transforming growth factor receptor (TGFR; e.g., TGFR beta).

In some embodiments, the particles, e.g., beads, comprise a surface conjugated or otherwise attached to a binding molecule that binds or is recognized by an antigen-binding domain of a recombinant receptor, e.g., a CAR, and one or more additional binding molecules that binds to a checkpoint molecule, such as an inhibitory receptor or activating receptor or ligand thereof, In some embodiments, the binding molecule contains the extracellular domain or binding portion thereof of an inhibitory receptor or ligand thereof or activating receptor or ligand thereof. In some embodiments, the binding molecule is fused to a multimerization domain, such as an Fc region or domain. In particular embodiments, the particles, e.g., beads, comprise a surface conjugated or otherwise attached binding molecule that that binds or is recognized by an antigen-binding domain of a recombinant receptor, e.g., a CAR, and one or more additional binding molecules that bind to OX-40, ICOS, DAP10, CD28 or 4-1BB, CTLA-4, PD-1, LAG-3, Tim-3, BTLA or TIGIT. In some embodiments, the particles, e.g., beads, comprise a surface conjugated or otherwise attached binding molecule that that binds or is recognized by an antigen-binding domain of a recombinant receptor, e.g., a CAR, and one or more additional binding molecules that bind to OX-40L, ICOSL, B7-1, B7-2 or 4-1BBL, PD-L1, PD-L2, CD155, CD112 or LIGHT.

In particular embodiments, the particles, e.g., beads, comprise a surface conjugated or otherwise attached binding molecule that that binds or is recognized by an antigen-binding domain of a recombinant receptor, e.g., a CAR, and one or more additional binding molecules, e.g., antibodies or fragments or variants thereof, that bind to CD3 and/or CD28. In some embodiments, the one or more additional molecules bind to CD2 or CD28. In particular embodiments, the particles, e.g., beads, comprise a surface conjugated or otherwise attached binding molecule that that binds or is recognized by an antigen-binding domain of a recombinant receptor, e.g., a CAR, and one or more additional binding molecules that bind to CD28.

In some embodiments, the particle contains a surface conjugated binding molecule and an additional surface conjugated agent that binds to CD2. In certain embodiments, the particle contains a surface conjugated binding molecule and a surface conjugated anti-CD2 antibody. In particular embodiments, the particle contains a surface conjugated binding molecule and an additional surface conjugated agent that binds to CD28. In various embodiments, the particle contains a surface conjugated binding molecule and a surface conjugated anti-CD28 antibody. In some embodiments, the particle contains a surface conjugated binding molecule and additional surface conjugated agents that binds to CD2 and CD28. In various embodiments, the particle contains a surface conjugated binding molecule and surface conjugated anti-CD2 and anti-CD28 antibodies. In certain embodiments, the binding molecule is or contains a BCMA, ROR1, or CD22 antigen or epitope containing fragment. In certain embodiments, the binding molecule is an anti-ID that binds to or recognizes a recombinant receptor, e.g., a CAR.

In particular embodiments, the particle contains a surface conjugated anti-ID, e.g., an anti-ID that binds to or recognizes a CAR such as an anti_CD19 CAR, and a surface conjugated anti-CD2 antibody. In certain embodiments, the particle contains a surface conjugated anti-ID, e.g., an anti-ID that binds to or recognizes a CAR such as an anti_CD19 CAR, and a surface conjugated anti-CD28 antibody. In various embodiments, the particle contains a surface conjugated anti-ID, e.g., an anti-ID that binds to or recognizes a CAR such as an anti_CD19 CAR, and surface conjugated anti CD2 and anti-CD28 antibodies.

In some embodiments, the ratio, such as the molar or weight ratio, of the binding molecule and the additional agent or molecule is from or from about 1:10 to 10:1, such as 1:5 to 5:1 or 1:2 or 2:1, or is about 1:1. In certain embodiments, the ratio, such as the molar or weight ratio, of the binding molecule to the two additional agents is or is about 1:1:1.

### C. Methods of Conjugation

Provided herein are particles (e.g., bead particles) that are conjugated and/or attached to binding molecules (e.g., polypeptide antigen or antibody) that bind or recognize a recombinant receptor (e.g., a CAR). A variety of means, well known in the art, may be used to conjugate binding molecules, e.g., polypeptide antigens and anti-idiotype antibodies, to particles (e.g., bead particles). These methods include any standard chemistries which do not destroy or severely limit the biological activity or structure of the binding molecule, and which allow for a sufficient number of binding molecules to be conjugated to the particle in a manner which allows for an interaction of the binding molecule, e.g., antigen peptide or protein, with the recombinant receptor. In some embodiments, a method of conjugation is selected that conjugates the C-terminal region of the binding molecule, e.g., a polypeptide binding molecule, to the particle. One of skill in the art will understand that the exact chemistries for conjugation may depend upon the nature of the particle material, the functional groups exposed at the surface of the particle, the presence or absence of C-terminal fusions to the binding molecule, and the presence or absence of conjugating moieties. Whichever chemistry is chosen, it is important that the conjugation method does not alter the function and/or structural confirmation of the binding molecule. For example, if the binding molecule is an antigen, the method selected for conjugating the antigen to the particle must not prevent the antigen from being recognized by its conjugate receptor. In certain embodiments, if the binding molecule is an antibody, e.g. an anti-ID, the conjugation method must not prevent the antibody from binding its target antigen.

In some embodiments, the binding molecule (e.g., a polypeptide antigen or anti-ID) is attached to the particle (e.g., a bead particle) by passive absorption to the plain surface of the particle. Attachment by this method typically relies on hydrophobic interactions that bind the binding molecule to the bead. While this method relatively simple, in some embodiments, the method provides little control over the final orientation of the attached molecule with respect to other techniques of attaching the binding molecule, such as any of the techniques discussed herein, such as in Section I-C.

### 1. Binding to Particle Surface

In certain embodiments, binding molecules are bound to the carrier via a covalent chemical bond. In particular embodiments, the binding molecule is a polypeptide and a reactive group or moiety of an amino acid is conjugated directly to a reactive group or moiety on the surface of the particle by a direct chemical reaction. In certain embodiments, an amino acid carboxyl group (e.g., a C-terminal carboxyl group), hydroxyl, thiol, or amine group ( such as an amino acid side chain group) of the binding molecule is conjugated directly to a hydroxyl or carboxyl group of a PLA or PGA polymer, a terminal amine or carboxyl group of a dendrimer, or a hydroxyl, carboxyl or phosphate group of a phospholipid on the surface of the particle by direct chemical reaction. In some embodiments, a conjugating moiety conjugates, e.g., covalently binds, to both the binding molecule and the particle, thereby linking them together.

In certain embodiments, the surface of the particle comprises chemical moieties and/or functional groups that allow attachment (e.g., covalent, non-covalent) of the binding molecule (e.g., polypeptide antigen or antibody). In some embodiments, the number, orientation, spacing, etc. of chemical moieties and/or functional groups on the particle vary according to particle chemistry and the properties of the binding molecule. In particular embodiments, the particles, e.g., beads, have introduced modified surfaces. In particular embodiments, the particle surfaces contain exposed functional groups. Suitable surface exposed functional groups include, but are not limited to, carboxyl, amino, hydroxyl, sulfate groups, tosyl, epoxy, and chloromethyl groups.

In some embodiments, the binding molecule is a polypeptide and is conjugated to the surface-exposed functional groups. In some embodiments, the surface exposed functional group must be activated, i.e., it must undergo a chemical reaction to yield an intermediate product capable of directly binding a polypeptide. Particular embodiments contemplate that activation of surface exposed functional groups on a particle to allow for conjugation of a binding molecule is a matter of routine skill in the art, and that one of skill would readily identify suitable reagents and protocols to perform any necessary activation steps to conjugate a binding molecule to a particle.

In some embodiments, in order to bind a polypeptide binding molecule to a carboxylated particle, e.g., bead particle, the surface exposed carboxyl groups of the particle require activation by contacting the functional group with an agent that will yield an intermediate ester that is capable of directly binding an amine group. Reactive (i.e. activated) carboxyl groups on the surface of a particle may be conjugated to free amines (e.g., from Lys residues) on the polypeptide. Suitable agents include, for example but not limited to, carbodiimide (EDC), ethylene carbodiimide (ECDI), hexamethylene diisocyanate, propyleneglycol di-glycidylether which contain 2 epoxy residues, epichlorohydrin, N-Hydroxysuccinimide (NHS) or sulfo-NHS or ethyl (dimethylaminopropyl). In some embodiments, a polypeptide binding molecule is covalently attached to a particle at a surface-exposed carboxyl group. In particular embodiments, the polypeptide binding molecule is covalently attached at the surface-exposed carboxyl group by first contacting the carboxyl group with an agent to generate an intermediate ester.

In some embodiments, a functional group of the polypeptide binding molecule is activated prior to conjugating the polypeptide to a surface-exposed functional group. For example, a carboxyl group of the polypeptide molecule may be activated with the agents described above to generate intermediate esters capable of directly binding to surface exposed amino groups of the particle. In some embodiments, a polypeptide binding molecule is conjugated to the particle at a surface exposed amine group. In particular embodiments, a carboxyl group of the polypeptide binding molecule is contacted with an agent to generate an intermediate ester prior to covalently attaching the particle to the surface exposed carboxyl group of the particle. Alternatively, free amine groups on the surface of a carrier may be covalently bound to antigen peptides and proteins, or antigen peptide or protein fusion proteins, using sulfosuccinimidyl (4-iodoacetyl)aminobenzoate (sulfo-SIAB) chemistry.

In particular embodiments, a polypeptide binding molecule is covalently attached to the particle, e.g., a bead particle, at a surface exposed functional group that does not require activation by an agent prior to forming a covalent attachment. Examples of such functional groups include, but are not limited to, tosyl, epoxy, and chloromethyl groups. In particular embodiments, covalent attachment can be performed in the presence of specific buffers, at specific pH ranges, at specific temperature ranges, and for specific amounts of time that can readily be identified and performed for any such functional group by one of skill in the art. For example, in some embodiments, tosyl groups on the particle surface bind to amino or to sulfhydryl groups of a binding molecule (e.g., a polypeptide antigen or antibody) depending on pH. Neutral pH is used bind sulfhydryl groups of a polypeptide to the tosyl group, whereas a more basic pH is used for binding amino groups of a polypeptide to the tosyl group. In particular embodiments, a polypeptide binding molecule (e.g., an antigen or antibody) is covalently attached to a particle (e.g., a bead particle) at surface-exposed tosyl group, epoxy group, or chloromethyl group of the particle. In particular embodiments, the polypeptide binding molecule is attached to a tosylated particle (i.e. a particle comprising surface exposed tosyl groups).

In some embodiments, a non-covalent bond between a ligand bound to the antigen peptide or protein and an anti-ligand attached to the carrier may conjugate the antigen to the carrier. In some embodiments, a biotin ligase recognition sequence tag may be joined to the C-terminus of an antigen peptide or protein, and this tag may be biotinylated by biotin ligase. The biotin may then serve as a ligand to non-covalently conjugate the antigen peptide or protein to avidin or streptavidin which is adsorbed or otherwise bound to the surface of the carrier as an anti-ligand. Alternatively, if the antigen peptides and proteins are fused to an immunoglobulin domain bearing an Fc region, as described above, the Fc domain may act as a ligand, and protein A, either covalently or non-covalently bound to the surface of the carrier, may serve as the anti-ligand to non-covalently conjugate the antigen peptide or protein to the carrier. Other means are well known in the art which may be employed to non-covalently conjugate antigen peptides and proteins to carriers, including metal ion chelation techniques (e.g., using a poly-His tag at the C-terminus of the antigen peptide or protein or antigen peptide or protein fusion proteins, and a Ni -coated carrier), and these methods may be substituted for those described here.

In some embodiments, the binding molecule is conjugated to the particle by a linker. In certain embodiments, the linkers can include, but are not limited to, a variety of bifunctional protein coupling agents such as N-succinimidyl-3-(2-pyridyldithio)propionate (SPDP), succinimidyl-4-(N- maleimidomethyl)cyclohexane-1-carboxylate, iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p- azidobenzoyl)hexanediamine), bis-diazonium derivatives (such as bis-(p-diazoniumbenzoyl)- ethylenediamine), diisocyanates (such as toluene 2,6-diisocyanate), and bis-active fluorine compounds (such as 1,5-difluoro-2,4-dinitrobenzene). Particular coupling agents include N- succinimidyl-3-(2-pyridyldithio)propionate (SPDP) and N-succinimidyl-4-(2-pyridylthio)pentanoate (SPP) to provide for a disulfide linkage.

### 2. Reversible Binding

In some embodiments, the binding molecule is reversibly attached or otherwise associated with a particle, e.g., a bead particle. In certain embodiment, the binding molecule is reversibly attached, or otherwise associated with, a reagent that is attached to the particle. In particular embodiments, the reagent is exposed on the particle surface. In certain embodiments, the reagent contains a plurality of binding sites capable of reversibly binding to the binding molecule. In some embodiments, the reagent is a multimerization reagent. In some embodiments, the binding interaction between the binding molecule and the reagent is a non-covalent interaction. In some embodiments, the binding interaction between the binding molecule and the reagent, e.g., a non-covalent binding interaction is reversible. In some embodiments, the binding molecule is reversibly attached to a particle that is an oligomer or a polymer comprised of proteins, e.g., streptavidin.

In some embodiments, the reversible association between the binding molecule and the reagent can be mediated in the presence of a substance, such as a competition reagent (also called an eluent reagent), that is or contains a binding site that also is able to bind to the same binding site (or sites) of the reagent that is bound by the binding molecule. Generally, the substance (e.g., competition reagent) can act as a competitor due to a higher binding affinity for the binding site present in the reagent and/or due to being present at higher concentrations than the binding molecule, thereby detaching and/or dissociating the binding molecule from the reagent. In some embodiments, the affinity of the substance (e.g., competition reagent) for the at least one binding site on the reagent is greater than the affinity of the binding molecule for the at least one binding site on the reagent. Thus, in some embodiments, the bond between the binding of the reagent and the binding molecule can be disrupted by addition of the substance (e.g., competition reagent), thereby rendering the association of the agent (e.g., receptor-binding agent or selection agent) and reagent reversible.

Reagents that can be used in such reversible systems are described and known in the art, see e.g., U.S. Patent Nos. 5,168,049; 5,506,121; 6,103,493; 7,776,562; 7,981,632; 8,298,782; 8,735,540; 9,023,604; and International published PCT Appl. Nos. WO2013/124474 and WO2014/076277. Non-limiting examples of reagents and binding partners capable of forming a reversible interaction, as well as substances (e.g., competition reagents) capable of reversing such binding, are described below.

In some embodiments, the reagent is attached to and exposed on the surface of a particle, e.g., a bead particle, and contains a plurality of binding sites that are able to specifically bind to the binding molecule, such that the reagent is capable of reversibly binding to a plurality of binding molecules, e.g., is a multimerization reagent. In some embodiments, the reagent is an oligomer or polymer of individual molecules (e.g., monomers) or complexes that make up an individual molecule (e.g., tetramer) that are attached to the surface of a particle, each containing at least one binding site capable of binding to the binding molecule. In some embodiments, particle is composed of the reagent, i.e. the particle is an oligomer or a polymer of the reagent.

In some embodiments, the reagent is a streptavidin, a streptavidin mutein or analog, avidin, an avidin mutein or analog (such as neutravidin) or a mixture thereof, in which such reagent contains one or more binding sites capable of a reversible association with a binding molecule. In some embodiments, the binding molecule comprises a biotin, a biotin derivative or analog, or a streptavidin-binding peptide or other molecule that is able to specifically bind to the reagent. In some embodiments, the binding molecule comprises a biotin, a biotin derivative or analog, or a streptavidin-binding peptide or other molecule that is able to specifically bind to streptavidin, a streptavidin mutein or analog, avidin or an avidin mutein or analog. In certain embodiments, binding molecule is a polypeptide, e.g., a polypeptide antigen or antibody, that comprises a fusion domain that is a biotin, a biotin derivative or analog, or a streptavidin-binding peptide or other molecule that is able to specifically bind to streptavidin, a streptavidin mutein or analog, avidin or an avidin mutein or analog. In some embodiments, the binding molecule is a polypeptide that comprises a fusion domain that is a biotin, a biotin derivative or analog, or a streptavidin-binding peptide or other molecule that is able to specifically bind to streptavidin, a streptavidin mutein or analog, avidin or an avidin mutein or analog, and the reagent is or comprises streptavidin, avidin, an analog or mutein of streptavidin, or an analog or mutein or avidin that reversibly binds biotin, a biotin analog or a biologically active fragment thereof. In certain embodiments, the fusion domain is located at the C-terminus of the binding molecule. In some embodiments, the reagent is or comprises an analog or mutein of streptavidin or an analog or mutein of avidin that reversibly binds a streptavidin-binding peptide. In some embodiments, the substance (e.g., competitive reagent) can be a biotin, a biotin derivative or analog or a streptavidin-binding peptide capable of competing for binding with the binding molecule for the one or more binding sites of the reagent. In some embodiments, fusion domain of the binding molecule and the substance (e.g., competitive reagent) are different, and the substance (e.g., competitive reagent) exhibits a higher binding affinity for the reagent as compared to the affinity of the binding molecule to the reagent. In certain embodiments, the fusion domain and the substance, e.g., competitive reagent, are the same.

In some embodiments, the streptavidin can be wild-type streptavidin, streptavidin muteins or analogs, such as streptavidin-like polypeptides. Likewise, avidin, in some aspects, includes wild-type avidin or muteins or analogs of avidin such as neutravidin, a deglycosylated avidin with modified arginines that typically exhibits a more neutral isoelectric point and is available as an alternative to native avidin. Generally, deglycosylated, neutral forms of avidin include those commercially available forms such as "Extravidin", available through Sigma Aldrich, or "NeutrAvidin" available from Thermo Scientific or Invitrogen, for example.

In some embodiments, the reagent is a streptavidin or a streptavidin mutein or analog. In some embodiments, wild-type streptavidin (wt-streptavidin) has the amino acid sequence disclosed by Argarana et al, Nucleic Acids Res. 14 (1986) 1871-1882 (SEQ ID NO: 21). In general, streptavidin naturally occurs as a tetramer of four identical subunits, i.e. it is a homo-tetramer, where each subunit contains a single binding site for biotin, a biotin derivative or analog or a biotin mimic. An exemplary sequence of a streptavidin subunit is the sequence of amino acids set forth in SEQ ID NO: 21, but such a sequence also can include a sequence present in homologs thereof from other *Streptomyces* species. In particular, each subunit of streptavidin may exhibit a strong binding affinity for biotin with an equilibrium dissociation constant (K_{D}) on the order of about 10⁻¹⁴ M. In some cases, streptavidin can exist as a monovalent tetramer in which only one of the four binding sites is functional (Howarth et al. (2006) Nat. Methods, 3:267-73; Zhang et al. (2015) Biochem. Biophys. Res. Commun., 463:1059-63)), a divalent tetramer in which two of the four binding sites are functional (Fairhead et al. (2013) J. Mol. Biol., 426:199-214), or can be present in monomeric or dimeric form (Wu et al. (2005) J. Biol. Chem., 280:23225-31; Lim et al. (2010) Biochemistry, 50:8682-91).

In some embodiments, the binding molecule comprises a fusion domain that is a Strep-tag, e.g., such as disclosed in U.S. Pat. No. 5,506,121, which is a peptide sequence that acts as biotin mimics and demonstrate a binding affinity for streptavidin. In some cases, the binding affinity can be further improved by making a mutation within the streptavidin molecule, see e.g., U.S. Pat. No. 6,103,493 or International published PCT App. No. WO2014/076277. In some embodiments, binding affinity can be determined by methods known in the art.

In some embodiments, the reagent, such as a streptavidin or streptavidin mutein, exhibits binding affinity for a fusion domain of the binding molecule, In some embodiments, the fusion domain comprises a peptide sequence contains a sequence with the general formula set forth in SEQ ID NO: 11, such as contains the sequence set forth in SEQ ID NO: 12. In some embodiments, the peptide sequence has the general formula set forth in SEQ ID NO: 13, such as set forth in SEQ ID NO: 43. In one example, the peptide sequence is set forth in SEQ ID NO: 9. In one example, the peptide sequence is set forth in SEQ ID NO: 10, also known as STREP-TAG^{®} II streptavidin polypeptide. In some embodiments, the peptide ligand contains a sequential arrangement of at least two streptavidin-binding modules, wherein the distance between the two modules is at least 0 and not greater than 50 amino acids, wherein one binding module has 3 to 8 amino acids and contains at least the sequence His-Pro-Xaa (SEQ ID NO: 11), where Xaa is glutamine, asparagine, or methionine, and wherein the other binding module has the same or different streptavidin peptide ligand, such as set forth in SEQ ID NO: 13 (see e.g., International Published PCT Appl. No. WO02/077018; U.S. Patent No. 7,981,632). In some embodiments, the peptide ligand contains a sequence having the formula set forth in any of SEQ ID NO: 15 or 16. In some embodiments, the peptide ligand has the sequence of amino acids set forth in any of SEQ ID NOS: 15-22.

In some embodiments, the reagent is a streptavidin or streptavidin mutein (or a portion thereof) that binds to or recognizes to other streptavidin ligands, such as but not limited to, biotin, iminobiotin, lipoic acid, desthiobiotin, diaminobiotin, HABA (hydroxyazobenzene-benzoic acid) and/or dimethyl-HABA. In some embodiments, the streptavidin mutein exhibits a binding affinity for another streptavidin ligand, such as biotin or desthiobiotin, that is greater than the binding affinity of the streptavidin mutein for a biotin mimic peptide ligand, such as set forth in any of SEQ ID NOS: 7-19. Thus, in some embodiments, biotin or a biotin analog or derivative (e.g., desthiobiotin) can be employed as a competition reagent in the provided methods. For example, as an example, the interaction of a mutein streptavidin designated Strep-tactin^{®} (e.g., containing the sequence set forth in SEQ ID NO: 23.

In some embodiments, the reagent comprises at least two chelating groups K that may be capable of binding to a transition metal ion. In some embodiments, the reagent may be capable of binding to an oligohistidine affinity tag, a glutathione-S-transferase, calmodulin or an analog thereof, calmodulin binding peptide (CBP), a FLAG-peptide, an HA-tag, maltose binding protein (MBP), an HSV epitope, a myc epitope, and/or a biotinylated carrier protein.

### 3. Binding Molecule Orientation

In some embodiments, the binding molecule is bound or attached to the particle, e.g., a bead particle, in an orientation that is optimized to allow for an interaction between the binding molecule and a cell that expresses a recombinant receptor, e.g., a CAR, that is bound by or recognized by the binding molecule. In some embodiments, the optimized orientation allows for binding between the binding molecule and the recombinant receptor with minimal or no interference by the particle to which the binding molecule is bound. In some embodiments, the relative position of the particle with respect to the region of the binding molecule that binds to or recognizes the CAR, e.g., the antigen or anti-ID, does not prevent access for this region to contact cells that express the recombinant receptor, e.g., the CAR. In some embodiments, the binding molecule is attached to the particle at a different region, e.g., a fusion domain, than the region that binds to or recognizes the recombinant receptor of the target cell, e.g., the antigen or anti-ID. In certain embodiments, the binding molecule is not attached to the particle at the region that binds to or recognizes the recombinant receptor. In certain embodiments, the binding molecule is less likely to be attached to the particle at the region that binds to or recognizes the recombinant receptor as compared to another region of the binding molecule.

Particular embodiments contemplate that when a binding molecule is bound to a particle, e.g., a bead particle, at one end of the binding molecule, e.g., at the C-terminus, this provides an optimal orientation for the binding molecule to bind to the recombinant receptor. In some embodiments, the binding molecule is bound to the particle at the opposite end from the region that that binds or recognizes the recombinant receptor. In certain embodiments, the binding molecule is attached to the particle so that the region that binds to the recombinant receptor, e.g., the antigen or anti-ID, is positioned away from the particle. In particular embodiments, the binding molecule is attached to the particle at one end, e.g., at its C-terminus, and the region that binds to the recombinant receptor is at the opposite end, e.g., the N-terminus, of the binding molecule. In particular embodiments, the binding molecule is attached to the particle so that the antigen or anti-ID of the binding molecule is positioned in an outward orientation in relation to the particle. Some embodiments contemplate that the outward orientation for the region that binds the recombinant receptor from the particle provides the greatest probability for interactions between the binding molecules and cells that expresses a recombinant receptor.

In some embodiments, the binding molecule, e.g., a polypeptide, comprises a region that binds to or recognizes the recombinant receptor of the target cell, e.g., and antigen or anti-ID, and is attached or bound to the particle at a separate region, e.g., a fusion domain. In certain embodiments, the region that binds to or recognizes the recombinant receptor and the separate region, e.g., the fusion domain, are on opposite ends of the binding molecule. In certain embodiments, the binding molecule comprises a region that binds to or recognizes the recombinant receptor of the target cell which is at or near the N-terminus of the binding molecule and a separate region, e.g., a fusion domain, which is at or near the C-terminal domain. In some embodiments, the binding molecule comprises a region that binds to or recognizes the recombinant receptor of the target cell which is at or near the C-terminus of the binding molecule and the separate region, e.g., a fusion domain, which is at or near the N-terminal domain. In some embodiments, a region of a binding molecule, e.g., a polypeptide antigen or antibody, is near the N-terminus or the C-terminus if the region is located within one, two, three, four, five, six, seven, eight, nine, ten, fifteen, twenty, twenty-five, thirty, thirty-five, forty, forty-five, fifty, sixty, seventy, eighty, ninety, or one hundred amino acids from the N-terminus or the C-terminus.

In some embodiments, the binding molecule is a polypeptide, e.g., a polypeptide antigen or antibody, that comprises a fusion domain that binds to the particle. In some embodiments, the binding molecule binds to the particle at one or more sites within the fusion domain. In certain embodiments, the binding molecule is more likely to bind to the particle at one or more sites within the fusion domain than at one or more sites of the binding molecule that fall outside of the fusion domain. In some embodiments, the binding molecule is at least 1%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 99%, at least 99.9% or at least 100% more likely to bind to the particle at one or more sites within the fusion domain than at one or more sites of the binding molecule that fall outside of the fusion domain. In certain embodiments, the binding molecule is at least 1-fold, at least 2-fold, at least 3-fold, at least 4-fold, at least 5-fold, at least 6-fold, at least 7-fold, at least 8-fold, at least 9-fold, at least 10-fold, at least 20-fold, at least 30-fold, at least 40-fold, at least 50-fold, at least 100-fold, at least 500-fold or at least 1,000-fold more likely to bind to the particle at one or more sites within the fusion domain than at one or more sites of the binding molecule that fall outside of the fusion domain.

In certain embodiments, the binding molecule is linked to a fusion domain or tag. In some embodiments, the binding molecule., is or contains an antigen or an anti-ID that is recognized and/or bound by a recombinant receptor, such as an antigen receptor or a CAR, is linked, directly or indirectly, to a fusion domain or tag. In some embodiments, the binding molecule is a fusion polypeptide containing the antigen or portion thereof and fusion domain or tag. In some embodiments, the binding molecule is conjugated to the particle at a site (e.g., a side chain of an amino acid) at or within the fusion domain or tag. In certain embodiments, attachment of the binding molecule to the particle at the fusion domain or tag results in an optimal orientation for the binding molecule that is conjugated to the particle to be bound or recognized by the recombinant receptor or CAR. In some embodiments, a binding molecule comprising an antigen or anti-ID that is recognized by an antigen receptor, e.g. CAR, is linked to a fusion domain or tag at the C-terminus, in which case, conjugation to the particle via the fusion domain or tag preferentially orients or exposes the N-terminal region of the bound binding molecule for interactions with the antigen receptor, e.g. CAR.

Fusion domains or tags are well known in the art, and can be linked to an antigen in the provided binding molecules to confer a desired property, e.g., isolation of the fusion polypeptide by affinity chromatography, or covalent attachment to a functional group on a particle surface. Well known examples of such fusion domains include, but are not limited to, polyhistidine, Glu-Glu, avidin, glutathione S transferase (GST), thioredoxin, protein A, protein G, an immunoglobulin heavy chain constant region (Fc), maltose binding protein (MBP), or human serum albumin. In some embodiments, the fusion domain is a polypeptide tag. Well known examples of polypeptide tags include, but are not limited to, AviTag (SEQ ID NO: 3), a Calmodulin-tag (SEQ ID NO: 4),a polyglutamate tag (SEQ ID NO: 5), a FLAG-tag (SEQ ID NO: 6), an HA-tag (SEQ ID NO: 7), a His-tag, (5-10 histidines), a Myc-tag (SEQ ID NO: 8) ,and fluorescent protein-tags (e.g., EGFP).

In some embodiments, the fusion domain or tag comprises a streptavidin-binding peptide sequence. In some embodiments, the sequence is a STREP-TAG^{®} streptavidin-binding peptide sequence as exemplified by SEQ ID NOS: 9 and 10. In some embodiments, the fusion domain comprises a streptavidin-binding peptide sequence as exemplified by the amino acid sequences set forth in SEQ ID NOS: 11-19.

In some embodiments, the binding molecule comprises a GST fusion domain which binds to a surface exposed glutathione of the particle. In particular embodiments, the GST fusion domain is located at or near the C-terminus of the binding molecule. In some embodiments, the binding molecule comprises a strep-tag fusion domain, which binds to a surface exposed biotin or streptavidin ligand of the particle. In some embodiments, the strep-tag fusion domain is at or near the C-terminus of the binding molecule. In particular embodiments, the binding molecule comprises a Protein A fusion domain which binds to an Fc polypeptide attached to the surface of the particle. In some embodiments, the Protein A fusion domain is at or near the C-terminus of the binding molecule. In certain embodiments, the binding molecule comprises a Protein G fusion domain which binds to an albumin or an Fc polypeptide attached to the surface of the particle. In some embodiments, the Protein A fusion domain is at or near the C-terminus of the binding molecule.

In particular embodiments, the binding molecule contains a hydrophobic region or a region that is more hydrophobic than the rest of the binding molecule. In some embodiments, the binding molecule comprises a region that is more hydrophobic than the sites of the binding molecule that fall outside of the hydrophobic region. In some embodiments, the hydrophobic region is a fusion domain. In particular embodiments, the hydrophobic region is an Fc domain. In certain embodiments, the binding molecule is more likely to bind or attach to the particle, at one more sites in a hydrophobic region than in a different region of the binding molecule if the surface of the particle is hydrophobic. In various embodiments, the hydrophobic region of the binding molecule binds a surface exposed functional group of the particle. In some embodiments, the function group is or contains a tosyl group.

In some embodiments, the binding molecule comprises an Fc domain, wherein the Fc domain is present at the C-terminus of the fusion polypeptide. In some embodiments, the binding molecule is an antibody, e.g., and anti-idiotype antibody, comprising an Fc domain. In particular embodiments, the Fc domain binds or attaches to a surface exposed Protein G or Protein A of the particle. In some embodiments, one or more functional groups of one or more amino acids present on the Fc domain binds to a functional group of the particle. In particular embodiments, the Fc domain of the fusion polypeptide or antibody is more hydrophobic than the antigen or antibody region of the binding molecule, and sites within the Fc domain are more likely to bind to a particle, e.g., at surface exposed function groups, when the particle surface is hydrophobic than sites of the binding molecule that are outside of the Fc domain.

In particular embodiments, a binding molecule comprising an Fc domain is bound to a particle at one or more sites within the Fc region. Binding of a binding molecule at a site located on the Fc region to a particle, e.g., a bead particle, may readily be performed through standard techniques in the art. For example, Fc domains tend to be hydrophobic, and in where the Fc domain is more hydrophobic than the other domains of the binding molecule (e.g., the polypeptide antigen), then binding between amino acid side chains located within the Fc domain and surface exposed funtional groups located on the particle (e.g., tosyl groups) may occur if the particle surface is hydrophobic. In some embodiments, the surface of the particle is hydrophobic. In some embodiments, the surface of the particle is non-hydrophilic. In particular embodiments, the surface of the particle is hydrophobic and comprises surface exposed tosyl groups.

In some embodiments, the particle is non-hydrophobic. In certain embodiments, the particle is hydrophilic. In some embodiments, the surface of the particle is non-hydrophobic. In some embodiments, the surface of the particle is hydrophilic.

### 4. Conjugated Particles

In some embodiments, a binding molecule (e.g., a polypeptide antigen or antibody) is attached to a particle, e.g., a bead particle. In certain embodiments, the polypeptide is covalently attached to the particle. In some embodiments, the polypeptide is non-covalently attached to the particle. In some embodiments, at least 1 binding molecule, at least 10 binding molecules, at least 10² binding molecules, at least 10³ binding molecules, at least 10⁴ binding molecules, at least 10⁵ binding molecules, at least 10⁶ binding molecules, at least 10⁷ binding molecules, at least 10⁸ binding molecules, or at least 10⁹ binding molecules are attached to each particle. In particular embodiments, between or between about 10² binding molecules and 10⁹ binding molecules, t 10² binding molecules and 10⁷ binding molecules, 10³ binding molecules and 10⁹ binding molecules, 10³ binding molecules and 10⁸ binding molecules, or 10³ binding molecules and 10⁶ binding molecules are covalently attached to each particle, each inclusive. In particular embodiments, between or between about 10³ binding molecules and 10⁶ binding molecules, inclusive, are covalently attached to each particle. In particular embodiments, between or between about 10⁴ binding molecules and 10⁶ binding molecules, inclusive, are covalently attached to each particle. In certain embodiments, between or between about 10⁴ binding molecules and 10⁵ binding molecules are covalently attached to each particle. In some embodiments, between about 10⁵ binding molecules and 10⁶ binding, inclusive molecules are covalently attached to each particle.

In certain embodiments, the binding molecule is covalently attached to the particle, e.g., bead particle. In particular embodiments, an amount of at least 0.001 µg, at least 0.01 µg, at least 0. 1 µg, at least 0.5 µg, at least 1 µg, at least 1.5 µg, at least 2 µg, at least 2.5 µg, at least 3 µg, at least 3.5 µg, at least 4 µg, at least 4.5 µg, at least 5 µg, at least 6 µg, at least 7 µg, at least 8 µg, at least 9 µg, at least 10 µg, or at least 50 µg of the polypeptide binding molecules are covalently attached to the particles, e.g., beads, for every 10⁷ particles. In some embodiments, between or between about 0.001 µg and 100 µg, 0.01 µg and 50 µg, 0.1 µg and 10 µg, 0.5 µg and 10 µg, 0.1 µg and 1 µg, 1 µg and 10 µg, 0.5 µg and 5 µg, or 1 µg and 5 µg, each inclusive, of the polypeptide binding molecules are covalently attached to the particles, e.g., beads, for every 10⁷ particles. In particular embodiments, between or between about 1 µg and about 10 µg, inclusive, of the polypeptide binding molecules are covalently attached to the particles, e.g., beads, for every 10⁷ particles.

In certain embodiments, the binding molecule is bound or attached to the particle, e.g., bead particle. In particular embodiments, an average amount of, of about, or of at least 0.0001 pg, 0.001 pg, 0.005 pg, 0.01 pg, 0.02 pg, 0.03 pg, 0.04 pg, 0.05 pg, 0.06 pg, 0.07 pg, 0.08 pg, 0.09 pg, 0.1 pg, 0.2 pg, 0.3 pg, 0.4 pg, 0.5 pg, 0.6 pg, 0.7 pg, 0.8 pg, 0.9 pg, 1.0 pg, or 5.0 pg of the polypeptide binding molecules are bound or attached to the each particle. In some embodiments, between or between about 0.0001 pg and about 10 pg, 0.001 pg and 1 pg, 0.001 pg and 1 pg, 0.001 µg and 0.1 pg, 0.01 pg and 0.1 pg, 1 pg and 10 pg, 0.5 pg and 5 pg, or b 1 pg and 5 pg, each inclusive, of the binding molecules are bound or attached to the particle. In particular embodiments, an average amount of equal to or less than 0.0001 pg, 0.001 pg, 0.005 pg, 0.01 pg, 0.02 pg, 0.03 pg, 0.04 pg, 0.05 pg, 0.06 pg, 0.07 pg, 0.08 pg, 0.09 pg, 0.1 pg, 0.2 pg, 0.3 pg, 0.4 pg, 0.5 pg, 0.6 pg, 0.7 pg, 0.8 pg, 0.9 pg, 1.0 pg, or 5.0 pg of the polypeptide binding molecules are bound or attached to the each particle. In particular embodiments, the particle has a diameter of about 2.8 µm and between or between about .001 and 0.1 pg, inclusive, of the binding molecules are bound or attached to the particle. In particular embodiments, the particle has a diameter of about 4.5 µm and between or between about .01 and about 1 pg, inclusive, of the binding molecules are bound or attached to the particle.

In particular embodiments, the binding molecule is bound or attached to the particle, e.g., bead particle, in an amount of at least 10⁻¹⁶ mol, at least 10⁻¹⁵ mol, at least 10⁻¹⁴ mol, at least 10⁻¹³ mol, at least 10⁻¹² mol, at least 10⁻¹¹ mol, at least 10⁻¹⁰ mol, at least 10⁻⁹ mol, at least 10⁻⁸ mol, at least 10⁻⁷ mol, at least 10⁻⁶ mol, at least 10⁻⁵ mol, at least 10⁻⁴ mol, at least 10⁻³ mol, at least 10⁻² mol, at least 10⁻¹ mol, or at least 1 mol for every 10⁷ particles. In some embodiments, between or between about 1 × 10⁻¹³ mol and 1 × 10⁻⁹ mol, 1 × 10⁻¹² mol and 1 × 10⁻⁹ mol, 1 × 10⁻¹³ mol and 1 × 10⁻¹⁰ mol, or1 × 10⁻¹² mol and 1 × 10⁻⁹ mol, each inclusive, are bound or attached to the particles for every 10⁷ particles.

In some embodiments, the binding molecule is bound or attached to the particle, e.g., bead particle, in an amount of at least 10⁻²⁰ mol, at least 10⁻¹⁹ mol, at least 10⁻¹⁸ mol, at least 10⁻¹⁷ mol, at least 10⁻¹⁶ mol, at least 10⁻¹⁵ mol, at least 10⁻¹⁴ mol, at least 10⁻¹³ mol, at least 10⁻¹² mol, at least 10⁻¹¹ mol, or at least 10⁻¹⁰ mol to each particle. In some embodiments, between or between about 10⁻²¹ mol and 10⁻¹⁰ mol, 10⁻²⁰ mol and 10⁻¹⁸ mol, 10⁻¹⁹ mol and 10⁻¹⁷ mol, or 10⁻²¹ mol and 10⁻¹⁹ mol, each inclusive, are covalently attached to the particles, e.g., beads, for each particle. In some embodiments, the particles, e.g., beads, have a diameter of about 2.8 µm and the binding molecule is bound or attached to the particle in an amount of between or between about 10⁻²⁰ mol and 10⁻¹⁸ mol, inclusive. In certain embodiments, the particles, e.g., beads, have a diameter about 4.5 µm and the binding molecule is bound or attached to the particle in an amount of between or between about 10⁻¹⁹ mol and 10⁻¹⁷ mol, inclusive.

In certain embodiments, the binding molecule is incubated with particles, e.g., beads, e.g., tosylactivated beads, to attach and/or conjugate the binding molecules to the particles, e.g., beads. In particular embodiments, the binding molecules and the particles, e.g., beads, are incubated at a concentration of or of about 1 µg, 2 µg, 2.5 µg, 5 µg, 10 µg, 20 µg, 25 µg, 30 µg, 40 µg, 50 µg, 60 µg, 70 µg, 75 µg, 100 µg, 125 µg, 150 µg, 175 µg, or 200 µg of binding molecules per between 1×10⁸ and 1×10¹⁰ particles, inclusive, and the binding molecules are attached and/or conjugated to the particles, e.g., beads, with an efficiency of or of about or greater than 50%, 60%, 70%, 80%, 90%, 95%, 97%, 98%, 99%, 99.9% or of or of about 100%. In some embodiments, the binding molecules and the particles, e.g., beads, are incubated at a concentration of or of about 1 µg, 2 µg, 2.5 µg, 5 µg, 10 µg, 20 µg, 25 µg, 30 µg, 40 µg, 50 µg, 60 µg, 70 µg, 75 µg, 100 µg, 125 µg, 150 µg, 175 µg, or 200 µg of binding molecules per between 4×10⁸ and 5×10⁸ particles, inclusive, and the binding molecules are attached and/or conjugated to the particles, e.g., beads, with an efficiency of or of about or greater than 50%, 60%, 70%, 80%, 90%, 95%, 97%, 98%, 99%, 99.9% or of or of about 100%. In particular embodiments, the binding molecules and the particles, e.g., beads, are incubated at a concentration of or of about 1 µg, 2 µg, 2.5 µg, 5 µg, 10 µg, 20 µg, 25 µg, 30 µg, 40 µg, 50 µg, 60 µg, 70 µg, 75 µg, 100 µg, 125 µg, 150 µg, 175 µg, or 200 µg of binding molecules per between 3.5×10⁹ and 4.5×10⁹ particles, inclusive, and the binding molecules are attached and/or conjugated to the particles with an efficiency of or of about or greater than 50%, 60%, 70%, 80%, 90%, 95%, 97%, 98%, 99%, 99.9% or of or of about 100%. In particular embodiments, between 1 µg and 5 µg, between 5 µg and 25 µg, between 1 µg and 50 µg, between 10 µg and 50 µg, 0.1 µg and 10 µg, or 50 µg and 200 µg, each inclusive, of the binding molecule are incubated with between or between about 4×10⁸ and 5×10⁸ particles or between 3.5×10⁹ and 4.5×10⁹ particles, each inclusive, and the binding molecules are attached and/or conjugated to the particles, e.g., beads, with an efficiency of or of about or greater than 50%, 60%, 70%, 80%, 90%, 95%, 97%, 98%, 99%, 99.9% or of or of about 100%.

In some embodiments, the average amount of binding molecule conjugated or attached to the particle is, is about, or is at least 2×10⁻¹⁶ g/particle,1×10⁻¹⁵ g/particle, 2×10⁻¹⁵ g/particle, 5×10⁻¹⁵ g/particle, 1×10⁻¹⁴ g/particle,1×10⁻¹⁴ g/particle, 5×10⁻¹⁴ g/particle, 1×10⁻¹³ g/particle, 2×10⁻¹³ g/particle, 5×10⁻¹³ g/particle, 1×10⁻¹³ g/particle, and 2×10⁻¹³ g/particle ±50%, ±40%, ±30%, ±25%, ±20%, ±10%, ±5%, or ±1%. In certain embodiments, the average amount of binding molecule conjugated or attached to the particle is or is about 2×10⁻¹⁶ g/particle ±50%, ±40%, ±30%, ±25%, ±20%, ±10%, ±5%, or ±1%. In particular embodiments, the average amount of binding molecule conjugated or attached to the particle is or is about 1×10⁻¹⁵ g/particle ±50%, ±40%, ±30%, ±25%, ±20%, ±10%, ±5%, or ±1%. In certain embodiments, the average amount of binding molecule conjugated or attached to the particle is or is about 2×10⁻¹⁵ g/particle ±50%, ±40%, ±30%, ±25%, ±20%, ±10%, ±5%, or ±1%. In particular embodiments, the average amount of binding molecule conjugated or attached to the particle is or is about 1×10⁻¹⁴ g/particle ±50%, ±40%, ±30%, ±25%, ±20%, ± 10%, ±5%, or ±1%. In certain embodiments, the average amount of binding molecule conjugated or attached to the particle is equal to or less than 5×10⁻¹³ g/particle, 2×10⁻¹³ g/particle, 1×10⁻¹³ g/particle, 5×10⁻¹⁴ g/particle, 2×10⁻¹⁴ g/particle, 1×10⁻¹⁴ g/particle, 5×10⁻¹⁵ g/particle, 2×10⁻¹⁵ g/particle, 1×10⁻¹⁵ g/particle, or 2×10⁻¹⁶ g/particle.

In some embodiments, the particles, e.g., beads, comprise binding molecules that are anti-idiotypic antibodies, or active fragments thereof, that bind to the antigen-binding domain of a CAR. In certain embodiments, the anti-idiotype anti-CAR antibody, or active fragment thereof, is bound to a surface exposed tosyl group of the particle at a site (e.g., a side chain amino group or a sulfhydryl group of an amino acid) located within the Fc domain of the antibody. In particular embodiments, the particles, e.g., beads, are monodisperse and superparamagnetic. In particular embodiments, the particles, e.g., beads, and have a diameter of about 2.8 µm, between about 10⁴ and about 10⁶ copies, inclusive, of the binding molecule, i.e., an anti-idiotypic anti-CAR antibody, per particle. In some embodiments, the particles, e.g., beads, have a diameter of about 4.5 µm, and comprise about between about 5×10⁵ and about 5×10⁶ copies, inclusive, of the anti-idiotypic anti-CAR antibody per particle.

In certain embodiments, the particles, e.g., beads, comprise a binding molecule that is or contains BCMA polypeptide antigen. In particular embodiments, the binding molecule comprises a human BCMA extracellular domain and an Fc domain, which, in some cases, is a C-terminal Fc domain. In particular embodiments, the particles, e.g., beads, have a diameter of about 280 µm, and comprise about 10⁵ copies of the binding molecule, e.g., the BCMA fusion polypeptide, per particle. In certain embodiments, the BCMA fusion polypeptide is bound to a surface exposed tosyl group of the particle at a site (e.g., a side chain amino group or a sulfhydryl group of an amino acid) located within the Fc domain of the fusion polypeptide. In particular embodiments, the particles, e.g., beads, are monodisperse and superparamagnetic. In particular embodiments, the particles, e.g., beads, and have a diameter of about 2.8 µm, between about 10⁴ and about 10⁶ copies, inclusive, of the binding molecule, i.e., a BCMA fusion polypeptide, per particle. In some embodiments, the particles, e.g., beads, have a diameter of about 4.5 µm, and comprise between or between about 5 × 10⁵ and 5×10⁶ copies, inclusive, of the BCMA fusion polypeptide per particle.

### II. EX VIVO STIMULATION OR EXPANSION OF CELLS

Provided herein is a method of stimulating or expanding cells that express a recombinant receptor, e.g., a CAR, comprising incubating an input composition comprising cells expressing a CAR with particles, e.g., beads, that comprise a binding molecule that specifically binds or recognizes the recombinant receptor, e.g. bead-conjugated reagents as provided, such as anti-ID conjugated beads or BCMA-conjugated beads. In some embodiments, binding between the binding molecule and the recombinant receptor, e.g., a CAR, induces expansion of the cells expressing the CAR, thereby producing an output composition comprising expanded cells. In particular embodiments, the recombinant receptor is a CAR. In certain embodiments, the binding molecule is an anti-idiotype antibody or antigen-binding fragment thereof that binds to the antigen-binding fragment of the recombinant receptor. In certain embodiments, the binding molecule is an antigen that binds to or is recognized by the CAR.

In some embodiments, particles, e.g., beads, comprising a binding molecule are contacted to or incubated with an input composition comprising one or more cells to generate an output composition. In certain embodiments, the input composition or input cells refer to a composition and/or a plurality of cells desired to be treated, incubated, or contacted under conditions that will stimulate, activate, cause, generate, and/or produce one or more changes to at least a portion of the cells of the input composition, thereby converting the input composition into an output composition. In some embodiments, the input cells are a composition of immune cells, for example, a composition of T cells that contain cells expressing a recombinant receptor, e.g., a CAR. In particular embodiments, at least a portion of the cells in the input composition are activated, expanded, and/or enriched in the generated output composition by practice of the provided methods.

In certain embodiments, the particles, e.g., beads, comprising a binding molecule are contacted to or incubated with cells from an input composition to generate an output composition by expanding, enriching, and/or activating at least a portion, e.g., a subgroup or a fraction, of the input cells. In particular embodiments, the cells from the input composition comprise at least a portion of cells that express a recombinant receptor, e.g., a CAR, and/or at least a portion of cells that contain a heterologous nucleic acid molecule that encodes a recombinant receptor. In some embodiments, the input composition comprises cells that are to be treated, contacted, or incubated with a particle comprising a binding molecule, wherein the treatment, contact, or incubation with the agent will alter at least a portion of the cells of the input composition, thereby generating an output composition. Such alterations may include, but are not limited to, expanding and/or enriching a portion of cells, activation of at least a portion of the cells, and/or increasing or decreasing the expression of at least one gene of at least a portion of the cells. In certain embodiments, the output composition comprises at least a portion of cells from the input composition that has undergone an alteration following the alteration by the treatment, contact or incubation with the agent.

In some embodiments, the expansion, enrichment, stimulation, and/or activation achieved by incubating cells with particles, e.g., beads, comprising a binding molecule may be titrated, adjusted, and/or controlled by selecting particles, e.g., beads, with a particular number, level, or amount of binding molecules that are conjugated or otherwise attached to each particle. In particular embodiments, increasing the number of binding molecules conjugated or otherwise attached to each particle increases the expansion, enrichment, stimulation, and/or activation achieved by incubating the cells with the particles, e.g., beads. In certain embodiments, decreasing the number binding molecules conjugated or otherwise attached to each particle decreases the expansion, enrichment, stimulation, and/or activation achieved by incubating the cells with the particles, e.g., beads. In particular embodiments, expansion, enrichment, stimulation, and/or activation is measured by any suitable known means. In particular embodiments, an increase in expansion, enrichment, stimulation, and/or activation is a statistically significant increase and/or at least or at least about a 1%, 5%, 10%, 20%, 30%, 40%, 50%, 60%, 70%, 80%, 90%, 100%, 1-fold, 2-fold, 3-fold, 5-fold, 10-fold, 100-fold increase, e.g., as compared to a control and/or incubation under different conditions, of a measurement associated with expansion, enrichment, stimulation, and/or activation taken of the cells during or following incubation with the particles, e.g., beads. In certain embodiments, a decrease in expansion, enrichment, stimulation, and/or activation is a statistically significant decrease and/or at least or at least about a 1%, 5%, 10%, 20%, 30%, 40%, 50%, 60%, 70%, 80%, 90%, 95%, 99% or 99.9% decrease of a measurement associated with expansion, enrichment, stimulation, and/or activation taken of the cells during or following incubation with the particles, e.g., beads.

In some embodiments, the input composition comprises eukaryotic cells, such as mammalian cells. In certain embodiments, the input composition comprises human cells. In some embodiments, the input composition comprises cells that are derived from the blood, bone marrow, lymph, or lymphoid organs. In particular embodiments, the input composition comprises cells of the immune system, i.e., cells of innate or adaptive immunity, e.g., myeloid or lymphoid cells, including lymphocytes, typically T cells and/or NK cells. In some embodiments, the input composition comprises stem cells, such as multipotent and pluripotent stem cells, including induced pluripotent stem cells (iPSCs). In particular embodiments, the input composition comprises CD3⁺ cells. In certain embodiments, the output composition comprises CD4⁺ cells. In some embodiments, the input composition comprises CD8⁺ cells. In some embodiments, the input composition comprises CD3+ cells that also express low levels or no CD28.

In some embodiments, the binding molecule stimulates activation and/or expansion of cells expressing low levels of CD28 or cells that are CD28 negative. In certain embodiments, the cells are not contacted with anti-CD3/anti-CD28 conjugated reagents prior to contacting the cells with the binding molecule.

In some embodiments, the methods and particles, e.g., beads, comprising a binding molecule (e.g. e.g. bead-conjugated reagents as provided, such as anti-ID conjugated beads or BCMA-conjugated beads) are capable of stimulating T cells deficient in or that have downregulated one or more natural signaling molecules such as one or more costimulatory receptors or antigen receptors or cytokine receptors but that express the chimeric receptor, e.g., the CAR, recognized by the binding molecule of the provided bead-conjugated reagents. In some embodiments, cells of the input composition are low or negative for surface expression of CD28 or other costimulatory molecule or other signaling molecule. Thus in some embodiments, the provided reagents and methods have certain advantages compared to certain other activation or stimulatory agents or methods that which may require or depend upon surface expression of CD28 or other endogenous signaling molecule, to provide the desired signal and/or the full extent of such signal, e.g., to provide costimulatory signal and/or to achieve full activation. In some embodiments, the provided agents and methods are advantageous in such regards compared to anti-CD3/anti-CD28 reagents (e.g. beads); in some aspects, the provided particles, e.g., beads, comprising a binding molecule (e.g. e.g. bead-conjugated reagents as provided, such as anti-ID conjugated beads or BCMA-conjugated beads) are advantageous in being able to stimulate or achieve a desired effect such as activation or proliferation of cells that are low or negative for CD28 or other natural signaling molecule. In some aspects, signaling through the CAR by stimulation with an anti-ID antibody results in both a primary and secondary (costimulatory) signal via the CAR using only the single reagent. In some embodiments, the input composition comprises CD3+ cells that express low levels of CD28 or other endogenous signaling molecule. In some embodiments, the input composition comprises CD3+ cells that are CD28 negative or are negative for other endogenous signaling molecule. In some embodiments, particles, e.g., beads, comprising a binding molecule (e.g. e.g. bead-conjugated reagents as provided, such as anti-ID conjugated beads or BCMA-conjugated beads) stimulate activation and/or expansion of cells expressing low levels of CD28 or cells that are CD28 negative.

In particular embodiments, the input composition is or includes primary human T cells. In some embodiments, the input composition is or includes enriched CD4+ T cells. In some embodiments, the input composition is or includes enriched CD8+ T cells. In certain embodiments, the input composition comprises CD4⁺ cells and CD8⁺ cells. In particular embodiments, the ratio of the CD4⁺ cells to the CD8⁺ cells in the input composition is greater than about 50:1, or between or between about 25:1 and 50:1, 10: and 25:1, 5:1 and 10:1, 3:1 and 5:1, 2:1 and 3:1, 1:1 and 2:1, 1:1 and 1:2, 2:1 and 1:2, 1:2 and 1:3, 1:3 and 1:5, 1:5 and 1:10, 1:10 and 1:25, or 1:25 and 1:50, each inclusive.

In some embodiments, an input composition comprises a population of cells that have been transduced or transfected, or cells that are derived from cells that have been transduced or transfected, with one or more nucleic acids encoding a recombinant receptor, e.g., a CAR, that is bound by or recognized by the binding molecule of the particles, e.g., beads. In some embodiments, the input composition comprises a population of cells that have been transduced or transfected, or cells that are derived from cells that have been transduced or transfected with one or more nucleic acids encoding a recombinant receptor, that is bound by or recognized by the binding molecule. In particular embodiments, the cells from the input composition have been transfected or transduced by any method as described herein, e.g., in Section III. In certain embodiments, the one or more nucleic acids have been transfected into the cell with a virus as described herein, e.g., in Section III. In some embodiments, the one or more nucleic acids have been transfected into the cell with a non-viral plasmid, e.g., an episome or a transposon, as described herein, e.g., in Section III. In particular embodiments, an input composition comprising cells that express a recombinant receptor, e.g., a CAR, at the cell surface that is bound by or recognized by the binding molecule of the particles, e.g., beads.

In particular embodiments, the input composition comprises a cell that expresses a recombinant receptor, e.g., a CAR, such as one that is derived from any of the methods described herein, e.g., in Section III.

In particular embodiments, an input composition does not initially comprise cells that express a recombinant receptor e.g., a CAR. In some embodiments, the cells of an input composition that do not initially comprise cells that express an recombinant receptor are contacted, incubated or treated with particles, e.g., beads, comprising a binding molecule at least during a portion of a transfection or transduction of the cells with one or more nucleic acids that comprises a gene expressing recombinant receptor that is bound by or recognized by the binding molecule. In some embodiments, the transfection or transduction is performed as described herein, e.g., in Section III. In some embodiments, during at least a portion of the incubation with the binding molecule, upon introduction of the nucleic acid into cells, the recombinant receptor becomes expressed on the surface of the cells where it is able to interact with the binding molecule. In particular embodiments, the transfection or transduction is performed without any prior expansion, activation, and/or isolation steps. In certain embodiments, the cells are not incubated, treated, or contacted, with one or more polyclonal stimulatory molecules capable of activating one or more intracellular signaling domains of one or more components of a TCR complex prior to the transfection or transduction. In particular embodiments, the cells are not contacted with one or more polyclonal stimulatory molecules that are anti-CD3 and anti-CD28 antibodies prior to transduction or transfection.

In certain embodiments, the input composition comprises both cells that do and cells that do not express the recombinant receptor, e.g., a CAR, that is bound by or recognized by the binding molecule. In some embodiments, treating, contacting, or incubating the cells of the input composition with the particles, e.g., beads, comprising the binding molecule will stimulate activation and/or expansion of cells expressing the recombinant receptor, e.g., a CAR, but will not stimulate activation and/or expansion of cells that do not express the recombinant receptor. In certain embodiments, treating, contacting, or incubating the cells of the input composition with the particles, e.g., beads, comprising the binding molecule that binds to or recognizes the recombinant receptor will stimulate activation and/or expansion of cells that do not express the recombinant receptor to a lesser degree than cells that express the recombinant receptor.

In particular embodiments a portion of the cells of the input composition express, or contain heterologous DNA that encodes, a recombinant receptor, e.g., a CAR. In certain embodiments, less than 0.01%, less than 0.1%, less than 1%, less than 5%, less than 10%, less than 20%, less than 25%, less than 30%, less than 35%, less than 40%, less than 45%, less than 50%, less than 55%, less than 60%, less than 65%, less than 70%, less than 80%, or less than 90% of the cells express, or contain one or more nucleic acids that contain a gene encoding, a recombinant receptor. Cells that express the recombinant receptor or comprises heterologous DNA that encodes a recombinant receptor may be identified by any means known in the art, for example but not limited to detection of an mRNA encoding the recombinant receptor in the cell, directly detecting the heterologous receptor, e.g., by detecting binding of a labeled probe or antibody that binds to the recombinant receptor, or by detection of a surrogate marker that is encoded by the heterologous DNA. In some embodiments, cells that express the recombinant receptor identified by detecting a surrogate marker. In certain embodiments, the surrogate marker is a truncated EGFR polypeptide.

In particular embodiments, the input cells are or include cells that were transfected and/or transduced with a low amount of viral particles, e.g., beads, ratio of copies of the viral vector particles, e.g., beads, to cells, and/or infectious units (IU), prior to contact with the particles, e.g., beads. In particular embodiments, the input cells, e.g., cells that are contacted, incubated, and/or treated with particles, e.g., beads, containing a binding molecule, are cells that have been transduced with a lower amount of viral particles, ratio of copies of the viral vector particles, e.g., beads, to cells, and/or IU, than input cells that are expanded and/or enriched by polyclonal stimulation, e.g., particles coated with anti-CD3 and/or anti-CD28 antibody. For example, in some embodiments, the input composition that is incubated with the particles, e.g., beads, containing binding molecules is generated from cells that were transduced with or with at least 0.5, 1, 2, 3, 4, 5, 10, 15, 20, 30, 40, 50, or 60 fewer IU per cell than an input composition that is expanded and/or enriched by polyclonal stimulation. In some embodiments, the input composition that is incubated with the particles, e.g., beads, containing a binding molecule is generated from cells that were transduced with a titer of viral vector particles, e.g., beads, with or with at least 1 x 10⁵ IU/mL, 5 x 10⁵ IU/mL, 1 x 10⁶ IU/mL, 5 x 10⁶ IU/mL, 6 x 10⁶ IU/mL, 7 x 10⁶ IU/mL, 8 x 10⁶ IU/mL, 9 x 10⁶ IU/mL, or 1 x 10⁷ IU/mL less than the input composition that is expanded and/or enriched by polyclonal stimulation.

In particular embodiments, transducing cells with a high IU/cell will lead to high transduction efficiency but, in some embodiments, may also lead to transfected cells with a high vector copy number (VCN), which can present safety risks and may not meet regulatory standards. In particular embodiments, lowering the IU/cell that cells are transduced with will reduce transduction efficiency but will lower VCN. In particular embodiments, increasing the IU/cell that cells are transduced with will increase transduction efficiency but will also increase VCN.

In particular embodiments, the incubation, contacting, or treatment of cells from the input composition with the particles, e.g., beads, comprising a binding molecule is performed under conditions for stimulation, expansion, and/or activation of cells which conditions can include one or more of particular media, temperature, oxygen content, carbon dioxide content, time, agents, e.g., nutrients, amino acids, antibiotics, ions, and/or stimulatory factors, such as cytokines, chemokines, antigens, binding partners, fusion proteins, recombinant soluble receptors, and any other agents designed to activate the cells.

In some embodiments, the cells of the input composition do not express a recombinant receptor, e.g., a CAR, and are incubated, contacted, or treated with particles, e.g., beads, comprising a binding molecule while they are contacted with the one or more nucleic acids that comprise a gene encoding a recombinant receptor. In certain embodiments, the binding molecule of the particles, e.g., beads, binds to or recognizes the recombinant receptor that is encoded by the gene. In some embodiments, the one or more nucleic acids are delivered in a virus particle. In particular embodiments, the one or more nucleic acids are an episomal vector. In some embodiments, the one or more nucleic acids are a transposon. In certain embodiments, the cells of the input composition are treated, incubated, or contacted with the particles, e.g., beads, comprising a binding molecule at least portion of the time that the one or more nucleic acids contacts the cells of the input composition. In particular embodiments, the cells are contacted with the one or more nucleic acids as described herein, e.g., in Section III.

In some embodiments, the cells of the input composition have been transfected or transduced with one nucleic acid comprising a gene encoding a recombinant receptor, e.g., a CAR; the input composition comprises cells that express a recombinant receptor, e.g., a CAR; and the cells are contacted, incubated, or treated with particles, e.g., beads, comprising binding molecules that bind to or recognize the recombinant receptor. In some embodiments, the cells of the input composition are treated, incubated, or contacted with the particles, e.g., beads, comprising a binding molecule after the cells transduced or transfected. In some embodiments, the cells of the input composition are treated, incubated, or contacted with the particles, e.g., beads, comprising a binding molecule after the cells have been transduced or transfected with the one or more nucleic acids as described herein, e.g., in Section III. In particular embodiments, the cells of the input composition are treated, incubated, or contacted with the particles, e.g., beads, comprising a binding molecule immediately, within about 1 minute, within about 5 minutes, within about 30 minutes, within about 1 hour, within about 2 hours, within about 4 hours, within about 6 hours, within about 8 hours, within about 12 hours, within about 24 hours, within about 2 days, within about 3 days, within about 4 days, within about 5 days, within about 6 days, within about 1 week, within about 2 weeks, within about 3 weeks, within about 4 weeks, within about 5 weeks, or within about 6 weeks after the cells are contacted with the one or more nucleic acids.

In some embodiments, the cells of the input composition are transduced or transfected with the one or more nucleic acids that comprise a gene encoding a recombinant receptor, e.g., as described in Section III, and the cells are subsequently cryofrozen and stored for a time prior to thawing and expanding the cells. In some embodiments, cells of the input composition have been cryofrozen, and the cells are contacted, incubated, or treated with the particles, e.g., beads, comprising a binding molecule within or within about 1 minute, 5 minutes, 30 minutes, 1 hour, 2 hours, 4 hours, 6 hours, 8 hours, 12 hours, 24 hours, 2 days, 3 days, 4 days, 5 days, 6 days, 1 week, 2 weeks, 3 weeks, 4 weeks, 5 weeks, or t 6 weeks after the cells are thawed.

In some embodiments, the contacting, incubating or treating of cells of the input composition with the particles (e.g. beads) comprising a binding molecule is carried out in a container, e.g., a cell culture dish, a cell culture well, or a bag.

In some embodiments, the cells of the input composition are contacted, incubated, or treated with the particles, e.g., beads, at a ratio of total cells of the input composition to particles, e.g., beads, from between or between about 100:1 to 1:100, 50:1 to 1:50, 25:1 to 1:25, 10:1 to 1:10, 5:1 to 1:5, 3:1 to 1:3, or 2:1 to 1:2. In particular embodiments, the ratio is from or from about 1:0.1 to about 1:5. In some embodiments, the ratio of total cells of the input composition to particles, e.g., beads, is about 1:1.

In some embodiments, between or between about 10² and 10¹², 10³ and 10¹⁰, 10⁴ and 10⁹, 10³ and 10⁶, 10⁴ and 10⁸, 10² and 10⁴, 10³ and 10⁵, 10⁴ and 10⁶, 10⁵ and 10⁷, or 10⁶ and 10⁸ cells, each inclusive, from the input composition are contacted, incubated, or treated with the particles, e.g., beads, comprising a binding molecule. In certain embodiments, less than about 10² , or between or between about 10² and 10³, 10³ and 10⁴, 10⁴ and 10⁵, 10⁵ and 10⁶, 10⁶ and 10⁷, 10⁷ and 10⁸, 10⁸ and 10⁹,10⁹ and 10¹⁰, 10¹⁰ and 10¹¹, or 10¹¹ and 10¹² cells from the input composition, each inclusive, are contacted, incubated, or treated with the particles, e.g., beads, comprising a binding molecule.

In some embodiments, the incubation or contacting with cells of an input composition is carried out with a sufficient amount of particles, e.g., beads, comprising the binding molecule to permit binding of the binding molecule to one or more cells in the input composition and/or to induce the stimulation, activation and/or proliferation of one or more cells in the input composition. The particular number or ratio of particles, e.g., beads, to cells of the input composition can be empirically determined depending on the particular amount of binding molecule per bead, the particular antigen, the source of cells of the input composition and other factors within the level of a skilled artisan.

In some embodiments, the amount of particles, e.g., beads, added to cells of an input composition is an amount to provide between or between about 1 binding molecule and 10¹² binding molecules per cell of the input composition during the incubation or contacting, such as between or between about 10² binding molecules and 10¹⁰ binding molecules, 10³ binding molecules and 10⁸ binding molecules, 10⁴ binding molecules and 10⁶ binding molecules, 1 binding molecule and 10² binding molecules, 10² binding molecules and 10³ binding molecules, 10³ binding molecules and 10⁴ binding molecules, 10⁴ binding molecules and 10⁵ binding molecules, 10⁵ binding molecules and 10⁶ binding molecules, 10⁵ binding molecules and 10⁶ binding molecules, 10⁶ binding molecules and 10⁷ binding molecules, 10⁷ binding molecules and 10⁸ binding molecules, 10⁹ binding molecules and 10¹⁰ binding molecules, 10¹⁰ binding molecules and 10¹¹ binding molecules, or 10¹¹ binding molecules and 10¹² binding molecules per cell, each inclusive, in the input composition during the incubation or contacting. In some embodiments, the amount of particles, e.g., beads, added to the input composition is an amount to provide between about 10⁴ binding molecules and about 10⁶ binding molecules for each cell in the input composition during the incubation or contacting. In some embodiments, the amount of particles, e.g., beads, contains about 10⁵ binding molecules for each cell in the input composition during the incubation or contacting.

In some embodiments, the amount of the particles, e.g., beads, comprising the binding molecule added to the input composition during the incubation or contacting is an amount of particles, e.g., beads, that contains between or between about 0.0001 pg and about 10 pg, 0.001 pg and 1 pg, 0.001 pg and 1 pg, 0.001 pg and 0.1 pg, 0.005 pg and 0.05 pg, 0.005 pg and 0.02 pg, or 0.01 pg and 0.05 pg of binding molecules, each inclusive, for each cell in the in the input composition during the incubation or contacting. In some embodiments, the total amount of particles, e.g., beads, added to the cells of the input composition is an amount to provide between or between about 0.0001 µg and 10 µg, 0.001 µg and 1 µg, 0.001 µg and 1 µg, 0.001 µg and 0.1 µg, 0.005 µg and 0.05 µg, 0.005 µg and 0.02 µg, or 0.01 µg and 0.05 µg of binding molecules, each inclusive.

In some embodiments, the amount of particles, e.g., beads, comprising the binding molecule added to the input composition during the incubation or contacting is an amount of particles, e.g., beads, that contains at least 10⁻²⁰ mol, at least 10⁻¹⁹ mol, at least 10⁻¹⁸ mol, at least 10⁻¹⁷ mol, at least 10⁻¹⁶ mol, at least 10⁻¹⁵ mol, at least 10⁻¹⁴ mol, at least 10⁻¹³ mol, at least 10⁻¹² mol, at least 10⁻¹¹ mol, or at least 10⁻¹⁰ mol of binding molecules for each cell in the input composition during the incubation or contacting. In some embodiments, the amount contains between or between about 10⁻²¹ mol and 10⁻¹⁰ mol, 10⁻²⁰ mol and 10⁻¹⁸ mol, 10⁻¹⁹ mol and 10⁻¹⁷ mol, or 10⁻²¹ mol and 10⁻¹⁹ mol of binding molecules, each inclusive, for each cell in the input composition during the incubation or contacting. In some embodiments, the total amount of particles, e.g., beads, added to the cells of the input composition is an amount to provide at least 10⁻¹⁶ mol, at least 10⁻¹⁵ mol, at least 10⁻¹⁴ mol, at least 10⁻¹³ mol, at least 10⁻¹² mol, at least 10⁻¹¹ mol, at least 10⁻¹⁰ mol, at least 10⁻⁹ mol, at least 10⁻⁸ mol, at least 10⁻⁷ mol, at least 10⁻⁶ mol, at least 10⁻⁵ mol, at least 10⁻⁴ mol, at least 10⁻³ mol, at least 10⁻² mol, at least 10⁻¹ mol, or at least 1 mol of binding molecules.

In some embodiments, the contacting or incubation of the particles, e.g., beads, comprising a binding molecule with the input composition is carried out in a volume that is between or between about 0.01 mL and 100 mL, such as 0.01 mL and 50 mL, 0.01 mL and 25 mL, 0.01 mL and 10 mL, 0.01 mL and 5 mL, 0.01 mL and 1 mL, 0.01 mL and 0.5 mL, 0.01 mL and 0.1 mL, 0.01 mL and 0.05 mL, 0.05 mL and 100 mL, 0.05 mL and 50 mL, 0.05 mL and 25 mL, 0.05 mL and 10 mL, 0.05 mL and 5 mL, 0.05 mL and 1 mL, 0.05 mL and 0.5 mL, 0.05 mL and 0.1 mL, 0.1 mL and 100 mL, 0.1 mL and 50 mL, 0.1 mL and 25 mL, 0.1 mL and 10 mL, 0.1 mL and 5 mL, 0.1 mL and 1 mL, 0.1 mL and 0.5 mL, 0.5 mL and 100 mL, 0.5 mL and 50 mL, 0.5 mL and 25 mL, 0.5 mL and 10 mL, 0.5 mL and 5 mL, 0.5 mL and 1 mL, 1 mL and 100 mL, 1 mL and 50 mL, 1 mL and 25 mL, 1 mL and 10 mL, 1 mL and 5 mL, 5 mL and 100 mL, 5 mL and 50 mL, 5 mL and 25 mL, 5 mL and 10 mL, 10 mL and 100 mL, 10 mL and 50 mL, 10 mL and 25 mL, 25 mL and 100 mL, 25 mL and 50 mL or 50 mL and 100 mL, each inclusive. In some embodiments, the volume is provided by a solution, such as media or a pharmaceutically acceptable buffer.

In some embodiments, the particles, e.g., beads, comprising the binding molecule are from a composition of particles, e.g., beads, that have a concentration of binding molecules of at least 0.001 µg/ml, at least 0.01 µg/ml, at least 0.1 µg/ml, at least 0.5 µg/ml, at least 1 µg/ml, at least 1.5 µg/ml, at least 2 µg/ml, at least 3 µg/ml, at least 4 µg/ml, at least 5 µg/ml, at least 6 µg/ml, at least 7 µg/ml, at least 8 µg/ml, at least 9 µg/ml, at least 10 µg/ml, at least 50 µg/ml, at least 100 µg/ml, at least 500 µg/ml, at least 1 mg/ml, or at least 10 mg/ml. In some embodiments, the composition of particles contains between or between about 5×10⁷and 1×10¹⁰ particles or beads per ml, inclusive. In particular embodiments, the particles, e.g., beads, comprising the binding molecule are from a composition of particles, e.g., beads, that have a concentration of binding molecules of between or between about 0.001 µg/ml to 10 mg/ml, 0.001 µg/ml and 1 µg/ml, 0.001 µg/ml and 1 µg/ml, 0.001 µg/ml and 0.1 µg/ml, 0.01 µg/ml and 1 µg/ml, 1 µg/ml and 10 µg/ml, 0.5 µg/ml and 5 µg/ml, or 1 µg/ml and 5 µg/ml. In some embodiments, the composition of particles contains between or between about 5×10⁷and 1×10¹⁰, 1×10⁸ and 5×10⁹, or 4×10⁸ and 4×10⁹ particles or beads per ml, each inclusive.

In certain embodiments, the amount of particles, e.g., beads, comprising the binding molecule added to the input composition during the incubation or contacting is an amount of particles, e.g., beads, that provides a concentration of 10⁻¹³ M, at least 10⁻¹² M, at least 10⁻¹¹ M, at least 10⁻¹⁰ M, at least 10⁻⁹ M, at least 10⁻⁸ M, at least 10⁻⁷ M, at least 10⁻⁶ M, at least 10⁻⁵ M, at least 10⁻⁴ M, at least 10⁻³ M, at least 10⁻² M, at least 0.1 M, at least 1 M, or at least 10 M of binding molecule. In some embodiments, the amount of particles, e.g., beads, comprising the binding molecule added to the input composition during the incubation or contacting is an amount of particles, e.g., beads, that provides a concentration between or between about 10⁻¹⁰ M and 10⁻³ M, 10⁻⁹ M and 10⁻⁶ M, 10⁻¹⁰ M and 10⁻⁷ M, or 10⁻⁹ M and about 10⁻⁶ M, inclusive.

In particular embodiments, the cells from the input composition are contacted, incubated, or treated with the particles, e.g., beads, comprising the binding molecule for at least or at least about 2 hours, 4 hours, 6 hours, 12 hours, 24 hours, 2 days, 3 days, 4 days, 5 days, 6 days, 7 days, 8 days, 9 days, 10 days, 11 days, 12 days, 13 days, 14 days, 3 weeks, or 4 weeks. In particular embodiments, the cells from the input composition are contacted, incubated, or treated with the particles, e.g., beads, comprising the binding molecule for less than or less than about 30 minutes, 1 hour, 2 hours, 6 hours, 12 hours, 1 day, 2 days, 3 days, 4 days, 5 days, 6 days, or less than or about 12 days. In some embodiments, the cells from the input composition are contacted, incubated, or treated with the particles, e.g., beads, comprising the binding molecule for between or between about 1 day and about 14 days, 3 days and 7 days, or 4 days and 6 days, inclusive. In some embodiments, the cells from the input composition are contacted, incubated, or treated with the particles, e.g., beads, comprising the binding molecule for about 5 days. In certain embodiments, the particles, e.g., beads, comprising a binding molecule are contacted or incubated with cells from the input composition, e.g. comprising cells that express a recombinant receptor, e.g., a CAR, for an amount of time to expand one or more cells of the input composition, such as to expand cells of the input composition that express the recombinant receptor. In particular embodiments, the particles, e.g., beads, comprising a binding molecule are contacted or incubated with cells from the input composition, e.g. comprising cells that express a recombinant receptor, e.g., a CAR, for an amount of time to chronically input composition

In particular embodiments, cells from an input composition, e.g. comprising cells that express a recombinant receptor, e.g., a CAR, are incubated, contacted, or treated with particles, e.g., beads, comprising a binding molecule at temperatures greater than room temperature to expand the cells of the input composition that express the recombinant receptor. In some embodiments, the treatment, incubation, or contacting is performed at a temperature greater than about 25 °C, such as generally greater than or greater than about 32 °C, 35 °C or 37 °C. In some embodiments, the treatment, contacting, or incubation is performed at a temperature of at or about 37 °C ± 2 °C, such as at a temperature of at or about 37 °C.

In some embodiments, the cells of the input composition, e.g. comprising cells expressing a recombinant receptor, e.g., a CAR, are incubated, treated, or contacted with the particles, e.g., beads, containing the binding molecule and one or more additional agents (e.g., stimulatory and/or accessory agents), e.g., ligand, which is capable of activating an intracellular signaling domain of a TCR complex.

In some embodiments, the additional agent is attached to the surface of the particle.

In certain embodiments, the one or more additional agents are separate from the particles, e.g., beads. In some embodiments, the one or more additional agents are one or more cytokines. In particular embodiments, the one or more additional agents are or include one or more recombinant cytokines. In some embodiments, the one or more cytokines are human recombinant cytokines. In certain embodiments, the one or more cytokines bind to and/or are capable of binding to receptors that are expressed by and/or are endogenous to T cells. In particular embodiments, the one or more cytokines are or include one or more members of the 4-alpha-helix bundle family of cytokines. In some embodiments, the one or more cytokines may include, but are not limited to, interleukin-2 (IL-2), interleukin-4 (IL-4), interleukin-7 (IL-7), interleukin-9 (IL-9), interleukin 12 (IL-12), interleukin 15 (IL-15), granulocyte colony-stimulating factor (G-CSF), and granulocyte-macrophage colony-stimulating factor (GM-CSF). In some embodiments, the one or more cytokines is or includes one or more of IL-2, IL-7, and IL-15.

In some embodiments, at least a portion of the cells of the input composition express a CAR, and the cells are incubated, treated, or contacted with particles, e.g., beads, with attached binding molecules, wherein the binding molecules are anti-idiotypic antibodies, or active fragments thereof, that bind to the antigen-binding domain of the CAR. In some embodiments, the cells are incubated for between about 3-7 days, inclusive. In particular embodiments, the particles, e.g., beads, have a diameter of about 280 µm, the particles, e.g., beads, comprise about 10⁵ copies of the binding molecules, i.e., an anti-idiotypic anti-CAR antibody, per particle, and cells are incubated, contacted, and/or treated with the cells at a ratio of about 1: 1 particles, e.g., beads, to cells. In some embodiments, the particles, e.g., beads, have a diameter of about 450 µm, the particles, e.g., beads, comprise about between about 1×10⁶ and about 2×10⁶ copies of the anti-idiotypic anti-CAR antibody, per particle, and cells are incubated, contacted, and/or treated with the cells at a ratio of about 1:1 particles, e.g., beads, to cells.

Particular embodiments are drawn to a method of expanding cells, comprising incubating an input composition comprising cells expressing a CAR with an antigen-binding domain that specifically binds or recognizes BCMA with particles, e.g., beads, that comprise a binding molecule that bind to or recognize the CAR. In some embodiments, the binding molecule is a fusion polypeptide comprising a human BCMA extracellular domain and a C-terminal Fc domain. In some embodiments, the cells are incubated for between about 3-7 days. In particular embodiments, the particles, e.g., beads, have a diameter of about 280 µm, the particles, e.g., beads, comprise about 10⁵ copies of the binding molecules, i.e., BCMA fusion polypeptides, per particle, and cells are incubated, contacted, and/or treated with the cells at a ratio of about 1: 1 particles, e.g., beads, to cells. In some embodiments, the particles, e.g., beads, have a diameter of about 450 µm, the particles, e.g., beads, comprise about between about 1×10⁶ and about 2×10⁶ copies of the binding molecules, i.e., BCMA fusion polypeptides, per particle, and cells are incubated, contacted, and/or treated with the cells at a ratio of about 1: 1 particles, e.g., beads, to cells.

In particular embodiments, the cells from the input composition are contacted, incubated, or treated with the particles, e.g., beads, comprising the binding molecule for an extended period of time to carry out a long-term stimulation, e.g., longer than 10 days. In some embodiments, the cells expressing a recombinant receptor are incubated, such as are incubated continuosuly or without disruption, with the particles or beads containing the binding molecule for at least 10 days, 11 days, 12 days, 13 days, 14 days, 15 days or 16 days. In some aspects, the length of time chosen for the incubation is a time at which one or more functions or activities of cells of the composition exhibits features of chronically stimulated cells or cells having prolonged exposure to antigen at the termination or end of the incubation. In some embodiments, the cells expressing a recombinant receptor are incubated with the particles or beads to perform a long term stimulation method, such as to model features of chronically stimulated cells or cells having prolonged exposure to antigen.

### Exemplary Features of Output Compositions

In particular embodiments, contacting cells of the input composition with particles, e.g., beads, comprising a binding molecule (e.g. e.g. bead-conjugated reagents as provided, such as anti-ID conjugated beads or BCMA-conjugated beads) results in an output composition comprising at least one property e.g., total cell number, the portion or subset of cells that express a recombinant receptor, rate of cell proliferation, and/or expression of a gene such as an activation marker or exhaustion marker, that is different from a corresponding cell of an input composition. In some embodiments, the methods result in proliferation, activation, stimulation, cytokine release, or other functional outcome such as upregulation of an activation marker or cytokine release or production, of cells expressing the chimeric receptor such as the CAR recognized by the binding molecule. In some aspects, such proliferation or other functional response or readout is induced in such cells to a degree that is similar to or greater than that induced by incubation of the cells with an agent and/or conditions that stimulates proliferation of T cells, such as anti-CD3/CD28 beads and/or crosslinked anti-CD3.

In some embodiments, the particles, e.g., beads, comprising a binding molecule are removed from the output composition. In some embodiments, the particles, e.g., beads, are magnetizable or magnetically responsive e.g., paramagnetic or superparamagnetic particles, e.g., beads. In some embodiments, the particles, e.g., beads, are streptavidin oligomers. Methods for removing particles, e.g., beads, (e.g., bead particles, e.g., beads, or magnetizable particles) from cells are known. In some embodiments, the use of competing non-labeled antibodies can be used, which, for example, bind to the binding molecule and alter its affinity for its recombinant receptor on the cell, thereby permitting for gentle detachment of the binding molecule and, therefore, the particle. In some cases, after detachment, the competing antibodies may remain associated with binding molecule of the particle while the unreacted unlabeled antibody is or may be washed away, and the cell is free of antibody and the binding molecule. Exemplary of such a reagent is DETACaBEAD (Friedl et al. 1995; Entschladen et al. 1997). In some embodiments, particles (e.g., bead particles) can be removed in the presence of a cleavable linker (e.g., DNA linker), whereby the particle-bound antibodies are conjugated to the linker (e.g., CELLection, Dynal). In some cases, the linker region provides a cleavable site to remove the particles (e.g., bead particles) from the cells after isolation, for example, by the addition of DNase or other releasing buffer. In some embodiments, other enzymatic methods can also be employed for release of a particle (e.g., bead particle) from cells. In some embodiments, the particles (e.g., bead particles or magnetizable particles) are biodegradable.

In some embodiments, the cells from the output composition that were incubated, contacted, or treated with the particles, e.g., beads, comprising the binding molecule are compared to an output composition that was derived from an identical input composition, i.e., an input composition with the same cellular makeup, that was incubated, treated, or contacted, with one or more polyclonal stimulatory molecules capable of activating one or more intracellular signaling domains of one or more components of a TCR complex. In certain embodiments, the identical input composition was not treated with particles, e.g., beads, comprising binding molecules. In some embodiments, the cells from the identical input composition were contacted, incubated, or treated with the one or more polyclonal stimulatory molecules under the same conditions (e.g., same media, same temperature, same duration, and the same amount and/or concentration of the one or more polyclonal stimulatory molecules as the amount and/or concentration of the binding molecules) as the cells from the input composition that were treated with the particles, e.g., beads, comprising the binding molecule. In some embodiments, the cells of the identical input composition were contacted with one or more polyclonal stimulatory molecules that are attached to a surface. In certain embodiments, the cells of the identical input composition were contacted with one or more polyclonal stimulatory molecules that are attached to a surface of a particle (e.g., a bead particle). In particular embodiments, the cells of the identical input composition were contacted with one or more polyclonal stimulatory molecules that are anti-CD3 and anti-CD28 antibodies.

In particular embodiments, the number of cells in the output composition is at least about 1%, at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 95%, at least about 100%, at least about 2-fold, at least about 3-fold, at least about 4-fold, at least about 5-fold, at least about 6-fold, at least about 7-fold, at least about 8-fold, at least about 9-fold, at least about 10-fold, at least about 15-fold, at least about 20-fold, at least about 25-fold, at least about 50-fold, or at least about 100-fold greater than the number of cells in the input composition.

In certain embodiments, the output composition comprises a greater number of cells following the incubation, contacting, or treatment with the particles, e.g., beads, comprising the binding molecule than an output composition that was derived from an identical input composition, i.e., an input composition with the same cellular makeup, that was incubated, treated, or contacted, with one or more polyclonal stimulatory molecules capable of activating one or more intracellular signaling domains of one or more components of a TCR complex e.g., anti-CD3 and anti-CD28 antibodies. In some embodiments, the number of cells of the output composition that was treated with particles, e.g., beads, comprising the binding molecule is at least or at least about 1%, 5%, 10%, 15%, 20%, 25%, 30%, 35%, 40%, 45%, 50%, 55%, 60%, 65%, 70%, 75%, 80%, 85%, 95%, 100%, 2-fold, 3-fold, 4-fold,5-fold, 6-fold, 7-fold, 8-fold, 9-fold, 10-fold, 20-fold, 50-fold, or 100-fold greater than the number of cells from the output composition from the identical input composition that was incubated, treated, or contacted with the one or more polyclonal stimulatory molecules.

In particular embodiments, the cells of an input composition are treated, contacted, or incubated with particles, e.g., beads, comprising a binding molecule that binds to or is recognized by the recombinant receptor expressed by a subset of the cells of an input composition, thereby resulting in an output composition comprising a greater subset of cells expressing the recombinant receptor than in the input composition. In certain embodiments, the subset of cells expressing the recombinant receptor is at least or at least about 1%, 5%, 10%, 15%, 20%, 25%, 30%, 35%, 40%, 45%, 50%, 55%, 60%, 65%, 70%, 75%, 80%, 85%, 95%, 100%, 2-fold, 3-fold, 4-fold,5-fold, 6-fold, 7-fold, 8-fold, 9-fold, 10-fold, 20-fold, 50-fold, or 100-fold greater in the output composition than in the input composition.

In some embodiments, the output composition comprises a larger subset of cells expressing the recombinant receptor following incubation, contacting, or treatment with the particles, e.g., beads, comprising the binding molecule than the input composition prior to the incubation, contacting, or treatment with the particles, e.g., beads, comprising the binding molecule. In some embodiments, at least or at least about 1%, 5%, 10%, 15%, 20%, 25%, 30%, 35%, 40%, 45%,50%, 55%, 60%, 65%, 70%, 75%, 80%, 85%, 95%, 98%, 99%, or 99.9% of the cells of the output composition express the recombinant receptor.

In certain embodiments, the output composition comprises a larger subset of cells expressing the recombinant receptor that is bound by or recognized by the binding molecule, following the incubation, contacting, or treatment with the particles, e.g., beads, comprising the binding molecule than the subset of cells expressing the recombinant receptor in an output composition that was derived from an identical input composition, that was incubated, treated, or contacted with one or more polyclonal stimulatory molecules capable of activating one or more components of a TCR complex, e.g., anti-CD3 and anti-CD28 antibodies. In some embodiments, the subset of cells expressing the recombinant receptor in an output composition of cells that were treated with particles, e.g., beads, comprising the binding molecule is at least or at least about 1%, 5%, 10%, 15%, 20%, 25%, 30%, 35%, 40%, 45%, 50%, 55%, 60%, 65%, 70%, 75%, 80%, 85%, 95%, 100%, 2-fold, 3-fold, 4-fold,5-fold, 6-fold, 7-fold, 8-fold, 9-fold, 10-fold, 20-fold, 50-fold, or 100-fold larger than the subset of cells expressing the recombinant receptor from the output composition that was derived from the identical input composition that was incubated, treated, or contacted with the one or more polyclonal stimulatory molecules.

In some embodiments, the cells that express the recombinant receptor, e.g., the CAR, in the output composition that was incubated, treated, and/or contacted with the particles, e.g., beads, containing a binding molecule contain at least or at least about 1%, 5%, 10%, 15%, 20%, 25%, 30%, 35%, 40%, 45%,50%, 55%, 60%, 65%, 70%, 75%, 80%, 85%, 95%, 98%, 99%, or 99.9% lower VCN than cells of an output composition that received polyclonal stimulation, e.g., incubation with anti-CD3 and/or anti-CD28 antibodies. In some embodiments, the average VCN of CAR expressing cells of the output no more than at or about 10, 5, 4, 2.5, 1.5, or 1.

In some embodiments, the output composition comprises a greater subset of activated cells following incubation, contacting, or treatment with the particles, e.g., beads, comprising the binding molecule than the input composition prior to the incubation, contacting, or treatment with the particles, e.g., beads, comprising the binding molecule. In some embodiments, the activated cells are cells that express at least one activation marker, i.e., a polynucleotide or a polypeptide associated with activation. In some embodiments, the activated cells express at least one, at least two, at least three, at least four, at least five, at least six, at least seven, at least eight, at least nine, at least ten, or greater than ten different activation markers. In some embodiments, the activation marker is Human Leukocyte Antigen - antigen D Related (HLA-DR), CD25, CD69, CD71, CD40L or 4-1BB (CD137). In particular embodiments, the activation marker is IL-2, IFN gamma, or TNF-alpha. In some embodiments, the activation marker is intracellular expression of IL-2, IFN gamma, or TNF-alpha. In certain embodiments, the activation marker is secreted IL-2, IFN gamma, or TNF-alpha. In certain embodiments, the subset of cells of expressing the activation marker is at least or at least about 1%, 5%, 10%, 15%, 20%, 25%, 30%, 35%, 40%, 45%, 50%, 55%, 60%, 65%, 70%, 75%, 80%, 85%, 95%, 100%, 2-fold, 3-fold, 4-fold,5-fold, 6-fold, 7-fold, 8-fold, 9-fold, 10-fold, 20-fold, 50-fold, or 100-fold greater in the output composition than in the input composition.

In some embodiments, the output composition comprises a greater number of cell activated cells following incubation, contacting, or treatment with the particles, e.g., beads, comprising the binding molecule than the input composition prior to the incubation, contacting, or treatment with the particles, e.g., beads, comprising the binding molecule. In some embodiments, at least or at least about 1%, 5%, 10%, 15%, 20%, 25%, 30%, 35%, 40%, 45%,50%, 55%, 60%, 65%, 70%, 75%, 80%, 85%, 95%, 98%, 99%, or 99.9% of the cells of the output composition are activated. In some embodiments, less than or less than about 1%, 5%, 10%, 15%, 20%, 25%, 30%, 35%, 40%, 45%, 50%, 55%, 60%, 65%, or 70% of the cells are activated.

In some embodiments, the output composition comprises a smaller subset of activated cells following the incubation, contacting, or treatment with the particles, e.g., beads, comprising the binding molecule than an output composition that was derived from an identical input composition, i.e., an input composition with the same cellular makeup, that was incubated, treated, or contacted, with one or more polyclonal stimulatory molecules capable of activating one or more intracellular signaling domains of one or more components of a TCR complex. In some embodiments, the subset of activated cells of the output composition that was treated with particles, e.g., beads, comprising the binding molecule is less than or less than about 1%, 5%, 10%, 15%, 20%, l 25%, 30%, 35%, 40%, 45%, 50%, 55%, 60%, 65%, 70%, 75%, 80%, 85%, 95%, 99%,or 99.9% of the subset of activated cells of the output composition derived from the identical input composition that was incubated, treated, or contacted with the one or more polyclonal stimulatory molecules.

In particular embodiments, cells of the output composition are more activated following incubation, contacting, or treatment with the particles, e.g., beads, comprising the binding molecule than the input composition prior to the incubation, contacting, or treatment with the particles, e.g., beads, comprising the binding molecule. In some embodiments, the cells are more activated when they express a greater level of at least one activation marker than cells that are less activated or cells that are not activated. In some embodiments, the more activated cells express at least one, at least two, at least three, at least four, at least five, at least six, at least seven, at least eight, at least nine, at least ten, or greater than ten different activation markers at levels greater than cells that are less activated or not activated. In some embodiments, the activated cells express a greater amount of at least one of Human Leukocyte Antigen-antigen D Related (HLA-DR), CD25, CD69, CD71, CD40L or 4-1BB (CD137) than cells from the input composition. In particular embodiments, the activated cells express a greater level of IL-2, IFN gamma, or TNF-alpha, e.g., mRNA or polypeptides. In some embodiments, the activated cells express a greater amount of intracellular of IL-2, IFN gamma, or TNF-alpha. In certain embodiments, the activated cells secrete a greater amount of IL-2, IFN gamma, or TNF-alpha. In certain embodiments, the activated cells express an amount of an activation marker that is at least or at least about 1%, 5%, 10%, 15%, 20%, 25%, 30%, 35%, 40%, 45%, 50%, 55%, 60%, 65%, 70%, 75%, 80%, 85%, 95%, 100%, 2-fold, 3-fold, 4-fold,5-fold, 6-fold, 7-fold, 8-fold, 9-fold, 10-fold, 20-fold, 50-fold, or 100-fold greater than the amount of the activation marker expressed by cells from the input composition.

In some embodiments, the output composition comprises cells that are less activated following the incubation, contacting, or treatment with the particles, e.g., beads, comprising the binding molecule than an output composition that was derived from an identical input composition, i.e., an input composition with the same cellular makeup, that was incubated, treated, or contacted, with one or more polyclonal stimulatory molecules capable of activating one or more intracellular signaling domains of one or more components of a TCR complex. In some embodiments, the expression of at least one activation marker in the cells of the output composition that was treated with particles, e.g., beads, comprising the binding molecule is less than or less than about 1%, 5%, 10%, 15%, 20%, 1 25%, 30%, 35%, 40%, 45%, 50%, 55%, 60%, 65%, 70%, 75%, 80%, 85%, 95%, 99%, or 99.9% of the expression of at least one activation marker in cells from the output composition from the identical input composition that was incubated, treated, or contacted with the one or more polyclonal stimulatory molecules.

In some embodiments, a greater subset of cells expressing the recombinant receptor (that is recognized by the binding molecule) from the output composition are activated following incubation, contacting, or treatment with the particles, e.g., beads, comprising the binding molecule than the cells expressing the recombinant receptor of the input composition prior to the incubation, contacting, or treatment with the particles, e.g., beads, comprising the binding molecule. In certain embodiments, the subset of cells of expressing the recombinant receptor that are activated is at least or at least about 1%, 5%, 10%, 15%, 20%, 25%, 30%, 35%, 40%, 45%, 50%, 55%, 60%, 65%, 70%, 75%, 80%, 85%, 95%, 100%, 2-fold, 3-fold, 4-fold,5-fold, 6-fold, 7-fold, 8-fold, 9-fold, 10-fold, 20-fold, 50-fold, or 100-fold greater in the output composition than the subset of activated cells expressing the recombinant receptor from the input composition.

In some embodiments, the cells expressing the recombinant receptor (that is bound by or recognized by the binding molecule) of the output composition comprises a greater subset of activated cells following incubation, contacting, or treatment with the particles, e.g., beads, comprising the binding molecule than the input composition prior to the incubation, contacting, or treatment with the particles, e.g., beads, comprising the binding molecule. In some embodiments, at least or at least about 1%, 5%, 10%, 15%, 20%, 25%, 30%, 35%, 40%, 45%, 50%, 55%, 60%, 65%, 70%, 75%, 80%, 85%, 95%, 98%, 99%, or 99.9% of the cells expressing the recombinant receptor from the output composition are activated. In some embodiments, is less than or less than about 1%, 5%, 10%, 15%, 20%, l 25%, 30%, 35%, 40%, 45%, 50%, 55%, 60%, 65%, 70% of the cells expressing the recombinant receptor are activated.

In some embodiments, a smaller subset of the cells expressing the recombinant receptor (that binds to or is recognized by the binding molecule) from the output composition is activated following the incubation, contacting, or treatment with the particles, e.g., beads, comprising the binding molecule as compared to the cells expressing the recombinant receptor from an output composition that was derived from an identical input composition, i.e., an input composition with the same cellular makeup, that was incubated, treated, or contacted, with one or more polyclonal stimulatory molecules capable of activating one or more intracellular signaling domains of one or more components of a TCR complex. In some embodiments, the subset of cells expressing the recombinant receptor from the output composition that are activated is less than or less than about 1%, 5%, 10%, 15%, 20%, 25%, 30%, 35%, 40%, 45%, 50%, 55%, 60%, 65%, 70%, 75%, 80%, 85%, 95%, 99%,or 99.9% than the subset of cells expressing the recombinant receptor from the output composition derived from the identical input composition that was incubated, treated, or contacted with the one or more polyclonal stimulatory molecules.

In particular embodiments, cells expressing the recombinant receptor (that binds or is or recognized by the binding molecule) of the output composition are more activated following incubation, contacting, or treatment with the particles, e.g., beads, comprising the binding molecule than the cells expressing the recombinant receptor from the input composition prior to the incubation, contacting, or treatment with the particles, e.g., beads, comprising the binding molecule. In some embodiments, cells that express the recombinant receptor from the output composition express a greater amount of at least one activation marker as compared to the cells that express the recombinant receptor from the input composition. In particular embodiments, cells that express the recombinant receptor from the output composition express a greater amount of at least one of Human Leukocyte Antigen-antigen D Related (HLA-DR), CD25, CD69, CD71, CD40L or 4-1BB (CD137) than cells expressing the recombinant receptor from the input composition.

In particular embodiments, the cells expressing the recombinant receptor express a greater level of IL-2, IFN gamma, or TNF-alpha. In some embodiments, the cells expressing the recombinant receptor express a greater amount of intracellular of IL-2, IFN gamma, or TNF-alpha. In certain embodiments, the cells expressing the recombinant receptor secrete a greater amount of IL-2, IFN gamma, or TNF-alpha. In certain embodiments, the cells that express the recombinant receptor from the output composition express an amount of an activation marker that is at least or at least about 1%, 5%, 10%, 15%, 20%, 25%, 30%, 35%, 40%, 45%, 50%, 55%, 60%, 65%, 70%, 75%, 80%, 85%, 95%, 100%, 2-fold, 3-fold, 4-fold,5-fold, 6-fold, 7-fold, 8-fold, 9-fold, 10-fold, 20-fold, 50-fold, or 100-fold greater than the amount of the activation marker expressed by cells expressing the recombinant receptor from the input composition.

In some embodiments, the output composition comprises cells that express the recombinant receptor (that is bound by or recognized by the binding molecule) that are less activated following the incubation, contacting, or treatment with the particles, e.g., beads, comprising the binding molecule than cells expressing the recombinant receptor from an output composition that was derived from an identical input composition, i.e., an input composition with the same cellular makeup, that was incubated, treated, or contacted, with one or more polyclonal stimulatory molecules capable of activating one or more intracellular signaling domains of one or more components of a TCR complex. In some embodiments, the expression of at least one activation marker in the cells that express the recombinant receptor from the output composition that was treated with particles, e.g., beads, comprising the binding molecule is less than or less than about 1%, 5%, 10%, 15%, 20%, l 25%, 30%, 35%, 40%, 45%, 50%, 55%, 60%, 65%, 70%, 75%, 80%, 85%, 95%, 99%,or 99.9% of the expression of the at least one activation marker in cells expressing the recombinant receptor from the output composition derived from the identical input composition that was incubated, treated, or contacted with the one or more polyclonal stimulatory molecules.

In some embodiments, the cells of the output composition that were incubated, contacted, or treated with particles, e.g., beads, comprising a binding molecule display a reduced or lower level of exhaustion as compared to cells expressing the recombinant receptor from an output composition that was derived from an identical input composition, i.e., an input composition with the same cellular makeup, that was incubated, treated, or contacted, under the same conditions, with one or more polyclonal stimulatory molecules capable of activating one or more intracellular signaling domains of one or more components of a TCR complex. In particular embodiments, the cells of the output composition that were incubated, contacted, or treated with a particles, e.g., beads, comprising a binding molecule display a reduced or lower expression of an exhaustion marker as compared to cells from an output composition that was derived from an identical input composition that was incubated, treated, or contacted with one or more polyclonal stimulatory molecules. In some embodiments, the exhaustion marker is a polypeptide that is associated with exhaustion, or is a polynucleotide that encodes a polypeptide that is associated with exhaustion. In some embodiments, expression of an exhaustion marker comprises surface expression of an exhaustion marker polypeptide. In some embodiments, the exhaustion maker is an inhibitory receptor, i.e., a receptor that, when bound by its corresponding ligand, reduces at least one immune or cellular activity of the cell. In certain embodiments, the exhaustion marker is PD-1, CTLA-4, TIM-3, LAG-3, BTLA or TIGIT. In particular embodiments, cells from the output composition were was contacted, treated, or incubated with the particles, e.g., beads, comprising the binding molecule have is less than or less than about 1%, 5%, 10%, 15%, 20%, 25%, 30%, 35%, 40%, 45%, 50%, 55%, 60%, 65%, 70%, 75%, 80%, 85%, 95%, 99%,or 99.9% of the expression of at least one exhaustion marker than cells from an output composition that was contacted, treated, or incubated with one or more polyclonal stimulatory molecules.

In particular embodiments, the cells expressing the recombinant receptor of the output composition that were incubated, contacted, or treated with particles, e.g., beads, comprising a binding molecule display a reduced or lower level of exhaustion as compared to cells expressing the recombinant receptor from an output composition that was derived from an identical input composition that was incubated, treated, or contacted with one or more polyclonal stimulatory molecules capable of activating one or more intracellular signaling domains of one or more components of a TCR complex. In particular embodiments, the cells expressing the recombinant receptor of the output composition that were incubated, contacted, or treated with a particles, e.g., beads, comprising a binding molecule display a reduced or lower expression of an exhaustion marker as compared to cells expressing the recombinant receptor from an output composition that was derived from an identical input composition that was incubated, treated, or contacted, under the same conditions, with one or more polyclonal stimulatory molecules capable of activating one or more intracellular signaling domains of one or more components of a TCR complex. In particular embodiments, cells that express a recombinant receptor from the output composition that was contacted, treated, or incubated with the particles, e.g., beads, comprising the binding molecule have less than or less than about 1%, 5%, 10%, 15%, 20%, l 25%, 30%, 35%, 40%, 45%, 50%, 55%, 60%, 65%, 70%, 75%, 80%, 85%, 95%, 99%,or 99.9% of the expression of the at least one exhaustion marker than cells that express a recombinant receptor from an output composition that was contacted, treated, or incubated with one or more polyclonal stimulatory molecules.

### III. METHODS FOR ASSESSING LONG-TERM STIMULATION

Provided herein is a long term stimulation method (also referred to herein as a method for long term stimulation) that is useful, inter alia, for assessing phenotypes, characteristics, or activities of a cell composition, e.g., a cell composition. In some embodiments, long-term stimulation method is or includes incubating a cell composition, e.g., an input composition containing cells expressing a recombinant receptor such as a CAR, under conditions to stimulate a recombinant receptor-dependent activity (e.g., CAR-dependent activity) in the cells. In some aspects, the cell composition. e.g, the input composition, contains T cells expressing a recombinant receptor (e.g., a CAR) comprising an extracellular antigen-binding domain that specifically binds or recognizes an antigen. In some aspects, the incubation results in a T cell composition, e.g., an output compositions, containing cells that exhibit features of chronically stimulated cells or of cells having prolonged exposure to antigen.

In some embodiments, the long-term stimulation method is or includes incubating a cell composition, e.g., an input composition containing cells expressing a recombinant receptor such as a CAR, under conditions to stimulate a recombinant receptor-dependent activity (e.g., CAR-dependent activity) in the cells. In such embodiments, a recombinant receptor-dependent activity is an activity that is specific to stimulation of the recombinant receptor, e.g. CAR, such as via the presence of an antigen or other agent recognized by the antigen binding domain of the recombinant receptor, e.g. CAR, or that specifically stimulates the recombinant receptor. In some aspects, the cell composition, e.g., the input composition, contains T cells expressing a recombinant receptor (e.g., a CAR) comprising an extracellular antigen-binding domain that specifically binds or recognizes an antigen. In some aspects, the incubation results in a T cell composition, e.g., an output compositions, containing cells that exhibit features of chronically stimulated cells or of cells having prolonged exposure to antigen. In certain embodiments, the incubation is performed continuously without interruption.

In certain embodiments, the methods for long term stimulation provided herein are useful, inter alia, to identify cell compositions that may have desirable features when administered in vivo, such as a maintained or extended persistence, viability, or activity. In some embodiments, the methods are performed on two or more different cell compositions to identify differences that may enhance or prolong persistence, activity, or viability, or decrease exhaustion or differentiation, such as when cells are administered in vivo. In some embodiments, such differences may include, but are not limited to, aspects of the manufacturing process, such as the timing, conditions, or reagents used in different steps of the engineering process, differences in the recombinant receptor (e.g., differences in the amino acid sequence differences in the constructs or vectors used to deliver the recombinant receptor, or different methods of gene delivery), differences in cells (e.g., differences in cell type or subtype or cell source, such as differences in the samples or subjects where the cells are obtained), different storage conditions (e.g., number of prior freeze-thaw cycles, storage temperature, formulation of storage media, storage container, cyropreservative, or cell density), or assay conditions (such as the presence or absence of a control or test compound during the incubation).

In some embodiments, the binding molecule is an antigen (such as a recombinant antigen or fragment thereof) that is bound by or is recognized by the recombinant receptor. In certain embodiments, the binding molecule is an anti-ID that binds to or recognizes the recombinant receptor. In some embodiments, such features may include evidence of decreased viability, activity, or persistence or increased exhaustion or differentiation. In various embodiments, the cells are incubated with the binding molecules in the presence of a media without additional agents that promote cell division, growth, expansion, or activation. In some embodiments, the cells are incubated with the particles for an extended amount of time. e.g., 14 days, without any additional manipulations, e.g., media changes, bead replacement, or splitting or replating the cells.

Particular embodiments contemplate that the long term stimulation methods provided herein are useful, inter alia, for modeling ex vivo the conditions that cells of a cell therapy undergo when administered to a subject, e.g., a human subject, in vivo. Thus, in some aspects, the provided assay is useful to identify cell compositions that may have desirable features when administered in vivo, such as but not limited a maintained or extended persistence, viability, or activity.

In particular embodiments, the long term stimulation method is performed in two or more cell compositions, e.g., input compositions, to identify cell compositions that increase or maintain viability, activity, or persistence, or increase or maintain expression of markers e.g., biomarkers, indicative of increased viability, activity, or persistence. In certain embodiments, the long term stimulation method is performed in two or more cell compositions to identify differences in cell compositions that decrease or prevent exhaustion or differentiation (e.g., such as differentiation to a senescent state), or decrease expression of markers indicative of increased exhaustion or differentiation. In some embodiments, the binding molecule is attached or conjugated to a solid surface, such as a surface of a cell culture plate or dish. In particular embodiments, the binding molecules are conjugated or attached to particles, e.g., beads.

In certain embodiments, the methods include steps for incubating the cells in the presence of particles, e.g., beads, containing a binding molecule, e.g., a binding molecule that binds to or recognizes the recombinant receptor. In some embodiments, the particles, e.g., beads, containing the binding molecule are or include bead-conjugated reagents as provided, such as anti-ID conjugated beads or BCMA-conjugated beads. In particular embodiments, the cells or compositions of cells are incubated with a particle with an attached binding molecule that is described herein, e.g., in Section I. In certain embodiments, the particle is a particle described herein, e.g., in Section I-A. In certain embodiments, the binding molecule is a binding molecule described herein, e.g., in Section I-B. In particular embodiments, the binding molecule binds to or recognizes the recombinant receptor.

In particular embodiments, the input composition is incubated with the binding molecule, such as with particles or beads that contain the binding molecule, for, for about, or for at least 10 days, 11 days, 12 days, 13 days, 14 days, 15 days, 16 days, 17 days, 18 days, 19 days, 20 days, or 21 days. In various embodiments, the input composition is incubated with the binding molecule, e.g., particles or beads that contain the binding molecule, for between or between about 10 days and 21 days, 12 days and 18 days, or 14 days and 16 days, inclusive. In certain embodiments, the cells are incubated with the binding molecule, e.g., particles or beads that contain the binding molecule, for, for about, or for at least 14 days. In particular embodiments, the cells of the cell composition are incubated with the binding molecule at temperatures greater than room temperature. In some embodiments, the incubation is performed at a temperature greater than about 25 °C, such as generally greater than or greater than about 32 °C, 35 °C or 37 °C. In some embodiments, the incubation is performed at a temperature of at or about 37 °C ± 2 °C, such as at a temperature of at or about 37 °C.

In some embodiments, the cells are incubated with the binding molecules, such as with particles or beads containing binding molecules, in the presence of a media without additional agents that promote cell, e.g., T cell, division, growth, expansion, or activation. In some embodiments, the cells are incubated with the binding molecules in the absence of any recombinant cytokines. In particular embodiments, the incubation takes place without interruption, e.g., without replating, media replacement, or addition of fresh binding molecule, etc. In certain embodiments, the incubation takes place under static conditions. In particular embodiments, the incubation takes place without any perfusion, mixing, rocking, or shaking. In some aspects, the particles, e.g., beads, containing a binding molecule are present for the entire duration of the incubation. In certain embodiments, the binding molecules are not changed or replaced during the incubation. Particular embodiments contemplate that since, in some aspects, the media does not contain any recombinant cytokines, and cytokines present in the media during the incubation would have been produced by the cells, e.g., in response to an interaction between the recombinant receptor of the cell and the binding molecule of the particle.

In some embodiments, the provided methods for long term stimulation may be used to compare different cells or cell compositions. For example, in some embodiments, two or more cell compositions that each contain cells expressing the same recombinant receptor, e.g., a same CAR, may be compared by incubating the cells with the same binding molecule, e.g., particles or beads containing the same binding molecule, that binds to or recognizes the recombinant receptor. In certain embodiments, the two or more cell compositions are generated by different engineering processes, such as processes involving different conditions, different timings and durations, or different equipment or reagents. In particular embodiments, the cell compositions are incubated with particles, e.g., beads, containing a binding molecule in the presence of one or more test agents or compounds, such as agents suspected of reducing exhaustion or promoting persistence. In particular embodiments, the cell compositions may be compared to a control or reference cell composition. In some aspects, control or reference cell compositions may include, but are not limited to, cell compositions that do not undergo any incubation, cell compositions that are not incubated in the presence of particles, e.g., beads, containing a binding molecule, cell compositions that do not contain cells expressing the recombinant receptor, cell compositions that are generated from a different engineering process, and/or cell compositions that are incubated in the presence of a vehicle or control compound.

In some embodiments, the long term stimulation method is performed in the presence of one or more test agents or compounds. In particular embodiments, the test agent or compound is an agent suspected of or is a candidate for changing or influencing one or more characteristics, phenotypes, or activities of cells of the cell composition, e.g., cells expressing the recombinant receptor during or following the incubation. In some aspects, the test agent or compound increases or decreases, or is suspected of increasing or decreasing, one or more phenotypes, characteristics, or activities of T cells, such as growth, division, expansion, division, persistence, differentiation, activation, or exhaustion, or activities, such as antigen-stimulated activities including but not limited to cytolytic activity or cytokine production. In certain embodiments, a test agent or compound is a natural or chemically modified polypeptide, an antibody, a natural or chemically modified small oligopeptide, a natural, unnatural, or chemically modified amino acid, a polynucleotide, a natural or chemically modified oligonucleotide, RNAi, shRNA, siRNA, a small nucleotide, a natural or chemically modified mononucleotide, a lipopeptide, an antimicrobial, a small molecule, or a pharmaceutical molecule.

In particular embodiments, two or more cell compositions that each contain cells expressing the different recombinants receptor, e.g., different CARs, may be compared by incubating the cells with binding molecules, such as with particles or beads containing different binding molecules that bind to or recognizes the different recombinant receptors.

In some embodiments, the output composition is composed of cells that have undergone all or a portion of an long term stimulation method, e.g., an long term stimulation method described herein. In certain embodiments, cells undergoing the long term stimulation method are assessed at different time points during the incubation. For example, in some aspects, a phenotype, characteristic, or activity of cells from one or more cell compositions are assessed at an intermediate time point, such as a time point that occurs at, at about, or at least a 5%, 10%, 20%, 25%, 30%, 40%, 50%, 60%, 70%, 75%, 80%, 90%, completion of the incubation. In certain embodiments, the cells are assessed once, twice, three times, four times, five times, six times, seven times, eight times, nine times, ten times or more than ten times during the incubation. In certain embodiments, the cells are assessed following different intervals during the incubation, such as interval of, of about, or of at least 6 hours, 12 hours, 18 hours, 24 hours, 36 hours, 48 hours, 1 day, 2 days, 3 days, 4 days, 5 days, 6 days, 7 days, or 8 days. In some embodiments, the cells are assessed every day. In some embodiments, the cells are assessed every three days. In certain embodiments, the cells are assessed every 7 days.

In certain embodiments, cells of the output composition, e.g., cells that have undergone the long term stimulation method, are assessed for an activity, e.g., an antigen simulated activity, a phenotype, or a characteristic. In some embodiments, and antigen stimulated activity is assessed in cells during or after the method is completed, e.g., during or after the incubation with the binding molecules. In particular embodiments, results of the assessment are compared to an assessment of the same or a similar activity measured in cells from a different cell composition, e.g., a control or reference cell composition.

In some embodiments, the activity is an antigen-stimulated activity. Particular embodiments contemplate that the antigen-stimulated activity of cells, such as T cells expressing a recombinant receptor, can be assessed by any of a number of known suitable techniques. In some embodiments, the production of one or more cytokines is measured, detected, and/or quantified by intracellular cytokine staining. In some aspects, intracellular cytokine staining (ICS) by flow cytometry is a technique well-suited for studying cytokine production at the single-cell level. In certain aspects, ICS is useful to detect the production and accumulation of cytokines within the endoplasmic reticulum after cell stimulation, such as with an cell expressing the antigen or a particle, e.g., a bead particle, with a conjugated antigen, allowing for the identification of cell populations that are positive or negative for production of a particular cytokine or for the separation of high producing and low producing cells based on a threshold. ICS can also be used in combination with other flow cytometry protocols for immunophenotyping using cell surface markers, e.g., CD4 or CD8, to access cytokine production in a particular subgroup of cells. Other single-cell techniques for measuring or detecting cytokine production include, but are not limited to ELISPOT, limiting dilution, and T cell cloning.

In some embodiments, the antigen-stimulated activity is the production of one or more cytokines. Cytokines that may be produced in response to antigen stimulation may include, but are not limited to, IL-1, IL-1β, IL-2, sIL-2Ra, IL-3, IL-5, IL-6, IL-7, IL-8, IL-10, IL-12, IL-13, IL 27, IL-33, IL-35, TNF, TNF alpha, CXCL2, CCL2, CCL3, CCL5, CCL17, CCL24, PGD2, LTB4, interferon gamma (IFN-γ), granulocyte macrophage colony stimulating factor (GM-CSF), macrophage inflammatory protein (MIP)-1a, MIP-1b, Flt-3L, fracktalkine, and/or IL-5. In some embodiments, the one or more cytokines are or include one or more of IL-2, IFN-gamma, or TNF-alpha. In some embodiments, cytokine secretion is assessed by measuring, detecting, or quantifying the amount or concentration of extracellular cytokines following a co-culture with antigen expressing cells or following an incubation of particles, e.g., beads, containing attached antigen or antigen fragments.

In particular embodiments, the antigen-stimulated activity is cytolytic (cytotoxic) activity. In some embodiments, cytolytic activity is assessed by exposing, incubating, and/or contacting the cells of the composition, e.g., cells expressing the recombinant receptor, with a target cell that expresses the antigen or an epitope that is recognized by the recombinant receptor. The cytolytic activity can be measured by directly or indirectly measuring the target cell number over time. For example, the target cells may be incubated with a detectable marker prior to being incubated with recombinant receptor expressing cells, such a marker that is detectable when the target cell is lysed, or a detectable marker that is only detectable in viable target cells. These readouts provide direct or indirect of target cell number and/or target cell death, and can be measured at different time points during the assay. A reduction of target cell number and/or an increase of target cell death indicate the cytolytic activity of the cells. Suitable methods for performing cytolytic assays are known in the art, and include, but are not limited to chromium-51 release assays, non-radioactive chromium assays, flow cytometric assays that use fluorescent dyes such as carboxyfluorescein succinimidyl ester (CFSE), PKH-2, and PKH-26.

In certain embodiments, cells, e.g., cells expressing the recombinant receptor, of the composition are assessed for one or more characteristics or phenotypes during or after the assay, e.g., during or after an incubation with particles, e.g., beads, containing a binding receptor. In some embodiments, the one or more characteristics or phenotypes are or relate to one or more of activation, exhaustion, or differentiation. In certain embodiments, the one or more phenotypes or characteristics are assessed by measuring, detecting, or quantifying the presence, absence, amount, or level of one or more markers.

In some embodiments, the expression of a marker, e.g., a biomarker, that is positively or negatively associated with activation, exhaustion, or differentiation, is or includes assessing, measuring, determining, and/or quantifying a level, amount, or concentration of a marker in the sample. In certain embodiments, the marker is a gene product, e.g., any biomolecule that is assembled, generated, and/or synthesized with information encoded by a gene, and may include polynucleotides and/or polypeptides. In certain embodiments, the level, amount, or concentration of the marker may be transformed (e.g., normalized) or directly analyzed (e.g., raw). In some embodiments, the marker is a protein. In certain embodiments, the marker is a polynucleotide, e.g., an mRNA or a protein, that is encoded by the gene.

In particular embodiments, the amount or level of a marker that is a polynucleotide may be assessed, measured, determined, and/or quantified by any suitable known means. For example, in some embodiments, the amount or level of a polynucleotide marker can be assessed, measured, determined, and/or quantified by polymerase chain reaction (PCR), including reverse transcriptase (rt) PCR, droplet digital PCR, real-time and quantitative PCR methods (including, e.g., TAQMAN^{®}, molecular beacon, LIGHTUP^{™}, SCORPION^{™}, SIMPLEPROBES^{®}; see, e.g., U.S. Pat. Nos.5,538,848; 5,925,517; 6,174,670; 6,329,144; 6,326,145 and 6,635,427); northern blotting; Southern blotting, e.g., of reverse transcription products and derivatives; array based methods, including blotted arrays, microarrays, or in situ-synthesized arrays; and sequencing, e.g., sequencing by synthesis, pyrosequencing, dideoxy sequencing, or sequencing by ligation, or any other methods known in the art, such as discussed in Shendure et al., Nat. Rev. Genet. 5:335-44 (2004) or Nowrousian, Euk. Cell 9(9): 1300-1310 (2010), including such specific platforms as HELICOS^{®}, ROCHE^{®} 454, ILLUMINA^{®}/SOLEXA^{®}, ABI SOLiD^{®}, and POLONATOR^{®} sequencing. In particular embodiments, the levels of nucleic acid gene products are measured by qRT-PCR. In some embodiments, the qRT-PCR uses three nucleic acid sets for each gene, where the three nucleic acids comprise a primer pair together with a probe that binds between the regions of a target nucleic acid where the primers bind- known commercially as a TAQMAN^{®} assay.

In particular embodiments, the expression of two or more polynucleotide markers are measured or assessed simultaneously. In certain embodiments, a multiplex PCR, e.g., a multiplex rt-PCR assessing, measuring, determining, and/or quantifying the level, amount, or concentration of two or more gene products. In some embodiments, microarrays (e.g., AFFYMETRIX^{®}, AGILENT^{®} and ILLUMINA^{®}-style arrays) are used for assessing, measuring, determining, and/or quantifying the level, amount, or concentration of two or more gene products. In some embodiments, microarrays are used for assessing, measuring, determining, and/or quantifying the level, amount, or concentration of a cDNA polynucleotide that is derived from an RNA gene product.

In some embodiments, the expression of one or more polynucleotide markers is determined by sequencing a marker mRNA or a cDNA polynucleotide that is derived from the marker mRNA. In some embodiments, the sequencing is performed by a non-Sanger sequencing method and/or a next generation sequencing (NGS) technique. Examples of Next Generation Sequencing techniques include, but are not limited to Massively Parallel Signature Sequencing (MPSS), Polony sequencing, pyrosequencing, Reversible dye-terminator sequencing, SOLiD sequencing, Ion semiconductor sequencing, DNA nanoball sequencing, Helioscope single molecule sequencing, Single molecule real time (SMRT) sequencing, Single molecule real time (RNAP) sequencing, and Nanopore DNA sequencing.

In some embodiments, the NGS technique is RNA sequencing (RNA-Seq). In particular embodiments, the expression of the one or more polynucleotide gene products is measured, determined, and/or quantified by RNA-Seq. RNA-Seq, also called whole transcriptome shotgun sequencing determines the presence and quantity of RNA in a sample. RNA sequencing methods have been adapted for the most common DNA sequencing platforms [HiSeq systems (Illumina), 454 Genome Sequencer FLX System (Roche), Applied Biosystems SOLiD (Life Technologies), IonTorrent (Life Technologies)]. These platforms require initial reverse transcription of RNA into cDNA. Conversely, the single molecule sequencer HeliScope (Helicos BioSciences) is able to use RNA as a template for sequencing. A proof of principle for direct RNA sequencing on the PacBio RS platform has also been demonstrated (Pacific Bioscience). In some embodiments, the one or more RNA gene products are assessed, measured, determined, and/or quantified by RNA-seq. In some embodiments, the RNA-seq is a tag-based RNA-seq or a shotgun RNA-seq.

In particular embodiments, the marker is a protein or fragment thereof. In certain embodiments, one or more protein markers are measured by any suitable means known in the art. Suitable methods for assessing, measuring, determining, and/or quantifying the level, amount, or concentration or more or more protein markers include, but are not limited to, detection with immunoassays, nucleic acid-based or protein-based aptamer techniques, HPLC (high precision liquid chromatography), peptide sequencing (such as Edman degradation sequencing or mass spectrometry (such as MS/MS), optionally coupled to HPLC), and microarray adaptations of any of the foregoing (including nucleic acid, antibody or protein-protein (i.e., non- antibody) arrays). In some embodiments, the immunoassay is or includes methods or assays that detect proteins based on an immunological reaction, e.g., by detecting the binding of an antibody or antigen binding antibody fragment to a gene product. Immunoassays include, but are not limited to, quantitative immunocytochemistry or immunohistochemistry, ELISA (including direct, indirect, sandwich, competitive, multiple and portable ELISAs (see, e.g., U.S. Patent No. 7,510,687), western blotting (including one, two or higher dimensional blotting or other chromatographic means, optionally including peptide sequencing), enzyme immunoassay (EIA), RIA (radioimmunoassay), and SPR (surface plasmon resonance).

In some embodiments, the protein marker is measured, detected, or quantified by flow cytometry. In some aspects, flow cytometry is a laser- or impedance-based, biophysical technology employed in marker detection by suspending cells in a stream of fluid and passing them through an electronic detection apparatus. Markers present on cells may be labeled, such as with a fluorescence tagged antibody for detection by flow cytometry. In some aspects, flow cytometry is employed to measure, detect, or quantify the presence, absence, amount, or level of a marker present in a population of cells. In some aspects the population of cells may be the total or total viable cells of the cell composition or from a subset of cells from the cell composition, e.g., cells positive for the recombinant receptor, CD4+ T cells, or CD8+ T cells.

In particular embodiments, the marker is positively associated or correlated with activation or an activation-like state. In some embodiments, the marker is or includes CD25, CD26, CD27, CD28, CD30, CD71, CD154, CD40L, CD127, LAG3, or Ki67. In some embodiments, the marker is positively associated or correlated with exhaustion or a condition related to exhaustion. In particular embodiments, the marker is or includes one or more of CTLA-4, FOXP3, PD-1, TIGIT, LAB-3, 2B4, BTLA, TIM3, VISTA, or CD96. In some embodiments, the marker is associated with differentiation of a T cell. In particular embodiments, the marker is or includes one or more of CD25, CD45RO, CD56, KLRG1, CD95 and/or one or more of CD45RA, CD27, CD28, CD62L, and CCR7.In some embodiments, cells, e.g., cells of the output composition, are assessed for cells that are surface positive for a T cell activation marker selected from the group consisting of CD45RA, CD27, CD28, CD62L, and CCR7; and/or that are surface negative for a marker selected from the group consisting of CD25, CD45RO, CD56, KLRG1; and/or have low expression of CD95; and/or are negative for intracellular expression of a cytokine selected from the group consisting of IL-2, IFN-γ, IL-4, IL-10. In some the output composition is assessed for cells that are CD45RA+, CD27+, CCR7+, and/or CD45RO-.

In some embodiments, the cells of a cell composition, e.g., output cell composition, display features of cells that have undergone prolonged or chronic stimulation following the long term stimulation method, e.g., following an incubation with the particles, e.g., beads, containing a binding molecule. In some embodiments, the cells expressing the recombinant receptor of a cell composition, e.g., a reference or control cell composition, display features of cells that have undergone prolonged or chronic stimulation following the assay, e.g., following an incubation with the particles, e.g., beads, containing a binding molecule.

In some embodiments, cells from output compositions that have undergone the long term stimulation method, e.g., an incubation with the particles, e.g., beads, containing a binding molecule, display reduced antigen-stimulated activity as compared to cells of the composition that did not undergo the long term stimulation method. In certain embodiments, the antigen-stimulated activity is reduced by, by about, or by at least 10%, 15%, 20%, 25%, 30%, 35%, 40%, 45%, 50%, 60%, 70%, 80%, 90%, 95%, 99%, 99.9% or by or by about 100% as compared to cells of the composition that did not undergo the long term stimulation method. In certain embodiments, the antigen-stimulated activity is reduced by a detectable, statistically significant amount as compared to cells of the composition that did not undergo the long term stimulation method. In some embodiments, the antigen-stimulated cytokine production is reduced as compared to cells of the composition that did not undergo the long term stimulation method. In particular embodiments, the antigen-stimulated cytokine secretion is reduced as compared to cells of the composition that did not undergo the long term stimulation method. In particular embodiments, the antigen stimulated cytolytic activity is reduced as compared to cells of the composition that did not undergo the long term stimulation method.

In particular embodiments, cells from output compositions, such as cells from or control or reference compositions, display a reduced level, expression, or amount of cells positive for a marker associated with naive-like T cells, as compared to cells of the composition that did not undergo the long term stimulation method. In certain embodiments, the amount of cells positive for the marker such as a marker, e.g., having a detectable amount of the marker or an amount or expression above a threshold level or amount of staining, is reduced by, by about, or by at least 10%, 15%, 20%, 25%, 30%, 35%, 40%, 45%, 50%, 60%, 70%, 80%, 90%, 95%, 99%, 99.9% or by or by about 100% as compared to cells of the composition that did not undergo the long term stimulation method. In certain embodiments, the amount of cells positive for the marker is reduced by a detectable, statistically significant amount as compared to cells of the composition that did not undergo the long term stimulation method.

In some embodiments, the average, mean, or median expression, amount, or level of a marker such as a marker associated with activation or a marker positively associated naive-like state of the cells or a subset of the cells of the output composition is reduced by, by about, or by at least 10%, 15%, 20%, 25%, 30%, 35%, 40%, 45%, 50%, 60%, 70%, 80%, 90%, 95%, 99%, 99.9% or by or by about 100% as compared to cells of the composition that did not undergo the long term stimulation method. In certain embodiments, the amount of cells positive for the marker associated with activation is reduced by a detectable, statistically significant amount as compared to cells of the composition that did not undergo the long term stimulation method.

In certain embodiments, cells from output compositions, e.g., output compositions or control or reference compositions, that have undergone the long term stimulation method, e.g., an incubation with the particles, e.g., beads, containing a binding molecule, display an increased level, expression, or amount of cells positive for a marker, such as a marker positively associated with exhaustion or a marker positively associated with differentiated T cells, as compared to cells of the composition that did not undergo the long term stimulation method. In certain embodiments, the amount of cells positive for the marker, e.g., having a detectable amount of the marker or an amount or expression above a threshold level or amount of staining, is increased by, by about, or by at least 10%, 15%, 20%, 25%, 30%, 35%, 40%, 45%, 50%, 60%, 70%, 80%, 90%, 95%, 100%, 150%, 200%, 1-fold, 2-fold, 3-fold, 4-fold, 5-fold, 10-fold, 20-fold, 50-fold, or 100-fold as compared to cells of the composition that did not undergo the long term stimulation method. In certain embodiments, the amount of cells positive for the marker is increased by a detectable, statistically significant amount as compared to cells of the composition that did not undergo the long term stimulation method.

In various embodiments, the average, mean, or median expression, amount, or level of a marker, such as a marker positively associated with exhaustion or a marker positively associated with differentiated T cells, of the cells or a subset of the cells that underwent the long term stimulation method is increased by, by about, or by at least 10%, 15%, 20%, 25%, 30%, 35%, 40%, 45%, 50%, 60%, 70%, 80%, 90%, 95%, 100%, 150%, 200%, 1-fold, 2-fold, 3-fold, 4-fold, 5-fold, 10-fold, 20-fold, 50-fold, or 100-fold as compared to cells of the composition that did not undergo the long term stimulation method. In certain embodiments, the amount of cells positive for the marker positively associated with exhaustion is increased by a detectable, statistically significant amount as compared to cells of the composition that did not undergo the long term stimulation method.

In certain embodiments, a cell composition containing cells expressing a recombinant receptor is incubated in the presence of a particle containing a binding molecule for between 7 days and 21 days. In particular embodiments, cells of a cell composition containing CAR expressing cells are incubated with particles, e.g., beads, containing a binding molecule for at least 14 days in the absence of recombinant cytokines. In certain embodiments, following the long term stimulation method, the cells or a subset of the cells, e.g., CAR+ cells, are assessed for an antigen-stimulated activity, such as cytokine production or cytolytic activity. In particular embodiments, following the ex-vivo assay, the cells or a subset of the cells of the composition are assessed for the expression of one or more markers. In particular embodiments, two or more cell compositions that undergo the long term stimulation method are compared to identify variations or reagents that improve persistence, viability, or activity of cell compositions when they are administered to a subject in vivo.

### IV. GENETICALLY ENGINEERED CELLS AND RECOMBINANT RECEPTORS

In some embodiments, the provided methods, e.g. for stimulating or expanding recombinant receptor-expressing cells, are carried out on an input composition comprising cells expressing a recombinant receptor and/or in connection with methods for genetically engineering cells with a recombinant receptor. In some aspects, genetically engineering a cell with a recombinant receptor involves contacting a composition of cells with a nucleic acid molecule, e.g., a virus or a non-viral plasmid, that contains a gene encoding a recombinant receptor, e.g., a CAR, under conditions to deliver the nucleic acid molecule into one or more cells of the composition.

In some embodiments, the cells include one or more nucleic acids introduced via genetic engineering in accord with the provided methods, and thereby express recombinant or genetically engineered products of such nucleic acids. In some embodiments, the nucleic acids are heterologous, i.e., normally not present in a cell or sample obtained from the cell, such as one obtained from another organism or cell, which for example, is not ordinarily found in the cell being engineered and/or an organism from which such cell is derived. In some embodiments, the nucleic acids are not naturally occurring, such as a nucleic acid not found in nature, including one comprising chimeric combinations of nucleic acids encoding various domains from multiple different cell types. In particular embodiments, the nucleic acids contain a gene that encodes a recombinant receptor, e.g., a CAR.

In some embodiments, the provided methods may be carried out simultaneously, sequentially or concurrently with one or more processing steps for manufacturing or preparing genetically engineered cells. The processing steps of the methods may include any one or more of a number of cell processing steps, alone or in combination. In particular embodiments, the processing steps include transduction or transfection of the cells with one or more nucleic acids, e.g., a heterologous polynucleotide comprising a gene encoding a recombinant receptor. In certain embodiments, cells are transduced with viral vector particles containing a retroviral vector, such as one encoding a recombinant product for expression in the cells. In certain embodiments, the cells are transfected with one or more non-viral nucleic acids, e.g., an episomal plasmid or a transposon. The methods may further and/or alternatively include other processing steps, such as steps for the isolation, separation, selection, washing, suspension, dilution, concentration, and/or formulation of the cells. In some cases, the methods also can include an ex vivo step for cultivation, stimulation or expansion of cells (e.g., stimulation of the cells, for example, to induce their proliferation and/or activation), which, in some cases, can be carried out in accord with the provided methods. In some embodiments, the methods include isolating cells from the subject, preparing, processing, culturing, and/or engineering them, and re-introducing them into the same subject, before or after cryopreservation.

In some embodiments, the method includes processing steps carried out in an order in which: cells, e.g., primary cells, are first isolated, such as selected or separated, from a biological sample; selected cells are incubated with viral vector particles for transduction; and transduced cells are formulated in a composition. In some cases, transduced cells are activated, expanded or propagated ex vivo, such as by stimulation in the presence of a stimulation reagent, such as in accord with the provided methods. In some embodiments, the method can include one or more processing steps from among washing, suspending, diluting and/or concentrating cells, which can occur prior to, during, or simultaneous with or subsequent to one or more of the isolation, such as separation or selection, transduction, stimulation, and/or formulation steps.

In particular embodiments, the cells to be transfected or transduced are not isolated, selected, or enriched prior to contact with the one or more nucleic acids. In some embodiments, the cells are not selected prior to contacting the cells with the one or more nucleic acids. In some embodiments, the cells to be transfected or transduced are not enriched prior to contacting the cells with the one or more nucleic acids.

In some embodiments, one or more or all of the processing steps, e.g., isolation, selection and/or enrichment, processing, incubation in connection with transduction and engineering, stimulation and/or activation and formulation steps is carried out using a system, device, or apparatus in an integrated or self-contained system, and/or in an automated or programmable fashion. In some aspects, the system or apparatus includes a computer and/or computer program in communication with the system or apparatus, which allows a user to program, control, assess the outcome of, and/or adjust various aspects of the processing, isolation, engineering, and formulation steps. In one example, the system is a system as described in International Patent Application, Publication Number WO2009/072003, or US 20110003380 A1. In one example, the system is a system as described in International Publication Number WO2016/073602.

In some embodiments, one or more of the cell processing steps in connection with preparing, processing and/or incubating cells in connection with the provided method, including in connection with preparing a composition containing genetically engineered cells, can be carried out in the internal cavity of a centrifugal chamber, such as a substantially rigid chamber that is generally cylindrical in shape and rotatable around an axis of rotation, which can provide certain advantages compared to other available methods. In some embodiments, all processing steps are carried out in the same centrifugal chamber. In some embodiments, one or more processing steps are carried out in different centrifugal chambers, such as multiple centrifugal chambers of the same type. Such methods include any of those as described in International Publication Number WO2016/073602.

Exemplary centrifugal chambers include those produced and sold by Biosafe SA, including those for use with the Sepax^{®} and Sepax^{®} 2 system, including an A-200/F and A-200 centrifugal chambers and various kits for use with such systems. Exemplary chambers, systems, and processing instrumentation and cabinets are described, for example, in US Patent No. 6,123,655, US Patent No. 6,733,433 and Published U.S. Patent Application, Publication No.: US 2008/0171951, and published international patent application, publication no. WO 00/38762, the contents of each of which are incorporated herein by reference in their entirety. Depending on the particular process (e.g., dilution, wash, transduction, formulation), it is within the level of a skilled artisan to choose a particular kit that is appropriate for the process. Exemplary kits for use with such systems include, but are not limited to, single-use kits sold by BioSafe SA under product names CS-430.1, CS-490.1, CS-600.1 or CS-900.2.

In some embodiments, the system is included with and/or placed into association with other instrumentation, including instrumentation to operate, automate, control and/or monitor aspects of the various processing steps performed in the system. This instrumentation in some embodiments is contained within a cabinet. In some embodiments, the instrumentation includes a cabinet, which includes a housing containing control circuitry, a centrifuge, a cover, motors, pumps, sensors, displays, and a user interface. An exemplary device is described in US Patent No. 6,123,655, US Patent No. 6,733,433 and US 2008/0171951.

In some embodiments, the system comprises a series of containers, e.g., bags, tubing, stopcocks, clamps, connectors, and a centrifuge chamber. In some embodiments, the containers, such as bags, include one or more containers, such as bags, containing the cells to be transduced or transfected and the vector particles, e.g., viral vector particles or non-viral plasmids, in the same container or separate containers, such as the same bag or separate bags. In some embodiments, the system further includes one or more containers, such as bags, containing medium, such as diluent and/or wash solution, which is pulled into the chamber and/or other components to dilute, resuspend, and/or wash components and/or compositions during the methods. The containers can be connected at one or more positions in the system, such as at a position corresponding to an input line, diluent line, wash line, waste line and/or output line.

**In** some embodiments, the system, such as a closed system, is sterile. In some embodiments, all connections of components of the system, such as between tubing line and a container via a connector, are made under sterile conditions. In some embodiments, connections are made under laminar flow. In some embodiments, connections are made using a sterile connection device that produces sterile connections, such as sterile welds, between a tubing and a container. In some embodiments, a sterile connection device effects connection under thermal condition high enough to maintain sterility, such as temperatures of at least 200 °C, such as at least 260 °C or 300 ° C.

In some embodiments, the system may be disposable, such as a single-use kit. In some embodiments, a single-use kit can be utilized in a plurality of cycles of a process or processes, such as at least 2, 3, 4, 5 or more times, for example, in processes that occur in a continuous or a semi-continuous manner. In some embodiments, the system, such as a single-use kit, is employed for processing of cells from a single patient. In aspects of the methods, the processes need not be performed in the same closed system, such as in the same centrifugal chamber, but can be performed under a different closed system, such as in a different centrifugal chamber; in some embodiments, such different centrifugal chambers are at the respective points in the methods placed in association with the same system, such as placed in association with the same centrifuge. In some embodiments, all processing steps are performed in a closed system, in which all or a subset of each one or more processing step is performed in the same or a different centrifugal chamber.

### A. Samples and Cell Preparations

Provided herein are cells, including engineered cells that contain a recombinant receptor. Also provided are population of such cells and compositions containing such cells. Among the compositions are input compositions containing cells in which one or more cells is known or likely or will express a recombinant receptor capable of being recognized or bound by a binding molecule present on one or more particles to which the cells are incubated or contacted. Also among the compositions are compositions produced by the provided methods, including output compositions in which is contained stimulated or expanded cells, including compositions enriched for cells containing a recombinant receptor bound or recognized by the binding molecule of the particle, such as in which cells expressing the recombinant receptor, e.g. chimeric receptor, make up at least 50, 60, 70, 80, 90, 91, 92, 93, 94, 95, 96, 97, 98, 99, or more percent of the total cells in the composition or cells of a certain type such as T cells or CD8+ or CD4+ cells. Thus, provided are genetically engineered cells expressing the a recombinant receptors e.g., CARs.

Among the compositions are pharmaceutical compositions and formulations for administration, such as for adoptive cell therapy. Also provided are methods for engineering, producing or generating such cells, therapeutic methods for administering the cells and compositions to subjects, e.g., patients, and methods for detecting, selecting, isolating or separating such cells.

The cells generally are eukaryotic cells, such as mammalian cells, and typically are human cells. In some embodiments, the cells are derived from the blood, bone marrow, lymph, or lymphoid organs, are cells of the immune system, such as cells of the innate or adaptive immunity, e.g., myeloid or lymphoid cells, including lymphocytes, typically T cells and/or NK cells. Other exemplary cells include stem cells, such as multipotent and pluripotent stem cells, including induced pluripotent stem cells (iPSCs).

The cells typically are primary cells, such as those isolated directly from a subject and/or isolated from a subject and frozen. In some embodiments, the cells include one or more subsets of T cells or other cell types, such as whole T cell populations, CD4+ cells, CD8+ cells, and subpopulations thereof, such as those defined by function, activation state, maturity, potential for differentiation, expansion, recirculation, localization, and/or persistence capacities, antigen-specificity, type of antigen receptor, presence in a particular organ or compartment, marker or cytokine secretion profile, and/or degree of differentiation. With reference to the subject to be treated, the cells may be allogeneic and/or autologous. Among the methods include off-the-shelf methods. In some aspects, such as for off-the-shelf technologies, the cells are pluripotent and/or multipotent, such as stem cells, such as induced pluripotent stem cells (iPSCs).

Among the sub-types and subpopulations of T cells and/or of CD4+ and/or of CD8+ T cells are naive T (T_{N}) cells, effector T cells (T_{EFF}), memory T cells and sub-types thereof, such as stem cell memory T (T_{SCM}), central memory T (T_{CM}), effector memory T (T_{EM}), or terminally differentiated effector memory T cells, tumor-infiltrating lymphocytes (TIL), immature T cells, mature T cells, helper T cells, cytotoxic T cells, mucosa-associated invariant T (MAIT) cells, naturally occurring and adaptive regulatory T (Treg) cells, helper T cells, such as TH1 cells, TH2 cells, TH3 cells, TH17 cells, TH9 cells, TH22 cells, follicular helper T cells, alpha/beta T cells, and delta/gamma T cells.

In some embodiments, the cells are natural killer (NK) cells. In some embodiments, the cells are monocytes or granulocytes, e.g., myeloid cells, macrophages, neutrophils, dendritic cells, mast cells, eosinophils, and/or basophils.

In some embodiments, the cells are derived from cell lines, e.g., T cell lines. The cells in some embodiments are obtained from a xenogeneic source, for example, from mouse, rat, non-human primate, and pig.

In some embodiments, the cells may be isolated from a sample, such as a biological sample, e.g., one obtained from or derived from a subject. In some embodiments, the subject from which the cell is isolated is one having the disease or condition or in need of a cell therapy or to which cell therapy will be administered. The subject in some embodiments is a human in need of a particular therapeutic intervention, such as the adoptive cell therapy for which cells are being isolated, processed, and/or engineered.

Accordingly, the cells in some embodiments are primary cells, e.g., primary human cells. The samples include tissue, fluid, and other samples taken directly from the subject, as well as samples resulting from one or more processing steps, such as separation, centrifugation, genetic engineering (e.g., transduction with viral vector), washing, and/or incubation. The biological sample can be a sample obtained directly from a biological source or a sample that is processed. Biological samples include, but are not limited to, body fluids, such as blood, plasma, serum, cerebrospinal fluid, synovial fluid, urine and sweat, tissue and organ samples, including processed samples derived therefrom.

In some examples, cells from the circulating blood of a subject are obtained, e.g., by apheresis or leukapheresis. In certain embodiments, the blood cells contain lymphocytes, including T cells, monocytes, granulocytes, B cells, other nucleated white blood cells, red blood cells, and/or platelets, and in some aspects contain cells other than red blood cells and platelets. In some embodiments, prior to the selection and/or enrichment of cells, the sample or the cells in the sample can be rested or held prior to further processing steps. In some embodiments, the sample is maintained at or held at a temperature of from or from about 2° C to 8° C for up to 48 hours, such as for up to12 hours, 24 hours or 36 hours. In certain embodiments, the cells are not selected and/or enriched prior to contacting the cells with the one or more nucleic acids. In some embodiments, the sample or the cells can be rested or held prior to contacting or incubating the cells with one or more nucleic acids. In certain embodiments, the sample is maintained at or held at a temperature of from or from about 2° C to 8° C for up to 48 hours, such as for up to 12 hours, 24 hours or 36 hours prior to contacting or incubating the cells with one or more nucleic acids.

In some embodiments, the preparation methods include steps for freezing, e.g., cryopreserving, the cells, either before or after isolation, selection and/or enrichment, incubation for transduction and engineering and/or stimulation, activation and/or expansion of cells. In some embodiments, the cells are suspended in a freezing solution, e.g., following a washing step to remove plasma and platelets. Any of a variety of known freezing solutions and parameters in some aspects may be used. One example involves using PBS containing 20% DMSO and 8% human serum albumin (HSA), or other suitable cell freezing media. This is then diluted 1: 1 with media so that the final concentration of DMSO and HSA are 10% and 4%, respectively. The cells are generally then frozen to -80° C. at a rate of 1° per minute and stored in the vapor phase of a liquid nitrogen storage tank.

In some embodiments, isolation of the cells includes one or more preparation and/or non-affinity based cell separation steps. In some examples, cells are washed, centrifuged, and/or incubated in the presence of one or more reagents, for example, to remove unwanted components, enrich for desired components, lyse or remove cells sensitive to particular reagents. In some examples, cells are separated based on one or more property, such as density, adherent properties, size, sensitivity and/or resistance to particular components.

In some embodiments, the methods include density-based cell separation methods, such as the preparation of white blood cells from peripheral blood by lysing the red blood cells and centrifugation through a Percoll or Ficoll gradient.

In some embodiments, the isolation methods include the separation of different cell types based on the expression or presence in the cell of one or more specific molecules, such as surface markers, e.g., surface proteins, intracellular markers, or nucleic acid. In some embodiments, any known method for separation based on such markers may be used. In some embodiments, the separation is affinity- or immunoaffinity-based separation. For example, the isolation in some aspects includes separation of cells and cell populations based on the cells' expression or expression level of one or more markers, typically cell surface markers, for example, by incubation with an antibody or binding partner that specifically binds to such markers, followed generally by washing steps and separation of cells having bound the antibody or binding partner, from those cells having not bound to the antibody or binding partner.

Such separation steps can be based on positive selection, in which the cells having bound the reagents are retained for further use, and/or negative selection, in which the cells having not bound to the antibody or binding partner are retained. In some examples, both fractions are retained for further use. In some aspects, negative selection can be particularly useful where no antibody is available that specifically identifies a cell type in a heterogeneous population, such that separation is best carried out based on markers expressed by cells other than the desired population.

The separation need not result in 100% enrichment or removal of a particular cell population or cells expressing a particular marker. For example, positive selection of or enrichment for cells of a particular type, such as those expressing a marker, refers to increasing the number or percentage of such cells, but need not result in a complete absence of cells not expressing the marker. Likewise, negative selection, removal, or depletion of cells of a particular type, such as those expressing a marker, refers to decreasing the number or percentage of such cells, but need not result in a complete removal of all such cells.

In some examples, multiple rounds of separation steps are carried out, where the positively or negatively selected fraction from one step is subjected to another separation step, such as a subsequent positive or negative selection. In some examples, a single separation step can deplete cells expressing multiple markers simultaneously, such as by incubating cells with a plurality of antibodies or binding partners, each specific for a marker targeted for negative selection. Likewise, multiple cell types can simultaneously be positively selected by incubating cells with a plurality of antibodies or binding partners expressed on the various cell types.

For example, in some aspects, specific subpopulations of T cells, such as cells positive or expressing high levels of one or more surface markers, e.g., CD28⁺, CD62L⁺, CCR7⁺, CD27⁺, CD127⁺, CD4⁺, CD8⁺, CD45RA⁺, and/or CD45RO⁺ T cells, are isolated by positive or negative selection techniques.

For example, CD3⁺, CD28⁺ T cells can be positively selected using anti-CD3/anti-CD28 conjugated magnetic beads (e.g., DYNABEADS^{®} M-450 CD3/CD28 T Cell Expander). In particular embodiments, cells are contacted with anti-CD3/anti-CD28 conjugated magnetic beads (e.g., DYNABEADS^{®} M-450 CD3/CD28 T Cell Expander) to expand CD3⁺, CD28⁺ T cells prior to contacting the cells with the one or more nucleic acids. In certain embodiments, the cells are not contacted with anti-CD3/anti-CD28 conjugated magnetic beads prior to contacting the cells with the one or more nucleic acids.

In some embodiments, isolation is carried out by enrichment for a particular cell population by positive selection, or depletion of a particular cell population, by negative selection. In some embodiments, positive or negative selection is accomplished by incubating cells with one or more antibodies or other binding agent that specifically bind to one or more surface markers expressed or expressed (marker⁺) at a relatively higher level (marker^{high}) on the positively or negatively selected cells, respectively.

In some embodiments, T cells are separated from a PBMC sample by negative selection of markers expressed on non-T cells, such as B cells, monocytes, or other white blood cells, such as CD14. In some aspects, a CD4⁺ or CD8⁺ selection step is used to separate CD4⁺ helper and CD8⁺ cytotoxic T cells. Such CD4⁺ and CD8⁺ populations can be further sorted into sub-populations by positive or negative selection for markers expressed or expressed to a relatively higher degree on one or more naive, memory, and/or effector T cell subpopulations.

In some embodiments, CD8⁺ cells are further enriched for or depleted of naive, central memory, effector memory, and/or central memory stem cells, such as by positive or negative selection based on surface antigens associated with the respective subpopulation. In some embodiments, enrichment for central memory T (T_{CM}) cells is carried out to increase efficacy, such as to improve long-term survival, expansion, and/or engraftment following administration, which in some aspects is particularly robust in such sub-populations. *See* Terakura et al. (2012) Blood.1:72-82; Wang et al. (2012) J Immunother. 35(9):689-701. In some embodiments, combining T_{CM}-enriched CD8⁺ T cells and CD4⁺ T cells further enhances efficacy.

In embodiments, memory T cells are present in both CD62L⁺ and CD62L⁻ subsets of CD8⁺ peripheral blood lymphocytes. PBMC can be enriched for or depleted of CD62L⁻CD8⁺ and/or CD62L⁺CD8⁺ fractions, such as using anti-CD8 and anti-CD62L antibodies.

In some embodiments, the enrichment for central memory T (T_{CM}) cells is based on positive or high surface expression of CD45RO, CD62L, CCR7, CD28, CD3, and/or CD 127; in some aspects, it is based on negative selection for cells expressing or highly expressing CD45RA and/or granzyme B. In some aspects, isolation of a CD8⁺ population enriched for T_{CM} cells is carried out by depletion of cells expressing CD4, CD14, CD45RA, and positive selection or enrichment for cells expressing CD62L. In one aspect, enrichment for central memory T (T_{CM}) cells is carried out starting with a negative fraction of cells selected based on CD4 expression, which is subjected to a negative selection based on expression of CD14 and CD45RA, and a positive selection based on CD62L. Such selections in some aspects are carried out simultaneously and in other aspects are carried out sequentially, in either order. In some aspects, the same CD4 expression-based selection step used in preparing the CD8⁺ cell population or subpopulation, also is used to generate the CD4⁺ cell population or subpopulation, such that both the positive and negative fractions from the CD4-based separation are retained and used in subsequent steps of the methods, optionally following one or more further positive or negative selection steps.

In a particular example, a sample of PBMCs or other white blood cell sample is subjected to selection of CD4⁺ cells, where both the negative and positive fractions are retained. The negative fraction then is subjected to negative selection based on expression of CD14 and CD45RA or CD19, and positive selection based on a marker characteristic of central memory T cells, such as CD62L or CCR7, where the positive and negative selections are carried out in either order.

CD4⁺ T helper cells are sorted into naïve, central memory, and effector cells by identifying cell populations that have cell surface antigens. CD4⁺ lymphocytes can be obtained by standard methods. In some embodiments, naive CD4⁺ T lymphocytes are CD45RO⁻, CD45RA⁺, CD62L⁺, and CD4⁺ T cells. In some embodiments, central memory CD4⁺ cells are CD62L⁺ and CD45RO⁺. In some embodiments, effector CD4⁺ cells are CD62L⁻ and CD45RO⁻.

In one example, to enrich for CD4⁺ cells by negative selection, a monoclonal antibody cocktail typically includes antibodies to CD14, CD20, CD11b, CD16, HLA-DR, and CD8. In some embodiments, the antibody or binding partner is bound to a solid support or matrix, such as a magnetic bead or paramagnetic bead, to allow for separation of cells for positive and/or negative selection. For example, in some embodiments, the cells and cell populations are separated or isolated using immunomagnetic (or affinitymagnetic) separation techniques (reviewed in Methods in Molecular Medicine, vol. 58: Metastasis Research Protocols, Vol. 2: Cell Behavior In Vitro and In Vivo, p 17-25 Edited by: S. A. Brooks and U. Schumacher © Humana Press Inc., Totowa, NJ).

In some aspects, the sample or composition of cells to be separated is incubated with small, magnetizable or magnetically responsive material, such as magnetically responsive particles or microparticles, such as paramagnetic beads (e.g., such as Dynalbeads or MACS beads). The magnetically responsive material, e.g., particle, generally is directly or indirectly attached to a binding partner, e.g., an antibody, that specifically binds to a molecule, e.g., surface marker, present on the cell, cells, or population of cells that it is desired to separate, e.g., that it is desired to negatively or positively select.

In some embodiments, the magnetic particle or bead comprises a magnetically responsive material bound to a specific binding member, such as an antibody or other binding partner. There are many well-known magnetically responsive materials used in magnetic separation methods. Suitable magnetic particles include those described in Molday, U.S. Pat. No. 4,452,773, and in European Patent Specification EP 452342 B, which are hereby incorporated by reference. Colloidal sized particles, such as those described in Owen U.S. Pat. No. 4,795,698, and Liberti et al., U.S. Pat. No. 5,200,084 are other examples.

The incubation generally is carried out under conditions whereby the antibodies or binding partners, or molecules, such as secondary antibodies or other reagents, which specifically bind to such antibodies or binding partners, which are attached to the magnetic particle or bead, specifically bind to cell surface molecules if present on cells within the sample.

In some aspects, the sample is placed in a magnetic field, and those cells having magnetically responsive or magnetizable particles attached thereto will be attracted to the magnet and separated from the unlabeled cells. For positive selection, cells that are attracted to the magnet are retained; for negative selection, cells that are not attracted (unlabeled cells) are retained. In some aspects, a combination of positive and negative selection is performed during the same selection step, where the positive and negative fractions are retained and further processed or subject to further separation steps.

In certain embodiments, the magnetically responsive particles are coated in primary antibodies or other binding partners, secondary antibodies, lectins, enzymes, or streptavidin. In certain embodiments, the magnetic particles are attached to cells via a coating of primary antibodies specific for one or more markers. In certain embodiments, the cells, rather than the beads, are labeled with a primary antibody or binding partner, and then cell-type specific secondary antibody- or other binding partner (e.g., streptavidin)-coated magnetic particles, are added. In certain embodiments, streptavidin-coated magnetic particles are used in conjunction with biotinylated primary or secondary antibodies.

In some embodiments, the magnetically responsive particles are left attached to the cells that are to be subsequently incubated, cultured and/or engineered; in some aspects, the particles are left attached to the cells for administration to a patient. In some embodiments, the magnetizable or magnetically responsive particles are removed from the cells. Methods for removing magnetizable particles from cells are known and include, e.g., the use of competing non-labeled antibodies, and magnetizable particles or antibodies conjugated to cleavable linkers. In some embodiments, the magnetizable particles are biodegradable.

In some embodiments, the affinity-based selection is via magnetic-activated cell sorting (MACS) (Miltenyi Biotec, Auburn, CA). Magnetic Activated Cell Sorting (MACS) systems are capable of high-purity selection of cells having magnetized particles attached thereto. In certain embodiments, MACS operates in a mode wherein the non-target and target species are sequentially eluted after the application of the external magnetic field. That is, the cells attached to magnetized particles are held in place while the unattached species are eluted. Then, after this first elution step is completed, the species that were trapped in the magnetic field and were prevented from being eluted are freed in some manner such that they can be eluted and recovered. In certain embodiments, the non-target cells are labelled and depleted from the heterogeneous population of cells.

In certain embodiments, the isolation or separation is carried out using a system, device, or apparatus that carries out one or more of the isolation, cell preparation, separation, processing, incubation, culture, and/or formulation steps of the methods. In some aspects, the system is used to carry out each of these steps in a closed or sterile environment, for example, to minimize error, user handling and/or contamination. In one example, the system is a system as described in International Patent Application, Publication Number WO2009/072003, or US 20110003380 A1. In one example, the system is a system as described in International Publication Number WO2016/073602.

In some aspects, the separation and/or other steps is carried out using CliniMACS system (Miltenyi Biotec), for example, for automated separation of cells on a clinical-scale level in a closed and sterile system. Components can include an integrated microcomputer, magnetic separation unit, peristaltic pump, and various pinch valves. The integrated computer in some aspects controls all components of the instrument and directs the system to perform repeated procedures in a standardized sequence. The magnetic separation unit in some aspects includes a movable permanent magnet and a holder for the selection column. The peristaltic pump controls the flow rate throughout the tubing set and, together with the pinch valves, ensures the controlled flow of buffer through the system and continual suspension of cells.

The CliniMACS system in some aspects uses antibody-coupled magnetizable particles that are supplied in a sterile, non-pyrogenic solution. In some embodiments, after labelling of cells with magnetic particles the cells are washed to remove excess particles. A cell preparation bag is then connected to the tubing set, which in turn is connected to a bag containing buffer and a cell collection bag. The tubing set consists of pre-assembled sterile tubing, including a pre-column and a separation column, and are for single use only. After initiation of the separation program, the system automatically applies the cell sample onto the separation column. Labelled cells are retained within the column, while unlabeled cells are removed by a series of washing steps. In some embodiments, the cell populations for use with the methods described herein are unlabeled and are not retained in the column. In some embodiments, the cell populations for use with the methods described herein are labeled and are retained in the column. In some embodiments, the cell populations for use with the methods described herein are eluted from the column after removal of the magnetic field, and are collected within the cell collection bag.

In certain embodiments, separation and/or other steps are carried out using the CliniMACS Prodigy system (Miltenyi Biotec). The CliniMACS Prodigy system in some aspects is equipped with a cell processing unity that permits automated washing and fractionation of cells by centrifugation. The CliniMACS Prodigy system can also include an onboard camera and image recognition software that determines the optimal cell fractionation endpoint by discerning the macroscopic layers of the source cell product. For example, peripheral blood is automatically separated into erythrocytes, white blood cells and plasma layers. The CliniMACS Prodigy system can also include an integrated cell cultivation chamber which accomplishes cell culture protocols such as, e.g., cell differentiation and expansion, antigen loading, and long-term cell culture. Input ports can allow for the sterile removal and replenishment of media and cells can be monitored using an integrated microscope. See, e.g., Klebanoff et al. (2012) J Immunother. 35(9): 651-660, Terakura et al. (2012) Blood.1:72-82, and Wang et al. (2012) J Immunother. 35(9):689-701.

In some embodiments, a cell population described herein is collected and enriched (or depleted) via flow cytometry, in which cells stained for multiple cell surface markers are carried in a fluidic stream. In some embodiments, a cell population described herein is collected and enriched (or depleted) via preparative scale (FACS)-sorting. In certain embodiments, a cell population described herein is collected and enriched (or depleted) by use of microelectromechanical systems (MEMS) chips in combination with a FACS-based detection system (see, e.g., WO 2010/033140, Cho et al. (2010) Lab Chip 10, 1567-1573; and Godin et al. (2008) J Biophoton. 1(5):355-376. In both cases, cells can be labeled with multiple markers, allowing for the isolation of well-defined T cell subsets at high purity.

In some embodiments, the antibodies or binding partners are labeled with one or more detectable marker, to facilitate separation for positive and/or negative selection. For example, separation may be based on binding to fluorescently labeled antibodies. In some examples, separation of cells based on binding of antibodies or other binding partners specific for one or more cell surface markers are carried in a fluidic stream, such as by fluorescence-activated cell sorting (FACS), including preparative scale (FACS) and/or microelectromechanical systems (MEMS) chips, e.g., in combination with a flow-cytometric detection system. Such methods allow for positive and negative selection based on multiple markers simultaneously.

In some embodiments, the cells are activated or stimulated prior to one or more steps for genetically engineering the cells, e.g., by transducing or transfecting the cells. In particular embodiments, the cells are cultured or incubated under stimulatory condition prior to one or more steps for genetically engineering the cells. In some embodiments, the stimulating conditions or agents include one or more agents, e.g., ligands, capable of activating an intracellular signaling domain of a TCR complex. In some aspects, the agent turns on or initiates TCR/CD3 intracellular signaling cascade in a T cell. Such agents can include antibodies, such as those specific for a TCR, e.g. anti-CD3. In some embodiments, the stimulating conditions include one or more agent, e.g. ligand, which is capable of stimulating a costimulatory receptor, e.g., anti-CD28. In some embodiments, such agents and/or ligands may be, bound to solid support such as a bead, and/or one or more cytokines. Optionally, the activation or stimulation may further comprise the step of adding anti-CD3 and/or anti CD28 antibody to the culture medium (e.g., at a concentration of at least about 0.5 ng/ml). In some embodiments, the stimulating agents include IL-2, IL-15 and/or IL-7. In some aspects, the IL-2 concentration is at least about 10 units/mL.

### B. Nucleic Acid Encoding a Heterologous Protein

Also provided are one or more polynucleotides (e.g., nucleic acid molecules) encoding recombinant receptors, vectors for genetically engineering cells to express such receptors and methods for producing the engineered cells. In some embodiments, the vector contains the nucleic acid encoding the recombinant receptor. In particular embodiments, the vector is a viral vector a non-viral vector. In some cases, the vector is a viral vector, such as a retroviral vector, e.g., a lentiviral vector or a gammaretroviral vector.

In some embodiments, the cells are contacted with the vector without prior activation of the cells and/or within a time period that is initiated no more than 24 hours after obtaining the cells from a subject and/or in which the cells have not been subjected to a temperature greater than 15°C to 25° C, such as greater than or greater than about for more than 37 ° ± 2.0 °C, for no more than 24 hours after the cells are obtained from a subject. In certain embodiments, the vector is contacted to the cells without first, i.e. prior to the genetic engineering (e.g., transduction or transfection) , activating and/or stimulating the T cells with an ex vivo stimulation reagent (e.g. anti-CD3/anti-CD28 reagent) prior to and/or in conjunction with contacting or incubating the cells with the viral particles. In some embodiments, the vectors include viral vectors, e.g., retroviral or lentiviral, non-viral vectors or transposons, e.g. *Sleeping Beauty* transposon system, vectors derived from simian virus 40 (SV40), adenoviruses, adeno-associated virus (AAV), lentiviral vectors or retroviral vectors, such as gamma-retroviral vectors, retroviral vector derived from the Moloney murine leukemia virus (MoMLV), myeloproliferative sarcoma virus (MPSV), murine embryonic stem cell virus (MESV), murine stem cell virus (MSCV), spleen focus forming virus (SFFV) or adeno-associated virus (AAV).

In some embodiments, the viral vector or the non-viral DNA contains a nucleic acid that encodes a heterologous recombinant protein. In some embodiments, the heterologous recombinant molecule is or includes a recombinant receptor, *e.g.,* an antigen receptor, SB-transposons, e.g., for gene silencing, capsid-enclosed transposons, homologous double stranded nucleic acid, *e.g.,* for genomic recombination or reporter genes (*e.g.,* fluorescent proteins, such as GFP) or luciferase).

In some embodiments, the viral vector or the non-viral DNA contains a nucleic acid that encodes a recombinant receptor and/or chimeric receptor, such as a heterologous receptor protein. The recombinant receptor, such as heterologous receptor, may include antigen receptors, such as functional non-TCR antigen receptors, including chimeric antigen receptors (CARs), and other antigen-binding receptors such as transgenic T cell receptors (TCRs). The receptors may also include other receptors, such as other chimeric receptors, such as receptors that bind to particular ligands and having transmembrane and/or intracellular signaling domains similar to those present in a CAR.

In some embodiments, the nucleic acid is inserted or located in a region of a viral vector, such as generally in a non-essential region of the viral genome. In some embodiments, the nucleic acid is inserted into the viral genome in the place of certain viral sequences to produce a virus that is replication defective. In certain embodiments, the nucleic acid is located within a non-viral DNA, e.g., within a transposon.

Antigen receptors, including CARs and recombinant TCRs, and production and introduction thereof, in some embodiments include those described, for example, in international patent application publication numbers WO200014257, WO2013126726, WO2012/129514, WO2014031687, WO2013/166321, WO2013/071154, WO2013/123061 U.S. patent application publication numbers US2002131960, US2013287748, US20130149337, U.S. Patent Nos.: 6,451,995, 7,446,190, 8,252,592, , 8,339,645, 8,398,282, 7,446,179, 6,410,319, 7,070,995, 7,265,209, 7,354,762, 7,446,191, 8,324,353, and 8,479,118, and European patent application number EP2537416, and/or those described by Sadelain et al., Cancer Discov. 2013 April; 3(4): 388-398; Davila et al. (2013) PLoS ONE 8(4): e61338; Turtle et al., Curr. Opin. Immunol., 2012 October; 24(5): 633-39; Wu et al., Cancer, 2012 March 18(2): 160-75.

### 1. Chimeric Antigen Receptor

In some embodiments, engineered cells, such as T cells, are provided that express a CAR with specificity for a particular antigen (or marker or ligand), such as an antigen expressed on the surface of a particular cell type. In some embodiments, the antigen is a polypeptide. In some embodiments, it is a carbohydrate or other molecule. In some embodiments, the antigen is selectively expressed or overexpressed on cells of the disease or condition, e.g., the tumor or pathogenic cells, as compared to normal or non-targeted cells or tissues. In other embodiments, the antigen is expressed on normal cells and/or is expressed on the engineered cells.

In particular embodiments, the recombinant receptor, such as chimeric receptor, contains an intracellular signaling region, which includes a cytoplasmic signaling domain (also interchangeably called an intracellular signaling domain), such as a cytoplasmic (intracellular) region capable of inducing a primary activation signal in a T cell, for example, a cytoplasmic signaling domain of a T cell receptor (TCR) component (e.g. a cytoplasmic signaling domain of a zeta chain of a CD3-zeta (CD3ζ) chain or a functional variant or signaling portion thereof) and/or that comprises an immunoreceptor tyrosine-based activation motif (ITAM).

In some embodiments, the chimeric receptor further contains an extracellular ligand-binding domain that specifically binds to a ligand (e.g. antigen) antigen. In some embodiments, the chimeric receptor is a CAR that contains an extracellular antigen-recognition domain that specifically binds to an antigen. In some embodiments, the ligand, such as an antigen, is a protein expressed on the surface of cells. In some embodiments, the CAR is a TCR-like CAR and the antigen is a processed peptide antigen, such as a peptide antigen of an intracellular protein, which, like a TCR, is recognized on the cell surface in the context of a major histocompatibility complex (MHC) molecule.

Exemplary antigen receptors, including CARs, and methods for engineering and introducing such receptors into cells, include those described, for example, in international patent application publication numbers WO200014257, WO2013126726, WO2012/129514, WO2014031687, WO2013/166321, WO2013/071154, WO2013/123061 U.S. patent application publication numbers US2002131960, US2013287748, US20130149337, U.S. Patent Nos.: 6,451,995, 7,446,190, 8,252,592, 8,339,645, 8,398,282, 7,446,179, 6,410,319, 7,070,995, 7,265,209, 7,354,762, 7,446,191, 8,324,353, and 8,479,118, and European patent application number EP2537416,and/or those described by Sadelain et al., Cancer Discov. 2013 April; 3(4): 388-398; Davila et al. (2013) PLoS ONE 8(4): e61338; Turtle et al., Curr. Opin. Immunol., 2012 October; 24(5): 633-39; Wu et al., Cancer, 2012 March 18(2): 160-75. In some aspects, the antigen receptors include a CAR as described in U.S. Patent No.: 7,446,190, and those described in International Patent Application Publication No.: WO/2014055668 A1. Examples of the CARs include CARs as disclosed in any of the aforementioned publications, such as WO2014031687, US 8,339,645, US 7,446,179, US 2013/0149337, U.S. Patent No.: 7,446,190, US Patent No.: 8,389,282, Kochenderfer et al., 2013, Nature Reviews Clinical Oncology, 10, 267-276 (2013); Wang et al. (2012) J. Immunother. 35(9): 689-701; and Brentjens et al., Sci Transl Med. 2013 5(177). See also WO2014031687, US 8,339,645, US 7,446,179, US 2013/0149337, U.S. Patent No.: 7,446,190, and US Patent No.: 8,389,282.

In some embodiments, the CAR is constructed with a specificity for a particular antigen (or marker or ligand), such as an antigen expressed in a particular cell type to be targeted by adoptive therapy, *e.g.,* a cancer marker, and/or an antigen intended to induce a dampening response, such as an antigen expressed on a normal or non-diseased cell type. Thus, the CAR typically includes in its extracellular portion one or more antigen binding molecules, such as one or more antigen-binding fragment, domain, or portion, or one or more antibody variable domains, and/or antibody molecules. In some embodiments, the CAR includes an antigen-binding portion or portions of an antibody molecule, such as a single-chain antibody fragment (scFv) derived from the variable heavy (VH) and variable light (VL) chains of a monoclonal antibody (mAb).

In some embodiments, the antibody or antigen-binding portion thereof is expressed on cells as part of a recombinant receptor, such as an antigen receptor. Among the antigen receptors are functional non-TCR antigen receptors, such as chimeric antigen receptors (CARs). Generally, a CAR containing an antibody or antigen-binding fragment that exhibits TCR-like specificity directed against peptide-MHC complexes also may be referred to as a TCR-like CAR. In some embodiments, the extracellular antigen binding domain specific for an MHC-peptide complex of a TCR-like CAR is linked to one or more intracellular signaling components, in some aspects via linkers and/or transmembrane domain(s). In some embodiments, such molecules can typically mimic or approximate a signal through a natural antigen receptor, such as a TCR, and, optionally, a signal through such a receptor in combination with a costimulatory receptor.

In some embodiments, the recombinant receptor, such as a chimeric receptor (e.g. CAR), includes a ligand-binding domain that binds, such as specifically binds, to an antigen (or a ligand). Among the antigens targeted by the chimeric receptors are those expressed in the context of a disease, condition, or cell type to be targeted via the adoptive cell therapy. Among the diseases and conditions are proliferative, neoplastic, and malignant diseases and disorders, including cancers and tumors, including hematologic cancers, cancers of the immune system, such as lymphomas, leukemias, and/or myelomas, such as B, T, and myeloid leukemias, lymphomas, and multiple myelomas.

In some embodiments, the antigen (or a ligand) is a polypeptide. In some embodiments, it is a carbohydrate or other molecule. In some embodiments, the antigen (or a ligand) is selectively expressed or overexpressed on cells of the disease or condition, *e.g*., the tumor or pathogenic cells, as compared to normal or non-targeted cells or tissues. In other embodiments, the antigen is expressed on normal cells and/or is expressed on the engineered cells.

In some embodiments, the CAR contains an antibody or an antigen-binding fragment (*e.g.* scFv) that specifically recognizes an antigen, such as an intact antigen, expressed on the surface of a cell.

In some embodiments, the antigen is or includes αvβ6 integrin (avb6 integrin), B cell maturation antigen (BCMA), B7-H3, B7-H6, carbonic anhydrase 9 (CA9, also known as CAIX or G250), a cancer-testis antigen, cancer/testis antigen 1B (CTAG, also known as NY-ESO-1 and LAGE-2), carcinoembryonic antigen (CEA), a cyclin, cyclin A2, C-C Motif Chemokine Ligand 1 (CCL-1), CD19, CD20, CD22, CD23, CD24, CD30, CD33, CD38, CD44, CD44v6, CD44v7/8, CD123, CD133, CD138, CD171, chondroitin sulfate proteoglycan 4 (CSPG4), epidermal growth factor protein (EGFR), type III epidermal growth factor receptor mutation (EGFR vIII), epithelial glycoprotein 2 (EPG-2), epithelial glycoprotein 40 (EPG-40), ephrinB2, ephrin receptor A2 (EPHa2), estrogen receptor, Fc receptor like 5 (FCRL5; also known as Fc receptor homolog 5 or FCRH5), fetal acetylcholine receptor (fetal AchR), a folate binding protein (FBP), folate receptor alpha, ganglioside GD2, O-acetylated GD2 (OGD2), ganglioside GD3, glycoprotein 100 (gp100), glypican-3 (GPC3), G Protein Coupled Receptor 5D (GPRC5D), Her2/neu (receptor tyrosine kinase erb-B2), Her3 (erb-B3), Her4 (erb-B4), erbB dimers, Human high molecular weight-melanoma-associated antigen (HMW-MAA), hepatitis B surface antigen, Human leukocyte antigen A1 (HLA-A1), Human leukocyte antigen A2 (HLA-A2), IL-22 receptor alpha(IL-22Rα), IL-13 receptor alpha 2 (IL-13Rα2), kinase insert domain receptor (kdr), kappa light chain, L1 cell adhesion molecule (L1-CAM), CE7 epitope of L1-CAM, Leucine Rich Repeat Containing 8 Family Member A (LRRC8A), Lewis Y, Melanoma-associated antigen (MAGE)-A1, MAGE-A3, MAGE-A6, MAGE-A10, mesothelin (MSLN), c-Met, murine cytomegalovirus (CMV), mucin 1 (MUC1), MUC16, natural killer group 2 member D (NKG2D) ligands, melan A (MART-1), neural cell adhesion molecule (NCAM), oncofetal antigen, Preferentially expressed antigen of melanoma (PRAME), progesterone receptor, a prostate specific antigen, prostate stem cell antigen (PSCA), prostate specific membrane antigen (PSMA), Receptor Tyrosine Kinase Like Orphan Receptor 1 (ROR1), survivin, Trophoblast glycoprotein (TPBG also known as 5T4), tumor-associated glycoprotein 72 (TAG72), Tyrosinase related protein 1 (TRP1, also known as TYRP1 or gp75), Tyrosinase related protein 2 (TRP2, also known as dopachrome tautomerase, dopachrome delta-isomerase or DCT), vascular endothelial growth factor receptor (VEGFR), vascular endothelial growth factor receptor 2 (VEGFR2), Wilms Tumor 1 (WT-1), a pathogen-specific or pathogen-expressed antigen, or an antigen associated with a universal tag, and/or biotinylated molecules, and/or molecules expressed by HIV, HCV, HBV or other pathogens. Antigens targeted by the receptors in some embodiments include antigens associated with a B cell malignancy, such as any of a number of known B cell marker. In some embodiments, the antigen is or includes CD20, CD19, CD22, ROR1, CD45, CD21, CD5, CD33, Igkappa, Iglambda, CD79a, CD79b or CD30.

In some embodiments, the antigen is a pathogen-specific antigen. In some embodiments, the antigen is a viral antigen (such as a viral antigen from HIV, HCV, HBV, etc.), bacterial antigens, and/or parasitic antigens.

In some embodiments, the CAR contains a TCR-like antibody, such as an antibody or an antigen-binding fragment (*e.g*. scFv) that specifically recognizes an intracellular antigen, such as a tumor-associated antigen, presented on the cell surface as a MHC-peptide complex. In some embodiments, an antibody or antigen-binding portion thereof that recognizes an MHC-peptide complex can be expressed on cells as part of a recombinant receptor, such as an antigen receptor. Among the antigen receptors are functional non-TCR antigen receptors, such as chimeric antigen receptors (CARs). Generally, a CAR containing an antibody or antigen-binding fragment that exhibits TCR-like specificity directed against peptide-MHC complexes also may be referred to as a TCR-like CAR.

Reference to "Major histocompatibility complex" (MHC) refers to a protein, generally a glycoprotein, that contains a polymorphic peptide binding site or binding groove that can, in some cases, complex with peptide antigens of polypeptides, including peptide antigens processed by the cell machinery. In some cases, MHC molecules can be displayed or expressed on the cell surface, including as a complex with peptide, *i.e.* MHC-peptide complex, for presentation of an antigen in a conformation recognizable by an antigen receptor on T cells, such as a TCRs or TCR-like antibody. Generally, MHC class I molecules are heterodimers having a membrane spanning α chain, in some cases with three α domains, and a non-covalently associated β2 microglobulin. Generally, MHC class II molecules are composed of two transmembrane glycoproteins, α and β, both of which typically span the membrane. An MHC molecule can include an effective portion of an MHC that contains an antigen binding site or sites for binding a peptide and the sequences necessary for recognition by the appropriate antigen receptor. In some embodiments, MHC class I molecules deliver peptides originating in the cytosol to the cell surface, where a MHC-peptide complex is recognized by T cells, such as generally CD8⁺ T cells, but in some cases CD4+ T cells. In some embodiments, MHC class II molecules deliver peptides originating in the vesicular system to the cell surface, where they are typically recognized by CD4⁺ T cells. Generally, MHC molecules are encoded by a group of linked loci, which are collectively termed H-2 in the mouse and human leukocyte antigen (HLA) in humans. Hence, typically human MHC can also be referred to as human leukocyte antigen (HLA).

The term "MHC-peptide complex" or "peptide-MHC complex" or variations thereof, refers to a complex or association of a peptide antigen and an MHC molecule, such as, generally, by non-covalent interactions of the peptide in the binding groove or cleft of the MHC molecule. In some embodiments, the MHC-peptide complex is present or displayed on the surface of cells. In some embodiments, the MHC-peptide complex can be specifically recognized by an antigen receptor, such as a TCR, TCR-like CAR or antigen-binding portions thereof.

In some embodiments, a peptide, such as a peptide antigen or epitope, of a polypeptide can associate with an MHC molecule, such as for recognition by an antigen receptor. Generally, the peptide is derived from or based on a fragment of a longer biological molecule, such as a polypeptide or protein. In some embodiments, the peptide typically is about 8 to about 24 amino acids in length. In some embodiments, a peptide has a length of from or from about 9 to 22 amino acids for recognition in the MHC Class II complex. In some embodiments, a peptide has a length of from or from about 8 to 13 amino acids for recognition in the MHC Class I complex. In some embodiments, upon recognition of the peptide in the context of an MHC molecule, such as MHC-peptide complex, the antigen receptor, such as TCR or TCR-like CAR, produces or triggers an activation signal to the T cell that induces a T cell response, such as T cell proliferation, cytokine production, a cytotoxic T cell response or other response.

In some embodiments, a TCR-like antibody or antigen-binding portion, are known or can be produced by methods known in the art (see e.g. US Published Application Nos. US 2002/0150914; US 2003/0223994; US 2004/0191260; US 2006/0034850; US 2007/00992530; US20090226474; US20090304679; and International PCT Publication No. WO 03/068201).

In some embodiments, an antibody or antigen-binding portion thereof that specifically binds to a MHC-peptide complex, can be produced by immunizing a host with an effective amount of an immunogen containing a specific MHC-peptide complex. In some cases, the peptide of the MHC-peptide complex is an epitope of antigen capable of binding to the MHC, such as a tumor antigen, for example a universal tumor antigen, myeloma antigen or other antigen as described below. In some embodiments, an effective amount of the immunogen is then administered to a host for eliciting an immune response, wherein the immunogen retains a three-dimensional form thereof for a period of time sufficient to elicit an immune response against the three-dimensional presentation of the peptide in the binding groove of the MHC molecule. Serum collected from the host is then assayed to determine if desired antibodies that recognize a three-dimensional presentation of the peptide in the binding groove of the MHC molecule is being produced. In some embodiments, the produced antibodies can be assessed to confirm that the antibody can differentiate the MHC-peptide complex from the MHC molecule alone, the peptide of interest alone, and a complex of MHC and irrelevant peptide. The desired antibodies can then be isolated.

In some embodiments, an antibody or antigen-binding portion thereof that specifically binds to an MHC-peptide complex can be produced by employing antibody library display methods, such as phage antibody libraries. In some embodiments, phage display libraries of mutant Fab, scFv or other antibody forms can be generated, for example, in which members of the library are mutated at one or more residues of a CDR or CDRs. See *e.g.* US published application No. US20020150914, US2014/0294841; and Cohen CJ. et al. (2003) J Mol. Recogn. 16:324-332.

The term "antibody" herein is used in the broadest sense and includes polyclonal and monoclonal antibodies, including intact antibodies and functional (antigen-binding) antibody fragments, including fragment antigen binding (Fab) fragments, F(ab')₂ fragments, Fab' fragments, Fv fragments, recombinant IgG (rIgG) fragments, variable heavy chain (V_{H}) regions capable of specifically binding the antigen, single chain antibody fragments, including single chain variable fragments (scFv), and single domain antibodies (*e.g.,* sdAb, sdFv, nanobody) fragments. The term encompasses genetically engineered and/or otherwise modified forms of immunoglobulins, such as intrabodies, peptibodies, chimeric antibodies, fully human antibodies, humanized antibodies, and heteroconjugate antibodies, multispecific, *e.g.,* bispecific, antibodies, diabodies, triabodies, and tetrabodies, tandem di-scFv, tandem tri-scFv. Unless otherwise stated, the term "antibody" should be understood to encompass functional antibody fragments thereof. The term also encompasses intact or full-length antibodies, including antibodies of any class or sub-class, including IgG and sub-classes thereof, IgM, IgE, IgA, and IgD.

In some embodiments, the antigen-binding proteins, antibodies and antigen binding fragments thereof specifically recognize an antigen of a full-length antibody. In some embodiments, the heavy and light chains of an antibody can be full-length or can be an antigen-binding portion (a Fab, F(ab')2, Fv or a single chain Fv fragment (scFv)). In other embodiments, the antibody heavy chain constant region is chosen from, *e.g.,* IgG1, IgG2, IgG3, IgG4, IgM, IgA1, IgA2, IgD, and IgE, particularly chosen from, *e.g.,* IgG1, IgG2, IgG3, and IgG4, more particularly, IgG1 (*e.g.,* human IgG1). In another embodiment, the antibody light chain constant region is chosen from, *e.g.,* kappa or lambda, particularly kappa.

Among the provided antibodies are antibody fragments. An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')₂; diabodies; linear antibodies; variable heavy chain (V_{H}) regions, single-chain antibody molecules such as scFvs and single-domain V_{H} single antibodies; and multispecific antibodies formed from antibody fragments. In particular embodiments, the antibodies are single-chain antibody fragments comprising a variable heavy chain region and/or a variable light chain region, such as scFvs.

The term "variable region" or "variable domain" refers to the domain of an antibody heavy or light chain that is involved in binding the antibody to antigen. The variable domains of the heavy chain and light chain (V_{H} and V_{L}, respectively) of a native antibody generally have similar structures, with each domain comprising four conserved framework regions (FRs) and three CDRs. (See, *e.g.,* Kindt et al. Kuby Immunology, 6th ed., W.H. Freeman and Co., page 91 (2007). A single V_{H} or V_{L} domain may be sufficient to confer antigen-binding specificity. Furthermore, antibodies that bind a particular antigen may be isolated using a V_{H} or V_{L} domain from an antibody that binds the antigen to screen a library of complementary V_{L} or V_{H} domains, respectively. See, *e.g.,* Portolano et al., J. Immunol. 150:880-887 (1993); Clarkson et al., Nature 352:624-628 (1991).

Single-domain antibodies are antibody fragments comprising all or a portion of the heavy chain variable domain or all or a portion of the light chain variable domain of an antibody. In certain embodiments, a single-domain antibody is a human single-domain antibody. In some embodiments, the CAR comprises an antibody heavy chain domain that specifically binds the antigen, such as a cancer marker or cell surface antigen of a cell or disease to be targeted, such as a tumor cell or a cancer cell, such as any of the target antigens described herein or known in the art.

Antibody fragments can be made by various techniques, including but not limited to proteolytic digestion of an intact antibody as well as production by recombinant host cells. In some embodiments, the antibodies are recombinantly-produced fragments, such as fragments comprising arrangements that do not occur naturally, such as those with two or more antibody regions or chains joined by synthetic linkers, *e.g.,* peptide linkers, and/or that are may not be produced by enzyme digestion of a naturally-occurring intact antibody. In some embodiments, the antibody fragments are scFvs.

A "humanized" antibody is an antibody in which all or substantially all CDR amino acid residues are derived from non-human CDRs and all or substantially all FR amino acid residues are derived from human FRs. A humanized antibody optionally may include at least a portion of an antibody constant region derived from a human antibody. A "humanized form" of a non-human antibody, refers to a variant of the non-human antibody that has undergone humanization, typically to reduce immunogenicity to humans, while retaining the specificity and affinity of the parental non-human antibody. In some embodiments, some FR residues in a humanized antibody are substituted with corresponding residues from a non-human antibody (*e.g.,* the antibody from which the CDR residues are derived), *e.g.,* to restore or improve antibody specificity or affinity.

Thus, in some embodiments, the chimeric antigen receptor, including TCR-like CARs, includes an extracellular portion containing an antibody or antibody fragment. In some embodiments, the antibody or fragment includes an scFv. In some aspects, the chimeric antigen receptor includes an extracellular portion containing the antibody or fragment and an intracellular signaling region. In some embodiments, the intracellular signaling region comprises an intracellular signaling domain. In some embodiments, the intracellular signaling domain is or comprises a primary signaling domain, a signaling domain that is capable of inducing a primary activation signal in a T cell, a signaling domain of a T cell receptor (TCR) component, and/or a signaling domain comprising an immunoreceptor tyrosine-based activation motif (ITAM).

In some embodiments, the recombinant receptor such as the CAR, such as the antibody portion thereof, further includes a spacer, which may be or include at least a portion of an immunoglobulin constant region or variant or modified version thereof, such as a hinge region, e.g., an IgG4 hinge region, and/or a C_{H}1/C_{L} and/or Fc region. In some embodiments, the recombinant receptor further comprises a spacer and/or a hinge region. In some embodiments, the constant region or portion is of a human IgG, such as IgG4 or IgG1. In some aspects, the portion of the constant region serves as a spacer region between the antigen-recognition component, e.g., scFv, and transmembrane domain. The spacer can be of a length that provides for increased responsiveness of the cell following antigen binding, as compared to in the absence of the spacer. In some examples, the spacer is at or about 12 amino acids in length or is no more than 12 amino acids in length. Exemplary spacers include those having at least about 10 to 229 amino acids, about 10 to 200 amino acids, about 10 to 175 amino acids, about 10 to 150 amino acids, about 10 to 125 amino acids, about 10 to 100 amino acids, about 10 to 75 amino acids, about 10 to 50 amino acids, about 10 to 40 amino acids, about 10 to 30 amino acids, about 10 to 20 amino acids, or about 10 to 15 amino acids, and including any integer between the endpoints of any of the listed ranges. In some embodiments, a spacer region has about 12 amino acids or less, about 119 amino acids or less, or about 229 amino acids or less. Exemplary spacers include IgG4 hinge alone, IgG4 hinge linked to CH2 and CH3 domains, or IgG4 hinge linked to the CH3 domain. Exemplary spacers include, but are not limited to, those described in Hudecek et al. (2013) Clin. Cancer Res., 19:3153 or international patent application publication number WO2014031687. In some embodiments, the spacer has the sequence set forth in SEQ ID NO: 133, and is encoded by the sequence set forth in SEQ ID NO: 134. In some embodiments, the spacer has the sequence set forth in SEQ ID NO: 135. In some embodiments, the spacer has the sequence set forth in SEQ ID NO: 136.

In some embodiments, the constant region or portion is of IgD. In some embodiments, the spacer has the sequence set forth in SEQ ID NO: 137. In some embodiments, the spacer has a sequence of amino acids that exhibits at least 85%, 86%, 87%, 88%, 89%, 90%, 91%, 92%, 93%, 94%, 95%, 96%, 97%, 98%, 99% or more sequence identity to any of SEQ ID NOS: 133, 135, 136 and 137.

The antigen recognition domain generally is linked to one or more intracellular signaling components, such as signaling components that mimic activation through an antigen receptor complex, such as a TCR complex, in the case of a CAR, and/or signal via another cell surface receptor. Thus, in some embodiments, the antigen binding component (e.g., antibody) is linked to one or more transmembrane and intracellular signaling regions. In some embodiments, the transmembrane domain is fused to the extracellular domain. In one embodiment, a transmembrane domain that naturally is associated with one of the domains in the receptor, e.g., CAR, is used. In some instances, the transmembrane domain is selected or modified by amino acid substitution to avoid binding of such domains to the transmembrane domains of the same or different surface membrane proteins to minimize interactions with other members of the receptor complex.

The transmembrane domain in some embodiments is derived either from a natural or from a synthetic source. Where the source is natural, the domain in some aspects is derived from any membrane-bound or transmembrane protein. Transmembrane regions include those derived from (i.e. comprise at least the transmembrane region(s) of) the alpha, beta or zeta chain of the T-cell receptor, CD28, CD3 epsilon, CD45, CD4, CD5, CD8, CD9, CD 16, CD22, CD33, CD37, CD64, CD80, CD86, CD 134, CD137, CD 154. Alternatively the transmembrane domain in some embodiments is synthetic. In some aspects, the synthetic transmembrane domain comprises predominantly hydrophobic residues such as leucine and valine. In some aspects, a triplet of phenylalanine, tryptophan and valine will be found at each end of a synthetic transmembrane domain. In some embodiments, the linkage is by linkers, spacers, and/or transmembrane domain(s).

Among the intracellular signaling region are those that mimic or approximate a signal through a natural antigen receptor, a signal through such a receptor in combination with a costimulatory receptor, and/or a signal through a costimulatory receptor alone. In some embodiments, a short oligo- or polypeptide linker, for example, a linker of between 2 and 10 amino acids in length, such as one containing glycines and serines, e.g., glycine-serine doublet, is present and forms a linkage between the transmembrane domain and the cytoplasmic signaling domain of the CAR.

The receptor, e.g., the CAR, generally includes at least one intracellular signaling component or components. In some embodiments, the receptor includes an intracellular component of a TCR complex, such as a TCR CD3 chain that mediates T-cell activation and cytotoxicity, e.g., CD3 zeta chain. Thus, in some aspects, the antigen-binding domain of the CAR is linked to one or more cell signaling modules. In some embodiments, cell signaling modules include CD3 transmembrane domain, CD3 intracellular signaling domains, and/or other CD transmembrane domains. In some embodiments, the receptor, e.g., CAR, further includes a portion of one or more additional molecules such as Fc receptor γ, CD8, CD4, CD25, or CD16. For example, in some aspects, the CAR includes a chimeric molecule between CD3-zeta (CD3-ζ) or Fc receptor γ and CD8, CD4, CD25 or CD16.

In some embodiments, upon ligation of the CAR, the cytoplasmic domain or intracellular signaling region of the CAR activates at least one of the normal effector functions or responses of the immune cell, e.g., T cell engineered to express the CAR. For example, in some contexts, the CAR induces a function of a T cell such as cytolytic activity or T-helper activity, such as secretion of cytokines or other factors. In some embodiments, a truncated portion of an intracellular signaling region of an antigen receptor component or costimulatory molecule is used in place of an intact immunostimulatory chain, for example, if it transduces the effector function signal. In some embodiments, the intracellular signaling regions, e.g., comprising intracellular domain or domains, include the cytoplasmic sequences of the T cell receptor (TCR), and in some aspects also those of co-receptors that in the natural context act in concert with such receptor to initiate signal transduction following antigen receptor engagement, and/or any derivative or variant of such molecules, and/or any synthetic sequence that has the same functional capability.

In the context of a natural TCR, full activation generally requires not only signaling through the TCR, but also a costimulatory signal. Thus, in some embodiments, to promote full activation, a component for generating secondary or co-stimulatory signal is also included in the CAR. In other embodiments, the CAR does not include a component for generating a costimulatory signal. In some aspects, an additional CAR is expressed in the same cell and provides the component for generating the secondary or costimulatory signal.

T cell activation is in some aspects described as being mediated by two classes of cytoplasmic signaling sequences: those that initiate antigen-dependent primary activation through the TCR (primary cytoplasmic signaling sequences), and those that act in an antigen-independent manner to provide a secondary or co-stimulatory signal (secondary cytoplasmic signaling sequences). In some aspects, the CAR includes one or both of such signaling components.

In some aspects, the CAR includes a primary cytoplasmic signaling sequence that regulates primary activation of the TCR complex. Primary cytoplasmic signaling sequences that act in a stimulatory manner may contain signaling motifs which are known as immunoreceptor tyrosine-based activation motifs or ITAMs. Examples of ITAM containing primary cytoplasmic signaling sequences include those derived from TCR or CD3 zeta, FcR gamma or FcR beta. In some embodiments, cytoplasmic signaling molecule(s) in the CAR contain(s) a cytoplasmic signaling domain, portion thereof, or sequence derived from CD3 zeta.

In some embodiments, the CAR includes a signaling region and/or transmembrane portion of a costimulatory receptor, such as CD28, 4-1BB, OX40, DAP10, and ICOS. In some aspects, the same CAR includes both the signaling region and costimulatory components.

In some embodiments, the signaling region is included within one CAR, whereas the costimulatory component is provided by another CAR recognizing another antigen. In some embodiments, the CARs include activating or stimulatory CARs, and costimulatory CARs, both expressed on the same cell (*see* WO2014/055668).

In certain embodiments, the intracellular signaling region comprises a CD28 transmembrane and signaling domain linked to a CD3 (e.g., CD3-zeta) intracellular domain. In some embodiments, the intracellular signaling region comprises a chimeric CD28 and CD137 (4-1BB, TNFRSF9) co-stimulatory domains, linked to a CD3 zeta intracellular domain.

In some embodiments, the CAR encompasses one or more, e.g., two or more, costimulatory domains and an activation domain, e.g., primary activation domain, in the cytoplasmic portion. Exemplary CARs include intracellular components of CD3-zeta, CD28, and 4-1BB.

In some cases, CARs are referred to as first, second, and/or third generation CARs. In some aspects, a first generation CAR is one that solely provides a CD3-chain induced signal upon antigen binding; in some aspects, a second-generation CARs is one that provides such a signal and costimulatory signal, such as one including an intracellular signaling domain from a costimulatory receptor such as CD28 or CD137; in some aspects, a third generation CAR in some aspects is one that includes multiple costimulatory domains of different costimulatory receptors.

In some embodiments, the chimeric antigen receptor includes an extracellular portion containing the antibody or fragment described herein. In some aspects, the chimeric antigen receptor includes an extracellular portion containing the antibody or fragment described herein and an intracellular signaling domain. In some embodiments, the antibody or fragment includes an scFv or a single-domain V_{H} antibody and the intracellular domain contains an ITAM. In some aspects, the intracellular signaling domain includes a signaling domain of a zeta chain of a CD3-zeta (CD3ζ) chain. In some embodiments, the chimeric antigen receptor includes a transmembrane domain disposed between the extracellular domain and the intracellular signaling region.

In some aspects, the transmembrane domain contains a transmembrane portion of CD28. The extracellular domain and transmembrane can be linked directly or indirectly. In some embodiments, the extracellular domain and transmembrane are linked by a spacer, such as any described herein. In some embodiments, the chimeric antigen receptor contains an intracellular domain of a T cell costimulatory molecule, such as between the transmembrane domain and intracellular signaling domain. In some aspects, the T cell costimulatory molecule is CD28 or 4-1BB.

In some embodiments, the CAR contains an antibody, e.g., an antibody fragment, a transmembrane domain that is or contains a transmembrane portion of CD28 or a functional variant thereof, and an intracellular signaling domain containing a signaling portion of CD28 or functional variant thereof and a signaling portion of CD3 zeta or functional variant thereof. In some embodiments, the CAR contains an antibody, e.g., antibody fragment, a transmembrane domain that is or contains a transmembrane portion of CD28 or a functional variant thereof, and an intracellular signaling domain containing a signaling portion of a 4-1BB or functional variant thereof and a signaling portion of CD3 zeta or functional variant thereof. In some such embodiments, the receptor further includes a spacer containing a portion of an Ig molecule, such as a human Ig molecule, such as an Ig hinge, e.g. an IgG4 hinge, such as a hinge-only spacer.

In some embodiments, the transmembrane domain of the receptor, e.g., the CAR is a transmembrane domain of human CD28 or variant thereof, e.g., a 27-amino acid transmembrane domain of a human CD28 (Accession No.: P10747.1), or is a transmembrane domain that comprises the sequence of amino acids set forth in SEQ ID NO: 138 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO:138; in some embodiments, the transmembrane-domain containing portion of the recombinant receptor comprises the sequence of amino acids set forth in SEQ ID NO: 139 or a sequence of amino acids having at least at or about 85%, 86%, 87%, 88%, 89%, 90%, 91%, 92%, 93%, 94%, 95%, 96%, 97%, 98%, 99% or more sequence identity thereto.

In some embodiments, the chimeric antigen receptor contains an intracellular domain of a T cell costimulatory molecule. In some aspects, the T cell costimulatory molecule is CD28 or 4-1BB.

In some embodiments, the intracellular signaling region comprises an intracellular costimulatory signaling domain of human CD28 or functional variant or portion thereof, such as a 41 amino acid domain thereof and/or such a domain with an LL to GG substitution at positions 186-187 of a native CD28 protein. In some embodiments, the intracellular signaling domain can comprise the sequence of amino acids set forth in SEQ ID NO: 140 or 141 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 140 or 141. In some embodiments, the intracellular region comprises an intracellular costimulatory signaling domain of 4-1BB or functional variant or portion thereof, such as a 42-amino acid cytoplasmic domain of a human 4-1BB (Accession No. Q07011.1) or functional variant or portion thereof, such as the sequence of amino acids set forth in SEQ ID NO: 142 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 142.

In some embodiments, the intracellular signaling region comprises a human CD3 chain, optionally a CD3 zeta stimulatory signaling domain or functional variant thereof, such as an 112 AA cytoplasmic domain of isoform 3 of human CD3ζ (Accession No.: P20963.2) or a CD3 zeta signaling domain as described in U.S. Patent No.: 7,446,190 or U.S. Patent No. 8,911,993. In some embodiments, the intracellular signaling region comprises the sequence of amino acids set forth in SEQ ID NO: 143, 144 or 145 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 143, 144 or 145.

In some aspects, the spacer contains only a hinge region of an IgG, such as only a hinge of IgG4 or IgG1, such as the hinge only spacer set forth in SEQ ID NO:133. In other embodiments, the spacer is an Ig hinge, e.g., and IgG4 hinge, linked to a C_{H}2 and/or C_{H}3 domains. In some embodiments, the spacer is an Ig hinge, e.g., an IgG4 hinge, linked to C_{H}2 and C_{H}3 domains, such as set forth in SEQ ID NO: 135. In some embodiments, the spacer is an Ig hinge, e.g., an IgG4 hinge, linked to a C_{H}3 domain only, such as set forth in SEQ ID NO:136. In some embodiments, the spacer is or comprises a glycine-serine rich sequence or other flexible linker such as known flexible linkers.

In some embodiments, the recombinant receptor, e.g. CAR or other antigen receptor further includes a marker, such as a cell surface marker, which may be used to confirm transduction or engineering of the cell to express the receptor, such as a truncated version of a cell surface receptor, such as truncated EGFR (tEGFR). In some aspects, the marker includes all or part (e.g., truncated form) of CD34, a NGFR, or epidermal growth factor receptor (e.g., tEGFR). In certain embodiments, the tEGFR contains an amino acid sequence that is set forth in SEQ ID NO: 147 or 148. In particular embodiments, the tEGFR contains an amino acid sequence with or with about or at least 75%, 80%, 85%, 90%, 95%, 96%, 97%, 98%, 99%, or 99% identity to the sequence set forth in SEQ ID NO: 147 or 148. In some embodiments, the nucleic acid encoding the marker is operably linked to a polynucleotide encoding for a linker sequence, such as a cleavable linker sequence, e.g., T2A. For example, a marker, and optionally a linker sequence, can be any as disclosed in published patent application No. WO2014031687. For example, the marker can be a truncated EGFR (tEGFR) that is, optionally, linked to a linker sequence, such as a T2A cleavable linker sequence.

In certain cases where nucleic acid molecules encode two or more different polypeptide chains, each of the polypeptide chains can be encoded by a separate nucleic acid molecule. For example, two separate nucleic acids are provided, and each can be individually transferred or introduced into the cell for expression in the cell.

In some embodiments, such as those where the polynucleotide contains a first and second nucleic acid sequence, the coding sequences encoding each of the different polypeptide chains can be operatively linked to a promoter, which can be the same or different. In some embodiments, the nucleic acid molecule can contain a promoter that drives the expression of two or more different polypeptide chains. In some embodiments, such nucleic acid molecules can be multicistronic (bicistronic or tricistronic, see *e.g.,* U.S. Patent No. 6,060,273). In some embodiments, transcription units can be engineered as a bicistronic unit containing an IRES (internal ribosome entry site), which allows coexpression of gene products ((*e.g*. encoding the PSMA or modified form thereof and encoding the recombinant receptor) by a message from a single promoter. Alternatively, in some cases, a single promoter may direct expression of an RNA that contains, in a single open reading frame (ORF), two or three genes (*e.g.* encoding the PSMA or modified form thereof and encoding the recombinant receptor) separated from one another by sequences encoding a self-cleavage peptide (*e.g.,* 2A sequences) or a protease recognition site *(e.g.,* furin). The ORF thus encodes a single polypeptide, which, either during (in the case of 2A) or after translation, is processed into the individual proteins. In some cases, the peptide, such as a T2A, can cause the ribosome to skip (ribosome skipping) synthesis of a peptide bond at the C-terminus of a 2A element, leading to separation between the end of the 2A sequence and the next peptide downstream (*see,* for example, de Felipe. Genetic Vaccines and Ther. 2:13 (2004) and deFelipe et al. Traffic 5:616-626 (2004)). Various 2A elements are known. Examples of 2A sequences that can be used in the methods and system disclosed herein, without limitation, 2A sequences from the foot-and-mouth disease virus (F2A, *e.g.,* SEQ ID NO: 32), equine rhinitis A virus (E2A, *e.g.,* SEQ ID NO: 153), Thosea asigna virus (T2A, *e.g.,* SEQ ID NO: 146 or 149), and porcine teschovirus-1 (P2A, *e.g.,* SEQ ID NO: 151 or 152) as described in U.S. Patent Publication No. 20070116690.

### 2. T Cell Receptors (TCRs)

In some embodiments, engineered cells, such as T cells, are provided that express a T cell receptor (TCR) or antigen-binding portion thereof that recognizes an peptide epitope or T cell epitope of a target polypeptide, such as an antigen of a tumor, viral or autoimmune protein.

In some embodiments, a "T cell receptor" or "TCR" is a molecule that contains a variable α and β chains (also known as TCRα and TCRβ, respectively) or a variable γ and δ chains (also known as TCRα and TCRβ, respectively), or antigen-binding portions thereof, and which is capable of specifically binding to a peptide bound to an MHC molecule. In some embodiments, the TCR is in the αβ form. Typically, TCRs that exist in αβ and γδ forms are generally structurally similar, but T cells expressing them may have distinct anatomical locations or functions. A TCR can be found on the surface of a cell or in soluble form. Generally, a TCR is found on the surface of T cells (or T lymphocytes) where it is generally responsible for recognizing antigens bound to major histocompatibility complex (MHC) molecules.

Unless otherwise stated, the term "TCR" should be understood to encompass full TCRs as well as antigen-binding portions or antigen-binding fragments thereof. In some embodiments, the TCR is an intact or full-length TCR, including TCRs in the αβ form or γδ form. In some embodiments, the TCR is an antigen-binding portion that is less than a full-length TCR but that binds to a specific peptide bound in an MHC molecule, such as binds to an MHC-peptide complex. In some cases, an antigen-binding portion or fragment of a TCR can contain only a portion of the structural domains of a full-length or intact TCR, but yet is able to bind the peptide epitope, such as MHC-peptide complex, to which the full TCR binds. In some cases, an antigen-binding portion contains the variable domains of a TCR, such as variable α chain and variable β chain of a TCR, sufficient to form a binding site for binding to a specific MHC-peptide complex. Generally, the variable chains of a TCR contain complementarity determining regions involved in recognition of the peptide, MHC and/or MHC-peptide complex.

In some embodiments, the variable domains of the TCR contain hypervariable loops, or complementarity determining regions (CDRs), which generally are the primary contributors to antigen recognition and binding capabilities and specificity. In some embodiments, a CDR of a TCR or combination thereof forms all or substantially all of the antigen-binding site of a given TCR molecule. The various CDRs within a variable region of a TCR chain generally are separated by framework regions (FRs), which generally display less variability among TCR molecules as compared to the CDRs (see, e.g., Jores et al., Proc. Nat'l Acad. Sci. U.S.A. 87:9138, 1990; Chothia et al., EMBO J. 7:3745, 1988; see also Lefranc et al., Dev. Comp. Immunol. 27:55, 2003). In some embodiments, CDR3 is the main CDR responsible for antigen binding or specificity, or is the most important among the three CDRs on a given TCR variable region for antigen recognition, and/or for interaction with the processed peptide portion of the peptide-MHC complex. In some contexts, the CDR1 of the alpha chain can interact with the N-terminal part of certain antigenic peptides. In some contexts, CDR1 of the beta chain can interact with the C-terminal part of the peptide. In some contexts, CDR2 contributes most strongly to or is the primary CDR responsible for the interaction with or recognition of the MHC portion of the MHC-peptide complex. In some embodiments, the variable region of the β-chain can contain a further hypervariable region (CDR4 or HVR4), which generally is involved in superantigen binding and not antigen recognition (Kotb (1995) Clinical Microbiology Reviews, 8:411-426).

In some embodiments, a TCR also can contain a constant domain, a transmembrane domain and/or a short cytoplasmic tail (see, e.g., Janeway et al., Immunobiology: The Immune System in Health and Disease, 3rd Ed., Current Biology Publications, p. 4:33, 1997). In some aspects, each chain of the TCR can possess one N-terminal immunoglobulin variable domain, one immunoglobulin constant domain, a transmembrane region, and a short cytoplasmic tail at the C-terminal end. In some embodiments, a TCR is associated with invariant proteins of the CD3 complex involved in mediating signal transduction.

In some embodiments, a TCR chain contains one or more constant domain. For example, the extracellular portion of a given TCR chain (e.g., α-chain or β-chain) can contain two immunoglobulin-like domains, such as a variable domain (e.g., Vα or Vβ; typically amino acids 1 to 116 based on Kabat numbering Kabat et al., "Sequences of Proteins of Immunological Interest, US Dept. Health and Human Services, Public Health Service National Institutes of Health, 1991, 5th ed.) and a constant domain (e.g., α-chain constant domain or Cα, typically positions 117 to 259 of the chain based on Kabat numbering or β chain constant domain or C_{β}, typically positions 117 to 295 of the chain based on Kabat) adjacent to the cell membrane. For example, in some cases, the extracellular portion of the TCR formed by the two chains contains two membrane-proximal constant domains, and two membrane-distal variable domains, which variable domains each contain CDRs. The constant domain of the TCR may contain short connecting sequences in which a cysteine residue forms a disulfide bond, thereby linking the two chains of the TCR. In some embodiments, a TCR may have an additional cysteine residue in each of the α and β chains, such that the TCR contains two disulfide bonds in the constant domains.

In some embodiments, the TCR chains contain a transmembrane domain. In some embodiments, the transmembrane domain is positively charged. In some cases, the TCR chain contains a cytoplasmic tail. In some cases, the structure allows the TCR to associate with other molecules like CD3 and subunits thereof. For example, a TCR containing constant domains with a transmembrane region may anchor the protein in the cell membrane and associate with invariant subunits of the CD3 signaling apparatus or complex. The intracellular tails of CD3 signaling subunits (e.g. CD3γ, CD3δ, CD3ε and CD3ζ chains) contain one or more immunoreceptor tyrosine-based activation motif or ITAM that are involved in the signaling capacity of the TCR complex.

In some embodiments, the TCR may be a heterodimer of two chains α and β (or optionally γ and δ) or it may be a single chain TCR construct. In some embodiments, the TCR is a heterodimer containing two separate chains (α and β chains or γ and δ chains) that are linked, such as by a disulfide bond or disulfide bonds.

In some embodiments, the TCR can be generated from a known TCR sequence(s), such as sequences of Vα,β chains, for which a substantially full-length coding sequence is readily available. Methods for obtaining full-length TCR sequences, including V chain sequences, from cell sources are well known. In some embodiments, nucleic acids encoding the TCR can be obtained from a variety of sources, such as by polymerase chain reaction (PCR) amplification of TCR-encoding nucleic acids within or isolated from a given cell or cells, or synthesis of publicly available TCR DNA sequences.

In some embodiments, the TCR is obtained from a biological source, such as from cells such as from a T cell (e.g. cytotoxic T cell), T-cell hybridomas or other publicly available source. In some embodiments, the T-cells can be obtained from *in vivo* isolated cells. In some embodiments, the TCR is a thymically selected TCR. In some embodiments, the TCR is a neoepitope-restricted TCR. In some embodiments, the T- cells can be a cultured T-cell hybridoma or clone. In some embodiments, the TCR or antigen-binding portion thereof or antigen-binding fragment thereof can be synthetically generated from knowledge of the sequence of the TCR.

In some embodiments, the TCR is generated from a TCR identified or selected from screening a library of candidate TCRs against a target polypeptide antigen, or target T cell epitope thereof. TCR libraries can be generated by amplification of the repertoire of Vα and Vβ from T cells isolated from a subject, including cells present in PBMCs, spleen or other lymphoid organ. In some cases, T cells can be amplified from tumor-infiltrating lymphocytes (TILs). In some embodiments, TCR libraries can be generated from CD4+ or CD8+ cells. In some embodiments, the TCRs can be amplified from a T cell source of a normal of healthy subject, i.e. normal TCR libraries. In some embodiments, the TCRs can be amplified from a T cell source of a diseased subject, i.e. diseased TCR libraries. In some embodiments, degenerate primers are used to amplify the gene repertoire of Vα and Vβ, such as by RT-PCR in samples, such as T cells, obtained from humans. In some embodiments, scTv libraries can be assembled from naive Vα and Vβ libraries in which the amplified products are cloned or assembled to be separated by a linker. Depending on the source of the subject and cells, the libraries can be HLA allele-specific. Alternatively, in some embodiments, TCR libraries can be generated by mutagenesis or diversification of a parent or scaffold TCR molecule. In some aspects, the TCRs are subjected to directed evolution, such as by mutagenesis, e.g., of the α or β chain. In some aspects, particular residues within CDRs of the TCR are altered. In some embodiments, selected TCRs can be modified by affinity maturation. In some embodiments, antigen-specific T cells may be selected, such as by screening to assess CTL activity against the peptide. In some aspects, TCRs, e.g. present on the antigen-specific T cells, may be selected, such as by binding activity, e.g., particular affinity or avidity for the antigen.

In some embodiments, the TCR or antigen-binding portion thereof is one that has been modified or engineered. In some embodiments, directed evolution methods are used to generate TCRs with altered properties, such as with higher affinity for a specific MHC-peptide complex. In some embodiments, directed evolution is achieved by display methods including, but not limited to, yeast display (Holler et al. (2003) Nat Immunol, 4, 55-62; Holler et al. (2000) Proc Natl Acad Sci U S A, 97, 5387-92), phage display (Li et al. (2005) Nat Biotechnol, 23, 349-54), or T cell display (Chervin et al. (2008) J Immunol Methods, 339, 175-84). In some embodiments, display approaches involve engineering, or modifying, a known, parent or reference TCR. For example, in some cases, a wild-type TCR can be used as a template for producing mutagenized TCRs in which in one or more residues of the CDRs are mutated, and mutants with an desired altered property, such as higher affinity for a desired target antigen, are selected.

In some embodiments, peptides of a target polypeptide for use in producing or generating a TCR of interest are known or can be readily identified by a skilled artisan. In some embodiments, peptides suitable for use in generating TCRs or antigen-binding portions can be determined based on the presence of an HLA-restricted motif in a target polypeptide of interest, such as a target polypeptide described below. In some embodiments, peptides are identified using available computer prediction models. In some embodiments, for predicting MHC class I binding sites, such models include, but are not limited to, ProPred1 (Singh and Raghava (2001) Bioinformatics 17(12):1236-1237, and SYFPEITHI (see Schuler et al. (2007) Immunoinformatics Methods in Molecular Biology, 409(1): 75-93 2007). In some embodiments, the MHC-restricted epitope is HLA-A0201, which is expressed in approximately 39-46% of all Caucasians and therefore, represents a suitable choice of MHC antigen for use preparing a TCR or other MHC-peptide binding molecule.

HLA-A0201-binding motifs and the cleavage sites for proteasomes and immune-proteasomes using computer prediction models are known to those of skill in the art. For predicting MHC class I binding sites, such models include, but are not limited to, ProPred1 (described in more detail in Singh and Raghava, ProPred: prediction of HLA-DR binding sites. BIOINFORMATICS 17(12):1236-1237 2001), and SYFPEITHI (see Schuler et al. SYFPEITHI, Database for Searching and T-Cell Epitope Prediction. in Immunoinformatics Methods in Molecular Biology, vol. 409(1): 75-93 2007)

In some embodiments, the TCR or antigen binding portion thereof may be a recombinantly produced natural protein or mutated form thereof in which one or more property, such as binding characteristic, has been altered. In some embodiments, a TCR may be derived from one of various animal species, such as human, mouse, rat, or other mammal. A TCR may be cell-bound or in soluble form. In some embodiments, for purposes of the provided methods, the TCR is in cell-bound form expressed on the surface of a cell.

In some embodiments, the TCR is a full-length TCR. In some embodiments, the TCR is an antigen-binding portion. In some embodiments, the TCR is a dimeric TCR (dTCR). In some embodiments, the TCR is a single-chain TCR (sc-TCR). In some embodiments, a dTCR or scTCR have the structures as described in WO 03/020763, WO 04/033685, WO2011/044186.

In some embodiments, the TCR contains a sequence corresponding to the transmembrane sequence. In some embodiments, the TCR does contain a sequence corresponding to cytoplasmic sequences. In some embodiments, the TCR is capable of forming a TCR complex with CD3. In some embodiments, any of the TCRs, including a dTCR or scTCR, can be linked to signaling domains that yield an active TCR on the surface of a T cell. In some embodiments, the TCR is expressed on the surface of cells.

In some embodiments a dTCR contains a first polypeptide wherein a sequence corresponding to a TCR α chain variable region sequence is fused to the N terminus of a sequence corresponding to a TCR α chain constant region extracellular sequence, and a second polypeptide wherein a sequence corresponding to a TCR β chain variable region sequence is fused to the N terminus a sequence corresponding to a TCR β chain constant region extracellular sequence, the first and second polypeptides being linked by a disulfide bond. In some embodiments, the bond can correspond to the native inter-chain disulfide bond present in native dimeric αβ TCRs. In some embodiments, the interchain disulfide bonds are not present in a native TCR. For example, in some embodiments, one or more cysteines can be incorporated into the constant region extracellular sequences of dTCR polypeptide pair. In some cases, both a native and a non-native disulfide bond may be desirable. In some embodiments, the TCR contains a transmembrane sequence to anchor to the membrane.

In some embodiments, a dTCR contains a TCR α chain containing a variable α domain, a constant α domain and a first dimerization motif attached to the C-terminus of the constant α domain, and a TCR β chain comprising a variable β domain, a constant β domain and a first dimerization motif attached to the C-terminus of the constant β domain, wherein the first and second dimerization motifs easily interact to form a covalent bond between an amino acid in the first dimerization motif and an amino acid in the second dimerization motif linking the TCR α chain and TCR β chain together.

In some embodiments, the TCR is a scTCR. Typically, a scTCR can be generated using methods known to those of skill in the art, See e.g., Soo Hoo, W. F. et al. PNAS (USA) 89, 4759 (1992); Wiilfing, C. and Pliickthun, A., J. Mol. Biol. 242, 655 (1994); Kurucz, I. et al. PNAS (USA) 90 3830 (1993); International published PCT Nos. WO 96/13593, WO 96/18105, WO99/60120, WO99/18129, WO 03/020763, WO2011/044186; and Schlueter, C. J. et al. J. Mol. Biol. 256, 859 (1996). In some embodiments, a scTCR contains an introduced non-native disulfide interchain bond to facilitate the association of the TCR chains (see e.g. International published PCT No. WO 03/020763). In some embodiments, a scTCR is a non-disulfide linked truncated TCR in which heterologous leucine zippers fused to the C-termini thereof facilitate chain association (see e.g. International published PCT No. WO99/60120). In some embodiments, a scTCR contain a TCRα variable domain covalently linked to a TCRβ variable domain via a peptide linker (see e.g., International published PCT No. WO99/18129).

In some embodiments, a scTCR contains a first segment constituted by an amino acid sequence corresponding to a TCR α chain variable region, a second segment constituted by an amino acid sequence corresponding to a TCR β chain variable region sequence fused to the N terminus of an amino acid sequence corresponding to a TCR β chain constant domain extracellular sequence, and a linker sequence linking the C terminus of the first segment to the N terminus of the second segment.

In some embodiments, a scTCR contains a first segment constituted by an α chain variable region sequence fused to the N terminus of an α chain extracellular constant domain sequence, and a second segment constituted by a β chain variable region sequence fused to the N terminus of a sequence β chain extracellular constant and transmembrane sequence, and, optionally, a linker sequence linking the C terminus of the first segment to the N terminus of the second segment.

In some embodiments, a scTCR contains a first segment constituted by a TCR β chain variable region sequence fused to the N terminus of a β chain extracellular constant domain sequence, and a second segment constituted by an α chain variable region sequence fused to the N terminus of a sequence α chain extracellular constant and transmembrane sequence, and, optionally, a linker sequence linking the C terminus of the first segment to the N terminus of the second segment.

In some embodiments, the linker of a scTCRs that links the first and second TCR segments can be any linker capable of forming a single polypeptide strand, while retaining TCR binding specificity. In some embodiments, the linker sequence may, for example, have the formula -P-AA-P- wherein P is proline and AA represents an amino acid sequence wherein the amino acids are glycine and serine. In some embodiments, the first and second segments are paired so that the variable region sequences thereof are orientated for such binding. Hence, in some cases, the linker has a sufficient length to span the distance between the C terminus of the first segment and the N terminus of the second segment, or vice versa, but is not too long to block or reduces bonding of the scTCR to the target ligand. In some embodiments, the linker can contain from or from about 10 to 45 amino acids, such as 10 to 30 amino acids or 26 to 41 amino acids residues, for example 29, 30, 31 or 32 amino acids. In some embodiments, the linker has the formula -PGGG-(SGGGG)5-P- wherein P is proline, G is glycine and S is serine (SEQ ID NO:28). In some embodiments, the linker has the sequence
GSADDAKKDAAKKDGKS (SEQ ID NO:29)

In some embodiments, the scTCR contains a covalent disulfide bond linking a residue of the immunoglobulin region of the constant domain of the α chain to a residue of the immunoglobulin region of the constant domain of the β chain. In some embodiments, the interchain disulfide bond in a native TCR is not present. For example, in some embodiments, one or more cysteines can be incorporated into the constant region extracellular sequences of the first and second segments of the scTCR polypeptide. In some cases, both a native and a non-native disulfide bond may be desirable.

In some embodiments of a dTCR or scTCR containing introduced interchain disulfide bonds, the native disulfide bonds are not present. In some embodiments, the one or more of the native cysteines forming a native interchain disulfide bonds are substituted to another residue, such as to a serine or alanine. In some embodiments, an introduced disulfide bond can be formed by mutating non-cysteine residues on the first and second segments to cysteine. Exemplary non-native disulfide bonds of a TCR are described in published International PCT No. WO2006/000830.

In some embodiments, the TCR or antigen-binding fragment thereof exhibits an affinity with an equilibrium binding constant for a target antigen of between or between about 10-5 and 10-12 M and all individual values and ranges therein. In some embodiments, the target antigen is an MHC-peptide complex or ligand.

In some embodiments, nucleic acid or nucleic acids encoding a TCR, such as α and β chains, can be amplified by PCR, cloning or other suitable means and cloned into a suitable expression vector or vectors. The expression vector can be any suitable recombinant expression vector, and can be used to transform or transfect any suitable host. Suitable vectors include those designed for propagation and expansion or for expression or both, such as plasmids and viruses.

In some embodiments, the vector can a vector of the pUC series (Fermentas Life Sciences), the pBluescript series (Stratagene, LaJolla, Calif.), the pET series (Novagen, Madison, Wis.), the pGEX series (Pharmacia Biotech, Uppsala, Sweden), or the pEX series (Clontech, Palo Alto, Calif.). In some cases, bacteriophage vectors, such as λG10, λGT11, λZapII (Stratagene), λEMBL4, and λNM1149, also can be used. In some embodiments, plant expression vectors can be used and include pBI01, pBI101.2, pBI101.3, pBI121 and pBIN19 (Clontech). In some embodiments, animal expression vectors include pEUK-Cl, pMAM and pMAMneo (Clontech). In some embodiments, a viral vector is used, such as a retroviral vector.

In some embodiments, the recombinant expression vectors can be prepared using standard recombinant DNA techniques. In some embodiments, vectors can contain regulatory sequences, such as transcription and translation initiation and termination codons, which are specific to the type of host (e.g., bacterium, fungus, plant, or animal) into which the vector is to be introduced, as appropriate and taking into consideration whether the vector is DNA- or RNA-based. In some embodiments, the vector can contain a nonnative promoter operably linked to the nucleotide sequence encoding the TCR or antigen-binding portion (or other MHC-peptide binding molecule). In some embodiments, the promoter can be a non-viral promoter or a viral promoter, such as a cytomegalovirus (CMV) promoter, an SV40 promoter, an RSV promoter, and a promoter found in the long-terminal repeat of the murine stem cell virus. Other known promoters also are contemplated.

In some embodiments, to generate a vector encoding a TCR, the α and β chains are PCR amplified from total cDNA isolated from a T cell clone expressing the TCR of interest and cloned into an expression vector. In some embodiments, the α and β chains are cloned into the same vector. In some embodiments, the α and β chains are cloned into different vectors. In some embodiments, the generated α and β chains are incorporated into a retroviral, e.g. lentiviral, vector.

### C. Introduction of Nucleic Acids and Vectors into Cells

Various methods for the introduction of genetically engineered components, e.g., recombinant receptors, e.g., CARs or TCRs, are well known and may be used with the provided methods and compositions. Exemplary methods include those for transfer of nucleic acids encoding the polypeptides or receptors, including via viral vectors, e.g., retroviral or lentiviral, non-viral vectors or transposons, e.g. *Sleeping Beauty* transposon system. Methods of gene transfer can include transduction, electroporation or other method that results into gene transfer into the cell.

In some embodiments, gene transfer is accomplished by first stimulating the cell, such as by combining it with a stimulus that induces a response such as proliferation, survival, and/or activation, e.g., as measured by expression of a cytokine or activation marker, followed by transduction of the activated cells, and expansion in culture to numbers sufficient for clinical applications.

In some contexts, it may be desired to safeguard against the potential that overexpression of a stimulatory factor (for example, a lymphokine or a cytokine) could potentially result in an unwanted outcome or lower efficacy in a subject, such as a factor associated with toxicity in a subject. Thus, in some contexts, the engineered cells include gene segments that cause the cells to be susceptible to negative selection *in vivo,* such as upon administration in adoptive immunotherapy. For example in some aspects, the cells are engineered so that they can be eliminated as a result of a change in the *in vivo* condition of the patient to which they are administered. The negative selectable phenotype may result from the insertion of a gene that confers sensitivity to an administered agent, for example, a compound. Negative selectable genes include the Herpes simplex virus type I thymidine kinase (HSV-I TK) gene (Wigler et al., Cell 2 :223, I977) which confers ganciclovir sensitivity; the cellular hypoxanthine phosphribosyltransferase (HPRT) gene, the cellular adenine phosphoribosyltransferase (APRT) gene, bacterial cytosine deaminase, (Mullen et al., Proc. Natl. Acad. Sci. USA. 89:33 (1992)).

In some embodiments, the cells, e.g., T cells, may be transfected either during or after expansion, e.g. with nucleic acids encoding recombinant receptor, e.g., a T cell receptor (TCR) or a chimeric antigen receptor (CAR). This transfection for the introduction of the gene of the desired polypeptide or receptor can be carried out with any suitable gene-based transfer system, including via retroviral vector. In some embodiments, the genetically engineered cell population can, concurrently, simultaneously or subsequently, be stimulated with an antigenic stimulus e.g. via the de novo introduced receptor, such as in accord with the provided methods. In some embodiments, the binding molecule present on the provided particles, e.g., beads, provides an antigenic stimulus, such as provided in the form of a the cognate (cross-linking) antigen or ligand of the genetically introduced receptor (e.g. natural ligand of a CAR) or an anti-idiotyptic antibody that directly binds or recognizes the recombinant receptor.

Among additional nucleic acids, e.g., genes for introduction are those to improve the efficacy of therapy, such as by promoting viability and/or function of transferred cells; genes to provide a genetic marker for selection and/or evaluation of the cells, such as to assess *in vivo* survival or localization; genes to improve safety, for example, by making the cell susceptible to negative selection *in vivo* as described by Lupton S. D. et al., Mol. and Cell Biol., 11:6 (1991); and Riddell et al., Human Gene Therapy 3:319-338 (1992); see also the publications of PCT/US91/08442 and PCT/US94/05601 by Lupton et al. describing the use of bifunctional selectable fusion genes derived from fusing a dominant positive selectable marker with a negative selectable marker. *See,* e.g., Riddell et al., US Patent No. 6,040,177, at columns 14-17.

As described above, in some embodiments, the cells are incubated and/or cultured prior to or in connection with genetic engineering. The incubation steps can include culture, cultivation, stimulation, activation, propagation and/or freezing for preservation, e.g. cryopreservation.

### 1. Viral Vector Particles

In some embodiments, recombinant nucleic acids are transferred into cells using recombinant infectious virus particles, such as, e.g., vectors derived from simian virus 40 (SV40), adenoviruses, adeno-associated virus (AAV). In some embodiments, recombinant nucleic acids are transferred into T cells using recombinant lentiviral vectors or retroviral vectors, such as gamma-retroviral vectors (see, e.g., Koste et al. (2014) Gene Therapy 2014 Apr 3. doi: 10.1038/gt.2014.25; Carlens et al. (2000) Exp Hematol 28(10): 1137-46; Alonso-Camino et al. (2013) Mol Ther Nucl Acids 2, e93; Park et al., Trends Biotechnol. 2011 November 29(11): 550-557.

In some embodiments, the retroviral vector has a long terminal repeat sequence (LTR), e.g., a retroviral vector derived from the Moloney murine leukemia virus (MoMLV), myeloproliferative sarcoma virus (MPSV), murine embryonic stem cell virus (MESV), murine stem cell virus (MSCV), spleen focus forming virus (SFFV), or adeno-associated virus (AAV). Most retroviral vectors are derived from murine retroviruses. In some embodiments, the retroviruses include those derived from any avian or mammalian cell source. The retroviruses typically are amphotropic, meaning that they are capable of infecting host cells of several species, including humans. In one embodiment, the gene to be expressed replaces the retroviral gag, pol and/or env sequences. A number of illustrative retroviral systems have been described (e.g., U.S. Pat. Nos. 5,219,740; 6,207,453; 5,219,740; Miller and Rosman (1989) BioTechniques 7:980-990; Miller, A. D. (1990) Human Gene Therapy 1:5-14; Scarpa et al. (1991) Virology 180:849-852; Burns et al. (1993) Proc. Natl. Acad. Sci. USA 90:8033-8037; and Boris-Lawrie and Temin (1993) Cur. Opin. Genet. Develop. 3:102-109.

Methods of lentiviral transduction are known. Exemplary methods are described in, e.g., Wang et al. (2012) J. Immunother. 35(9): 689-701; Cooper et al. (2003) Blood. 101:1637-1644; Verhoeyen et al. (2009) Methods Mol Biol. 506: 97-114; and Cavalieri et al. (2003) Blood. 102(2): 497-505.

In some embodiments, the viral vector particles contain a genome derived from a retroviral genome based vector, such as derived from a lentiviral genome based vector. In some aspects of the provided viral vectors, the heterologous nucleic acid encoding a recombinant receptor, such as an antigen receptor, such as a CAR, is contained and/or located between the 5' LTR and 3' LTR sequences of the vector genome.

In some embodiments, the viral vector genome is a lentivirus genome, such as an HIV-1 genome or an SIV genome. For example, lentiviral vectors have been generated by multiply attenuating virulence genes, for example, the genes env, vif, vpu and nef can be deleted, making the vector safer for therapeutic purposes. Lentiviral vectors are known. See Naldini et al., (1996 and 1998); Zufferey et al., (1997); Dull et al., 1998, U.S. Pat. Nos. 6,013,516; and 5,994,136). In some embodiments, these viral vectors are plasmid-based or virus-based, and are configured to carry the essential sequences for incorporating foreign nucleic acid, for selection, and for transfer of the nucleic acid into a host cell. Known lentiviruses can be readily obtained from depositories or collections such as the American Type Culture Collection ("ATCC"; 10801 University Blvd., Manassas, Va. 20110-2209), or isolated from known sources using commonly available techniques.

Non-limiting examples of lentiviral vectors include those derived from a lentivirus, such as Human Immunodeficiency Virus 1 (HIV-1), HIV-2, an Simian Immunodeficiency Virus (SIV), Human T-lymphotropic virus 1 (HTLV-1), HTLV-2 or equine infection anemia virus (E1AV). For example, lentiviral vectors have been generated by multiply attenuating the HIV virulence genes, for example, the genes env, vif, vpr, vpu and nef are deleted, making the vector safer for therapeutic purposes. Lentiviral vectors are known in the art, see Naldini et al., (1996 and 1998); Zufferey et al., (1997); Dull et al., 1998, U.S. Pat. Nos. 6,013,516; and 5,994,136). In some embodiments, these viral vectors are plasmid-based or virus-based, and are configured to carry the essential sequences for incorporating foreign nucleic acid, for selection, and for transfer of the nucleic acid into a host cell. Known lentiviruses can be readily obtained from depositories or collections such as the American Type Culture Collection ("ATCC"; 10801 University Blvd., Manassas, Va. 20110-2209), or isolated from known sources using commonly available techniques.

In some embodiments, the viral genome vector can contain sequences of the 5' and 3' LTRs of a retrovirus, such as a lentivirus. In some aspects, the viral genome construct may contain sequences from the 5' and 3' LTRs of a lentivirus, and in particular can contain the R and U5 sequences from the 5' LTR of a lentivirus and an inactivated or self-inactivating 3' LTR from a lentivirus. The LTR sequences can be LTR sequences from any lentivirus from any species. For example, they may be LTR sequences from HIV, SIV, FIV or BIV. Typically, the LTR sequences are HIV LTR sequences.

In some embodiments, the nucleic acid of a viral vector, such as an HIV viral vector, lacks additional transcriptional units. The vector genome can contain an inactivated or self-inactivating 3' LTR (Zufferey et al. J Virol 72: 9873, 1998; Miyoshi et al., J Virol 72:8150, 1998). For example, deletion in the U3 region of the 3' LTR of the nucleic acid used to produce the viral vector RNA can be used to generate self-inactivating (SIN) vectors. This deletion can then be transferred to the 5' LTR of the proviral DNA during reverse transcription. A self-inactivating vector generally has a deletion of the enhancer and promoter sequences from the 3' long terminal repeat (LTR), which is copied over into the 5' LTR during vector integration. In some embodiments enough sequence can be eliminated, including the removal of a TATA box, to abolish the transcriptional activity of the LTR. This can prevent production of full-length vector RNA in transduced cells. In some aspects, the U3 element of the 3' LTR contains a deletion of its enhancer sequence, the TATA box, Sp1, and NF-kappa B sites. As a result of the self-inactivating 3' LTR, the provirus that is generated following entry and reverse transcription contains an inactivated 5' LTR. This can improve safety by reducing the risk of mobilization of the vector genome and the influence of the LTR on nearby cellular promoters. The self-inactivating 3' LTR can be constructed by any method known in the art. In some embodiments, this does not affect vector titers or the in vitro or in vivo properties of the vector.

Optionally, the U3 sequence from the lentiviral 5' LTR can be replaced with a promoter sequence in the viral construct, such as a heterologous promoter sequence. This can increase the titer of virus recovered from the packaging cell line. An enhancer sequence can also be included. Any enhancer/promoter combination that increases expression of the viral RNA genome in the packaging cell line may be used. In one example, the CMV enhancer/promoter sequence is used (U.S. Pat. No. 5,385,839 and U.S. Pat. No. 5,168,062).

In certain embodiments, the risk of insertional mutagenesis can be minimized by constructing the retroviral vector genome, such as lentiviral vector genome, to be integration defective. A variety of approaches can be pursued to produce a non-integrating vector genome. In some embodiments, a mutation(s) can be engineered into the integrase enzyme component of the pol gene, such that it encodes a protein with an inactive integrase. In some embodiments, the vector genome itself can be modified to prevent integration by, for example, mutating or deleting one or both attachment sites, or making the 3' LTR-proximal polypurine tract (PPT) non-functional through deletion or modification. In some embodiments, non-genetic approaches are available; these include pharmacological agents that inhibit one or more functions of integrase. The approaches are not mutually exclusive; that is, more than one of them can be used at a time. For example, both the integrase and attachment sites can be non-functional, or the integrase and PPT site can be non-functional, or the attachment sites and PPT site can be non-functional, or all of them can be non-functional. Such methods and viral vector genomes are known and available (see Philpott and Thrasher, Human Gene Therapy 18:483, 2007; Engelman et al. J Virol 69:2729, 1995; Brown et al J Virol 73:9011 (1999); WO 2009/076524; McWilliams et al., J Virol 77:11150, 2003; Powell and Levin J Virol 70:5288, 1996).

In some embodiments, the vector contains sequences for propagation in a host cell, such as a prokaryotic host cell. In some embodiments, the nucleic acid of the viral vector contains one or more origins of replication for propagation in a prokaryotic cell, such as a bacterial cell. In some embodiments, vectors that include a prokaryotic origin of replication also may contain a gene whose expression confers a detectable or selectable marker such as drug resistance.

The viral vector genome is typically constructed in a plasmid form that can be transfected into a packaging or producer cell line. Any of a variety of known methods can be used to produce retroviral particles whose genome contains an RNA copy of the viral vector genome. In some embodiments, at least two components are involved in making a virus-based gene delivery system: first, packaging plasmids, encompassing the structural proteins as well as the enzymes necessary to generate a viral vector particle, and second, the viral vector itself, i.e., the genetic material to be transferred. Biosafety safeguards can be introduced in the design of one or both of these components.

In some embodiments, the packaging plasmid can contain all retroviral, such as HIV-1, proteins other than envelope proteins (Naldini et al., 1998). In other embodiments, viral vectors can lack additional viral genes, such as those that are associated with virulence, e.g., vpr, vif, vpu and nef, and/or Tat, a primary transactivator of HIV. In some embodiments, lentiviral vectors, such as HIV-based lentiviral vectors, comprise only three genes of the parental virus: gag, pol and rev, which reduces or eliminates the possibility of reconstitution of a wild-type virus through recombination.

In some embodiments, the viral vector genome is introduced into a packaging cell line that contains all the components necessary to package viral genomic RNA, transcribed from the viral vector genome, into viral particles. Alternatively, the viral vector genome may comprise one or more genes encoding viral components in addition to the one or more sequences, e.g., recombinant nucleic acids, of interest. In some aspects, in order to prevent replication of the genome in the target cell, however, endogenous viral genes required for replication are removed and provided separately in the packaging cell line.

In some embodiments, a packaging cell line is transfected with one or more plasmid vectors containing the components necessary to generate the particles. In some embodiments, a packaging cell line is transfected with a plasmid containing the viral vector genome, including the LTRs, the cis-acting packaging sequence and the sequence of interest, i.e. a nucleic acid encoding an antigen receptor, such as a CAR; and one or more helper plasmids encoding the virus enzymatic and/or structural components, such as Gag, pol and/or rev. In some embodiments, multiple vectors are utilized to separate the various genetic components that generate the retroviral vector particles. In some such embodiments, providing separate vectors to the packaging cell reduces the chance of recombination events that might otherwise generate replication competent viruses. In some embodiments, a single plasmid vector having all of the retroviral components can be used.

In some embodiments, the retroviral vector particle, such as lentiviral vector particle, is pseudotyped to increase the transduction efficiency of host cells. For example, a retroviral vector particle, such as a lentiviral vector particle, in some embodiments is pseudotyped with a VSV-G glycoprotein, which provides a broad cell host range extending the cell types that can be transduced. In some embodiments, a packaging cell line is transfected with a plasmid or polynucleotide encoding a non-native envelope glycoprotein, such as to include xenotropic, polytropic or amphotropic envelopes, such as Sindbis virus envelope, GALV or VSV-G.

In some embodiments, the packaging cell line provides the components, including viral regulatory and structural proteins, that are required in trans for the packaging of the viral genomic RNA into lentiviral vector particles. In some embodiments, the packaging cell line may be any cell line that is capable of expressing lentiviral proteins and producing functional lentiviral vector particles. In some aspects, suitable packaging cell lines include 293 (ATCC CCL X), 293T, HeLA (ATCC CCL 2), D17 (ATCC CCL 183), MDCK (ATCC CCL 34), BHK (ATCC CCL-10) and Cf2Th (ATCC CRL 1430) cells.

In some embodiments, the packaging cell line stably expresses the viral protein(s). For example, in some aspects, a packaging cell line containing the gag, pol, rev and/or other structural genes but without the LTR and packaging components can be constructed. In some embodiments, a packaging cell line can be transiently transfected with nucleic acid molecules encoding one or more viral proteins along with the viral vector genome containing a nucleic acid molecule encoding a heterologous protein, and/or a nucleic acid encoding an envelope glycoprotein.

In some embodiments, the viral vectors and the packaging and/or helper plasmids are introduced via transfection or infection into the packaging cell line. The packaging cell line produces viral vector particles that contain the viral vector genome. Methods for transfection or infection are well known. Non-limiting examples include calcium phosphate, DEAE-dextran and lipofection methods, electroporation and microinjection.

When a recombinant plasmid and the retroviral LTR and packaging sequences are introduced into a special cell line (e.g., by calcium phosphate precipitation for example), the packaging sequences may permit the RNA transcript of the recombinant plasmid to be packaged into viral particles, which then may be secreted into the culture media. The media containing the recombinant retroviruses in some embodiments is then collected, optionally concentrated, and used for gene transfer. For example, in some aspects, after cotransfection of the packaging plasmids and the transfer vector to the packaging cell line, the viral vector particles are recovered from the culture media and titered by standard methods used by those of skill in the art.

In some embodiments, a retroviral vector, such as a lentiviral vector, can be produced in a packaging cell line, such as an exemplary HEK 293T cell line, by introduction of plasmids to allow generation of lentiviral particles. In some embodiments, a packaging cell is transfected and/or contains a polynucleotide encoding gag and pol, and a polynucleotide encoding a recombinant receptor, such as an antigen receptor, for example, a CAR. In some embodiments, the packaging cell line is optionally and/or additionally transfected with and/or contains a polynucleotide encoding a rev protein. In some embodiments, the packaging cell line is optionally and/or additionally transfected with and/or contains a polynucleotide encoding a non-native envelope glycoprotein, such as VSV-G. In some such embodiments, approximately two days after transfection of cells, *e.g.,* HEK 293T cells, the cell supernatant contains recombinant lentiviral vectors, which can be recovered and titered.

Recovered and/or produced retroviral vector particles can be used to transduce target cells using the methods as described. Once in the target cells, the viral RNA is reverse-transcribed, imported into the nucleus and stably integrated into the host genome. One or two days after the integration of the viral RNA, the expression of the recombinant protein, e.g., antigen receptor, such as CAR, can be detected.

In some embodiments, the provided methods involve methods of transducing cells by contacting, e.g., incubating, a cell composition comprising a plurality of cells with a viral particle. In some embodiments, the cells to be transfected or transduced are or comprise primary cells obtained from a subject, such as cells enriched and/or selected from a subject.

In some embodiments, the concentration of cells to be transduced of the composition is from or from about 1.0 x 10⁵ cells/mL to 1.0 x 10⁸ cells/mL, such as at least or about at least or about 1.0 x 10⁵ cells/mL, 5 x 10⁵ cells/mL, 1 x 10⁶ cells/mL, 5 x 10⁶ cells/mL, 1 x 10⁷ cells/mL, 5 x 10⁷ cells/mL or 1 x 10⁸ cells/mL.

In some embodiments, the viral particles are provided at a certain ratio of copies of the viral vector particles or infectious units (IU) thereof, per total number of cells to be transduced (IU/cell). For example, in some embodiments, the viral particles are present during the contacting at or about or at least at or about 0.5, 1, 2, 3, 4, 5, 10, 15, 20, 30, 40, 50, or 60 IU of the viral vector particles per one of the cells.

In some embodiments, the titer of viral vector particles is between or between about 1 x 10⁶ IU/mL and 1 x 10⁸ IU/mL, such as between or between about 5 x 10⁶ IU/mL and 5 x 10⁷ IU/mL, such as at least 6 x 10⁶ IU/mL, 7 x 10⁶ IU/mL, 8 x 10⁶ IU/mL, 9 x 10⁶ IU/mL, 1 x 10⁷ IU/mL, 2 x 10⁷ IU/mL, 3 x 10⁷ IU/mL, 4 x 10⁷ IU/mL, or 5 ×10⁷ IU/mL.

In some embodiments, transduction can be achieved at a multiplicity of infection (MOI) of less than 100, such as generally less than 60, 50, 40, 30, 20, 10, 5 or less.

In some embodiments, the method involves contacting or incubating, the cells with the viral particles. In some embodiments, the contacting is for 30 minutes to 72 hours, such as 30 minute to 48 hours, 30 minutes to 24 hours or 1 hour to 24 hours, such as at least or about at least 30 minutes, 1 hour, 2 hours, 6 hours, 12 hours, 24 hours, 36 hours or more.

In some embodiments, contacting is performed in solution. In some embodiments, the cells and viral particles are contacted in a volume of from or from about 0.5 mL to 500 mL, such as from or from about 0.5 mL to 200 mL, 0.5 mL to 100 mL, 0.5 mL to 50 mL, 0.5 mL to 10 mL, 0.5 mL to 5 mL, 5 mL to 500 mL, 5 mL to 200 mL, 5 mL to 100 mL, 5 mL to 50 mL, 5 mL to 10 mL, 10 mL to 500 mL, 10 mL to 200 mL, 10 mL to 100 mL, 10 mL to 50 mL, 50 mL to 500 mL, 50 mL to 200 mL, 50 mL to 100 mL, 100 mL to 500 mL, 100 mL to 200 mL or 200 mL to 500 mL.

In some embodiments, when the contacting can be effected with centrifugation, such as spinoculation (e.g., centrifugal inoculation). In some embodiments, the composition containing cells, viral particles and reagent can be rotated, generally at relatively low force or speed, such as speed lower than that used to pellet the cells, such as from or from about 600 rpm to 1700 rpm (e.g., at or about or at least 600 rpm, 1000 rpm, or 1500 rpm or 1700 rpm). In some embodiments, the rotation is carried at a force, e.g., a relative centrifugal force, of from or from about 100 g to 3200 g (e.g., at or about or at least at or about 100 g, 200 g, 300 g, 400 g, 500 g, 1000 g, 1500 g, 2000 g, 2500 g, 3000 g or 3200 g), as measured for example at an internal or external wall of the chamber or cavity. The term "relative centrifugal force" or RCF is generally understood to be the effective force imparted on an object or substance (such as a cell, sample, or pellet and/or a point in the chamber or other container being rotated), relative to the earth's gravitational force, at a particular point in space as compared to the axis of rotation. The value may be determined using well-known formulas, taking into account the gravitational force, rotation speed and the radius of rotation (distance from the axis of rotation and the object, substance, or particle at which RCF is being measured).

In certain embodiments, the input cells are treated, incubated, or contacted with particles, e.g., beads, that comprise binding molecules that bind to or recognize the recombinant receptor that is encoded by the viral DNA.

In some embodiments, the incubation of the cells with the viral vector particles results in or produces an output composition comprising cells transduced with the viral vector particles.

### 2. Non-Viral Vectors

In some embodiments, recombinant nucleic acids are transferred into T cells via electroporation *(see,* e.g., Chicaybam et al, (2013) PLoS ONE 8(3): e60298 and Van Tedeloo et al. (2000) Gene Therapy 7(16): 1431-1437). In some embodiments, recombinant nucleic acids are transferred into T cells via transposition (see, e.g., Manuri et al. (2010) Hum Gene Ther 21(4): 427-437; Sharma et al. (2013) Molec Ther Nucl Acids 2, e74; and Huang et al. (2009) Methods Mol Biol 506: 115-126). Other methods of introducing and expressing genetic material in immune cells include calcium phosphate transfection (e.g., as described in Current Protocols in Molecular Biology, John Wiley & Sons, New York. N.Y.), protoplast fusion, cationic liposome-mediated transfection; tungsten particle-facilitated microparticle bombardment (Johnston, Nature, 346: 776-777 (1990)); and strontium phosphate DNA co-precipitation (Brash et al., Mol. Cell Biol., 7: 2031-2034 (1987)).

Other approaches and vectors for transfer of the nucleic acids encoding the recombinant products are those described, e.g., in international patent application, Publication No.: WO2014055668, and U.S. Patent No. 7,446,190.

In some embodiments, recombinant nucleic acids are transferred into T cells via transposons. Transposons (transposable elements), are mobile segments of DNA that can move from one locus to another within genomes. These elements move via a conservative, "cut-and-paste" mechanism: the transposase catalyzes the excision of the transposon from its original location and promotes its reintegration elsewhere in the genome. Transposase-deficient elements can be mobilized if the transposase is provided by another transposase gene. Thus, transposons can be utilized to incorporate a foreign DNA into a host genome without the use of a viral transduction system. Examples of transposons suitable for use with mammalian cells, e.g., human primary leukocytes, include but are not limited to Sleeping Beauty and Piggybac.

Transposon-based transfection is a two-component system consisting of a transposase and a transposon. In some embodiments, the system comprises a transposon is engineered to comprise a foreign DNA (also referred herein as cargo DNA), e.g., a gene encoding a recombinant receptor, that is flanked by inverted repeat/direct repeat (IR/DR) sequences that are recognized by an accompanying tranposase. In some embodiments, a non-viral plasmid encodes a transposase under the control of a promoter. Transfection of the plasmid into a host cell results in a transitory expression of the transposase, thus for an initial period following transfection, the transposase is expressed at sufficiently levels to integrate the transposon into the genomic DNA. In some embodiments, the transposase itself is not integrated into the genomic DNA, and therefor expression of the transposase decreases over time. In some embodiments, the transposase expression is expressed by the host cell at levels sufficient to integrate a corresponding transposon for less than about 4 hours, less than about 8 hours, less than about 12 hours, less than about 24 hours, less than about 2 days, less than about 3 days, less than about 4 days, less than about 5 days, less than about 6 days, less than about 7 days, less than about 2 weeks, less than about 3 weeks, less than about 4 weeks, less than about weeks, or less than about 8 weeks. In some embodiments, the cargo DNA that is introduced into the host's genome is not subsequently removed from the host's genome, at least because the host dose not express an endogenous transposase capable of excising the cargo DNA.

Sleeping Beauty (SB) is a synthetic member of the Tc/1-mariner superfamily of transposons, reconstructed from dormant elements harbored in the salmonid fish genome. SB transposon-based transfection is a two-component system consisting of a transposase and a transposon containing inverted repeat/direct repeat (IR/DR) sequences that result in precise integration into a TA dinucleotide. The transposon is designed with an expression cassette of interest flanked by IR/DRs. The SB transposase binds specific binding sites that are located on the IR of the Sleeping beauty transposon. The SB transposase mediates integration of the transposon, a mobile element encoding a cargo sequence flanked on both sides by inverted terminal repeats that harbor binding sites for the catalytic enzyme (SB). Stable expression results when SB inserts gene sequences into vertebrate chromosomes at a TA target dinucleotide through a cut-and-paste mechanism. This system has been used to engineer a variety of vertebrate cell types, including primary human peripheral blood leukocytes. In some embodiments, the cells are contacted, incubated, and/or treated with an SB transposon comprising a cargo gene, e.g., a gene encoding a recombinant receptor or a CAR, flanked by SB IR sequences. In particular embodiments, the cells to be transfected are contacted, incubated, and/or treated with a plasmid comprising an SB transposon comprising a cargo gene, e.g., a gene encoding a CAR, flanked by SB IR sequences. In certain embodiments, the plasmid further comprises a gene encoding an SB transposase that is not flanked by SB IR sequences.

An exemplary SB IR sequence is provided by SEQ ID NO: 26. An exemplary polynucleotide sequence encoding an SB transposase, and an amino acid sequence of an exemplary SB transposase are provided by SEQ ID NOS: 24, and 25, respectively.

PiggyBac (PB) is another transposon system that can be used to integrate cargo DNA into a host's, e.g., a human's, genomic DNA. The PB transposase recognizes PB transposon-specific inverted terminal repeat sequences (ITRs) located on both ends of the transposon and efficiently moves the contents from the original sites and efficiently integrates them into TTAA chromosomal sites. The PB transposon system enables genes of interest between the two ITRs in the PB vector to be mobilized into target genomes. The PB system has been used to engineer a variety of vertebrate cell types, including primary human cells. In some embodiments, the cells to be transfected are contacted, incubated, and/or treated with an PB transposon comprising a cargo gene, e.g., a gene encoding a CAR, flanked by PB IR sequences. In particular embodiments, the cells to be transfected are contacted, incubated, and/or treated with a plasmid comprising a PB transposon comprising a cargo gene, e.g., a gene encoding a CAR, flanked by PB IR sequences. In certain embodiments, the plasmid further comprises a gene encoding an SB transposase that is not flanked by PB IR sequences.

An exemplary PB IR sequence is illustrated by SEQ ID NO: 27. An exemplary polynucleotide sequence encoding a PB transposase is illustrated by SEQ ID NO: 28. An amino acid sequence of an exemplary PB transposase is illustrated by SEQ ID NO: 29.

In some embodiments, the various elements of the transposon/transposase the employed in the subject methods, e.g., SB or PB vector(s), may be produced by standard methods of restriction enzyme cleavage, ligation and molecular cloning. One protocol for constructing the subject vectors includes the following steps. First, purified nucleic acid fragments containing desired component nucleotide sequences as well as extraneous sequences are cleaved with restriction endonucleases from initial sources, e.g., a vector comprising the transposase gene. Fragments containing the desired nucleotide sequences are then separated from unwanted fragments of different size using conventional separation methods, e.g., by agarose gel electrophoresis. The desired fragments are excised from the gel and ligated together in the appropriate configuration so that a circular nucleic acid or plasmid containing the desired sequences, e.g., sequences corresponding to the various elements of the subject vectors, as described above is produced. Where desired, the circular molecules so constructed are then amplified in a prokaryotic host, e.g., *E. coli.* The procedures of cleavage, plasmid construction, cell transformation and plasmid production involved in these steps are well known to one skilled in the art and the enzymes required for restriction and ligation are available commercially. (See, for example, R. Wu, Ed., Methods in Enzymology, Vol. 68, Academic Press, N.Y. (1979); T. Maniatis, E. F. Fritsch and J. Sambrook, Molecular Cloning: A Laboratory Manual, Cold Spring Harbor Laboratory Press, Cold Spring Harbor, N.Y. (1982); Catalog 1982-83, New England Biolabs, Inc.; Catalog 1982-83, Bethesda Research Laboratories, Inc. An example of how to construct the vectors employed in the subject methods is provided in the Experimental section, infra. The preparation of a representative Sleeping Beauty transposon system is also disclosed in WO 98/40510 and WO 99/25817).

In some embodiments, transduction with transposons is performed with a plasmid that comprises a transposase gene and a plasmid that comprises a transposon that contains a cargo DNA sequence that is flanked by inverted repeat/direct repeat (IR/DR) sequences that are recognized by the transposase. In certain embodiments, the cargo DNA sequence encodes a heterologous protein, e.g., a recombinant T cell receptor or a CAR. In some embodiments, the plasmid comprises transposase and the transposon. In some embodiments, the transposase is under control of a ubiquitous promoter, or any promoter suitable to drive expression of the transposase in the target cell. Ubiquitous promoters include, but are not limited to, EF1a, CMB, SV40, PGK1, Ubc, human β-actin, CAG, TRE, UAS, Ac5, CaMKIIa, and U6. In some embodiments, the cargo DNA comprises a selection cassette allowing for the selection of cells with stable integration of the cargo DNA into the genomic DNA. Suitable selection cassettes include, but are not limited to, selection cassettes encoding a kanamycin resistance gene, spectinomycin resistance gene, streptomycin resistance gene, ampicillin resistance gene, carbenicillin resistance gene, hygromycin resistance gene, bleomycin resistance gene, erythromycin resistance gene, and polymyxin B resistance gene.

In some embodiments, the components for transduction with a transposon, e.g., plasmids comprising an SB transposase and SB transposon, are introduced into the target cell. Any convenient protocol may be employed, where the protocol may provide for in vitro or in vivo introduction of the system components into the target cell, depending on the location of the target cell. For example, where the target cell is an isolated cell, the system may be introduced directly into the cell under cell culture conditions permissive of viability of the target cell, e.g., by using standard transformation techniques. Such techniques include, but are not necessarily limited to: viral infection, transformation, conjugation, protoplast fusion, electroporation, particle gun technology, calcium phosphate precipitation, direct microinjection, viral vector delivery, and the like. The choice of method is generally dependent on the type of cell being transformed and the circumstances under which the transformation is taking place (i.e. in vitro, ex vivo, or in vivo). A general discussion of these methods can be found in Ausubel, et al, Short Protocols in Molecular Biology, 3rd ed., Wiley & Sons, 1995.

In some embodiments, the SB transposon and the SB transposase source are introduced into a target cell of a multicellular organism, e.g., a mammal or a human, under conditions sufficient for excision of the inverted repeat flanked nucleic acid from the vector carrying the transposon and subsequent integration of the excised nucleic acid into the genome of the target cell. Some embodiments further comprise a step of ensuring that the requisite transposase activity is present in the target cell along with the introduced transposon. Depending on the structure of the transposon vector itself, i.e. whether or not the vector includes a region encoding a product having transposase activity, the method may further include introducing a second vector into the target cell which encodes the requisite transposase activity.

In some embodiments, the amount of vector nucleic acid comprising the transposon and the amount of vector nucleic acid encoding the transposase that is introduced into the cell is sufficient to provide for the desired excision and insertion of the transposon nucleic acid into the target cell genome. As such, the amount of vector nucleic acid introduced should provide for a sufficient amount of transposase activity and a sufficient copy number of the nucleic acid that is desired to be inserted into the target cell. The amount of vector nucleic acid that is introduced into the target cell varies depending on the efficiency of the particular introduction protocol that is employed, e.g., the particular ex vivo administration protocol that is employed.

Once the vector DNA has entered the target cell in combination with the requisite transposase, the nucleic acid region of the vector that is flanked by inverted repeats, i.e. the vector nucleic acid positioned between the Sleeping Beauty transposase recognized inverted repeats, is excised from the vector via the provided transposase and inserted into the genome of the targeted cell. As such, introduction of the vector DNA into the target cell is followed by subsequent transposase mediated excision and insertion of the exogenous nucleic acid carried by the vector into the genome of the targeted cell. In particular embodiments, the vector is integrated into the genomes of at least 1%, at least 2%, at least 3%, at least 4%, at least 5%, at least 6% at least 7% at least 8%, at least 9%, at least 10%, at least 15%, or at least 20% of the cells that are transfected with the SB transposon and/or SB transposase. In some embodiments, integration of the nucleic acid into the target cell genome is stable, i.e., the vector nucleic acid remains present in the target cell genome for more than a transient period of time and is passed on a part of the chromosomal genetic material to the progeny of the target cell.

**In** certain embodiments, the transposons are used to integrate nucleic acids, i.e. polynucleotides, of various sizes into the target cell genome. In some embodiments, the size of DNA that is inserted into a target cell genome using the subject methods ranges from about 0.1 kb to 200 kb, from about 0.5 kb to 100 kb, from about 1.0 kb to about 8.0 kb, from about 1.0 to about 200 kb, from about 1.0 to about 10 kb, from about 10 kb to about 50 kb, from about 50 kb to about 100 kb, or from about 100 kb to about 200 kb. In some embodiments, the size of DNA that is inserted into a target cell genome using the subject methods ranges from about from about 1.0 kb to about 8.0 kb. In some embodiments, the size of DNA that is inserted into a target cell genome using the subject methods ranges from about 1.0 to about 200 kb. In particular embodiments, the size of DNA that is inserted into a target cell genome using the subject methods ranges from about 1.0 kb to about 8.0 kb.

### V. METHODS FOR DETECTION AND QUANTIFICATION

Provided herein are methods for detecting a chimeric antigen receptor (CAR) comprising an extracellular antigen-binding domain that is an anti-BCMA antibody by contacting a composition or biological sample containing cells express expressing the CAR (CAR-expressing cells) with a BCMA-Fc fusion protein. In some embodiments, the method further includes detecting whether a complex is formed between the BCMA-Fc fusion protein and the CAR expressed by or on the cells in the composition or biological sample, such as detecting the presence or absence or level of such binding. In some embodiments, the detecting comprises detecting cells bound with the BCMA-Fc fusion. In some embodiments, the BCMA-Fc fusion is directly or indirectly labeled for detection.

In some embodiments, the BCMA antigen of the BCMA-Fc fusion protein is or comprises an extracellular domain of BCMA or a portion thereof comprising an epitope recognized by an antigen receptor, e.g. CAR. In certain embodiments, the recombinant BCMA-Fc fusion protein contains a BCMA antigen that contains the extracellular domain of a human BCMA or a portion thereof that is or contains an epitope recognized by the CAR. In some embodiments, the BCMA antigen of the BCMA-Fc fusion does not contain a transmembrane domain and cytoplasmic domain of native human BCMA. Thus, in some embodiments, the BCMA antigen of the BCMA-Fc fusion is, consists or consists essentially of the extracellular domain or a portion thereof of a native human BCMA. In some embodiments, the BCMA antigen of the BCMA-Fc fusion proteion is or comprises, in some cases consists or consists essentially of, a polypeptide with an amino acid sequence with at least 70%, 75%, 80%, 85%, 86%, 87%, 88%, 89%, 90%, 91%, 92%, 93%, 94%, 95%, 96%, 97%, 98%, 99% or more sequence identity to SEQ ID NO: 1 or is a fragment thereof containing at least 50, at least 55, at least 60, at least 65, at least 70, at least 75, at least 80, at least 85, at least 90, at least 95, at least 100, at least 110, at least 120, at least 130, at least 140, at least 150, at least 160, at least 170, or at least 180 contiguous amino acids of SEQ ID NO: 1 that is recognized or specifically bound by the antigen-binding domain of the CAR. In some embodiments, the BCMA antigen is or comprises, in some cases consists or consists essentially of, the sequence set forth in SEQ ID NO:1 or a portion thereof that is or contains an epitope recognized by an antigen receptor, e.g. CAR.

In some embodiments, the recombinant BCMA antigen or portion thereof is fused directly or indirectly to an immunoglobulin constant region or domain, such as, for example, the Fc domain or portions thereof from IgG, including IgG1, IgG2, IgG3 or IgG4 subtypes, IgA, IgE, IgD and IgM and modified forms thereof. In particular embodiments, the BCMA antigen is linked, directly or indirectly, to an Fc domain. In some embodiments, the polypeptide is a fusion polypeptide comprising the BCMA antigen or portion thereof and the Fc domain.

In some embodiments, the Fc domain is composed of the second and third constant domains (*i.e.,* CH2 and CH3 domains) of the heavy chain of a IgG, IgA or IgD isotype, e.g. CH2 or CH3 of IgG, IgA and IgD isotypes. In some embodiments, the Fc domain is composed of three heavy chain constant domain (*i.e.,* CH2, CH3, and CH4 domains) of an IgM or IgE isotype. In some embodiments, the Fc domain may further include a hinge sequence or portion thereof. In certain aspects, the Fc domain contains part or all of a hinge domain of an immunoglobulin molecule plus a CH2 and a CH3 domain. In some cases, the Fc domain can form a dimer of two polypeptide chains joined by one or more disulfide bonds. In some embodiments, the Fc domain is derived from an immunoglobulin (e.g., IgG, IgA, IgM, or IgE) of a suitable mammal (e.g., human, mouse, rat, goat, sheep, or monkey). In some embodiments, the Fc domain comprises C_{H}2 and C_{H}3 domains of IgG. In certain embodiments, the Fc domain is fused to the C-terminal of the BCMA antigen. In particular embodiments, the Fc domain is fused to the N-terminal of the BCMA antigen.

In some embodiments, the Fc domain is an IgG Fc domain, or a portion or variant thereof. In some embodiments, the Fc domain is a human IgG Fc domain, or a portion or a variant thereof, that comprises an amino acid sequence set forth in SEQ ID NO: 2 or an amino acid sequence that is at least 70%, 75%, 80%, 85%, 86%, 87%, 88%, 89%, 90%, 91%, 92%, 93%, 94%, 95%, 96%, 97%, 98%, 99% sequence identity to the sequence set forth in SEQ ID NO: 2. In particualr embodiments, the Fc domain is a wild-type human IgG Fc domain, or a portion or variant thereof. In particular embodiments, the Fc domain is a variant of the wild-type human IgG1 Fc domain.

In some embodiments, the fusion polypeptide comprises a variant Fc domain. In certain embodiments, the variant human IgG Fc domain contains a mutation, e.g., a substitution, deletion, or insertion, that reduces, decreases, and/or diminishes pairing between the Fc domain and a light chain. In some embodiments, the variant human IgG Fc domain contains a mutation that reduces the binding affinity between the Fc domain and an Fc Receptor. In particular embodiments, the variant human IgG Fc domain contains a mutation that reduces, decreases, and/or diminishes the interactions, or the probabiltity or likelihood of an interaction, between the Fc domain and an Fc Receptor. In some embodiments, the variant human IgG Fc domain contains a mutation that reduces the binding affinity between the Fc domain and a protein of the complement system. In particular embodiments, the variant human IgG Fc domain contains a mutation that reduces, decreases, and/or diminishes the interactions, or the probabiltity or likelihood of an interaction, between the Fc domain and a protein of the complement system.

In some embodiments, the BCMA-Fc comprises a variant human IgG1 Fc domain. In some embodiments, the variant human IgG Fc domain contains a cystine to serine substitution in the hinge region of the Fc domain. In some embodiments, the variant human IgG Fc domain contains a leucine to alanine substitution in the hinge region of the Fc domain. In particular embodiments, the variant human IgG Fc domain contains a glycine to alanine substitution in the hinge region. In certain embodiments, the variant human IgG Fc domain contains an alanine to a serine substitution in the CH2 region of the Fc domain. In some embodiments, the variant human IgG Fc domain comprises a proline to serine substitution in the CH2 region of the Fc domain. In some embodiments, the variant human IgG Fc domain comprises an amino acid sequence as set forth by SEQ ID NO: 28.

In some embodiments, the BCMA-Fc comprises a fusion polypeptide comprising an Fc domain, wherein the Fc domain is present at the C-terminus of the fusion polypeptide.

In some embodiments, the BCMA-Fc is a fusion polypeptide comprising a BCMA polypeptide, or a portion thereof, and an Fc domain. In some embodiments, the BCMA polypeptide or portion thereof comprises an amino acid sequence set forth in SEQ ID NO: 1 or a sequence of amino acids that exhibits at least 70%, 75%, 80%, 85%, 86%, 87%, 88%, 89%, 90%, 91%, 92%, 93%, 94%, 95%, 96%, 97%, 98%, 99% to SEQ ID NO: 1 and contains an epitope recognized by the antigen receptor, e.g. the CAR. In some embodiments, the Fc domain comprises an amino acid sequence set forth in SEQ ID NO: 2 or a sequence of amino acids that exhibits at least 70%, 75%, 80%, 85%, 86%, 87%, 88%, 89%, 90%, 91%, 92%, 93%, 94%, 95%, 96%, 97%, 98%, 99% sequence identity to SEQ ID NO:2. In some embodiments, the Fc domain comprises an amino acid sequence set forth in SEQ ID NO: 28 or a sequence of amino acids that exhibits at least 70%, 75%, 80%, 85%, 86%, 87%, 88%, 89%, 90%, 91%, 92%, 93%, 94%, 95%, 96%, 97%, 98%, 99% to SEQ ID NO:28. In some embodiments, the BCMA antigen includes BCMA, or a portion or variant thereof, and a tag or a fusion domain, e.g., an Fc domain. In particular embodiments, the BCMA antigen contains all or a portion of the amino acid sequence set forth in SEQ ID NO: 35 or a sequence of amino acids that exhibits at least 70%, 75%, 80%, 85%, 86%, 87%, 88%, 89%, 90%, 91%, 92%, 93%, 94%, 95%, 96%, 97%, 98%, 99% to SEQ ID NO:35, and that comprises an epitope recognize by an antigen receptor, e.g. CAR.

In some embodiments, the BCMA-F is a dimer comprising two or more BCMA antigens as described, that each are recognized and/or bound by a CAR. In some embodiments, the BCMA antigens are identical.

In certain embodiments, the methods for detecting CAR-expressing cells with a BCMA-Fc fusion described herein are used to assess the CAR-expressing cells in a subject. In some embodiments, provided herein are methods of use for the BCMA-Fc fusion for assessing, measuring, and/or quantifying the *in vivo* pharmacokinetics, expansion, and/or persistence of a CAR expressing cells of a therapeutic cell composition. In some embodiments, the *in vivo* pharmacokinetics, expansion, and/or persistence of the cells, and/or changes in cell phenotypes or functional activity of cells, such as CAR expressing cells administered for immunotherapy, *e.g.* CAR-T cell therapy, in the methods provided herein, can be measured with the BCMA-Fc fusion protein. In some embodiments, the pharmacokinetics, expansion, and/or persistence of the CAR expressing cells are measured or assessed by detecting, the presence and/or amount of cells expressing the CAR in the subject and/or in a biological sample obtained from the subject following the administration of the therapeutic cell composition during and/or after the administration of the therapy.

In some aspects, the BCMA-Fc fusion is used with flow cytometry to assess the quantity of CAR-expressing cells in the blood or serum or organ or tissue sample (*e.g.,* disease site, *e.g.,* tumor sample) of the subject. In some aspects, persistence is quantified as the number of CAR-expressing cells per microliter of the sample, *e.g.,* of blood or serum, or per total number of peripheral blood mononuclear cells (PBMCs) or white blood cells or T cells per microliter of the sample. In certain aspects, expansion is quantified as the increase in the number of CAR-expressing cells per microliter between samples, *e.g.,* of blood or serum, or per total number of peripheral blood mononuclear cells (PBMCs) or white blood cells or T cells per microliter of the samples over time. In some embodiments, the pharmacokinetics, expansion, and/or persistence are measured or assessed by detecting the amount of CAR expressing cells in the subject and/or in samples collected from the subject at multiple time points. In certain embodiments, the one or more samples are collected, obtained, and/or taken from the subject within 24 hours, 48 hours, 72 hours, 4 days, 5 days, 6 days, 7 days, 10 days, 14 days, 21 days, 3 weeks, 4 weeks, 5 weeks, 6 weeks, 7 weeks, 8 weeks, 9 weeks, 10 weeks, 11 weeks, 12 weeks, 3 months, 4 months, 6 months, one year, or over one year after the therapeutic cell composition is administered.

In some embodiments, there is provided a method of evaluating an anti-BCMA CAR T cell therapy in an individual, wherein the CAR comprises an anti-BCMA antibody or an antigen-binding fragment thereof, and the method comprises incubating a sample from the subject with a BCMA-Fc fusion protein and determining the amount of T cells bound with the BCMA-Fc fusion protein. In some embodiments, the BCMA-Fc fusion is labeled, and BCMA-Fc fusion-bound T cells are assayed by flow cytometry. In some embodiments, the sample is a blood-derived sample, or is or is derived from an apheresis or leukapheresis product. In some aspects, the administration is carried out following initiation of a first dose of the therapy.

In one embodiment, an BCMA-Fc is used to determine whether adjustment to a CAR T cell therapy in an individual is necessary, e.g. where low levels of the CAR T cells in the individual indicate the need to adjust the therapy. In some aspects, the therapy is adjusted (i) if the number of cells of the T cell therapy detectable in the blood or other biological sample, after having been detectable, is not detectable or is reduced, optionally reduced compared to a preceding time point after administration of the T cell therapy; (ii) the number of cells of the T cell therapy detectable in the blood or other biological sample is decreased by or more than 1.5-fold, 2.0-fold, 3.0-fold, 4.0-fold, 5.0-fold, 10-fold or more the peak or maximum number cells of the T cell therapy detectable in the blood or the biological sample of the subject after initiation of administration of the T cell therapy, optionally the first, second or subsequent dose; (iii) at a time after a peak or maximum level of the cells of the T cell therapy are detectable in the blood of the subject, the number of cells of or derived from the T cells detectable in the blood from the subject is less than less than 10%, less than 5%, less than 1% or less than 0.1% of total peripheral blood mononuclear cells (PBMCs) in the blood of the subject; and/or (iv) if the number of CD3+ or CD8+ cells of the cell therapy detectable in the blood is less than 20 cells per µL, 15 cells per µL, 10 cells per µL, less than 5 cells per µL or less than per 1 cells per µL. In some embodiments, the therapy is adjusted by administering one or more additional doses of the CAR-T cell therapy, administering an increased dose of the CAR-T cell therapy, administering an alternative CAR-T cell therapy specific for the same or different antigen, administering one or more immunomodulatory agent or other agent for promoting or increasing expansion or persistence of the CAR-T cells.

Various methods known in the art for detecting binding of a BCMA-Fc fusion to CAR-expressing cells can be used. In some embodiments, the method includes flow cytometry or an immunoassay. An indicator moiety, or label group, can be attached to the BCMA-Fc fusion and is selected so as to meet the needs of various uses of the method which are often dictated by the availability of assay equipment and compatible immunoassay procedures. In some embodiments, the label is a chosen from fluorescent moieties or proteins (*e.g.,* fluorescein, rhodamine, phycoerythrin, GFP, or BFP), or luminescent moieties (*e.g.*, Qdot^{™} nanoparticles supplied by the Quantum Dot Corporation, Palo Alto, Calif.). Various general techniques to be used in performing the various immunoassays noted above are known.

In some embodiments, the BCMA-Fc fusions need not be labeled, and the presence thereof can be detected using a labeled antibody which binds to the BCMA-Fc fusion. In some embodiments, the labeled antibody is specific to the Fc portion of the molecule.

In some embodiments, the BCMA-Fc fusion is immobilized or bound to a solid support, wherein a composition or biological sample containing one or more target cells comprising CAR-T cells are contacted with the solid support. In some embodiments, the solid support is a bead. In some embodiments, the solid support is the surface of a well or plate, e.g., a cell culture plate. In some embodiments, the solid support is a resin or matrix present in or contained within a chromatography column, for example, to permit chromatographic isolation or selection of CAR+ T cells. In some embodiments, the BCMA-Fc fusion protein is or is capable of being reversibly bound to a solid support. In some embodiments, the solid support is an affinity chromatography matrix comprising one or more binding sites capable of binding, e.g. reversibly binding, to a binding partner present in the BCMA-Fc fusion protein. In one exemplary embodiment, the BCMA-Fc fusion protein comprises a streptavidin-binding peptide or other streptavidin binding moiety capable of binding to a streptavidin or streptavidin mutein molecule present on or immobilized on the solid support, which, in some cases, can be dissociated in the presence of a competition substance, such as biotin. Exemplary of such systems include those described in U.S. published patent application No. US20150024411.

### VI. ARTICLES OF MANUFACTURE AND KITS

In some embodiments, also provided are systems, apparatuses, and kits useful in performing the provided methods. In some embodiments, provided are articles of manufacture, such as kits or devices, containing the particles, e.g., beads, described herein, i.e., particles, e.g., beads, with attached binding molecules. In some embodiments, the binding molecules comprise an antigen or an antibody that binds to or recognizes a recombinant receptor or a CAR. In some embodiments, the kits can be used in methods for expanding, selecting, and/or enriching cells, such as in accord with preparing genetically engineered cells for adoptive cell therapy. In particular embodiments, the genetically engineered cells express a recombinant receptor, e.g., a CAR, that is bound by or recognized by attached the binding molecules.

In some embodiments, the articles of manufacture include one or more containers, typically a plurality of containers, packaging material, and a label or package insert on or associated with the container or containers and/or packaging, generally including instructions for expanding cells, such as cells from a subject that are transduced or transfected with one or more nucleic acids e.g., one or more nucleic acids comprising a gene encoding a recombinant receptor or CAR.

In certain embodiments, the kit further comprises instructions for using the particles, e.g., beads, described herein for selecting or enriching cells expressing a recombinant receptor from a population of cells. In some embodiments, the kit comprises instructions for using the particles, e.g., beads, described herein to enrich cells that express a CAR. In particular embodiments, the kit comprises instructions for incubating, contacting, or treating a composition comprising cells with the particles, e.g., beads, described herein, where the less than all of the cells express the CAR. In some embodiments, the kit comprises instructions for activating, expanding, and or enriching cells that express a CAR. In certain embodiments, the kit comprises instructions for activating, expanding, and or enriching cells that express a CAR from a population of cells where less than 0.01%, less than 0.1%, less than 1%, less than 5%, less than 10%, less than 15%, less than 20%, less than 25%, less than 30%, less than 35%, less than 40%, less than 45%, less than 50%, less than 55%, less than 60%, less than 65%, less than 70%, less than 80%, or less than 90% of the cells express the CAR.

In some embodiments, the kit further contains± instructions for transfecting or transducing cells with one or more nucleic acids that encode a recombinant receptor, e.g., a CAR, that have not been expanded, activated, and/or enriched prior to the transfection or transduction, by contacting, incubating, or treating the cells with the particles, e.g., beads, described herein.

### VII. DEFINITIONS

Unless defined otherwise, all terms of art, notations and other technical and scientific terms or terminology used herein are intended to have the same meaning as is commonly understood by one of ordinary skill in the art to which the claimed subject matter pertains. In some cases, terms with commonly understood meanings are defined herein for clarity and/or for ready reference, and the inclusion of such definitions herein should not necessarily be construed to represent a substantial difference over what is generally understood in the art.

As used herein, reference to a "corresponding form" of an antibody means that when comparing a property or activity of two antibodies, the property is compared using the same form of the antibody. For example, if it is stated that an antibody has greater activity compared to the activity of the corresponding form of a first antibody, that means that a particular form, such as a scFv of that antibody, has greater activity compared to the scFv form of the first antibody.

As used herein, recitation that nucleotides or amino acid positions "correspond to" nucleotides or amino acid positions in a disclosed sequence, such as set forth in the Sequence listing, refers to nucleotides or amino acid positions identified upon alignment with the disclosed sequence to maximize identity using a standard alignment algorithm, such as the GAP algorithm. By aligning the sequences, one skilled in the art can identify corresponding residues, for example, using conserved and identical amino acid residues as guides. In general, to identify corresponding positions, the sequences of amino acids are aligned so that the highest order match is obtained (see, e.g. : Computational Molecular Biology, Lesk, A.M., ed., Oxford University Press, New York, 1988; Biocomputing: Informatics and Genome Projects, Smith, D.W., ed., Academic Press, New York, 1993; Computer Analysis of Sequence Data, Part I, Griffin, A.M., and Griffin, H.G., eds., Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991; Carrillo et al. (1988) SIAM J Applied Math 48: 1073).

"Effector functions" refer to those biological activities attributable to the Fc region of an antibody, which vary with the antibody isotype. Examples of antibody effector functions include: C1q binding and complement dependent cytotoxicity (CDC); Fc receptor binding; antibody-dependent cell-mediated cytotoxicity (ADCC); phagocytosis; down regulation of cell surface receptors (e.g. B cell receptor); and B cell activation.

The term "Fc region" herein is used to define a C-terminal region of an immunoglobulin heavy chain that contains at least a portion of the constant region. The term includes native sequence Fc regions and variant Fc regions. In one embodiment, a human IgG heavy chain Fc region extends from Cys226, or from Pro230, to the carboxyl-terminus of the heavy chain. However, the C-terminal lysine (Lys447) of the Fc region may or may not be present. Unless otherwise specified herein, numbering of amino acid residues in the Fc region or constant region is according to the EU numbering system, also called the EU index, as described in Kabat et al., Sequences of Proteins of Immunological Interest, 5th Ed. Public Health Service, National Institutes of Health, Bethesda, MD, 1991.

The terms "full length antibody," "intact antibody," and "whole antibody" are used herein interchangeably to refer to an antibody having a structure substantially similar to a native antibody structure or having heavy chains that contain an Fc region as defined herein.

An "isolated" antibody is one which has been separated from a component of its natural environment. In some embodiments, an antibody is purified to greater than 95% or 99% purity as determined by, for example, electrophoretic (e.g., SDS-PAGE, isoelectric focusing (IEF), capillary electrophoresis) or chromatographic (e.g., ion exchange or reverse phase HPLC). For review of methods for assessment of antibody purity, see, e.g., Flatman et al., J. Chromatogr. B 848:79-87 (2007).

An "isolated" nucleic acid refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

"Isolated nucleic acid encoding an anti-idiotype antibody" refers to one or more nucleic acid molecules encoding antibody heavy and light chains (or fragments thereof), including such nucleic acid molecule(s) in a single vector or separate vectors, and such nucleic acid molecule(s) present at one or more locations in a host cell.

The terms "host cell," "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.

As used herein, "percent (%) amino acid sequence identity" and "percent identity" when used with respect to an amino acid sequence (reference polypeptide sequence) is defined as the percentage of amino acid residues in a candidate sequence (e.g., the subject antibody or fragment) that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full length of the sequences being compared.

An amino acid substitution may include replacement of one amino acid in a polypeptide with another amino acid. The substitution may be a conservative amino acid substitution or a non-conservative amino acid substitution Amino acid substitutions may be introduced into a binding molecule, e.g., antibody, of interest and the products screened for a desired activity, e.g., retained/improved antigen binding, decreased immunogenicity, or improved ADCC or CDC.

Amino acids generally can be grouped according to the following common side-chain properties:
(1) hydrophobic: Norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe

In some embodiments, conservative substitutions can involve the exchange of a member of one of these classes for another member of the same class. In some embodiments, non-conservative amino acid substitutions can involve exchanging a member of one of these classes for another class. Non-conservative amino acid substitutions will involve exchanging a member of one of these classes for another class.

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors."

As used herein, the singular forms "a," "an," and "the" include plural referents unless the context clearly dictates otherwise. For example, "a" or "an" means "at least one" or "one or more." It is understood that aspects and variations described herein include "consisting" and/or "consisting essentially of" aspects and variations.

Throughout this disclosure, various aspects of the claimed subject matter are presented in a range format. It should be understood that the description in range format is merely for convenience and brevity and should not be construed as an inflexible limitation on the scope of the claimed subject matter. Accordingly, the description of a range should be considered to have specifically disclosed all the possible sub-ranges as well as individual numerical values within that range. For example, where a range of values is provided, it is understood that each intervening value, between the upper and lower limit of that range and any other stated or intervening value in that stated range is encompassed within the claimed subject matter. The upper and lower limits of these smaller ranges may independently be included in the smaller ranges, and are also encompassed within the claimed subject matter, subject to any specifically excluded limit in the stated range. Where the stated range includes one or both of the limits, ranges excluding either or both of those included limits are also included in the claimed subject matter. This applies regardless of the breadth of the range.

The term "about" as used herein refers to the usual error range for the respective value readily known to the skilled person in this technical field. Reference to "about" a value or parameter herein includes (and describes) embodiments that are directed to that value or parameter *per se.* For example, description referring to "about X" includes description of "X". In some embodiments, "about" refers to within ±20%, ±15%, ±10%, ±5%, or ±1% of the value or parameter.

The terms "polypeptide" and "protein" are used interchangeably to refer to a polymer of amino acid residues, and are not limited to a minimum length. Polypeptides, including the provided antibodies and antibody chains and other peptides, e.g., linkers, may include amino acid residues including natural and/or non-natural amino acid residues. The terms also include post-expression modifications of the polypeptide, for example, glycosylation, sialylation, acetylation, phosphorylation, and the like. In some aspects, the polypeptides may contain modifications with respect to a native or natural sequence, as long as the protein maintains the desired activity. These modifications may be deliberate, as through site-directed mutagenesis, or may be accidental, such as through mutations of hosts which produce the proteins or errors due to PCR amplification.

As used herein, a composition refers to any mixture of two or more products, substances, or compounds, including cells. It may be a solution, a suspension, liquid, powder, a paste, aqueous, non-aqueous or any combination thereof.

As used herein, a statement that a cell or population of cells is "positive" for a particular marker refers to the detectable presence on or in the cell of a particular marker, typically a surface marker. When referring to a surface marker, the term refers to the presence of surface expression as detected by flow cytometry, for example, by staining with an antibody that specifically binds to the marker and detecting said antibody, wherein the staining is detectable by flow cytometry at a level substantially above the staining detected carrying out the same procedure with an isotype-matched control under otherwise identical conditions and/or at a level substantially similar to that for cell known to be positive for the marker, and/or at a level substantially higher than that for a cell known to be negative for the marker.

As used herein, a statement that a cell or population of cells is "negative" for a particular marker refers to the absence of substantial detectable presence on or in the cell of a particular marker, typically a surface marker. When referring to a surface marker, the term refers to the absence of surface expression as detected by flow cytometry, for example, by staining with an antibody that specifically binds to the marker and detecting said antibody, wherein the staining is not detected by flow cytometry at a level substantially above the staining detected carrying out the same procedure with an isotype-matched control under otherwise identical conditions, and/or at a level substantially lower than that for cell known to be positive for the marker, and/or at a level substantially similar as compared to that for a cell known to be negative for the marker.

### VIII. EXEMPLARY EMBODIMENTS

Among the provided embodiments are:
1. A method of expanding cells, comprising incubating an input composition comprising cells expressing a recombinant antigen receptor comprising an extracellular antigen-binding domain that specifically binds or recognizes an antigen with a plurality of particles, e.g., beads, each of the plurality of particles, e.g., beads, comprising a binding molecule that specifically binds to the antigen-binding domain, wherein binding of the binding molecule to the antigen-binding domain induces expansion of the cells comprising the recombinant antigen receptor, thereby producing an output composition comprising expanded cells.
2. The method of embodiment 1, wherein the recombinant antigen receptor is a chimeric antigen receptor (CAR).
3. The method of embodiment 1 or embodiment 2, wherein the antigen-binding domain comprises an antibody or antigen-binding fragment thereof.
4. The method of embodiment 3, wherein the antigen-binding fragment is or comprises a single chain antibody fragment.
5. The method of embodiment 3 or embodiment 4, wherein the antigen-binding fragment thereof comprises antibody variable regions joined by a flexible linker.
6. The method of any of embodiments 3-5, wherein the antigen-binding fragment thereof is or comprises an scFv.
7. The method of any of embodiments 1-6, wherein the antigen is selected from among ROR1, B cell maturation antigen (BCMA), carbonic anhydrase 9 (CAIX), Her2/neu (receptor tyrosine kinase erbB2), Ll-CAM, CD19, CD20, CD22, mesothelin, CEA, and hepatitis B surface antigen, anti-folate receptor, CD23, CD24, CD30, CD33, CD38, CD44, EGFR, epithelial glycoprotein 2 (EPG-2), epithelial glycoprotein 40 (EPG-40), EPHa2, erb-B2, erb-B3, erb-B4, erbB dimers, EGFR vIII, folate binding protein (FBP), FCRL5, FCRH5, fetal acetylcholine receptor, GD2, GD3, HMW-MAA, IL-22R-alpha, IL-13R-alpha2, kinase insert domain receptor (kdr), kappa light chain, Lewis Y, L1-cell adhesion molecule, (L1-CAM), Melanoma-associated antigen (MAGE)-A1, MAGE-A3, MAGE-A6, Preferentially expressed antigen of melanoma (PRAME), survivin, TAG72, B7-H6, IL-13 receptor alpha 2 (IL-13Ra2), CA9, GD3, HMW-MAA, CD171, G250/CAIX, HLA-AI MAGE Al, HLA-A2 NY-ESO-1, PSCA, folate receptor-a, CD44v6, CD44v7/8, avb6 integrin, 8H9, NCAM, VEGF receptors, 5T4, Foetal AchR, NKG2D ligands, CD44v6, dual antigen, a cancer-testes antigen, mesothelin, murine CMV, mucin 1 (MUC1), MUC16, PSCA, NKG2D, NY-ESO-1, MART-1, gp100, oncofetal antigen, ROR1, TAG72, VEGF-R2, carcinoembryonic antigen (CEA), prostate specific antigen, PSMA, Her2/neu, estrogen receptor, progesterone receptor, ephrinB2, CD123, c-Met, GD-2, O-acetylated GD2 (OGD2), CE7, Wilms Tumor 1 (WT-1), a cyclin, cyclin A2, CCL-1, CD138, a pathogen-specific antigen and an antigen associated with a universal tag and/or the antigen is selected from among αvβ6 integrin (avb6 integrin), B cell maturation antigen (BCMA), B7-H3, B7-H6, carbonic anhydrase 9 (CA9, also known as CAIX or G250), a cancer-testis antigen, cancer/testis antigen 1B (CTAG, also known as NY-ESO-1 and LAGE-2), carcinoembryonic antigen (CEA), a cyclin, cyclin A2, C-C Motif Chemokine Ligand 1 (CCL-1), CD19, CD20, CD22, CD23, CD24, CD30, CD33, CD38, CD44, CD44v6, CD44v7/8, CD123, CD138, CD171, chondroitin sulfate proteoglycan 4 (CSPG4), epidermal growth factor protein (EGFR), type III epidermal growth factor receptor mutation (EGFR vIII), epithelial glycoprotein 2 (EPG-2), epithelial glycoprotein 40 (EPG-40), ephrinB2, ephrin receptor A2 (EPHa2), estrogen receptor, Fc receptor like 5 (FCRL5; also known as Fc receptor homolog 5 or FCRH5), fetal acetylcholine receptor (fetal AchR), a folate binding protein (FBP), folate receptor alpha, ganglioside GD2, O-acetylated GD2 (OGD2), ganglioside GD3, glycoprotein 100 (gp100), G Protein Coupled Receptor 5D (GPRC5D), Her2/neu (receptor tyrosine kinase erb-B2), Her3 (erb-B3), Her4 (erb-B4), erbB dimers, Human high molecular weight-melanoma-associated antigen (HMW-MAA), hepatitis B surface antigen, Human leukocyte antigen A1 (HLA-A1), Human leukocyte antigen A2 (HLA-A2), IL-22 receptor alpha(IL-22Ra), IL-13 receptor alpha 2 (IL-13Ra2), kinase insert domain receptor (kdr), kappa light chain, L1 cell adhesion molecule (L1-CAM), CE7 epitope of L1-CAM, Leucine Rich Repeat Containing 8 Family Member A (LRRC8A), Lewis Y, Melanoma-associated antigen (MAGE)-A1, MAGE-A3, MAGE-A6, mesothelin, c-Met, murine cytomegalovirus (CMV), mucin 1 (MUC1), MUC16, natural killer group 2 member D (NKG2D) ligands, melan A (MART-1), neural cell adhesion molecule (NCAM), oncofetal antigen, Preferentially expressed antigen of melanoma (PRAME), progesterone receptor, a prostate specific antigen, prostate stem cell antigen (PSCA), prostate specific membrane antigen (PSMA), Receptor Tyrosine Kinase Like Orphan Receptor 1 (ROR1), survivin, Trophoblast glycoprotein (TPBG also known as 5T4), tumor-associated glycoprotein 72 (TAG72), vascular endothelial growth factor receptor (VEGFR), vascular endothelial growth factor receptor 2 (VEGFR2), Wilms Tumor 1 (WT-1), a pathogen-specific or pathogen-expressed antigen, or an antigen associated with a universal tag, and/or biotinylated molecules, and/or molecules expressed by HIV, HCV, HBV or other pathogen.
8. The method of any of embodiments 1-7, wherein the binding molecule does not bind or recognize a linker or spacer region of the recombinant antigen receptor, said linker or spacer region connecting the antigen-binding domain to the transmembrane domain of the antigen receptor.
9. The method of any of embodiments 1-8, wherein the binding molecule is an anti-idiotypic antibody or antigen-binding fragment thereof that specifically binds to the antigen-binding domain.
10. A method of expanding cells, comprising incubating an input composition comprising cells expressing a chimeric antigen receptor (CAR) comprising an antigen-binding domain that specifically binds or recognizes an antigen with a plurality of particles, e.g., beads, each of the plurality of particles, e.g., beads, comprising a binding molecule that is an anti-idiotypic antibody or antigen-binding fragment thereof that specifically binds to the antigen-binding domain, wherein binding of the anti-idiotyptic antibody or antigen-binding fragment thereof to the antigen-binding domain induces expansion of the cells comprising the chimeric antigen receptor, thereby producing an output composition comprising expanded cells.
11. The method of any of embodiments 1-8, wherein the binding molecule comprises a recombinant antigen or a portion thereof recognized by the antigen-binding domain.
12. A method of expanding cells, comprising incubating an input composition comprising cells expressing a chimeric antigen receptor (CAR) comprising an antigen-binding domain that specifically binds or recognizes an antigen with a plurality of particles, e.g., beads, each of the plurality of particles, e.g., beads, comprising a binding molecule comprising a recombinant antigen or a portion thereof recognized by the antigen-binding domain, wherein binding of the recombinant antigen or portion thereof to the antigen-binding domain induces expansion of the cells comprising the chimeric antigen receptor, thereby producing an output composition comprising expanded cells.
13. The method of embodiment 11 or embodiment 12, wherein the recombinant antigen is selected from among ROR1, B cell maturation antigen (BCMA), carbonic anhydrase 9 (CAIX), Her2/neu (receptor tyrosine kinase erbB2), Ll-CAM, CD19, CD20, CD22, mesothelin, CEA, and hepatitis B surface antigen, anti-folate receptor, CD23, CD24, CD30, CD33, CD38, CD44, EGFR, epithelial glycoprotein 2 (EPG-2), epithelial glycoprotein 40 (EPG-40), EPHa2, erb-B2, erb-B3, erb-B4, erbB dimers, EGFR vIII, folate binding protein (FBP), FCRL5, FCRH5, fetal acetylcholine receptor, GD2, GD3, HMW-MAA, IL-22R-alpha, IL-13R-alpha2, kinase insert domain receptor (kdr), kappa light chain, Lewis Y, L1-cell adhesion molecule, (L1-CAM), Melanoma-associated antigen (MAGE)-A1, MAGE-A3, MAGE-A6, Preferentially expressed antigen of melanoma (PRAME), survivin, TAG72, B7-H6, IL-13 receptor alpha 2 (IL-13Ra2), CA9, GD3, HMW-MAA, CD171, G250/CAIX, HLA-AI MAGE Al, HLA-A2 NY-ESO-1, PSCA, folate receptor-a, CD44v6, CD44v7/8, avb6 integrin, 8H9, NCAM, VEGF receptors, 5T4, Foetal AchR, NKG2D ligands, CD44v6, dual antigen, a cancer-testes antigen, mesothelin, murine CMV, mucin 1 (MUC1), MUC16, PSCA, NKG2D, NY-ESO-1, MART-1, gp100, oncofetal antigen, ROR1, TAG72, VEGF-R2, carcinoembryonic antigen (CEA), prostate specific antigen, PSMA, Her2/neu, estrogen receptor, progesterone receptor, ephrinB2, CD123, c-Met, GD-2, O-acetylated GD2 (OGD2), CE7, Wilms Tumor 1 (WT-1), a cyclin, cyclin A2, CCL-1, CD 13 8 and a pathogen-specific antigen or a portion of any of the foregoing recognized by the antigen-binding domain and/or the antigen is selected from among αvβ6 integrin (avb6 integrin), B cell maturation antigen (BCMA), B7-H3, B7-H6, carbonic anhydrase 9 (CA9, also known as CAIX or G250), a cancer-testis antigen, cancer/testis antigen 1B (CTAG, also known as NY-ESO-1 and LAGE-2), carcinoembryonic antigen (CEA), a cyclin, cyclin A2, C-C Motif Chemokine Ligand 1 (CCL-1), CD19, CD20, CD22, CD23, CD24, CD30, CD33, CD38, CD44, CD44v6, CD44v7/8, CD123, CD138, CD171, epidermal growth factor protein (EGFR), type III epidermal growth factor receptor mutation (EGFR vIII), epithelial glycoprotein 2 (EPG-2), epithelial glycoprotein 40 (EPG-40), ephrinB2, ephrin receptor A2 (EPHa2), estrogen receptor, Fc receptor like 5 (FCRL5; also known as Fc receptor homolog 5 or FCRH5), fetal acetylcholine receptor (fetal AchR), a folate binding protein (FBP), folate receptor alpha, ganglioside GD2, O-acetylated GD2 (OGD2), ganglioside GD3, glycoprotein 100 (gp100), G Protein Coupled Receptor 5D (GPRC5D), Her2/neu (receptor tyrosine kinase erb-B2), Her3 (erb-B3), Her4 (erb-B4), erbB dimers, Human high molecular weight-melanoma-associated antigen (HMW-MAA), hepatitis B surface antigen, Human leukocyte antigen A1 (HLA-A1), Human leukocyte antigen A2 (HLA-A2), IL-22 receptor alpha(IL-22Ra), IL-13 receptor alpha 2 (IL-13Ra2), kinase insert domain receptor (kdr), kappa light chain, L1 cell adhesion molecule (L1-CAM), CE7 epitope of L1-CAM, Leucine Rich Repeat Containing 8 Family Member A (LRRC8A), Lewis Y, Melanoma-associated antigen (MAGE)-A1, MAGE-A3, MAGE-A6, mesothelin, c-Met, murine cytomegalovirus (CMV), mucin 1 (MUC1), MUC16, natural killer group 2 member D (NKG2D) ligands, melan A (MART-1), neural cell adhesion molecule (NCAM), oncofetal antigen, Preferentially expressed antigen of melanoma (PRAME), progesterone receptor, a prostate specific antigen, prostate stem cell antigen (PSCA), prostate specific membrane antigen (PSMA), Receptor Tyrosine Kinase Like Orphan Receptor 1 (ROR1), survivin, Trophoblast glycoprotein (TPBG also known as 5T4), tumor-associated glycoprotein 72 (TAG72), vascular endothelial growth factor receptor (VEGFR), vascular endothelial growth factor receptor 2 (VEGFR2), Wilms Tumor 1 (WT-1), a pathogen-specific or pathogen-expressed antigen, or an antigen associated with a universal tag, and/or biotinylated molecules, and/or molecules expressed by HIV, HCV, HBV or other pathogen.
14. The method of any of embodiments 11-13, wherein the recombinant antigen is BCMA, CD22 or ROR1, or is a portion thereof recognized by the antigen-binding domain.
15. The method of any of embodiments 11-14, wherein the portion of the recombinant antigen recognized by the antigen-binding domain comprises the extracellular domain or a portion of the extracellular domain of the antigen.
16. The method of any of embodiments 11-14, wherein the portion of the recombinant antigen recognized by the antigen-binding domain consists essentially of the extracellular domain or a portion of the extracellular domain of the antigen.
17. A method of expanding cells, comprising incubating an input composition comprising cells expressing a chimeric antigen receptor (CAR) comprising an antigen-binding domain that specifically binds or recognizes B cell maturation antigen (BCMA) with a plurality of particles, e.g., beads, each of the plurality of particles, e.g., beads, comprising a binding molecule comprising the extracellular domain of BCMA or a portion of the extracellular domain recognized by the antigen-binding domain, wherein binding of the extracellular domain of BCMA or portion thereof to the antigen-binding domain induces expansion of the cells comprising the chimeric antigen receptor, thereby producing an output composition comprising expanded cells.
18. The method of embodiment 17, wherein the portion of BCMA consists essentially of the extracellular domain or a portion of the extracellular domain.
19. The method of any of embodiments 11-18, wherein the binding molecule is a fusion polypeptide comprising the recombinant antigen or the portion thereof linked to a moiety, optionally wherein the moiety facilitates attachment to the particle.
20. The method of embodiment 19, wherein the moiety is linked to the C-terminus of the recombinant antigen.
21. The method of embodiment 19 or embodiment 20, wherein the moiety is hydrophobic or is enriched in hydrophobic amino acids.
22. The method of any of embodiments 19-21, wherein the moiety is or comprises an Fc domain.
23. The method of embodiment 22, wherein the Fc region is derived from human IgG.
24. The method of any of embodiments 1-12, wherein the antigen is CD19.
25. A method of expanding cells, comprising incubating an input composition comprising cells expressing a chimeric antigen receptor (CAR) comprising an antigen-binding domain that specifically binds or recognizes CD19 with a plurality of particles, e.g., beads, each of the plurality of particles, e.g., beads, comprising a binding molecule that is an anti-idiotypic antibody or antigen-binding fragment thereof that specifically binds to the antigen-binding domain, wherein binding of the anti-idiotypic antibody or antigen-binding fragment thereof to the antigen-binding domain induces expansion of the cells comprising the chimeric antigen receptor, thereby producing an output composition comprising expanded cells.
26. The method of embodiment 9, embodiment 24 or embodiment 25, wherein the antigen-binding domain of the antigen receptor is or comprises antibody SJ25C1 or an antigen-binding fragment thereof.
27. The method of embodiment 9, embodiment 24 or embodiment 25, wherein the antigen-binding domain of the antigen receptor is or comprises antibody FMC63 or an antigen-binding fragment thereof.
28. The method of embodiment 26 or embodiment 27, wherein the antigen-binding fragment is or comprises an scFv.
29. The method of any of embodiments 1-28, wherein the antigen or recombinant antigen is human.
30. The method of any of embodiments 9, 10 and 25-29, wherein the anti-idiotypic antibody or antigen-binding fragment thereof comprises at least a portion of an immunoglobulin constant region.
31. The method of embodiment 30, wherein the at least a portion of an immunoglobulin constant region comprises an Fc region or a portion of the Fc comprising the CH2 and CH3 domains.
32. The method of embodiment 30 or embodiment 31, wherein the constant region or Fc region is derived from human IgG.
33. The method of any one of embodiments 9, 10 and 25-32, wherein the anti-idiotypic antibody or antigen-binding fragment thereof is an intact antibody or full-length antibody.
34. The method of any of embodiments 1-33, wherein the binding molecule is covalently or non-covalently attached to the particles, e.g., beads.
35. The method of any of embodiments 1-34, wherein the binding molecule is attached to each of the plurality of particles, e.g., beads, at or near the C-terminal amino acid residue of the binding molecule and/or attachment of the binding molecule to each of the plurality of particles, e.g., beads, is carried out such that the region or epitope of the binding molecule recognized by the antigen-binding domain of the antigen receptor is oriented so that it is capable of being recognized by the antigen receptor.
36. The method of any of embodiments 1-35, wherein the particles, e.g., beads, are synthetic particles, insoluble particles, solid particles or are non-cellular particles.
37. The method of any of embodiments 1-35, wherein the plurality of particles comprises beads.
38. The method of any of embodiments 1-37, wherein the plurality of particles, e.g., beads, comprises a mean diameter of between or between about 1 µm and 10 µm or between or between about 2 µm and 5 µm.
39. The method of any of embodiments 1-38, wherein the plurality of particles, e.g., beads, comprises a mean diameter of about 2.8 µm.
40. The method of any of embodiments 1-38, wherein the plurality of particles, e.g., beads, comprises a mean diameter of about 4.5 µm.
41. The method of any of embodiments 1-40, wherein the plurality of particles, e.g., beads, comprises a mean density of between about 0.5 g/cm³ and 5.0 g/cm³ or between or between about 1 g/cm³ and about 2 g/cm³.
42. The method of any of embodiments 1-41, wherein the plurality of particles, e.g., beads, comprises a mean density of about 1.3 g/cm³.
43. The method of embodiments 1-42, wherein the plurality of particles, e.g., beads, comprises a mean density of about 1.5 g/cm³.
44. The method of any of embodiments 1-43, wherein the plurality of particles, e.g., beads, is monodisperse.
45. The method of any of embodiments 1-44, wherein the binding molecule is covalently attached to the particles, e.g., beads.
46. The method of any of embodiments 1-45, wherein the particle comprises a surface exposed functional group for attachment of the binding molecule and/or wherein the binding molecule is covalently attached to the particle via a surface exposed functional group.
47. The method of embodiment 46, wherein the surface exposed functional group is an amino group, a carboxyl group, a thiol group, an aldehyde group, a chloromethyl group, an epoxy group, a hydroxyl group, a tosyl group or a hydrazine group.
48. The method of embodiment 46 or embodiment 47, wherein the surface exposed functional group is a tosyl group.
49. The method of any of embodiments 1-48, wherein the plurality of particles, e.g., beads, are biocompatible or non-toxic to cells.
50. The method of any of embodiments 1-49, wherein the plurality of particles, e.g., beads, comprise particles comprising glass, silica, polyesters of hydroxy carboxylic acids, polyanhydrides of dicarboxylic acids, copolymers of hydroxy carboxylic acids, copolymers dicarboxylic acids, or metal.
51. The method of any of embodiments 1-50, wherein the particles, e.g., beads, comprise a surface comprising a polymer, a polysaccharide, a silica, a fatty acid, a carbon or a combination thereof.
52. The method of embodiment 51, wherein the polymer is polyethylene glycol, poly(lactic-co-glycolic acid), polyglutaraldehyde, polyurethane, polystyrene, and polyvinyl alcohol or combinations thereof.
53. The method of any of embodiments 1-52, wherein the plurality of particles, e.g., beads, comprise particles comprising a hydrophobic surface.
54. The method of any of embodiments 1-53, wherein the plurality of particles, e.g., beads, comprise particles comprising a polystyrene surface.
55. The method of any of embodiments 1-54, the plurality of particles, e.g., beads, comprise particles that are magnetic and/or comprise a magnetic core, a paramagnetic core or a superparamagnetic core.
56. The method of any of embodiments 1-55, wherein the concentration of the binding molecule is between or between about 0.5 µg/mL and 500 µg/mL, 1 µg/mL and 200 µg/mL or 5 µg/mL and 100 µg/mL.
57. The method of any of embodiments 1-56, wherein the concentration of the binding molecule is at least or at least about 1 µg/mL, 5 µg/mL, 10 µg/mL, 25 µg/mL, 50 µg/mL, 100 µg/mL or 200 µg/mL.
58. The method of any of embodiments 1-57, wherein each of the plurality of particles, e.g., beads, comprises at least or about at least 10 copies, 10² copies, 10³ copies, 10⁴ copies, 10⁵ copies or 10⁶ copies of the binding molecule.
59. The method of any of embodiments 1-58, wherein at least a portion of the incubation is performed in the presence of an agent that specifically binds to an additional molecule on the cell to provide an accessory signal and/or to block an inhibitory signal.
60. The method of any of embodiments 1-59, wherein the agent is provided together with the particles, e.g., beads, optionally the agent is comprised by each of the plurality of particles, e.g., beads, or a subset thereof.
61. The method of any of embodiments 1-59, wherein the agent is provided separately from the plurality of particles, e.g., beads.
62. The method of any of embodiments 1-60, wherein the particles, e.g., beads, further comprise an agent that specifically binds to an additional molecule on the cell to provide an accessory signal and/or to block an inhibitory signal.
63. The method of any of embodiments 59-62, wherein the agent is a ligand or is an antibody or antigen-binding fragment thereof.
64. The method of any of embodiments 58-63, wherein the molecule is a costimulatory molecule or is an activating co-receptor or a ligand thereof.
65. The method of embodiment 64, wherein the costimulatory molecule or activating co-receptor is OX-40, ICOS, DAP10, CD28 or 4-1BB or is a ligand thereof, optionally OX-40L, ICOSL, B7-1, B7-2 or 4-1BBL.
66. The method of any of embodiments 58-63, wherein the molecule is an inhibitory receptor or a ligand thereof.
67. The method of embodiment 66, wherein the inhibitory receptor is CTLA-4, PD-1, LAG-3, Tim-3, BTLA or TIGIT or is a ligand thereof, optionally PD-L1, PD-L2, CD155, CD112 or LIGHT.
68. The method of any of embodiments 62-67, wherein the agent is covalently attached to the particles, e.g., beads.
69. The method of any of embodiments 62-68, wherein the ratio, optionally molar or weight ratio, of the binding molecule and the agent comprised by the particles, e.g., beads, is or is about 1:1.
70. The method of any of embodiments 1-69, wherein the ratio of total cells present in the input composition to particles, e.g., beads, is from or from about 5:1 to 1:5, 3:1 to 1:3 or 2:1 to 1:2.
71. The method of any of embodiments 1-70, wherein the ratio of total cells present in the input composition to particles, e.g., beads, is from or from about 1:0.1 to 1:5.
72. The method of any of embodiments 1-71, wherein the ratio of total cells present in the input composition to particles, e.g., beads, is or is about 1:1.
73. The method of any of embodiments 1-72, wherein the incubation is carried out for greater than or greater than about 2 hours, 4 hours, 8 hours, 12 hours, 24 hours, 2 days, 3 days, 4 days, 5 days, 6 days, 7 days, 8 days, 9 days, 10 days, 11 days or 12 days.
74. The method of any of embodiments 1-73, wherein the incubation is carried out at a temperature between or between about 30 °C and 39 °C.
75. The method of any of embodiments 1-74, wherein the incubation is carried out at a temperature of 37 ° ± 2.0 °C.
76. The method of any of embodiments 1-75, wherein the cells comprise immune cells or induced pluripotent stem cells (iPSC).
77. The method of any of embodiments 1-76, wherein the immune cell is a T cell or an NK cell.
78. The method of any of embodiments 1-77, wherein the cells comprise CD4+ and/or CD8+ T cells.
79. The method of any of clams 1-78, wherein the ratio of the CD4+ cells to the CD8+ cells is or is about 1:1, 1:2, 2:1, 1:3 or 3:1.
80. The method of any of embodiments 1-79, wherein the cells are primary cells obtained from a subject, optionally a human subject.
81. The method of any of embodiments 1-80, wherein the cells are human.
82. The method of any of embodiments 1-81, wherein the input composition is produced by a method comprising contacting a composition of cells with a nucleic acid molecule encoding the recombinant antigen receptor under conditions to introduce the nucleic acid molecule into one or more cells in the composition.
83. A method of genetically engineering a cell, comprising:
   (a) contacting a composition of cells with a nucleic acid molecule encoding a recombinant antigen receptor under conditions to introduce the nucleic acid molecule into one or more cells in the composition, thereby producing an input composition; and
   (b) incubating cells of the input composition according to the method of any of embodiments 1-74.
84. The method of embodiment 82 or embodiment 83, wherein at least a portion of the contacting and incubating are carried out simultaneously.
85. The method of any of embodiments 82-84, wherein the nucleic acid molecule is comprised in a viral vector, an episomal vector or a transposon.
86. The method of any of embodiments 82-85, wherein the contacting is carried out by transposon/transposase gene transfer.
87. The method of any of embodiments 82-85, wherein the contacting is carried out by transduction with a viral vector.
88. The method of embodiment 85 or embodiment 87, wherein the viral vector is a retrovirus, which optionally is a gamma-retroviral vector or a lentiviral vector.
89. The method of embodiment 87 or embodiment 88, wherein the contacting comprises a step of spinoculating the viral vector with the composition of cells.
90. The method of embodiment 89, wherein spinoculating comprises rotating, in an internal cavity of a centrifugal chamber, the viral vector particles and composition of cells, wherein the rotation is at a relative centrifugal force at an internal surface of the side wall of the cavity that is:
   between or between about 500 g and 2500 g, 500 g and 2000 g 500 g and 1600 g, 500 g an 1000 g, 600 g and 1600 g, 600 g and 1000 g, 1000 g and 2000 g or 1000 g and 1600 g, each inclusive; or
   at least or at least about 600 g, 800 g, 1000 g, 1200 g, 1600 g, or 2000 g.
91. The method of embodiment 89 or embodiment 90, wherein spinoculating is for a time that is:
   greater than or about 5 minutes, greater than or about 10 minutes, greater than or about 15 minutes, greater than or about 20 minutes, greater than or about 30 minutes, greater than or about 45 minutes, greater than or about 60 minutes, greater than or about 90 minutes or greater than or about 120 minutes; or
   between or between about 5 minutes and 60 minutes, 10 minutes and 60 minutes, 15 minutes and 60 minutes, 15 minutes and 45 minutes, 30 minutes and 60 minutes or 45 minutes and 60 minutes, each inclusive.
92. The method of any of embodiments 87-91, wherein the contacting is carried out in the presence of a transduction adjuvant.
93. The method of any of embodiments 82-92, wherein the composition of cells comprises a plurality of T cells and, prior to the contacting, the method does not comprise stimulating or activating the T cells.
94. The method of any of embodiments 82-93, wherein the composition of cells comprises a plurality of T cells and, prior to the contacting, the method does not comprise incubating the composition in the presence of an agent or agents capable of inducing a signal through a TCR complex and/or incubation in the presence of an agent or agents capable of inducing proliferation of T cells, CD4+ T cells, and/or CD8+ T cells; and/or CD3-binding molecules, CD28-binding molecules, recombinant IL-2, recombinant IL-15, and recombinant IL-7 or a combination thereof.
95. The method of clam 93 or embodiment 94, wherein, prior to the contacting, the method does not comprise stimulating the T cells in the presence of an anti-CD3 antibody and/or an anti-CD28 antibody.
96. The method of any of embodiments 82-95, wherein the composition of cells comprises a plurality of T cells, said plurality of cells having been obtained from a sample from a subject, wherein:
   the contacting is initiated no more than 24 hours after obtaining the sample from the subject; and/or
   prior to the contacting, the T cells have not been subjected to a temperature greater than or greater than about 15° C, about 18 ° C, about 22 ° C or about 25 ° C for a duration of more than 1 hour, 2 hours, 4 hours, 6 hours, 8 hours, 12 hours, or 24 hours after obtaining the sample from the subject; and/or
   prior to the contacting, the T cells have not been subjected to a temperature of, of about, greater than, or greater than about 37 ° ± 2.0 °C for a duration of more than 15 minutes, 30 minutes, 1 hour or 2 hours after obtaining the sample from the subject.
97. The method of any of embodiments 82-96, wherein, prior to said contacting, no more than 5 %, 10 %, 20 %, 30 %, or 40 % of the T cells are activated cells, express a surface marker selected from the group consisting of HLA-DR, CD25, CD69, CD71, CD40L and 4-1BB; comprise intracellular expression of a cytokine selected from the group consisting of IL-2, IFN-gamma, TNF-alpha, are in the G1 or later phase of the cell cycle and/or are capable of proliferating.
98. The method of any of embodiments 1-97, further comprising, prior to the incubation or the contacting, obtaining a biological sample from the subject comprising the cells and, optionally, selecting or enriching the cells, optionally T cells, from the sample.
99. The method of any of embodiments 1-98, wherein the percent of cells expressing the recombinant antigen receptor in the input composition is less than or less than about 75%, 70%, 60%, 50%, 40%, 30%, 20%, 15%, 10% or less.
100. The method of any of embodiments 1-99, wherein the composition of cells or the input composition comprises at least or at least about 1 x 10² cells, 1 x 10³ cells, 1 x 10⁴ cells, 1 x 10⁵ cells, 1 x 10⁶ cells or 1 x 10⁷ cells.
101. The method of any of embodiments 1-100, wherein the surface expression of an activation marker or exhaustion marker of cells present in the output composition is less than surface expression of the marker in a composition of cells produced after a similar incubation but in the presence of polyclonal stimulatory molecule capable of activating one or more intracellular signaling domains of one or more components of a TCR complex.
102. The method of embodiment 101, wherein the exhaustion marker is an inhibitory receptor.
103. The method of embodiment 101 or embodiment 102, wherein the exhaustion marker is PD-1, CTLA-4, TIM-3, LAG-3, BTLA or TIGIT.
104. The method of embodiment 103, wherein the activation marker is HLA-DR, CD25, CD69, CD71, CD40L or 4-1BB.
105. The method of any of embodiments 93-96, wherein the surface expression is at least or at least about 1.2-fold, 1.5-fold, 2.0-fold, 3.0-fold, 4.0-fold, 5.0-fold, 10.0-fold or more less.
106. The method of any of embodiments 1-105, wherein the number of cells in the output composition is substantially the same or is greater than the number of cells in a composition of cells produced by a similar incubation but in the presence of a polyclonal stimulatory molecule capable of activating one or more intracellular signaling domains of one or more components of a TCR complex.
107. The method of any of embodiments 101-106, wherein the polyclonal stimulatory molecule comprises an anti-CD3 antibody or fragment and/or an anti-CD28 antibody or fragment.
108. The method of any of embodiments 1-107, wherein the number of the cells in the output composition is greater than the number of the cells in the input composition by greater than or greater than about 1.2-fold, 1.5-fold, 2.0-fold, 3.0-fold, 4.0-fold, 5.0-fold, 10.0-fold, 25-fold, 50-fold, 100-fold or more.
109. The method of any of embodiments 1-108, wherein the percent of cells comprising the recombinant antigen receptor in the output composition is greater than or greater than about 50%, 60%, 70%, 80%, 90%, 95% or more.
110. The method of any of embodiments 1-109, wherein the number of cells in the output composition comprising the recombinant antigen receptor is increased or enriched by 1.2-fold, 2.0-fold, 3.0-fold, 4.0-fold, 5.0-fold, 6.0-fold, 7.0-fold, 8.0-fold, 9.0-fold, 10-fold or more compared to the number of the cells comprising the antigen receptor in the input composition.
111. The method of any of embodiments 1-110, wherein at least a portion of the incubation is carried out in the presence of one or more additional agents that modulate cell expansion or activity.
112. The method of embodiment 111, wherein the one or more additional agent is one or more cytokines.
113. The method of any of embodiments 1-112 that is performed in vitro or ex vivo.
114. The method of any of embodiments 1-113, wherein the antigen receptor is a CAR and the CAR further an intracellular signaling domain comprising an ITAM.
115. The method of embodiment 114, wherein the intracellular signaling domain comprises an intracellular domain of a CD3-zeta (CD3ζ) chain.
116. The method of embodiment 114 or embodiment 115, wherein the CAR further comprises a costimulatory signaling region.
117. The method of embodiment 116, wherein the costimulatory signaling region comprises a signaling domain of CD28 or 4-1BB.
118. The method of embodiment 117, wherein the costimulatory domain is CD28.
119. The method of any of embodiments 1-118, further comprising removing the plurality of particles, e.g., beads, from the output composition.
120. A composition of cells produced by the method of any of embodiments 1-119.
121. A surface modified particle, comprising a particle and a binding molecule bound to the surface of the particle, wherein the binding molecule specifically binds to an extracellular antigen-binding domain of an antigen receptor.
122. The surface modified particle of embodiment 121, wherein the antigen receptor is a chimeric antigen receptor (CAR).
123. The surface modified particle of embodiment 121 or embodiment 122 , wherein the binding molecule does not bind or recognize a linker or spacer region of the recombinant antigen receptor, said linker or spacer region connecting the antigen-binding domain to the transmembrane domain of the antigen receptor.
124. The surface modified particle of any of embodiments 121-123, wherein the binding molecule comprises a recombinant antigen or a portion thereof recognized by the antigen-binding domain.
125. The surface modified particle of embodiment 124, wherein the recombinant antigen is selected from among ROR1, B cell maturation antigen (BCMA), carbonic anhydrase 9 (CAIX), Her2/neu (receptor tyrosine kinase erbB2), Ll-CAM, CD19, CD20, CD22, mesothelin, CEA, and hepatitis B surface antigen, anti-folate receptor, CD23, CD24, CD30, CD33, CD38, CD44, EGFR, epithelial glycoprotein 2 (EPG-2), epithelial glycoprotein 40 (EPG-40), EPHa2, erb-B2, erb-B3, erb-B4, erbB dimers, EGFR vIII, folate binding protein (FBP), FCRL5, FCRH5, fetal acetylcholine receptor, GD2, GD3, HMW-MAA, IL-22R-alpha, IL-13R-alpha2, kinase insert domain receptor (kdr), kappa light chain, Lewis Y, L1-cell adhesion molecule, (L1-CAM), Melanoma-associated antigen (MAGE)-A1, MAGE-A3, MAGE-A6, Preferentially expressed antigen of melanoma (PRAME), survivin, TAG72, B7-H6, IL-13 receptor alpha 2 (IL-13Ra2), CA9, GD3, HMW-MAA, CD171, G250/CAIX, HLA-AI MAGE Al, HLA-A2 NY-ESO-1, PSCA, folate receptor-a, CD44v6, CD44v7/8, avb6 integrin, 8H9, NCAM, VEGF receptors, 5T4, Foetal AchR, NKG2D ligands, CD44v6, dual antigen, a cancer-testes antigen, mesothelin, murine CMV, mucin 1 (MUC1), MUC16, PSCA, NKG2D, NY-ESO-1, MART-1, gp100, oncofetal antigen, ROR1, TAG72, VEGF-R2, carcinoembryonic antigen (CEA), prostate specific antigen, PSMA, Her2/neu, estrogen receptor, progesterone receptor, ephrinB2, CD123, c-Met, GD-2, O-acetylated GD2 (OGD2), CE7, Wilms Tumor 1 (WT-1), a cyclin, cyclin A2, CCL-1, CD138 and a pathogen-specific antigen or a portion of any of the foregoing recognized by the antigen-binding domain and/or the antigen is selected from among αvβ6 integrin (avb6 integrin), B cell maturation antigen (BCMA), B7-H3, B7-H6, carbonic anhydrase 9 (CA9, also known as CAIX or G250), a cancer-testis antigen, cancer/testis antigen 1B (CTAG, also known as NY-ESO-1 and LAGE-2), carcinoembryonic antigen (CEA), a cyclin, cyclin A2, C-C Motif Chemokine Ligand 1 (CCL-1), CD19, CD20, CD22, CD23, CD24, CD30, CD33, CD38, CD44, CD44v6, CD44v7/8, CD123, CD138, CD171, epidermal growth factor protein (EGFR), type III epidermal growth factor receptor mutation (EGFR vIII), epithelial glycoprotein 2 (EPG-2), epithelial glycoprotein 40 (EPG-40), ephrinB2, ephrin receptor A2 (EPHa2), estrogen receptor, Fc receptor like 5 (FCRL5; also known as Fc receptor homolog 5 or FCRH5), fetal acetylcholine receptor (fetal AchR), a folate binding protein (FBP), folate receptor alpha, ganglioside GD2, O-acetylated GD2 (OGD2), ganglioside GD3, glycoprotein 100 (gp100), G Protein Coupled Receptor 5D (GPRC5D), Her2/neu (receptor tyrosine kinase erb-B2), Her3 (erb-B3), Her4 (erb-B4), erbB dimers, Human high molecular weight-melanoma-associated antigen (HMW-MAA), hepatitis B surface antigen, Human leukocyte antigen A1 (HLA-A1), Human leukocyte antigen A2 (HLA-A2), IL-22 receptor alpha(IL-22Ra), IL-13 receptor alpha 2 (IL-13Ra2), kinase insert domain receptor (kdr), kappa light chain, L1 cell adhesion molecule (L1-CAM), CE7 epitope of L1-CAM, Leucine Rich Repeat Containing 8 Family Member A (LRRC8A), Lewis Y, Melanoma-associated antigen (MAGE)-A1, MAGE-A3, MAGE-A6, mesothelin, c-Met, murine cytomegalovirus (CMV), mucin 1 (MUC1), MUC16, natural killer group 2 member D (NKG2D) ligands, melan A (MART-1), neural cell adhesion molecule (NCAM), oncofetal antigen, Preferentially expressed antigen of melanoma (PRAME), progesterone receptor, a prostate specific antigen, prostate stem cell antigen (PSCA), prostate specific membrane antigen (PSMA), Receptor Tyrosine Kinase Like Orphan Receptor 1 (ROR1), survivin, Trophoblast glycoprotein (TPBG also known as 5T4), tumor-associated glycoprotein 72 (TAG72), vascular endothelial growth factor receptor (VEGFR), vascular endothelial growth factor receptor 2 (VEGFR2), Wilms Tumor 1 (WT-1), a pathogen-specific or pathogen-expressed antigen, or an antigen associated with a universal tag, and/or biotinylated molecules, and/or molecules expressed by HIV, HCV, HBV or other pathogen.
126. The surface modified particle of embodiment 124 or embodiment 125 , wherein the recombinant antigen is BCMA, CD22 or ROR1, or is a portion thereof recognized by the antigen-binding domain.
127. The method of any of embodiments 124-126, wherein the portion of the recombinant antigen recognized by the antigen-binding domain comprises the extracellular domain or a portion of the extracellular domain of the antigen.
128. The method of any of embodiments 124-126, wherein the portion of the recombinant antigen recognized by the antigen-binding domain consists essentially of the extracellular domain or a portion of the extracellular domain of the antigen.
129. A surface modified particle, comprising a particle and a binding molecule bound to the surface of the particle, wherein the binding molecule comprises an extracellular domain or a portion thereof of B cell maturation antigen (BCMA).
130. The surface modified particle of any of embodiments 124-129, wherein the binding molecule is a fusion polypeptide comprising the recombinant antigen or the portion thereof linked to a moiety, optionally wherein the moiety facilitates attachment to the particle.
131. The surface modified particle of embodiment 130, wherein the moiety is linked to the C-terminus of the recombinant antigen.
132. The surface modified particle of embodiment 130 or embodiment 131, wherein the moiety is hydrophobic or is enriched in hydrophobic amino acids.
133. The surface modified particle of any of embodiments 130-132, wherein the moiety is or comprises an Fc domain.
134. The surface modified particle of embodiment 133, wherein the Fc region is derived from human IgG.
135. The surface modified particle of any of embodiments 130-134, wherein the recombinant antigen is human.
136. The surface modified particle of any of embodiments 121-123, wherein the binding molecule comprises an anti-idiotypic antibody or antigen-binding fragment thereof.
137. The surface modified particle of any of embodiments 121-123 and 136, wherein the antigen recognized by the antigen-binding domain is CD19.
138. The surface modified particle of embodiment 136 or embodiment 137, wherein the antigen-binding domain of the antigen receptor is or comprises antibody SJ25C1 or an antigen-binding fragment thereof.
139. The surface modified particle of embodiment 136 or embodiment 137, wherein the antigen-binding domain of the antigen receptor is or comprises antibody FMC63 or an antigen-binding fragment thereof.
140. The surface modified particle of embodiment 138 or embodiment 139, wherein the antigen-binding fragment is or comprises an scFv.
141. The surface modified particle of any of embodiments 136-140, wherein the anti-idiotypic antibody or antigen-binding fragment thereof comprises at least a portion of an immunoglobulin constant region.
142. The surface modified particle of embodiment 141, wherein the at least a portion of an immunoglobulin constant region comprises an Fc region or a portion of the Fc comprising the CH2 and CH3 domains.
143. The surface modified particle of embodiment 141 or embodiment 142, wherein the constant region or Fc region is derived from human IgG.
144. The surface modified particle of any one of embodiments 136-143, wherein the anti-idiotypic antibody or antigen-binding fragment thereof is an intact antibody or full-length antibody.
145. The surface modified particle of any of embodiments 121-144, wherein the binding molecule is covalently or non-covalently attached to the particles, e.g., beads.
146. The surface modified particle of any of embodiments 121-145, wherein the binding molecule is attached to each of the plurality of particles, e.g., beads, at or near the C-terminal amino acid residue of the binding molecule and/or attachment of the binding molecule to each of the plurality of particles, e.g., beads, is carried out such that the region or epitope of the binding molecule recognized by the antigen-binding domain of the antigen receptor is oriented so that it is capable of being recognized by the antigen receptor.
147. The surface modified particle of any of embodiments 121-146, wherein the particles, e.g., beads, are synthetic particles, insoluble particles, solid particles or are non-cellular particles.
148. The surface modified particle of any of embodiments 121-147, wherein the plurality of particles comprises beads.
149. The surface modified particle of any of embodiments 121-148, wherein the particle has a diameter of between or between about 1 µm and 10 µm or between or between about 2 µm and 5 µm.
150. The surface modified particle of any of embodiments 121-149, wherein the particle has a diameter of about 2.8 µm.
151. The surface modified particle of any of embodiments 121-149, wherein the particle has a diameter of about 4.5 µm.
152. The surface modified particle of any of embodiments 121-151, wherein the binding molecule is covalently attached to the particles, e.g., beads.
153. The surface modified particle of any of embodiments 121-152, wherein the particle comprises a surface exposed functional group for attachment of the binding molecule and/or wherein the binding molecule is covalently attached to the particle via a surface exposed functional group.
154. The surface modified particle of embodiment 153, wherein the surface exposed functional group is an amino group, a carboxyl group, a thiol group, an aldehyde group, a chloromethyl group, an epoxy group, a hydroxyl group, a tosyl group or a hydrazine group.
155. The surface modified particle of embodiment 154, wherein the surface exposed functional group is a tosyl group.
156. The surface modified particle of any of embodiments 121-155, wherein the particle is biocompatible or non-toxic to cells.
157. The surface modified particle of any of embodiments 121-156, wherein the plurality of particles, e.g., beads, comprise particles comprising glass, silica, polyesters of hydroxy carboxylic acids, polyanhydrides of dicarboxylic acids, copolymers of hydroxy carboxylic acids, copolymers dicarboxylic acids, or metal.
158. The surface modified particle of any of embodiments 121-157, wherein the particles, e.g., beads, comprise a surface comprising a polymer, a polysaccharide, a silica, a fatty acid, a carbon or a combination thereof.
159. The surface modified particle of embodiment 158, wherein the polymer is polyethylene glycol, poly(lactic-co-glycolic acid), polyglutaraldehyde, polyurethane, polystyrene, and polyvinyl alcohol or combinations thereof.
160. The surface modified particle of any of embodiments 121-149, wherein the particle comprises a hydrophobic surface.
161. The surface modified particle of any of embodiments 121-160, wherein the particles, e.g., beads, comprises a polystyrene surface.
162. The surface modified particle of any of embodiments 121-161, the particle is magnetic and/or comprises a magnetic core, a paramagnetic core or a superparamagnetic core.
163. The surface modified particle of any of embodiments 121-162, wherein the particle comprises at least or about at least 10 copies, 10² copies, 10³ copies, 10⁴ copies, 10⁵ copies or 10⁶ copies of the binding molecule.
164. The surface modified particle of any of embodiments 121-163, wherein the particle further comprises an agent that specifically binds to an additional molecule on the cell to provide an accessory signal and/or to block an inhibitory signal, thereby modulating expansion of the cells.
165. The surface modified particle of embodiment 165, wherein the agent is a ligand or is an antibody or antigen-binding fragment thereof.
166. The surface modified particle of embodiment 164 or embodiment 165, wherein the molecule is a costimulatory molecule or is an activating co-receptor or a ligand thereof.
167. The surface modified particle of embodiment 166, wherein the costimulatory molecule or activating co-receptor is OX-40, ICOS, DAP10, CD28 or 4-1BB, or is a ligand thereof, optionally OX-40L, ICOSL, B7-1, B7-2 or 4-1BBL.
168. The surface modified particle of embodiment 164 or embodiment 165, wherein the molecule is an inhibitory receptor or a ligand thereof.
169. The surface modified particle of embodiment 168, wherein the inhibitory receptor is CTLA-4, PD-1, LAG-3, Tim-3, BTLA or TIGIT, or is a ligand thereof, optionally PD-L1, PD-L2, CD155, CD112 or LIGHT.
170. The surface modified particle of any of embodiments 164-169, wherein the agent is covalently attached to the particles, e.g., beads.
171. The surface modified particle of any of embodiments 164-170, wherein the ratio, optionally molar or weight ratio, of the binding molecule and the agent comprised by the particle is or is about 1:1.
172. A composition, comprising a plurality of the surface modified particles, e.g., beads, of any of embodiments 113-153.
173. The composition of embodiment 172, wherein the concentration of the binding molecule is between or between about 0.5 µg/mL and 500 µg/mL, 1 µg/mL and 200 µg/mL or 5 µg/mL and 100 µg/mL.
174. The composition of embodiment 172 or embodiment 173, wherein the concentration of the binding molecule is at least or at least about 1 µg/mL, 5 µg/mL, 10 µg/mL, 25 µg/mL, 50 µg/mL, 100 µg/mL or 200 µg/mL.
175. The composition of any of embodiments 172-174 that is monodisperse.
176. A kit, comprising the particle of any of embodiments 121-171 or the composition of any of embodiments 154-158 and instructions for use.
177. The kit of embodiment 176, wherein instructions are for selecting or enriching, from a population of cells, cells expressing an antigen receptor comprising an antigen-binding domain specifically recognized by the binding molecule.
178. The kit of embodiment 176, wherein instructions are for expanding, from a population of cells, cells expressing an antigen receptor comprising an antigen-binding domain specifically recognized by the binding molecule.
179. The kit of embodiment 177 or embodiment 178, wherein the percent of cells expressing a recombinant antigen receptor in the population of cells in less than or less than about 75%, 70%, 60%, 50%, 40%, 30%, 20%, 15%, 10% or less.
180. A method for expanding cells, comprising incubating a population of cells with the particle of any of embodiments 121-171 or the composition of any of embodiments 172-175.
181. A method of selecting or enriching cells, comprising contacting a population of cells with the particle of any of embodiments 121-171 or the composition of any of embodiments 172-175.
182. A long-term stimulation method for assessing a cell composition, the method comprising:
   incubating, for a period of time of at least 10 days, an input composition under conditions to stimulate a CAR-dependent activity in cells in the input composition, said input composition comprising T cells expressing a chimeric antigen receptor (CAR) comprising an extracellular antigen-binding domain that specifically binds or recognizes an antigen, thereby producing an output composition; and
   assessing one or more phenotype or activity of one or more cells of the output composition.
183. The method of embodiment 182, wherein the conditions to stimulate a CAR-dependent activity comprises the presence of a binding molecule that specifically binds to the antigen-binding domain of the CAR.
184. The method of embodiment 183, wherein the binding molecule is attached to a support.
185. The method of embodiment 184, wherein the support is a solid support.
186. The method of embodiment 185, wherein the solid support is the surface of a well of a microplate or a bead.
187. The method of any of any of embodiments 183-186, wherein the solid support is a microplate having the binding molecule attached to the microplate, and the incubation is carried out in the microplate.
188. The method of any of embodiments 183-187, wherein the solid support is a bead having attached the binding molecule, and the incubation is carried out in the presence of a plurality of the beads.
189. The method of any of embodiments 183-187, wherein the binding molecule is or comprises a recombinant antigen or a portion thereof recognized by the antigen-binding domain.
190. The method of embodiment 189, wherein the recombinant antigen or portion thereof is BCMA, or is a portion thereof recognized by the antigen-binding domain.
191. The method of any of embodiments 183-190, wherein the binding molecule is or comprises an anti-iditopytic antibody or antigen-binding fragment thereof that specifically binds to the antigen-binding domain.
192. The method of embodiment191, wherein the antigen-binding domain of the antigen receptor is or comprises antibody SJ25C1 or an antigen-binding fragment thereof.
193. The method of embodiment 192, wherein the antigen-binding domain of the antigen receptor is or comprises antibody FMC63 or an antigen-binding fragment thereof.
194. The method of any of embodiments 183-193, wherein the method is carried out in vitro or ex vivo.
195. The method of any of embodiments 182-194, wherein the input composition is incubated in the presence of a media that does not comprise recombinant cytokines.
196. The method of any of embodiments 182-195, wherein the incubation is carried out continuously or is not interrupted for the period of time.
197. The method of any of embodiments 182-196, wherein during the incubation, cells are not replated, media is not changed and binding molecule is not added.
198. The method of any of embodiments182-197, comprising assessing one or more phenotypes of activation, exhaustion or differentiation state of the one or more cells of the output composition.
199. The method of embodiment 198, wherein the phenotype is exhaustion and the assessing comprises measuring the expression, optionally surface expression, of one or more markers selected from CTLA-4, FOXP3, PD-1, TIGIT, LAB-3, 2B4, BTLA, TIM3, VISTA, or CD96.
200. The method of embodiment 198, wherein with the phenotype is activation and the assessing comprising measuring the expression, optionally surface expression, of one or more markers selected from CD25, CD26, CD27, CD28, CD30, CD71, CD154, CD40L, CD127, LAG3, or Ki67.
201. The method of embodiment 198, wherein the phenotype is differentiation state and the assessing comprises measuring one or more markers selected from (i) one or more of CD25, CD45RO, CD56, KLRG1, CD95 and/or (ii) one or of CD45RA, CD27, CD28, CD62L, and CCR7, optionally wherein the one or more markers are markers are positively or inversely associated with naive-like T cells.
202. The method of any of embodiments 182-201, comprising assessing one or more activities of the one or more cells of the output composition.
203. The method of any of embodiments 182-202, wherein the one or more activities comprises a CAR-dependent activity, optionally an antigen-stimulated activity.
204. The method of embodiment 202 or 203, wherein the one or more activities comprises cytolytic activity or cytokine production.
205. The method of any of embodiments 182-204, wherein the period of time is at least or at least about 11 days, 12 days, 13 days, 14 days, or 15 days.
206. The method of any of embodiments 182-205, wherein the period of time is or is about 11 days, 12 days, 13 days, 14 days or 15 days.
207. The method of any of embodiments 182-206, wherein the input composition comprises cells that have been exposed or contacted with a test agent or compound prior to the incubation, optionally wherein the exposing or contacting is carried out during one or more steps of a process for producing the input composition comprising the T cells expressing the CAR.
208. The method of any of embodiments 182-207, wherein the method is carried out on a plurality of input compositions, each of said input compositions of the plurality being produced by a different process.
209. The method of any of embodiments 182-208, further comprising comparing the phenotype or activity of the output composition to the phenotype or activity of a control composition, optionally wherein the control composition is a composition of T cells that have been incubated for the at least 10 days under the same conditions to stimulate the CAR-dependent activity, said composition of T cells having not been produced in the presence of the test agent or compound or having been produced by an alternative process compared to the input composition.
210. The method of any of embodiments 182-209, further comprising identifying an output composition that exhibits reduced exhaustion, reduced activation or decreased differentiation, optionally wherein the decreased differentiation comprises increased expression of one more naive-like T cell markers.

### IX. EXAMPLES

The following examples are included for illustrative purposes only and are not intended to limit the scope of the invention.

### Example 1: Generation of BCMA conjugated beads

B cell maturation antigen (BCMA) was conjugated to beads by covalently coupling a BCMA-Fc fusion polypeptide, containing soluble human BCMA fused at its C-terminus to an Fc region of IgG, to the surface of commercially available tosyl-activated magnetic beads (ThermoFisher, Waltham MA). The beads are superparamagnetic, non-porous, monodisperse, tosylactivated beads that covalently bind primary amino and sulfhydryl groups. Conjugation was performed using beads having a diameter of approximately 2.8 µm (designated M-280) or 4.5 µm (designated M-450).

The BCMA-Fc (SEQ ID NO: 35) contained the extracellular domain of human BCMA (GenBank No. NP_001183.2) and a human IgG1 Fc connected with a linker as follows:
MLQMAGQCSQNEYFDSLLHACIPCQLRCSSNTPPLTCQRYCNASVTNSVKGTNA (extracellular domain of BCMA; SEQ ID NO: 1)
GGGGS (linker; SEQ ID NO: 27)

PKSSDKTHTCPPCPAPEAEGAPSVFLFPPKPKDTLMISRTPEVTCVVVDVSHEDPEVK
FNWYVDGVEVHNAKTKPREEQYNSTYRVVSVLTVLHQDWLNGKEYKCKVSNKAL
PSSIEKTISKAKGQPREPQVYTLPPSRDELTKNQVSLTCLVKGFYPSDIAVEWESNGQP
ENNYKTTPPVLDSDGSFFLYSKLTVDKSRWQQGNVFSCSVMHEALHNHYTQKSLSL
SPGK(Hum IgG1 Fc; SEQ ID NO: 28).

The clone encoding the recombinant human BCMA-Fc fusion construct with the N-terminal CD33 leader sequence (SEQ ID NO: 30) was inserted into an expression vector and expressed in HEK 293 cells. Supernatant from the transiently transfected HEK cells was harvested after 5 days and the BCMA-FC fusion protein was purified by protein A affinity chromatography and size exclusion chromatography (SEC). The resulting BCMA-Fc fusion protein was determined to have a purity of greater than 95% as assessed by gel permeation chromatography. To test binding, the BCMA-Fc fusion protein was incubated with T cells expressing anti-BCMA CARs and T cells expressing CARs that do not bind to BCMA. Results from flow cytometry indicated that the BCMA-Fc fusion protein specifically bound to anti-BCMA CAR expressing T cells.

Various concentrations of the BCMA-Fc fusion protein ranging from 5 µg to 200 µg were added to approximately 1 mL of tosylactivated beads (e.g. containing about 4×10⁹ tosylactivated beads having a diameter of 2.8 µm or about 4×10⁸ tosylactivated beads having a diameter of 4.5 µm). Covalent coupling was performed by overnight incubation at 37°C in phosphate buffered solution (PBS) containing 0.1% human serum albumin (HSA). Beads were washed and resuspended in 1 mL PBS with 0.1% HSA. Prior to and after conjugation, the bead concentration was determined using a Cellometer. The percentage of BCMA-Fc antigen that coupled to the beads was determined SDS page analysis In the examples below, the BCMA-conjugated beads used in various studies are referred to with reference either to the amount of BCMA-Fc antigen added per mL or the antigen concentration (µg/mL) during the conjugation, e.g. 5 µg or 5 µg/mL; 50 µg or 50 µg/mL; 200 µg or 200 µg/mL and so on.

For some of the studies described below, the beads were dual conjugated with BCMA-Fc and an anti-CD28 antibody at a 1:1 ratio using the procedures substantially as described above.

### Example 2: Anti-BCMA antibody-conjugated bead stimulation of CAR T cells

BCMA-conjugated beads (diameter of 4.5 µm), generated as described in Example 1, were incubated with anti-BCMA CAR-expressing T cells. T cells were isolated by immunoaffinity-based enrichment from leukapheresis samples from healthy donors. Isolated T cells were transduced with a viral vector encoding one of two anti-BCMA CARs, each containing a different scFv antigen-binding domain specific for BCMA, a spacer, a CD28 transmembrane region, a 4-1BB costimulatory signaling region and a CD3-zeta derived intracellular signaling domain (designated as anti-BCMA CAR #1 and anti-BCMA CAR #2, differing only in the scFv portion). The viral vector construct further encoded a truncated EGFR (EGFRt), which served as a surrogate marker for CAR expression; the EGFRt-coding region was separated from the CAR sequence by a T2A skip sequence. After transduction, cells were expanded, formulated at a CAR⁺ CD4⁺ T cell to CAR⁺ CD8⁺ T cell ratio of approximately 1:1 and the resulting compositions were frozen by cryopreservation. For the described studies, the transduction efficiency of the anti-BCMA CAR #1 and anti-BCMA CAR#2-expressing T cell compositions was either 45% or 56%, respectively, as determined using an anti-EGFR antibody for detection of the EGFRt surrogate marker.

The T cell composition containing anti-BCMA-CAR+ T cells was thawed and beads from a 100 µg/mL BCMA-conjugated bead composition were added to cells at a 1:1 bead:cell ratio and incubated for five days. As a positive control, cells were cultured with anti-CD3/anti-CD28 beads at a 1:1 bead:cell ratio for five days. T cells that did not express a CAR (mock study group) also were assessed after incubation with the BCMA-conjugated beads.

To assess proliferation, the cell composition containing anti-BCMA CAR-expressing T cells was labeled with the proliferation marker dye CELLTRACE VIOLET (CTV; ThermoFisher Scientific, Waltham MA) in accordance with the manufacturer's protocol prior to incubation with the BCMA-conjugated beads. Exemplary results shown in FIG. 1 demonstrated that incubation with BCMA-conjugated beads resulted in decreased fluorescent intensity of the CTV stain in CD3+ T cells, indicating a dilution of the proliferation dye representative of the degree of proliferation according to the number of cell divisions. For mock cells, very few CD3+ T cells survived after the incubation and no decrease in the CTV intensity was observed for mock cells that did not express an anti-BCMA CAR. These data indicated that incubation with BCMA-conjugated beads specifically increased cell proliferation in T cells expressing anti-BCMA CAR.

To assess if expansion of CAR+ T cells was specifically enriched following the incubation with BCMA-conjugated beads, cells of the T cell composition were stained for surface expression of CD4 or CD8 and for expression of the EGFRt transduction marker using an anti-EGFR antibody, and analyzed by flow cytometry. The extent of enrichment of CAR+ T cells (as detected by expression of the EGFRt surrogate marker) following incubation with BCMA-conjugated beads was compared to enrichment after polyclonal stimulation using the anti-CD3/anti-CD28 beads. The degree of cell proliferation (as determined by dilution in staining intensity of the CTV proliferation dye) and the degree of activation (as determined by surface expression of CD25 or PD-1) also was compared in the T cell compositions incubated with either BCMA-conjugated beads or the control anti-CD3/anti-CD28 beads.

As shown in Table E1, incubation of T cell compositions containing anti-BCMA CAR+ T cells with BCMA-conjugated beads for five days resulted in a greater percentage of CD4⁺ cells and CD8⁺ cells that express EGFRt as compared to incubation of the same T cell compositions with anti-CD3/ anti-CD28 antibody-conjugated beads, indicating BCMA-conjugated beads enrich for anti-BCMA CAR+ T cells.

**Table E1: Percentage of EGFRt+ cells following incubation with BCMA-conjugated beads**

| CAR T cells | Cell Type | Antibody-Conjugated beads | Percent EGFRt⁺ cells |
|---|---|---|---|
| anti-BCMA CAR #1 T cell composition | CD4⁺ | BCMA | 76.6 |
| | | Anti-CD3/CD28 | 63.5 |
| | CD8⁺ | BCMA | 55.3 |
| | | Anti-CD3/CD28 | 34.7 |
| anti-BCMA CAR #2 T cell composition | CD4⁺ | BCMA | 86.3 |
| | | Anti-CD3/CD28 | 68.2 |
| | CD8⁺ | BCMA | 58.3 |
| | | Anti-CD3/CD28 | 39.9 |

As shown in FIG. 2A, as compared to incubation with anti-CD3/anti-CD28 antibody-conjugated beads, incubation of the T cell composition containing anti-BCMA-CAR-expressing T cells with BCMA-conjugated beads for five days resulted in reduced expression of CD25, but a somewhat greater proliferative capacity as evidenced by a greater decrease in intensity of the CTV proliferation dye. FIG. 2B provides a histogram plot in which the x axis shows the intensity of the CTV stain and the y axis shows the number of cells normalized to depict the data in terms of "% of max," according to the FlowJo software (designated "normalized to mode"). The % of max denotes the number of cells in each bin (the numerical ranges for the parameter on the x axis) divided by the number of cells in the bin that contains the largest number of cells. FlowJo uses 256 bins, and each graph was scaled to the percentage of its maximum bin. As shown, a slightly decreased intensity of CTV staining (greater dilution evidencing cell proliferation) was observed in at least a subset of the cells compared to T cells incubated with anti-CD3/anti-CD28-conjugated beads. In addition, as shown in FIG. 2C, expression of PD-1 increased in CD4+ and CD8+ T cells incubated with anti-CD3/anti-CD28 antibody-conjugated beads, whereas incubation of CD4+ and CD8+ T cells with BCMA-conjugated beads did not result in increased expression of PD-1, particularly in CD8+ T cells.

To further assess the extent of enrichment of CAR+ T cells, three separate T cell compositions comprising T cells derived from three different donors engineered to express an anti-BCMA CAR were incubated with BCMA-conjugated beads. Cells were analyzed by flow cytometry following four, seven, or fourteen days of incubation (cells were replated at 7 days). As shown in FIG. 3A, the percentage of anti-BCMA CAR expressing T cells increased over time in all three T cell compositions . CD8+ CAR+ cells from each composition were analyzed by flow cytometry for surface expression of CD25, CD27, or CCR7 following four, seven, or fourteen days of incubation. As shown in FIG. 3B, the levels of CD25 and CCR7 surface expression decreased during the incubation as compared to measurements at day 4. By contrast, surface expression of CD27 in the CD8+ CAR+ cells remained relatively consistent.

### Example 3 : Effects of BCMA-conjugated bead titrations on CAR T cells

A T cell composition containing anti-BCMA CAR+ CD4/CD8 T cells was generated substantially as described in Example 2, except that the resulting formulated composition contained approximately 74% CD8+ cells and 21% CD4+ cells, in which 82% of the total cells were positive for the anti-BCMA CAR (as detected using the soluble BCMA-FC) and 68% were positive for EGFRt as determined using an anti-EGFR antibody. The composition containing anti-BCMA CAR+ T cells was frozen by cryopreservation and thawed before use in the following studies.

Various preparations of BCMA-conjugated beads (diameter of 4.5 µm; including beads from a 50 µg/ml, 25 µg/ml, 10 µg/ml, or 5 µg/ml BCMA-conjugated bead composition), prepared as described in Example 1, were added at a 1:1 bead:cell ratio to the cell composition containing anti-BCMA CAR-expressing T cells, and then incubated for 4 days. The T cells, which had been labeled with the CTV proliferation marker dye, were monitored by flow cytometry for intensity of the CTV signal as an indicator of cell proliferation. Cells also were assessed for surface expression of CD3, CD4, CD8, CD25, PD1 and the EGFRt surrogate marker by flow cytometry.

FIG. 4A depicts bar graphs of the levels of surface CD25 staining in CD4+ and CD8+ cells, and FIG. 4B depicts histogram plots, normalized to mode as described above, for surface expression of PD-1 in CD4+ or CD8+ T cells after incubation with the indicated concentrations of BCMA-conjugated beads. As shown in FIGS. 4A and 4B, incubation with BCMA-conjugated beads resulted in dose-dependent increase in surface expression of CD25 (top panels) and PD-1 (bottom panels) in both CD4+ and CD8+ cells.

As shown in FIG. 5A and FIG. 5B, there was no dose-dependent difference in total T cell counts as assessed by number of CD3+ cells (FIG. 5A), however, there was a slight dose-dependent decrease in CTV mean fluorescent intensity (MFI) in CD4+ cells (FIG. 5B) following incubation with BCMA-conjugated beads. As shown in FIG. 6, incubation with BCMA-conjugated beads resulted in a dose-dependent increase in CD25 surface expression in CD4+ cells.

Together, these data showed that BCMA-conjugated beads with a lower concentration of BCMA demonstrated lower activation marker expression and higher proliferation. These data indicate that certain attributes of a T cell composition containing anti-BCMA CAR+ T cells can be manipulated by titration of the amount of BCMA antigen conjugated to the beads.

### Example 4: Assessment of T cell Markers on anti-BCMA CAR+ T cell Stimulated with BCMA-Conjugated Beads

BCMA-conjugated beads (diameter of 4.5 µm) conjugated with various amounts of BCMA antigen as described in Example 1 were incubated with anti-BCMA CAR+ T cells, and the expression of T cell markers were assessed.

In one experiment, cryofrozen anti-BCMA CAR T cells, produced substantially as described in Example 2 and formulated at a 1:1 ratio of CD4+ and CD8+ T cells, were thawed and seeded at 1.5 x 10⁶ cells per well of a 12 well plate. The cells were incubated for three days in the presence of beads from a 50 µg/ml, 100 µg/ml or 200 µg/ml BCMA-conjugated bead composition. The incubation was carried out at a ratio of 1:1 T cells to beads. Analysis of CD25 surface expression on CD4+ and CD8+ cells by flow cytometry showed a similar induction of CD25 for all concentrations of BCMA-conjugated beads (FIG. 7A).

In another experiment, approximately 1.5 x 10⁶ CAR+ T cells, prepared as described above, were added to wells of a 12-well plate and were incubated with beads from a 200 µg BCMA-conjugated bead composition at a ratio of CAR+ T cell to BCMA-conjugated bead of 1:0.3, 1:1 or 1:3 (approximately 0.5×10⁶, 1.5×10⁶, and 4.5×10⁶ beads per well, respectively). As controls, 5 µg/mL anti-CD3 antibody was coated to wells (sub-optimal concentration for stimulation) or cells were seeded in the absence of any agent (no stimulation control). Each condition was incubated in the presence or absence of 5 µM lenalidomide. The cells were incubated for four days and then analyzed by flow cytometry for surface expression of CD4, CD8, Tim3, PD-1, CD25 and CD69. Incubation of anti-BCMA CAR+ T cells with BCMA-conjugated beads increased surface expression of CD25 (FIG. 7B), CD69 (FIG. 7C) and Tim3 (FIG. 7D) on CD4+ and CD8+ T cells. Cells that were stimulated in the presence of the greatest amount of beads provided at the ratio of CAR+ T cells to beads of 1:3 exhibited the greatest increase in expression of these markers. As shown in FIG. 7E, surface expression of PD-1 on anti-BCMA CAR+ T cells also was greatest when CD4+ cells were incubated at a ratio of 1:3 CAR+ T cells to beads. There also was a slight increase in PD-1 surface expression on CD8+ cells when stimulated with BCMA-conjugated beads at the higher BCMA bead concentration provided at the ratio of 1:3 CAR+ T cells to beads. The results are consistent with a finding that the BCMA concentration present during the incubation can differentially modulate surface marker expression on the CAR+ T cells.

As shown in FIG. 7F, the presence of 5 µM lenalidomide increased the proliferative capacity of T cells (observed by decrease in intensity of CTV dye) following incubation for three days with beads conjugated with 200 µg BCMA antigen at a ratio of 1:1 T cells to beads compared to incubation with the beads in the absence lenalidomide (vehicle control). As shown in FIGS. 7G and 7H, the presence of lenalidomide during the incubation further increased the extent of surface expression of CD25 in CD4+ and CD8+ T cells induced after incubation of anti-BCMA CAR+ T cells with BCMA-conjugated beads (FIG. 7G) or anti-CD3 stimulation (FIG. 7H).

In a further experiment, anti-BCMA CAR-T cell compositions produced substantially as described in Example 1 were plated in 96-well plates at a density of 5×10⁵ cells per well. The tested CAR-T cell compositions contained, on average, approximately 45% anti-BCMA CAR+ cells at plating. Cells from each composition were incubated for 18 hours in the presence of beads from a 5 µg/ml, 50 µg/ml, or 200 µg/ml BCMA-conjugated bead composition at a ratio of 1:1 T cells to beads. As a control, cells were incubated with anti-CD3/anti-CD28 antibody-conjugated beads (positive control) or no added agent (negative control). The incubations were carried out in the absence of lenalidomide or in the presence of 0.5 µM or 5 µM lenalidomide. Following the incubation, the cells were treated with reagents that allowed for extracellular and intracellular antibody staining by flow cytometry for the transcription factors Blimp1, EOMES, GATA-3, ikaros, helios, and Tbet and markers CD25, CD31, and PD-1.

Levels of markers after the incubation of a CAR+ T cell composition from one exemplary donor are shown for BLIMP-1 (FIG. 8A), CD25 (FIG. 8B), CD31 (FIG. 8C), PD-1 (FIG. 8D), Tbet (FIG. 8E), and EOMES (FIG. 8F), GATA-3 (FIG. 8G) Helios (FIG. 8H), and Ikaros (FIG. 8I). As shown, expression of a number of the assessed T effector cell-associated transcription factors and activation markers were increased following stimulation with the BCMA-conjugated beads. For many of the assessed markers, the extent of increased expression was similar to the expression induced by stimulation with anti-CD3/anti-CD28 beads. In some cases, the degree of stimulation with BCMA-conjugated beads was greatest in the presence of 5 µg beads. As shown in FIG. 8I, the expression level of Ikaros was decreased in the presence of lenalidomide in all conditions. Similar results were observed from a CAR+ T cell composition generated from a second donor, except that no change in Helios expression was observed from cells from this donor when stimulated under the tested conditions.

### Example 5: Assessment of Activity of anti-BCMA CAR T cells stimulated with BCMA-conjugated beads

The activity of anti-BCMA CAR T cells that were generated from a process that included expansion in the presence of BCMA-conjugated beads was assessed.

### A. Effector Responses

Cryofrozen anti-BCMA CAR T cells, produced substantially as described in Example 2 and formulated at a 1:1 ratio of CD4+ and CD8+ T cells, were thawed. Unless otherwise indicated, beads (diameter about 4.5 µm) from a 5 µg/ml or 50 µg/ml BCMA-conjugated bead composition, generated as described in Example 1, were added to the wells at a ratio of T cells to beads of 1:2 in the presence or absence of 5 µM lenalidomide. Cells were incubated up to 14 days and analyzed at various time points for cytokine secretion, cell expansion by flow cytometry for the EGFRt surrogate marker, and for cytolytic activity.

### a. Cytokine Expression

### (i) Presence of cytokines in supernatant

Twenty four hours after addition of BCMA-conjugated beads, the presence of TNF-α, IFNγ, and IL-2 in culture supernatants was assessed. As shown in FIGS. 9A-9C, incubation with BCMA-conjugated beads induced the secretion of IFNγ (FIG. 9A), IL-2 (FIG. 9B), and TNF-α (FIG. 9C) into culture supernatants. The degree of cytokine production was greater when the cells were incubated with beads from the 50 µg/mL BCMA-conjugated bead composition compared to the 5 µg/mL BCMA-conjugated bead composition, demonstrating that CAR stimulation via BCMA beads was dose-dependent. As shown, lenalidomide increased BCMA-induced CAR+ T cell cytokine production following stimulation with the BCMA-conjugated beads.

In a further exemplary study, two different anti-BCMA CAR T cell compositions were generated from different donors, each containing T cells expressing the same anti-BCMA CAR. The cells were thawed and incubated with beads (diameter about 4.5 µm) from a 5 µg/mL or 200 µg/mL BCMA-conjugated bead composition generated as described in Example 1. The incubation was carried out at a ratio of T cells to beads of 1:1 in the presence or absence of 1 µM or 5 µM lenalidomide. Twenty four hours after addition of BCMA-conjugated beads, IL-2 production by the anti-BCMA CAR+ T cells was assessed in culture supernatants. As shown in FIG. 9D, higher production of IL-2 was observed in the presence of high antigen stimulation (200 µg/mL BCMA-conjugated beads) compared to lower antigen stimulation (5 µg/mL BCMA-conjugated beads). Lenalidomide, at either 1 µM or 5 µM, increased cytokine production in the presence of both the high and low antigen stimulation.

### (ii) Intracellular cytokine levels

Anti-BCMA CAR+ T cells were incubated in the presence of 1 µM lenalidomide or a vehicle and 50 µg/mL BCMA-Fc conjugated beads for 2 hours, and cells were assessed by flow cytometry for phosphorylated STAT 5. To assess IFNγ and TNFα cytokine levels, anti-BCMA CAR+ T cells were incubated in the presence of 0.1 µM or 1 µM lenalidomide or a vehicle and 5 µg/mL, 50 µg/mL or 200 µg/mL BCMA-Fc conjugated beads for 24 hours. The cells were gated on transduced, live CD3+ cells, and assessed by flow cytometry for intracellular cytokine accumulation of IFNγ and TNFα in CD4+ and CD8+ cells.

As shown in FIG. 9E, a 2-hour stimulation with antigen increased the percent of cells positive for phosphor-STAT5 compared to the no stimulation control (shown with the dotted line). Results for intracellular cytokine levels of IFNγ and TNFα from anti-BCMA CAR T cells generated from a representative normal CAR-T cell donor are shown in FIG. 9F. In this study, anti-BCMA CAR-T cell cytokine production was increased by lenalidomide across a wide range of antigen levels and concentrations.

### b. Cell Proliferation

Total cell count, monitored at day 4 (FIG. 9G) and day 7 (FIG. 9H), was increased following stimulation of anti-BCMA CAR+ T cells with beads from a 50 µg/mL BCMA-conjugated bead composition, but not 5 µg/mL BCMA-conjugated bead composition, compared to the cells present at the time of initiation of the incubation (dashed line). A small increase in proliferation was observed in cells incubated with 50ug beads in the presence of lenalidomide at day 7.

To further assess proliferation, the cells containing anti-BCMA CAR-expressing T cells were labeled with the proliferation marker dye CELLTRACE VIOLET (CTV; ThermoFisher Scientific, Waltham MA) in accordance with the manufacturer's protocol prior to incubation with the BCMA-conjugated beads. Proliferation was assessed by dye dilution using flow cytometry on cells that were stimulated with beads from the 50 µg/mL BCMA-conjugated bead composition. Compared to proliferation in the absence of lenalidomide, there was a slight delay in proliferation as assessed by CTV dilution in the presence of lenalidomide at day 4 but not day 7 (FIG. 9I).

### c. Expansion

Four days and seven days after addition of BCMA-conjugated beads, the incubated cells were stained with CD4 or CD8 and with an anti-EGFR antibody to determine the percentage of cells positive for EGFRt as a surrogate for CAR+ T cells. At the time of plating, 26% of the CD4+ cells expressed anti-BCMA CAR and 39% of the CD8+ cells expressed anti-BCMA CAR as determined by staining with BCMA-Fc. The percent of EGFRt+CD4+ T cells increased from about 26% at the initiation of the incubation to greater than 40% by day 4 (FIG. 9J) and greater than 60% by day 7 (FIG. 9K) when the cells were incubated in the presence of beads from a 50 µg/mL BCMA-conjugated bead composition. As shown in FIG. 9K, the percent of EGFRt+CD8+ T cells increased by day 7 from about 38% at the initiation of the incubation to greater than 60% when the cells were incubated in the presence of beads from the 50 µg/mL BCMA-conjugated bead composition. The extent of cell expansion was greatest when cells were incubated in the presence of beads from a 50 µg/mL BCMA-conjugated bead composition compared to beads from the 5 µg/mL BCMA-conjugated bead composition. The presence of lenalidomide did not substantially impact the extent of CAR+ T cell expansion in this study.

### d. Cytolytic Activity

Cytolytic activity of CAR+ T cells after incubation with BCMA-conjugated beads was assessed by incubation with the BCMA-expressing target cell line RPMI-8226, which is a BCMA+ multiple myeloma cell line. After seven days of incubation of anti-BCMA CAR+ T cells with BCMA-conjugated beads (5 µg/ml or 50 µg/ml) in the presence or absence of lenalidomide, the beads were removed from the cultures and the cells were plated with the RPMI-8226 target cells at a ratio of effector cells to target cells of 3:1 or 1:1 in the further presence or absence of 5µM lenalidomide. To perform the cytolytic assay, the target RPMI-8226 cells were labeled with NucLight Red (NLR) to permit tracking of target cells by microscopy. Cytolytic activity was assessed by measuring the loss of viable target cells over a period of four days, as determined by red fluorescent signal (using the INCUCYTE^{®} Live Cell Analysis System, Essen Bioscience). The number of viable cells was normalized to cells at day 0 prior to incubation with the RPMI-8226 target cells.

Exemplary results at the 1:1 effector to target cell ratio are shown in FIG. 9L. As shown, the anti-BCMA CAR+ T cells demonstrated effective killing in the assay. Anti-BCMA CAR+ T cells that were stimulated with beads from the 5 µg/ml BCMA-conjugated bead composition were slightly less efficient at cell killing than anti-BCMA CAR+ T cells that were stimulated with beads from the 5 µg/ml BCMA-conjugated bead composition. For all conditions, preincubation with lenalidomide during the seven day incubation prior to the killing assay increased cytolytic activity of the CAR+ T cells. The presence of lenalidomide during the cell killing assay did not substantially affect killing activity. No cell killing was observed when RPMI 8226 cells were cultured alone or in the presence of lenalidomide, demonstrating that lenalidomide did not directly influence target cell viability in this assay.

In a further exemplary study, anti-BCMA CAR+ T cells were untreated, or were incubated for 24 hours or for seven days with beads (diameter about 4.5 µm) from a 50 µg/mL BCMA-conjugated bead composition generated as described in Example 1 at a ratio of beads to cells of 1:1. The anti-BCMA CAR T+ cells were removed from the cultures and incubated with RPMI-8226 target cells at a 0.3:1 or 1:1 effector cell to target cell ratio. To assess cytokine response to the target cells after incubation with BCMA-conjugated beads, IFN gamma levels were measured in the supernatant. As shown in FIG. 9M, anti-BCMA CAR+ T cells that were incubated for 24 hours with the BCMA-conjugated beads produced greater IFN-gamma compared to the other tested cells when cultured with RPMI-8226 target cells at either ratio. As shown in FIG. 9N for the 1:1 effector to target cell ratio, anti-BCMA CAR T cells demonstrated effective killing after incubation with BCMA-conjugated beads for 24 hours or 7 days demonstrating that the cells retained function after stimulation with the beads.

### B. Serial Restimulation

Anti-BCMA CAR T cell compositions were generated from three different donors, each containing T cells expressing the same anti-BCMA CAR, thawed, and were incubated for seven days with beads (diameter about 4.5 µm) from a 50 µg/mL BCMA-conjugated bead composition generated as described in Example 1 at a 1:1 ratio of beads to cells. The incubation was carried out in the presence of 5µM lenalidomide or in the absence of lenalidomide (vehicle control). Cells were harvested after 7 days and replated for three further rounds up to 28 days, each round involving resetting to initial seeding density and incubating for an additional 7 days in the presence of the same concentration of lenalidomide.

At each reset after the pretreatment, cytolytic activity was assessed by incubation with the BCMA-expressing target cell line RPMI-8226 (labeled with NucLight Red (NLR)) at an effector to target cell (E:T) ratio of 1:1 in the further presence of or absence of lenalidomide. Cytolytic activity was assessed by measuring the loss of viable target cells over a period of up to 80-150 hours, as determined by red fluorescent signal (using the IncuCyte^{®} Live Cell Analysis System, Essen Bioscience). Cells from each condition were plated in triplicate. The % cell killing compared to vehicle only control (set at 100%) was determined.

FIG. 10A show results for cytolytic activity of anti-BCMA CAR+ T cells from an exemplary donor after pretreatment for 7 days, 14 days or 21 days. As shown, anti-BCMA CAR+ T cells that were preincubated with lenalidomide for 7 days or 14 days exhibited greater cytolytic activity compared to cells that were not preincubated in the presence of lenalidomide. In this donor, an overall decrease in killing efficacy was observed by anti-BCMA CAR+ T cells that were preincubated with lenalidomide for 14 or 21 days compared to day 7. Similar effects on cytolytic activity of anti-BCMA CAR+ T cells after pretreatment with lenalidomide for 7 or 14 days were observed in the donor; cytolytic activity after 21 days lenalidomide pretreatment was not assessed in this donor. As shown in FIG. 10B, increased killing efficacy of anti-BCMA CAR+ T cells was observed in this donor in cells that were pre-incubated with lenalidomide at all time points.

### Example 6: Transduction and Expansion of Anti-BCMA CAR+ T Cells in the presence of BCMA-Conjugated beads

Human leukapheresis samples enriched in mononuclear cells were obtained from a whole blood sample from a subject using a leukapheresis collection system. On the same day as leukapheresis, cells of the leukapheresis sample were washed and resuspended in a buffer for use in affinity-based selection, the buffer containing Phosphate Buffered Saline (PBS), EDTA and human serum albumin. For immunoaffinity-based selection of T cells, the washed cells in the selection buffer were incubated for 30 minutes at room temperature with magnetic beads coupled to monoclonal antibodies specific for CD4 and CD8, and were enriched by positive selection using a magnetic separation column. The enriched CD4+ and CD8+ cells, at a ratio of 1:1, were resuspended in cryopreservation media and cells were cryopreserved in a controlled rate freezer and stored in liquid nitrogen until further use. Cryopreserved cells from 4 different donors were used.

Cryopreserved CD4+ and CD8+ T cells were thawed, washed and then contacted with lentiviral vector particles without prior activation of the cells with a T cell activation agent, e.g. without incubating the cells with anti-CD3/anti-CD28. For transduction, approximately 1 x 10⁶ of the thawed T cells were added to individual wells of a 96-well plate. Lentiviral vector particles, that had been premixed with a polycation transduction adjuvant (in this case, 100 µg/ml protamine sulfate) were added to wells. The lentiviral vector particles contained a nucleic acid encoding a transgene encoding an anti-BCMA CAR and a truncated EGFR (EGFRt) sequence for use as a transduction marker, separated from the CAR sequence by a self-cleaving T2A sequence. The final volume per well was adjusted to 100 µl. Five different composition containing cells engineered to express anti-BCMA CARs were prepared using the procedures described above.

Beads conjugated with BMCA-Fc and anti-CD28 (designated BCMA/anti-CD28-conjugated beads) were prepared substantially as described in Example 1. Immediately after the transduction, approximately 50 µL of the cells generated from each donor were added to 500 µL of media containing BCMA/anti-CD28-conjugated beads or anti-CD3/ anti-CD28 conjugated beads at a ratio of 1:1 beads per cell and incubated for 12 days.

Cell were counted every other day, and stained for expression of EGFRt (a surrogate marker for CAR expression) using an anti-EGFR antibody on days 3, 7, 10, and 12 post-transfection. The data showed that T cell compositions incubated for up to 12 days in the presence of the anti-CD3/anti-CD28 conjugated beads expanded in the culture with between approximately 95-fold to 140-fold expansion occurring by day 12 of culture. Expansion of control mock T cells that were not transduced, but incubated with the anti-CD3/anti-CD28 conjugated beads, also exhibited about 65-fold expansion at day 12 compared to the start of the incubation. The fold-expansion of T cell compositions that were incubated with BCMA/anti-CD28 beads immediately after transduction was about 1.0-fold to 7.0-fold by day 12.

As shown in FIG. 11, the T cells compositions that were incubated in the presence of anti-CD3/anti-CD28 exhibited high expression of the EGFRt transduction marker, even without activation of cells prior to transduction with the viral particles. This result is consistent with immediate stimulation being associated with surface CAR expression. T cells stimulated with BCMA/anti-CD28 beads exhibited longer kinetics to achieve the same percentage of cells positive for EGFRt, although by day 12 between about 60% and 90% of the cells were positive for EGFRt. The results indicate that, despite the slower growth of the cells following incubation with BCMA/anti-CD28 beads as compared to anti-CD3/anti-CD28 beads, CAR+ T cells could be enriched over the 12 days of culture, indicating that the BCMA/anti-CD28 beads expanded the CAR+ T cells.

T cells incubated under the conditions described above, without activation of the cells prior to transduction with lentiviral vector particles, also were stained at day 12 for surface expression of CD4 and CD8 and analyzed by flow cytometry. As a control, a T cell composition generated from a donor cells, also at a ratio of 1:1 of CD4+ and CD8+ cells, were activated prior to transduction by stimulation with anti-CD3/anti-CD28 beads for 24 hours before contacting the activated cells with lentiviral vector particles expressing an anti-BCMA CAR as described above; in this condition the cells were further incubated for 12 days but in the absence of any conjugated beads.

Table E2 summarizes results from the same donor cells incubated under the above conditions. As shown, incubation of cells with anti-CD3/anti-CD28 conjugated beads or BCMA/anti-CD28 beads, without activation of cells prior to transduction, altered the ratio of CD4/CD8 T cell compared to cells that were first activated 24 hour in the presence of anti-CD3/anti-CD28 conjugated beads prior to transduction. Furthermore, these results showed that incubation with the BCMA/ anti-CD28 antibody conjugated beads or the anti-CD3/ anti-CD28 antibody conjugated beads, without prior activation of the T cells, resulted in similar CAR expression levels and CD4/CD8 cell phenotype. As shown in Table E3, a similar reversal of the ratio of CD4/CD8 T cells was observed in T cells derived from 4 different donors when incubated with anti-CD3/anti-CD28 beads, without activation of cells prior to transduction, compared to cells that were first activated for 24 hours in the presence of anti-CD3/anti-CD28 conjugated beads before transduction.

**Table E2: Percentage of CD4+ and CD8+ cells in T cell cultures that were treated with antigen and/or antibody conjugated beads.**

| **Bead Treatment** | **% CD4+ cells** | **% CD8+ cells** |
|---|---|---|
| BCMA/anti-CD28 | 92.2 | 7.57 |
| Anti-CD3/Anti-CD28 | 76.7 | 23.0 |
| Anti-CD3/Anti-CD28 (24 hr) | 19.9 | 78.5 |

**Table E3: Percentage of CD4+ and CD8+ cells in T cell cultures that were treated with antibody conjugated beads.**

| **Donor** | **Bead Treatment** | **% CD4+ cells** | **% CD8+ cells** |
|---|---|---|---|
| 1 | Anti-CD3/Anti-CD28 | 65.6 | 29.7 |
| 2 | Anti-CD3/Anti-CD28 | 68.0 | 27.8 |
| 3 | Anti-CD3/Anti-CD28 | 61.8 | 32.6 |
| 4 | Anti-CD3/Anti-CD28 | 76.8 | 21.9 |
| 4 | Anti-CD3/Anti-CD28 (24 hr) | 19.9 | 76.8 |

### Example 7: Generation of ROR1 Conjugated Beads

Receptor tyrosine kinase like orphan receptor 1 (ROR1) was conjugated to beads by covalently coupling a ROR1-Fc fusion polypeptide, containing the extracellular domain of human ROR1 fused at its C-terminus to an Fc region of IgG, to the surface of commercially available tosyl-activated magnetic beads (ThermoFisher, Waltham MA). The beads were superparamagnetic, non-porous, monodisperse, tosylactivated beads that covalently bind primary amino and sulfhydryl groups. Conjugation was performed using beads having a diameter of approximately 2.8 µm (designated M-280) or 4.5 µm (designated M-450).

The ROR1-Fc contained a fragment of human ROR1 and a human IgG1 Fc connected with a linker as follows:

QETELSVSAELVPTSSWNISSELNKDSYLTLDEPMNNITTSLGQTAELHCKVS
GNPPPTIRWFKNDAPVVQEPRRLSFRSTIYGSRLRIRNLDTTDTGYFQCVATNGKEVVSS

TGVLFVKFGPPPTASPGYSDEYEEDGFCQPYRGIACARFIGNRTVYMESLHMQGEIENQI
TAAFTMIGTSSHLSDKCSQFAIPSLCHYAFPYCDETSSVPKPRDLCRDECEILENVLCQTE
YIFARSNPMILMRLKLPNCEDLPQPESPEAANCIRIGIPMADPINKNHKCYNSTGVDYRG
TVSVTKSGRQCQPWNSQYPHTHTFTALRFPELNGGHSYCRNPGNQKEAPWCFTLDENF
KSDLCDIPACDSKDSKEKNKMEILYGGGGSPKSSDKTHTCPPCPAPEAEGAPSVFLFPPK
PKDTLMISRTPEVTCVVVDVSHEDPEVKFNWYVDGVEVHNAKTKPREEQYNSTYRVV
SVLTVLHQDWLNGKEYKCKVSNKALPSSIEKTISKAKGQPREPQVYTLPPSRDELTKNQ
VSLTCLVKGFYPSDIAVEWESNGQPENNYKTTPPVLDSDGSFFLYSKLTVDKSRWQQG
NVFSCSVMHEALHNHYTQKSLSLSPGK (SEQ ID NO: 33).

Various concentrations of the ROR1-Fc fusion protein were added to approximately 1 mL of tosylactivated beads (e.g. 4×10⁹ tosylactivated beads having a diameter of 2.8 µm) Covalent coupling was performed by overnight incubation at 37°C in phosphate buffered solution (PBS) containing 0.1% human serum albumin (HSA). Beads were washed and resuspended in 1 mL PBS with 0.1% HSA. After conjugation, the bead concentration was determined using a Cellometer.

Anti-ROR1 CAR-expressing T cells are stimulated by incubation with ROR1 Fc-conjugated beads.

### Example 8: Generation of CD22 Conjugated Beads

CD22 was conjugated to beads by covalently coupling a CD22-Fc fusion polypeptide, containing the extracellular domain of human CD22 fused at its C-terminus to an Fc region of IgG, to the surface of commercially available tosyl-activated magnetic beads (ThermoFisher, Waltham MA). The beads were superparamagnetic, non-porous, monodisperse, tosylactivated beads that covalently bind primary amino and sulfhydryl groups. Conjugation was performed using beads having a diameter of approximately 2.8 µm (designated M-280) or 4.5 µm (designated M-450).

The CD22-Fc contained the extracellular domain of human CD22 and a human IgG1 Fc connected with a linker as follows:

DSSKWVFEHPETLYAWEGACVWIPCTYRALDGDLESFILFHNPEYNKNTSKFDGTRLYE
STKDGKVPSEQKRVQFLGDKNKNCTLSIHPVHLNDSGQLGLRMESKTEKWMERIHLNV
SERPFPPHIQLPPEIQESQEVTLTCLLNFSCYGYPIQLQWLLEGVPMRQAAVTSTSLTIKS
VFTRSELKFSPQWSHHGKIVTCQLQDADGKFLSNDTVQLNVKHTPKLEIKVTPSDAIVR

EGDSVTMTCEVSSSNPEYTTVSWLKDGTSLKKQNTFTLNLREVTKDQSGKYCCQVSND
VGPGRSEEVFLQVQYAPEPSTVQILHSPAVEGSQVEFLCMSLANPLPTNYTWYHNGKE
MQGRTEEKVHIPKILPWHAGTYSCVAENILGTGQRGPGAELDVQYPPKKVTTVIQNPMP
IREGDTVTLSCNYNSSNPSVTRYEWKPHGAWEEPSLGVLKIQNVGWDNTTIACAACNS
WCSWASPVALNVQYAPRDVRVRKIKPLSEIHSGNSVSLQCDFSSSHPKEVQFFWEKNG
RLLGKESQLNFDSISPEDAGSYSCWVNNSIGQTASKAWTLEVLYAPRRLRVSMSPGDQV
MEGKSATLTCESDANPPVSHYTWFDWNNQSLPYHSQKLRLEPVKVQHSGAYWCQGTN
SVGKGRSPLSTLTVYYSPETIGRRGGGGSPKSSDKTHTCPPCPAPEAEGAPSVFLFPPKPK
DTLMISRTPEVTCVVVDVSHEDPEVKFNWYVDGVEVHNAKTKPREEQYNSTYRVVSV
LTVLHQDWLNGKEYKCKVSNKALPSSIEKTISKAKGQPREPQVYTLPPSRDELTKNQVS
LTCLVKGFYPSDIAVEWESNGQPENNYKTTPPVLDSDGSFFLYSKLTVDKSRWQQGNV
FSCSVMHEALHNHYTQKSLSLSPGK (SEQ ID NO: 34).

Various concentrations of the CD22-Fc fusion protein were added to approximately 1 mL of tosylactivated beads (e.g. 4×10⁹ tosylactivated beads having a diameter of 2.8 µm) Covalent coupling was performed by overnight incubation at 37°C in phosphate buffered solution (PBS) Beads were washed and resuspended in 1 mL PBS with 0.1% HSA. After conjugation, the bead concentration was determined using a Cellometer.

Anti-CD22 CAR-expressing T cells are stimulated by incubation with CD22- Fc-conjugated beads.

### Example 9: Conjugation of Anti-Idiotype Antibody to Beads

One of two different anti-CD19 antibody anti-idiotype (ID) antibody, FMC63-derived scFv-specific anti-ID antibody, (sequences set forth in Table E4) was covalently coupled to the surface of commercially available tosyl-activated magnetic beads (ThermoFisher, Waltham MA) that are superparamagnetic, non-porous, monodisperse, tosylactivated beads. The beads covalently bind primary amino and sulfhydryl groups. Conjugation was performed using beads having a diameter of approximately 2.8 µm (designated M-280) or 4.5 µm (designated M-450).

**Table E4. Anti-ID B-1 and B-2 sequences**

| | Light Chain | | | Heavy Chain | | |
|---|---|---|---|---|---|---|
| | Full (SEQ ID NO) | Variable (SEQ ID NO) | Constant (SEQ ID NO) | Full (SEQ ID NO) | Variable (SEQ ID NO) | Constant (SEQ ID NO) |
| Anti-ID B-1 Amino acid | 125 | 98 | 124 | 122 | 73 | 121 |
| Anti-ID B-2 Amino acid | 131 | 99 | 124 | 128 | 74 | 127 |

200 µg anti-ID antibody was added to approximately 1 mL of the tocyl-activated beads (e.g. approximately 4×10⁹ tocyl-activated beads having a diameter of 2.8 µm or about 4×10⁸ tosylactivated beads having a diameter of 4.5 µm) and covalent coupling was performed by overnight incubation at 37°C in phosphate buffered solution (PBS) containing 0.1% human serum albumin (HSA). Beads were washed and resuspended in 1 mL PBS with 0.1% HSA. After conjugation, the bead concentration was determined using a Cellometer.

To assess stability of the anti-ID conjugated beads, the beads were pelleted, the supernatant was removed and loaded on a 4-12% Bis-Tris SDS-PAGE gel and the gel was stained with Coomassie blue. As a control for total protein conjugated on the beads, the pelleted beads were boiled in 4X (lithium dodecyl sulfate) LDS sample buffer at about 70° C for 20 minutes and approximately 12.5 µL or 25 µL boiled supernatant also was run on SDS-PAGE gel and assessed by Coomassie blue. Approximately 2.5 µg or 5.0 µg anti-ID antibody that had not been conjugated to the beads (positive control) or 5 µL 0.1% HSA (negative control) also were assessed by SDS-PAGE and Coomassie blue staining. No anti-ID antibody was detected in supernatant from conjugated samples that had not been boiled indicating that the conjugation was stable, whereas anti-ID antibody was detected in supernatant from conjugated samples that were boiled.

### Example 10 : Assessment of stimulation of T cells cultured with anti-idiotype antibody-conjugated beads

The FMC63-derived scFv-specific anti-ID B-1- conjugated beads were incubated with T cells. CD3-purified T cells were isolated by immunoaffinity-based enrichment from leukapheresis samples from healthy donors. Isolated cells were transduced with a viral vector encoding an anti-CD19 CAR having an scFv derived from FMC63. The viral vector construct further encoded a truncated EGFR (EGFRt), which served as a surrogate marker for CAR expression; the EGFRt-coding region was separated from the CAR sequence by a T2A skip sequence. After transductions, cells were expanded in culture and frozen by cryopreservation.

For T cells stimulation studies, thawed CD4+ or CD8+ CAR-expressing cells were separately seeded at approximately 50,000 total cells per well. In some cases, the culture media was additionally supplemented with cytokines as follows: for CD4+ cells, approximately 1200 IU/mL recombinant IL-7, 20 IU/mL recombinant IL-15 and 100 IU/mL recombinant IL-2; for CD8+ cells, approximately 200 IU/mL recombinant IL-2 and 20 IU/mL recombinant IL-15. Anti-ID B-1 conjugated beads were added to cells at a 1:1 or 1:5 cell:bead ratio and incubated up to 14 days with a 50% media exchange every 2-3 days. As a positive control, cells were cultured with anti-CD3/anti-CD28 magnetic beads at a 3:1 cell:bead ratio in the presence or absence of the indicated cytokines.

At various times of culture, CD4+ or CD8+ transduced cells (as detected by anti-EGFR for expression of the surrogate marker) were assessed for expansion, PD-1 expression and viability.

As shown in FIG.12A and 12B, expansion of CD4+ and CD8+ T cells, respectively, was observed when cells were cultured with anti-ID conjugated beads at either the 1:1 or 1:5 cell:bead ratio, particularly in the presence of cytokines. The extent of expansion was greater than when cells were cultured with the control anti-CD3/anti-CD28 magnetic beads.

Surface expression of PD-1 on the CD4+ cell subset was assessed by flow cytometry at days 3, 7, 10 and 14 of culture. As shown in FIG. 13, PD-1 expression was high on cells cultured with anti-CD3/anti-CD28 magnetic beads, however, the levels of PD-1 was substantially lower or undetectable in cells that were cultured with anti-ID conjugated beads.

As shown in FIG. 14, the percent viability of CD4+ and CD8+ T cells cultured with either the 1:1 or 1:5 ratio of anti-ID conjugated beads or the control anti-CD3/anti-CD28 conjugated beads remained consistently high during the period of culture when the cells were additionally cultured in the presence of cytokines. Under all conditions, however, viability of cells decreased in the absence of added cytokines, with the greatest loss in cell viability occurring at later days of cell culture.

### Example 11 : Assessment of cytokine production of T cells cultured with anti-idiotype antibody-conjugated beads

T cells engineered with an anti-CD19 CAR having an scFv derived from FMC63 were generated substantially as described in Example 10. Thawed CD8+ T cells were cultured with anti-ID B-1 conjugated beads for 4 hours in the presence of Golgi inhibitor. Intracellular cytokine levels of TNFα, IFNγ, and IL-2 was determined by flow cytometry in either the CAR⁺ T cell subset (as determined by positive surface staining with anti-EGFR for the surrogate marker) or the CAR⁻ T cell subset (as determined by negative surface staining with anti-EGFR). As a comparison, CAR+ T cells (EGFRt+) also were cultured with CD19-expressing target cells (K562 cells transduced to express CD19, K562-CD19) at an effector:T cell ratio of 1:2.

As shown in FIG. 15A, intracellular cytokine levels of TNFα, IFNγ and IL2 cytokines were induced in CAR+ T cells (EGFRt⁺), but not in CAR- T cells (EGFRt⁻), when the cells were cultured in the presence of the anti-ID B-1 conjugated beads. In this study, the extent of stimulation observed in the presence of anti-ID conjugated beads was similar to stimulation of CAR+ T cells using antigen-expressing K562-CD19 cells, which is an alternative CAR-specific stimulation reagent (FIG. 15B). These results demonstrated that anti-ID conjugated to beads are agonistic and specifically stimulate T cells expressing a CAR having an antigen-binding domain recognized by the anti-ID antibody. Further, the bead reagent provides for a better CAR-specific stimulation reagent compared to cell lines, which require cell culture and are prone to lot to lot variability.

### Example 12 : Assessment of Expansion after Serial Restimulation

The ability of cells to expand *ex vivo* following repeated stimulations may be a potential surrogate for capacity of CAR+ T cells to persist (e.g. following initial activation) and/or is indicative of function in vivo (Zhao et al. (2015) Cancer Cell, 28:415-28). CAR+ T cells were generated as described above and thawed CD4+ or CD8+ CAR-expressing T cells were plated separately at 50,000 CAR+ cells/well. Anti-ID B-1 conjugated beads were added to cells at a 1:1 or 1:5 cell:bead ratio in the presence or absence of cytokines as described in Example 10. As a control, anti-CD3/anti-CD28 magnetic beads were added to cells at a 3:1 cell:bead ratio in the presence or absence of the cytokines. Cells were harvested every 3-4 days and counted, and restimulated with new target cells using the same culture conditions after resetting cell number to initial seeding density for each round. A total of 4 rounds of stimulation during a 14 day culture period were carried out. For each round of stimulation, the number of doublings was determined.

As shown in FIG. 16, continued cell expansion of CAR-expressing (EGFRt+) CD4+ cells was observed after restimulation with anti-ID conjugated beads, although the degree of expansion was greater when the cells were cultured in the presence of cytokines. Also, the extent of expansion was greater than when cells were cultured with anti-CD3/anti-CD28 beads. For CD8+ T cells, similar expansion kinetics was observed when cells were cultured in the presence of either anti-ID conjugated beads or anti-CD3/anti-CD28 beads, although expansion was somewhat greater when cells were cultured with anti-ID conjugated beads, particularly in the absence of added recombinant cytokines.

### Example 13 : Further Analysis of CAR-Specific Cell Expansion Using Anti-idiotype antibody-Conjugated Beads

Similar studies as those described in Examples 11 and 12 were performed, except using CAR-expressing T cells generated from two different patient donors. CD3-purified T cells were isolated from peripheral blood mononuclear cells (PBMCs) of two donor patients, transduced with a viral vector encoding an anti-CD19 CAR having an scFv derived from FMC63, expanded in culture, frozen, and thawed.

Thawed CD4+, CD8+ or co-cultures of CD4/CD8 (1:1 ratio) were seeded in wells of a 6-well plate at approximately 5 x 10⁶ total cells/well in culture media that was additionally supplemented with cytokines as follows: for CD4+ cells or CD4+/CD8+ co-cultures media was supplemented with approximately 1200 IU/mL recombinant IL-7, 20 IU/mL recombinant IL-15 and 100 IU/mL recombinant IL-2; for CD8+ cells media was supplemented with 200 IU/mL recombinant IL-2 and 20 IU/mL recombinant IL-15. Anti-ID B-1 conjugated beads were added to cells at a 1:1 cell:bead ratio and incubated up to 9 days with a 50% media exchange every 2-3 days.

At various times of culture, the number of CD4+ or CD8+ transduced cells (as detected by anti-EGFR for expression of the EGFRt surrogate marker) present in the culture for each condition was assessed and fold expansion or frequency of the CAR-expressing cells as a percent of total cells was determined. Expression of PD-1 and CD25 and cell viability also was determined.

As shown in FIG. 17A, over 60-fold expansion of CAR-expressing (EGFRt+) CD4+ T cells was observed when CD4+ cells were cultured alone with anti-ID conjugated beads. For CD8+ T cells, substantially higher expansion of CAR-expressing (EGFRt+) CD8+ T cells occurred in the presence of anti-ID conjugated beads when the CD8+ T cells were co-cultured with CD4+ cells. As shown in FIG. 17B, the frequency of CAR-expressing (EGFRt+) CD4+ or CD8+ increased during the 9 days of culture in the presence of the anti-ID conjugated beads with >90% of transduced cells (EGFRt+) present in the culture at day 9. Viability of CD4+ and CD8+ cells also remained close to 100% during the culture, with somewhat greater viability of CD8+ T cells observed when co-cultured with CD4+ T cells (FIG. 17C). Similar results were observed for both donors. These results are consistent with an enrichment of CAR+ T cells, without prior CAR selection through the use of CAR specific anti-idiotype antibody-conjugated beads.

Surface expression of PD-1 and CD25 on transduced (EGFR+) CD4+ or CD8+ cells was assessed by flow cytometry at days 5, 7 and 9 of culture with the anti-ID conjugated beads. As shown in FIG. 18A, PD-1 expression on both CD4+ and CD8+ T cells decreased substantially over time of cultured with anti-ID conjugated beads. As shown in FIG. 18B, CD25 expression also was decreased after 9 days of culture in the presence of anti-ID conjugated beads. Similar results were observed for both donors.

### Example 14 : Comparison of Cytokine Levels and Phenotype After Culture with Anti-Idiotype Antibody Conjugated Beads

CD3-purified T cells were isolated from peripheral blood mononuclear cells (PBMCs) of two donor patients, transduced with a viral vector encoding an anti-CD19 CAR having an scFv derived from FMC63 and expanded in culture with anti-CD3 and anti-CD28 antibody coated beads. After expansion, the expanded T cells were frozen by cryopreservation. For the studies, frozen T cells were thawed and CD4+ and CD8+ T cells assessed for intracellular cytokine levels or surface phenotype (d=0) or thawed CD4+, CD8+, or CD4+/CD8+ co-culture T cells were cultured for an additional 9 days in the presence of anti-ID B-1 conjugated beads prior to assessing intracellular cytokine levels and surface marker phenotype following stimulation by PMA/ionomycin or CD19 transduced K562 cells (d=9).

For assessment of intracellular cytokine levels, Golgi inhibitor was added for 4 hours and then TNFα, IFNγ, and IL-2 was assessed by flow cytometry. For all conditions, the extent of intracellular cytokine expression was substantially greater when cells were stimulated in with PMA/ionomycin as compared to CAR-specific stimulation with CD19 transduced K562 cells. As shown in FIG. 19A, the levels of TNFα and IL-2 cytokines were similar in CD4+ or CD8+ cells immediately after thaw compared to corresponding CD4+ or CD8+ cells, or co-cultures of CD4/CD8 T cells, further cultured in the presence of anti-ID conjugated beads for 9 days. Increased levels of IFNγ was observed in thawed CD4+, CD8+ or CD4/CD8 co-culture T cells that were further cultured in the presence of anti-ID conjugated beads for 9 days compared to the level of IFNγ in CD4+ or CD8+ T cells immediately after thaw. These results demonstrated that T cell function was maintained compared to freshly thawed CAR+ T cells, following a 9 day expansion with anti-ID conjugated beads. Similar results were obtained in cells from the two donors.

Surface expression of the activation marker CD25, surface expression of inhibitory receptors PD-1 and LAG-3, and nuclear expression of the proliferation marker Ki-67 were also assessed in CD4+ or CD8+ cells immediately after thaw (d=0) or in CD4+ or CD8+ T cells expanded alone or as a CD4/CD8 co-culture for an additional 9 days in the presence of the anti-ID conjugated beads (d=9). As shown in FIG. 19B, reduced expression of CD25, but not Ki-67, was observed in cells that were further cultured in the presence of anti-ID conjugated beads for 9 days compared to T cells immediately after thaw. The reduced expression of CD25 was substantially greater in CD8+ cells than in CD4+ cells. In addition, decreased expression of PD-1 and LAG-3 also was observed in both CD4+ and CD8+ cells cultured alone or as a CD4/CD8 co-culture after incubation for 9 days with anti-ID conjugated beads compared to expression in cells immediately after thaw. This result demonstrated that after incubation with the anti-ID conjugated beads, the previously frozen transduced cells retained functional ability as evident by the high percentage of cells that were positive for the marker Ki-67, indicative of cell proliferation, but also exhibited a different activation state characterized by the low surface expression for the CD25 activation marker and the inhibitory receptor markers PD-1 and LAG-3.

### Example 15 : Effect of Stimulation of Anti-BCMA Conjugated Beads or Anti-BCMA/PD-L1 Conjugated Beads on PD-1 Expression and/or Signaling

Anti-BCMA CAR-T cells, generated from samples from representative healthy donors or multiple myeloma patient derived material, and cultured with 50 µg/mL BCMA-Fc conjugated beads (generated as described in Example 1) at a ratio of 1:1 bead:CAR+ T cell for 7 days, in the presence of 1 of 1 µM lenalidomide or a vehicle control. Expression of CD25, PD-1, Tim3 and Lag3 on CAR T cells (using an antibody for surrogate CAR marker) cultured under the different conditions then was assessed by flow cytometry.

Such anti-BCMA CAR- T cells prestimulated with beads in the presence or absence of lenalidomide, or freshly thawed anti-BCMA CAR-T cells generated from comparable donor samples, were then debeaded, washed, and cultured with RPMI-8226 target cells (labeled with NucLight Red (NLR) to permit their tracking by microscopy), in the presence of 1 µM lenalidomide or a vehicle control. Specifically, for pretreated cells in which pretreatment had been conducted in the presence of lenalidomide, the cells were cultured with the target cells in the presence of lenalidomide; likewise, for pretreated cells in which pretreatment had been conducted in the presence of vehicle, cells were cultured with the target cells in the presence of vehicle. Following the co-culture, cytolytic activity was assessed by measuring the loss of viable target cells over a period of seven days, as determined by red fluorescent signal. Percentage killing was normalized to anti-BCMA CAR T cells prestimulated on beads in the presence of vehicle. Cytokine production was assessed by ELISA from supernatant following culture with target cells for 24 hours. Experiments were performed twice in 3 donors. Linear fixed-effect or mixed-effect models were used to assess the significance of lenalidomide treatments on cytolytic activity and cytokine production, with treatment, donor, and time treated as fixed effects and animal treated as a random effect, nested with time when repeated measurements were derived from the same animal. P values were obtained by likelihood ratio tests comparing the full model with the effect of interest against the model without the effect of interest.

FIG. 20A shows results for CAR antigen-specific cytolytic activity and FIG. 20B shows results for cytokine production for anti-BCMA CAR-T cells that had been prestimulated with BCMA beads (compared to freshly-thawed (non-prestimulated) anti-BCMA CAR-T cells) in the co-cultures, comparing cells cultured in the presence versus absence of lenalidomide. Prestimulated CAR T cells showed decreased cytolytic activity (*P =* 2.1 × 10⁻⁴) and cytokine production (*P* = .03 for IFN-γ) compared with freshly thawed anti-BCMA CAR T cells.

In the absence of lenalidomide in pretreatment and subsequent co-culture, the results prestimulated CAR-T cells exhibited reduced cell killing and cytokine production compared to fresh CAR-T cells, indicating that chronic prestimulation leads to functional impairment. These results are consistent with an exhaustion-like phenotype having been induced by prestimulation on the BCMA-conjugated beads. The presence of lenalidomide during the prestimulation period preserved cytolytic function (*P* = .04), and there was a trend toward increased cytokine production compared with cells exposed to vehicle during the prestimulation period (FIG. 24B). The presence of lenalidomide in this assay was consistent with an observation that lenalidomide may reduce the functional exhaustion-like phenotypes in the prestimulated CAR-T cells.

As shown in FIG. 20C, the phenotype of anti-BCMA CAR T cells stimulated for 7 days on BCMA beads was assessed, and the addition of lenalidomide significantly increased CAR+ viability of anti-BCMA CAR T material across 3 healthy donors (P = 0.04). The addition of lenalidomide did not alter the total cell count across all donors in this 7-day period, and no significant differences were observed in percentage CAR+ between vehicle- and lenalidomide-treated CAR T cells. FIG. 20D shows representative results of flow cytometric analysis of surface CD25 and PD-1 expression (mean fluorescent intensity (MFI), for CD4+ or CD8+ anti-BCMA CAR T-cells after stimulation (pretreatment) with BCMA beads for 7 days, in the presence or absence of 1 µM lenalidomide. As shown, the results indicated that lenalidomide reduced PD-1 expression of BCMA-CAR-T cells, while increasing CD25 expression after prolonged stimulation. As shown in FIG. 20E, flow cytometric analysis across the three CAR T donors indicated that the addition of lenalidomide increased the surface expression of Tim3 in the CD8+ population (P = 4.0 × 10-4), with mixed effects on the CD4+ CAR+ population. Across all donors and in both the CD4+ and CD8+ CAR+ populations, lenalidomide increased CD25 (CD4+ and CD8+; P = 2.2 × 10-16) and the percentage positive for Lag3 expression (CD8+ P < 0.03; CD4+ P = 0.002). Notably, a decrease in the percentage of PD-1+ cells was also observed in the CD4+ population (P = 0.04), with 2 of 3 donors showing a decrease in the CD8+ population as well.

In another study, recombinant human BCMA-conjugated beads were used to stimulate CAR T cells at various concentrations to titrate the magnitude of stimulation, either low (5 µg/ mL), medium (50 µg/ mL), and high (200 µg/ mL) stimulation. At a medium stimulation condition, the secreted cytokine production 24 hours after stimulation was measured, and a 200% increase in IL-2 and TNF-α concentrations were observed compared with vehicle control, with donor-dependent increases in IFN-γ (FIG. 21A). Cells were stimulated with BCMA conjugated beads for 24 hours in the presence of 0.1 µM or 1.0 µM lenalidomide, or vehicle control. A protein transport inhibitor was added in the final hours of incubation, and cells were stained for intracellular IL-2, IFN-γ, and TNF-α.

Anti-BCMA CAR T cells activated on BCMA beads showed stimulation level-dependent effects on cytokine production, with 5-µg BCMA beads causing limited CAR T effector cytokine production compared with 50-µg and 200-µg BCMA beads (FIG. 21B). Lenalidomide increased the percentage of IFN-γ⁺ and TNF-α⁺ intracellular staining at all stimulation levels for both CD4⁺ and CD8⁺ CAR T cells. The magnitude of stimulation either increased or decreased IL-2 in response to lenalidomide, with the lenalidomide decreasing the percentage of IL-2⁺ CAR⁺ T cells at 50-µg and 200-µg stimulation but increasing the percentage of IL-2⁺ CAR⁺ T cells at the 5-µg stimulation condition. In the absence of stimulation, lenalidomide had no effect on CAR T cytokine production, indicating that cytokine enhancement provided by lenalidomide requires stimulation.

In another study, cells were cultured in the presence of BCMA beads generated as described in Example 1, with or without additional conjugation of human recombinant PD-L1-Fc. Healthy donor-or patient-derived CAR T cells were stimulated in the presence of BCMA-conjugated beads or BCMA/PD-L1 conjugated beads for 24 hours in the presence of 1 µM lenalidomide. Cytokine production was measured in the supernatant. Results are shown in FIG. 21C. As shown in FIG. 25C, evaluation of both healthy and patient donor CAR T cells demonstrated that addition of recombinant PD-L1 to recombinant BCMA beads reduced IFN-γ, IL-2, and TNF-α. It was shown that lenalidomide treatment potentiated secreted cytokine levels beyond those from CAR T cells treated with vehicle in the presence of PD-L1. The results were consistent with a conclusion that anti-BCMA CAR-T cytokine production following incubation with BCMA-conjugated beads was increased by lenalidomide in the presence of PD-L1-mediated inhibition.

### Example 16 : Assessment of cytokine production by anti-BCMA CAR T cells stimulated with BCMA-conjugated beads

Anti-BCMA CAR T cells were generated generally as described in Example 2. The anti-BCMA CAR T cells were formulated at a 1:1 ratio of CD4+ and CD8+ T cells and were thawed and cultured at 5.0 x 10⁵ cells per well in a 96-well plate. Anti-CD3/anti CD28 antibody conjugated beads or BCMA conjugated beads 4.5 or 2.8 µm diameter (from a 5 µg/ml, 50 µg/ml, or 200 µg/ml BCMA-conjugated bead composition generated as described in Example 1) were added to the cultures at a ratio of anti-BCMA CAR-T cells to beads of 1:1. Anti-BCMA CAR-T cells cultured in the absence of anti-CD3/anti-CD28 antibody or BCMA conjugated beads served as controls.

After a twenty-four hour incubation, the presence of IFN-gamma, IL-2, TNF-alpha, IL-6, GM-CSF, and IL-4 in culture supernatants were assessed using a multiplex cytokine immunoassay (Luminex^{®}). As shown in FIG. 22A, incubation with anti-CD3/anti-CD28 antibody conjugated beads resulted in higher concentrations of cytokines present in the supernatant as compared to incubation with BCMA-conjugated beads. In this assay, the magnitude of cytokine production from cells incubated with BCMA-conjugated beads was dose-dependent, correlating with increasing concentrations of BCMA (i.e., 5 µg/ml, 50 µg/ml, or 200 µg/ml) used for the bead conjugation. This result is consistent with a finding that BCMA-directed CAR activation could be controlled and titrated by varying the amount of BCMA conjugated to the beads. Comparisons of cytokine release following incubation with anti-CD3/anti-CD28 antibody conjugated beads or BCMA-conjugated beads revealed differences in magnitude and quality of cytokine production, with a more Th1-like cytokine release profile observed following stimulation with BCMA-conjugated beads.

Anti-BCMA CAR-T cells were cultured as described above and assessed for intracellular cytokine levels by flow cytometry. Anti-BCMA CAR T cells were incubated with anti-CD3/anti-CD28 antibody conjugated beads or BCMA-conjugated beads at a ratio of anti-BCMA CAR-T cells to beads of 1:1 for twenty hours followed by a four hour incubation with a protein transport inhibitor. Cells were stained for viability, CAR expression, CD4, and CD8, then fixed, permeabilized, and stained for intracellular levels of IFN-gamma, IL-2, and TNF-alpha. As shown in FIG. 22B, stimulation with BCMA-conjugated beads resulted in cytokine production in both CD4+ and CD8+ anti-BCMA CAR expressing cells. Similar to above, the magnitude of the intracellular cytokine levels appeared to correlate with the amount of BCMA conjugated to the beads.

### Example 17 : Generation of Anti-CD19 Antibody Anti-ID and Anti-CD2 Antibody Conjugated Beads

Anti-CD2 and anti-CD19 antibody anti-ID antibody coated beads were generated. Mouse anti-CD2 antibody (clone RPA-2.10, ED biosciences) and the anti-ID B-2 anti-idiotypic antibody described in Example 9 were covalently coupled to the surface of commercially available tosylactivated superparamagnetic having a diameter of approximately 4.5 µm (designated M-450; ThermoFisher, Waltham MA).

Approximately 6.67×10⁻¹⁰ mol of each antibody was added per 1 mL of the tocyl-activated beads (e.g. approximately 4×10⁸ beads/mL) and covalent coupling was performed by overnight incubation at 37°C in phosphate buffered solution (PBS) containing 0.1% human serum albumin (HSA). Beads were washed and resuspended in 1 mL PBS with 0.1% HSA. After conjugation, the bead concentration was determined using a Cellometer. Based on coupling concentration and bead count, the approximate number of molecules on the beads was1.58×10⁶ antibody molecules per bead. The antibody coated beads were suspended in solution at a concentration of 4.1 ×10⁸ beads/mL.

Stability of the antibody conjugated beads was assessed by pelleting the antibody-conjugated beads, running the resultant supernatant on an 4-12% Bis-Tris gel, and staining the gel for protein with Coomassie blue. As a control for total protein conjugated on the beads, a 10 µL sample of supernatant from pelleted beads that were boiled in LDS sample buffer also was assessed. Controls also included assessment of 1 µg of anti-CD2 antibody, 1 µg anti-ID B-2 antibody that had not been conjugated to the beads (positive controls), and 5 µL 0.1% HSA (negative control). Bands corresponding to the antibodies were detected in the sample with the supernatant from the boiled sample. No anti-ID antibody was detected in supernatant from conjugated samples that had not been boiled, consistent with a stable conjugation.

### Example 18 : Generation of Anti-CD19 Antibody Anti-ID and Anti-CD28 Antibody Conjugated Beads

Anti-CD28 and an anti-CD19 antibody anti-ID antibody coated beads were generated. Mouse anti-CD28 antibody (low endotoxin, azide free (LEAF) purified clone CD28.2 (Biolegend) and the anti-ID B-2 idiotypic antibody described in Example 9 were covalently coupled to the surface of commercially available tosylactivated superparamagnetic having a diameter of approximately 4.5 µm (designated M-450; ThermoFisher, Waltham MA). Approximately 6.67×10⁻¹⁰ mol of each antibody was added per 1 mL of the tocyl-activated beads (e.g. approximately 4×10⁸ beads/mL) and covalently coupled substantially as described in Example 17. Based on coupling concentration and bead count, the approximate number of molecules on the beads was 1.79×10⁶ antibody molecules per bead. The antibody coated beads were suspended in solution at a concentration of 3.2 ×10⁸ beads/mL as determined with a Cellometer.

### Example 19 : Generation of Beads containing surface conjugated Anti-CD19 Antibody Anti-ID, Anti-CD2, and Anti-CD28 Antibody

Paramagnetic beads coated with anti-CD2 , anti-CD28, and anti-CD19 antibody anti-ID antibody coated beads were generated. Mouse anti-CD2 antibody (clone RPA-2.10, ED biosciences), mouse anti-CD28 (LEAF purified clone CD28.2, Biolegend) and the anti-ID B-2 idiotypic antibody described in Example 9 were covalently coupled to the surface of commercially available tosylactivated superparamagnetic having a diameter of approximately 4.5 µm (designated M-450; ThermoFisher, Waltham MA).

Approximately 4.44×10⁻¹⁰ mol of each antibody was added per 1 mL of the tocyl-activated beads (e.g. approximately 4×10⁸ beads/mL) and were covalently coupled substantially as described in Example 17.. Based on coupling concentration and bead count, the approximate number of molecules on the beads was 1.56×10⁶ antibody molecules per bead. The antibody coated beads were suspended in solution at a concentration of 3.91 ×10⁸ beads/mL.

Stability of the antibody conjugated beads was assessed by pelleting the antibody-conjugated beads, running the resultant supernatant on an 4-12% Bis-Tris gel, and staining the gel for protein with Coomassie blue. As a control for total protein conjugated on the beads, 10 µL of supernatant from pelleted beads that were boiled in LDS sample buffer were assessed. Additional controls that were assessed also 1 µg of anti CD2 antibody, 1 µg anti-ID B-2 antibody that had not been conjugated to the beads (positive controls), and 5 µL 0.1% HSA (negative control). No anti-ID antibody was detected in supernatant from conjugated samples that had not been boiled indicating that the conjugation was stable, whereas bands corresponding to the antibodies were detected in the loaded supernatant from the boiled sample.

### Example 20 : In Vitro Assay for Chronic Stimulation of CAR+ T Cells Utilizing Anti-ID Conjugated Beads

Separate compositions of CD4+ and CD8+ cells were isolated from human donors, activated and transduced with a viral vector encoding an anti-CD19 CAR having an scFv derived from FMC63. CD4+ and CD8+ T cells from each donor were then separately harvested, formulated, and cryofrozen. The cryofrozen engineered CD4+ and CD8+ T cells were thawed and formulated at a 1:1 ratio of CD4+ and CD8+ T cells from the same donor to generate a T cell composition containing CAR+ T cells. Anti-ID conjugated beads against the anti-CD19 CAR were incubated with cells at a 1:1 bead:cell ratio for 14 days.

Secondary response of CAR-T cells harvested at day 14 following CAR-specific stimulation with anti-ID conjugated beads (Day 14; secondary) was assessed after stimulation with K562-CD19 antigen-expressing target cells at an effector to target ratio of 1:1 (to assess cytokine levels) or 3:1 to assess cytolytic activity). The primary response of T cells from the T cell composition that had not been incubated with the anti-ID conjugated beads also was determined by similar stimulation with antigen-expressing cells (Day 0; "primary"). To assess cytolytic activity, the target cells were labeled with NucLight Red (NLR) to permit tracking by fluorescent microscopy. Killing activity was assessed by measuring the loss of viable target cells over 72 hours, as determined by loss of fluorescent signal over time by kinetic fluorescence microscopy (using the INCUCYTE^{®} Live Cell Analysis System, Essen Bioscience). Killing index was determined as the inverse of the area under the curve (AUC) for target fluorescence over time. Intracellular cytokine levels of IL-2 and TNF-alpha also was assessed by flow cytometry in co-cultured T cells after incubation in the presence of Golgi inhibitor.

As shown in FIG. 23A, target cell killing by a T cell composition containing CAR+ T cells collected following CAR-specific stimulation for 14 days with anti-ID conjugated beads was reduced compared to cytolytic activity of CAR+ T cells that did not undergo prior CAR-specific stimulation. Intracellular cytokine levels of IL-2 and TNF-alpha also were reduced in CAR+ T cells that had received long-term CAR-specific stimulation with the anti-ID conjugated beads (FIG. 23B). These results are consistent with an observation that long-term CAR-specific stimulation, such as by incubation with anti-ID conjugated beads for 14 days, leads to chronic stimulation of the CAR and loss of sustained function.

The chronic stimulation assay described above was used to assess the effects of various compounds on improving CAR+ T cell function after long-term stimulation. Anti-CD19 CAR+ T cell compositions were generated as described above, except in the presence of a different compound or a vehicle control. Cells from each generated CAR-T cell composition were incubated with anti-ID conjugated paramagnetic beads at 1:1 bead to cell ratio for 14 days.

Primary response of CAR-T cell compositions at thaw (no stimulation with anti-ID conjugated beads) or secondary response of CAR-stimulated CAR-T compositions (following 14 day CAR-specific stimulation with anti-ID conjugated beads) was assessed after stimulation with antigen-expressing cells. CAR-T cells compositions were cultured 1:1 with K562-CD19 antigen-expressing cells in the presence of Golgi inhibitor, and polyfunctional cytokine production was assessed by flow cytometry following intracellular cytokine staining for IL-2, IFN-gamma and TNF-alpha. A polyfunctional score was determined from cumulative levels of cytokines as determined in CD8+ cells after the data were normalized by scaling within donor cohorts (FIG. 24A). Total secreted IL-2, TNF and IFN-gamma cytokines from cell culture supernatant of co-cultures after 20 hours of incubation with targets cells was determined, and the average of the scaled scores for all three cytokines was calculated as shown in FIG. 24B. As shown in FIGS. 24A and 24B, certain compounds resulted in improved primary or secondary responses based on the ability of CAR-T cell compositions to produce cytokines. Improvements in primary or secondary cytolytic response, following co-culture with target cells at a 3:1 effector:target cell ratio as described above, also was observed among T cell compositions produced in the presence of certain compounds (FIG. 24C).

These results demonstrate the utility of the chronic stimulation assay to evaluate CAR-T cell compositions, including different CAR-T cell compositions produced under different conditions or in the presence of compounds or other agents, for their ability to exhibit long-term survival and/or sustain function after chronic CAR-T cell stimulation, such as may occur following prolonged exposure to antigen in vivo.

### Example 21 : Functional Assessment of Chimeric Antigen Receptor (CAR)-Transduced T Cells (CAR T Cells) Expanded in the Presence of Small Molecule Compounds

Engineered T cell compositions containing T cells that express an anti-CD19 CAR were generated from three separate donors in the presence of different compounds or a vehicle control.

The ability of the cells from the different T cell compositions to expand following stimulation of the CAR was assessed by incubating cells of the generated anti-CD19 CAR-T cell compositions with beads surface conjugated with an anti-idiotype antibody specific to the anti-CD19 CAR. The anti-ID conjugated beads were incubated with cells at a 1:1 bead: cell ratio in wells of 24-well G-rex expansion vessels (Argos Technologies) for 15 days. The total live T cells per well was determined by counting cells in the cultures every 5 days (FIG. 25A). The mean area under the curve (AUC) of the function of T cell number over time was calculated relative to the AUC of cells expanded with media only (FIG. 25B).

As shown in FIG. 25A, stimulation of cells with anti-idiotypic antibody conjugated beads resulted in an initial expansion that was followed by a decline in cell number. T cells from generated anti-CD19 CAR T cell compositions that had been previously expanded by incubation with the compounds had a larger mean AUC compared to T cells previously expanded with the vehicle control (FIG. 25B). The results indicate that the presence of a long-term stimulation assay can be used to identify compounds that, in some cases, improve the ability of generated T cell compositions to expand and survive following a single CAR-specific stimulation.

Secondary cytokine response after stimulation with antigen-expressing cells was assessed on CAR-T cells from the T cell compositions harvested at day 11 following expansion with anti-ID conjugated beads. The anti-ID stimulated cells were incubated with irradiated K562-CD19 target cells at an effector:target ratio of 1:1 for approximately 16 hours. Supernatant was collected and TNF-alpha, IFN-gamma, and IL-2 cytokine production were measured using a Luminex Multiplex Assay. The fold-change of cytokine production observed in co-culture supernatants was determined from generated anti-CD19 CAR-T cell compositions expanded in the presence of compounds or vehicle control compared to cells expanded in media only. As shown in FIG. 25C, this assay identified T cell compositions, generated from anti-CD19 CAR T cell compositions that had been previously expanded in the presence of certain compounds, that exhibited improved secondary cytokine production following subsequent stimulation with antigen. In addition, the assay identified a T cell composition from some donor-derived cells, that when engineered and expanded in the presence of a compound, exhibited an increased frequency of CD8+ T cells that were CD107a+IFNγ+ cells at day 11, as determined by intracellular cytokine staining following incubation with Golgi inhibitor for 4 hours substantially as described above (FIG. 25D). These results are consistent with the use of a long-term stimulation assay to identify effects of compounds used in the process to engineer T cells to improve function of the engineered T cell composition.

The present invention is not intended to be limited in scope to the particular disclosed embodiments, which are provided, for example, to illustrate various aspects of the invention. Various modifications to the compositions and methods described will become apparent from the description and teachings herein. Such variations may be practiced without departing from the true scope and spirit of the disclosure and are intended to fall within the scope of the present disclosure.

### SEQUENCES

| **#** | **SEQUENCE** | **ANNOTATION** |
|---|---|---|
| 1 | MLQMAGQCSQNEYFDSLLHACIPCQLRCSSNTPPLTCQRYCNASVT NSVKGTNA | Extracellular domain of human BCMA (GenBank No. NP 001183.2) |
| 2 | EPKSCDKTHTCPPCPAPELLGGPSVFLFPPKPKDTLMISRTPEVTCVV VDVSHEDPEVKFNWYVDGVEVHNAKTKPREEQYNSTYRVVSVLTV LHQDWLNGKEYKCKVSNKALPAPIEKTISKAKGQPREPQVYTLPPSR EEMTKNQVSLTCLVKGFYPSDIAVEWESNGQPENNYKTTPPVLDSD GSFFLYSKLTVDKSRWQQGNVFSCSVMHEALHNHYTQKSLSLSPGK | Human IgG1 Fc |
| 3 | GLNDIFEAQKIEWHE | AviTag |
| 4 | KRRWKKNFIAVSAANRFKKISSSGAL | Calmodulin Tag |
| 5 | FFFFFF | Polyglutamine tag |
| 6 | DYKDDDDK | Flag-tag |
| 7 | YPYDVPDYA | HA-tag |
| 8 | EQKLISEEDL | Myc-tag |
| 9 | Trp-Arg-His-Pro-Gln-Phe-Gly-Gly (WRHPQFGG) | STREP-TAG^{®} I streptavidin-binding peptide sequence |
| 10 | Trp-Ser-His-Pro-Gln-Phe-Glu-Lys (WSHPQFEK) | STREP-TAG^{®} II streptavidin-binding peptide sequence |
| 11 | His-Pro-Baa (HPX) | - Streptavidin Binding peptide - X is selected from glutamine, asparagine and methionine |
| 12 | His-Pro-Gln-Phe (HPQF) | Streptavidin Binding peptide |
| 13 | Oaa-Xaa-His-Pro-Gln-Phe-Yaa-Zaa (XX HPQFXX) | Streptavidin Binding peptide Oaa is Trp, Lys or Arg |
| | | Xaa is any amino acid; |
| | | Yaa is Gly or Glu Zaa is Gly, Lys or Arg |
| 14 | Trp-Xaa-His-Pro-Gln-Phe-Yaa-Zaa (WXHPQFXX) | Streptavidin Binding peptide |
| | | Xaa is any amino acid; Yaa is Gly or Glu, Zaa is Gly, Lys or Arg |
| 15 | Trp-Ser-His-Pro-Gln-Phe-Glu-Lys-(Xaa)n-Trp-Ser-His-Pro-Gln-Phe-Glu-Lys- (Xaa is any amino acid; n is either 8 or 12) (WSHPQFEKXₙWSHPQFEK) | Streptavidin Binding peptide (Xaa is any amino acid; n is either 8 or 12) |
| 16 | Trp-Ser-His-Pro-Gln-Phe-Glu-Lys-(GlyGlyGlySer)n-Trp-Ser-His-Pro-Gln-Phe-Glu-Lys (n is 2 or 3) (WSHPQFEK(GGGS)ₙWSHPQFEK | Streptavidin Binding peptide |
| | | (n is 2 or 3) |
| 17 | SAWSHPQFEKGGGSGGGSGGGSWSHPQFEK | Streptavidin Binding peptide |
| 18 | SAWSHPQFEKGGGSGGGSGGSAWSHPQFEK | Streptavidin Binding peptide |
| 19 | WSHPQFEKGGGSGGGSGGGSWSHPQFEK | Streptavidin Binding peptide |
| 20 | WSHPQFEKGGGSGGGSWSHPQFEK | Streptavidin Binding peptide |
| 21 | WSHPQFEKGGGSGGGSGGSAWSHPQFEK | Streptavidin Binding peptide |
| 22 | DPSKDSKAQVSAAEAGITGTWYNQLGSTFIVTAGADGALTGTYESA VGNAESRYVLTGRYDSAPATDGSGTALGWTVAWKNNYRNAHSAT TWSGQYVGGAEARINTQWLLTSGTTEANAWKSTLVGHDTFTKVKP SAASIDAAKKAGVNNGNPLDAVQQ | Wild type Streptavidin Sequence (Streptavidin-Species: Streptomyces avidinii - UniProt No. P22629 |
| 23 | EAGITGTWYNQLGSTFIVTAGADGALTGTYVTARGNAESRYVLTGR YDSAPATDGSGTALGWTVAWKNNYRNAHSATTWSGQYVGGAEAR INTQWLLTSGTTEANAWKSTLVGHDTFTKVKPSAAS | mutein streptavidin designated Strep-tactin^{®} |
| 24 | MGKSKEISQDLRKKIVDLHKSGSSLGAISKRLKVPRSSVQTIVRKYK HHGTTQPSYRSGRRRVLSPRDERTLVRKVQINPRTTAKDLVKMLEE TGTKVSISTVKRVLYRHNLKGRSARKKPLLQNRHKKARLRFATAHG DKDRTFWRNVLWSDETKIELFGHNDHRYVWRKKGEACKPKNTIPT VKHGGGSIMLWCGFAAGGTGALHKIDGIMRKENYVDILKQHLKTS VRKLKLGRKWVFQMDNDPKHTSKVVAKWLKDNKVKVLEWPSQSP DLNPIENLWAELKKRVRARRPTNLTQLHQLCQEEWAKIHPTYCGKL VEGYPKRLTQVKQFKGNATKY | Sleeping Beauty Transposase |
| 25 | ATGGGAAAATCAAAAGAAATCAGCCAAGACCTCAGAAAAA AAATTGTAGACCTCCACAAGTCTGGTTCATCCTTGGGAGCAATTT CCAAACGCCTGAAAGTACCACGTTCATCTGTACAATCTGGTTCAT CCTTGGGAGCAATTTCCAAACGCCTGAAAGTACCACGTTCATCTG TACAAACAATAGTACGCAAGTATAAACACCATGGGACCACGCAG CCGTCATACCGCTCAGGAAGG AGACGCGTTCTGTCTCCTAGAGATGAACGT ACTTTGGTGCGAAAAGTGCAAATCAATCCCAGAACAACAGCAAA GGACCTTGTGAAGATGCTGGAGGAAACAGGTACAAAAGTATCTA TA TCCACAGTAAAACGAGTCCTATATCGACATAACCTGAAAGGCCG | Gene encoding Sleeping Beauty Transpose |
| | CTCAGCAAGGAAGAAGCCACTGCTCCAAAACCGACATAAGAAAG CCAGACTACGGTTTGCAACTGCACATGGGACAAAGATCGTACT TTTTGGAGAAATGTCCTCTGGTCTGATGAAACAAAAATAGAACTG TTT GGCCATAATGACCATCGTTATGTTTGGAGGAAGAAGGGGGAGGC TTGCAAGCCGAAGAACACCATCCCAACCGTGAAGCACGGGGGTG GCAGCATCATGTTGTGGGGGTGCTTTGCTGCAGGAGGGACTGGTG CACTTCA CAAAATAGATGGCATCATGAGGAAGGAAAATTATGTGGAT ATATTGAAGCAACATCTCAAGACATGAGTCAGGAAGTTAAAGCT TGGTCGCAAATGGGTCTTCCAAATGGACAATGACCCCAAGCATA CTTCCAAAGTTGTGGCAAAATGGCTTAAGGACAACAAAGTCAAG GTATTGGAGTGGCCATCACAAAGCCCTG ACCTCAATCCTATAGAAAAT TTGTGGGCAGAACTGAAAAAGCGTGTGCGAGCAAGGAGGCCTAC AAACCTGACTCAGTTACACCAGCTCTGTCAGGAGGAATGGGCCA AAATTCACCCAACTTATTGTGGGAAGCTTGTGGAAGGCTACCCGA AACGTTTGACCCAAGTTAAACAATTTAAAGGCAATGCTACCAAAT AC TAG | |
| 26 | cagttgaagtcggaagtttacatacacttaagttggagtcattaaaactcgtttttcaactacTccacaaatttctt gttaacaaacaatagttttggcaagtcagttaggacatctactttgtgcatgacacaagtcatttttccaacaattg tttacagacagattatttcacttataattcactgtatcacaattccagtgggtcagaagtttacatacactaagttga ctgtgcctttaaacagcttggaaaattccagaaaatgatgtcatggctttagaagcttctgatagactaattgaca tcatttgagtcaattggaggtgtacctgtggatgtatttcaagg | Sleeping Beauty inverted repeat sequence |
| 27 | GGGGS | Linker sequence |
| 28 | PKSSDKTHTCPPCPAPEAEGAPSVFLFPPKPKDTLMISRTPEVTCVVV DVSHEDPEVKFNWYVDGVEVHNAKTKPREEQYNSTYRVVSVLTVL HQDWLNGKEYKCKVSNKALPSSIEKTISKAKGQPREPQVYTLPPSRD ELTKNQVSLTCLVKGFYPSDIAVEWESNGQPENNYKTTPPVLDSDGS FFLYSKLTVDKSRWQQGNVFSCSVMHEALHNHYTQKSLSLSPGK | Modified Human IgG1 Fc |
| 29 | DSSKWVFEHPETLYAWEGACVWIPCTYRALDGDLESFILFHNPEYN KNTSKFDGTRLYESTKDGKVPSEQKRVQFLGDKNKNCTLSIHPVHL NDSGQLGLRMESKTEKWMERIHLNVSERPFPPHIQLPPEIQESQEVTL TCLLNFSCYGYPIQLQWLLEGVPMRQAAVTSTSLTIKSVFTRSELKFS PQWSHHGKIVTCQLQDADGKFLSNDTVQLNVKHTPKLEIKVTPSDAI VREGDSVTMTCEVSSSNPEYTTVSWLKDGTSLKKQNTFTLNLREVT KDQSGKYCCQVSNDVGPGRSEEVFLQVQYAPEPSTVQILHSPAVEG SQVEFLCMSLANPLPTNYTWYHNGKEMQGRTEEKVHIPKILPWHAG TYSCVAENILGTGQRGPGAELDVQYPPKKVTTVIQNPMPIREGDTVT LSCNYNSSNPSVTRYEWKPHGAWEEPSLGVLKIQNVGWDNTTIACA ACNSWCSWASPVALNVQYAPRDVRVRKIKPLSEIHSGNSVSLQCDF SSSHPKEVQFFWEKNGRLLGKESQLNFDSISPEDAGSYSCWVNNSIG QTASKAWTLEVLYAPRRLRVSMSPGDQVMEGKSATLTCESDANPP VSHYTWFDWNNQSLPYHSQKLRLEPVKVQHSGAYWCQGTNSVGK GRSPLSTLTVYYSPETIGRR | Human CD22 extracellular domain |
| 30 | MPLLLLLPLLWAGALA | CD33 Signal peptide |
| 31 | QETELSVSAELVPTSSWNISSELNKDSYLTLDEPMNNITTSLGQTAEL HCKVSGNPPPTIRWFKNDAPVVQEPRRLSFRSTIYGSRLRIRNLDTTD TGYFQCVATNGKEVVSSTGVLFVKFGPPPTASPGYSDEYEEDGFCQP YRGIACARFIGNRTVYMESLHMQGEIENQITAAFTMIGTSSHLSDKC SQFAIPSLCHYAFPYCDETSSVPKPRDLCRDECEILENVLCQTEYIFAR | Human ROR1 fragment |
| | SNPMILMRLKLPNCEDLPQPESPEAANCIRIGIPMADPINKNHKCYNS TGVDYRGTVSVTKSGRQCQPWNSQYPHTHTFTALRFPELNGGHSYC RNPGNQKEAPWCFTLDENFKSDLCDIPACDSKDSKEKNKMEILY | |
| 32 | VKQTLNFDLLKLAGDVESNPGP | F2A |
| 33 | QETELSVSAELVPTSSWNISSELNKDSYLTLDEPMNNITTSLGQTAEL HCKVSGNPPPTIRWFKNDAPVVQEPRRLSFRSTIYGSRLRIRNLDTTD TGYFQCVATNGKEVVSSTGVLFVKFGPPPTASPGYSDEYEEDGFCQP YRGIACARFIGNRTVYMESLHMQGEIENQITAAFTMIGTSSHLSDKC SQFAIPSLCHYAFPYCDETSSVPKPRDLCRDECEILENVLCQTEYIFAR SNPMILMRLKLPNCEDLPQPESPEAANCIRIGIPMADPINKNHKCYNS TGVDYRGTVSVTKSGRQCQPWNSQYPHTHTFTALRFPELNGGHSYC RNPGNQKEAPWCFTLDENFKSDLCDIPACDSKDSKEKNKMEILYGG GGSPKSSDKTHTCPPCPAPEAEGAPSVFLFPPKPKDTLMISRTPEVTC VVVDVSHEDPEVKFNWYVDGVEVHNAKTKPREEQYNSTYRVVSVL TVLHQDWLNGKEYKCKVSNKALPSSIEKTISKAKGQPREPQVYTLPP SRDELTKNQVSLTCLVKGFYPSDIAVEWESNGQPENNYKTTPPVLDS DGSFFLYSKLTVDKSRWQQGNVFSCSVMHEALHNHYTQKSLSLSPG K | ROR1-Fc fusion polypeptide |
| 34 | DSSKWVFEHPETLYAWEGACVWIPCTYRALDGDLESFILFHNPEYN KNTSKFDGTRLYESTKDGKVPSEQKRVQFLGDKNKNCTLSIHPVHL NDSGQLGLRMESKTEKWMERIHLNVSERPFPPHIQLPPEIQESQEVTL TCLLNFSCYGYPIQLQWLLEGVPMRQAAVTSTSLTIKSVFTRSELKFS PQWSHHGKIVTCQLQDADGKFLSNDTVQLNVKHTPKLEIKVTPSDAI VREGDSVTMTCEVSSSNPEYTTVSWLKDGTSLKKQNTFTLNLREVT KDQSGKYCCQVSNDVGPGRSEEVFLQVQYAPEPSTVQILHSPAVEG SQVEFLCMSLANPLPTNYTWYHNGKEMQGRTEEKVHIPKILPWHAG TYSCVAENILGTGQRGPGAELDVQYPPKKVTTVIQNPMPIREGDTVT LSCNYNSSNPSVTRYEWKPHGAWEEPSLGVLKIQNVGWDNTTIACA ACNSWCSWASPVALNVQYAPRDVRVRKIKPLSEIHSGNSVSLQCDF SSSHPKEVQFFWEKNGRLLGKESQLNFDSISPEDAGSYSCWVNNSIG QTASKAWTLEVLYAPRRLRVSMSPGDQVMEGKSATLTCESDANPP VSHYTWFDWNNQSLPYHSQKLRLEPVKVQHSGAYWCQGTNSVGK GRSPLSTLTVYYSPETIGRRGGGGSPKSSDKTHTCPPCPAPEAEGAPS VFLFPPKPKDTLMISRTPEVTCVVVDVSHEDPEVKFNWYVDGVEVH NAKTKPREEQYNSTYRVVSVLTVLHQDWLNGKEYKCKVSNKALPS SIEKTISKAKGQPREPQVYTLPPSRDELTKNQVSLTCLVKGFYPSDIA VEWESNGQPENNYKTTPPVLDSDGSFFLYSKLTVDKSRWQQGNVFS CSVMHEALHNHYTQKSLSLSPGK | CD22-Fc fusion polypeptide |
| 35 | MLQMAGQCSQNEYFDSLLHACIPCQLRCSSNTPPLTCQRYCNASVT NSVKGTNAGGGGSPKSSDKTHTCPPCPAPEAEGAPSVFLFPPKPKDT LMISRTPEVTCVVVDVSHEDPEVKFNWYVDGVEVHNAKTKPREEQ YNSTYRVVSVLTVLHQDWLNGKEYKCKVSNKALPSSIEKTISKAKG QPREPQVYTLPPSRDELTKNQVSLTCLVKGFYPSDIAVEWESNGQPE NNYKTTPPVLDSDGSFFLYSKLTVDKSRWQQGNVFSCSVMHEALH NHYTQKSLSLSPGK | BCMA-Fc fusion polypeptide |
| 36 | EVKLQQSGAELVRPGSSVKISCKASGYAFSSYWMNWVKQRPGQGL EWIGQIYPGDGDTNYNGKFKGQATLTADKSSSTAYMQLSGLTSEDS AVYFCARKTISSVVDFYFDYWGQGTTVTVSS | SJ25C1 VH |
| 37 | DIELTQSPKFMSTSVGDRVSVTCKASQNVGTNVAWYQQKPGQSPKP LIYSATYRNSGVPDRFTGSGSGTDFTLTITNVQSKDLADYFCQQYNR YPYTSGGGTKLEIKR | SJ25C1 VL |
| 38 | EVKLQESGPGLVAPSQSLSVTCTVSGVSLPDYGVSWIRQPPRKGLEW | FMC63 VH |
| | LGVIWGSETTYYNSALKSRLTIIKDNSKSQVFLKMNSLQTDDTAIYY CAKHYYYGGSYAMDYWGQGTSVTVSS | |
| 39 | DIQMTQTTSSLSASLGDRVTISCRASQDISKYLNWYQQKPDGTVKLL IYHTSRLHSGVPSRFSGSGSGTDYSLTISNLEQEDIATYFCQQGNTLP YTFGGGTKLEIT | FMC63 VL |
| 40 | SYWMN | SJ25C1 HC-CDR1 |
| 41 | QIYPGDGDTNYNGKFKG | SJ25C1 HC-CDR2 |
| 42 | KTISSVVDFYFDY | SJ25C1 HC-CDR3 |
| 43 | KASQNVGTNVA | SJ25C1 LC-CDR1 |
| 44 | SATYRNS | SJ25C1 LC-CDR2 |
| 45 | QQYNRYPYT | SJ25C1 LC-CDR3 |
| 46 | DYGVS | FMC63 HC-CDR1 |
| 47 | VIWGSETTYYNSALKS | FMC63 HC-CDR2 |
| 48 | HYYYGGSYAMDY | FMC63 HC-CDR3 |
| 49 | RASQDISKYLN | FMC63 LC-CDR1 |
| 50 | HTSRLHS | FMC63 LC-CDR2 |
| 51 | QQGNTLPYT | FMC63 LC-CDR3 |
| 52 | QVQLQQPGSELVRPGGSVKLSCKASDYTFTSYWMHWVRQRPGQGL EWIGNIYPGSGGTNYDEKFKRKATLTVDTSSSTAYMQLRSLTSEDSA VYYCTREVTTVAYYYSMDYWGQGTSVTVSS | anti-ID VH |
| 53 | EVTTVAYYYSMDY | anti-ID HC-CDR3 |
| 54 | TREVTTVAYYYSMD | anti-ID HC-CDR3 |
| 55 | SYWMH | anti-ID HC-CDR1 |
| 56 | DYTFTSY | anti-ID HC-CDR1 |
| 57 | DYTFTSYWMH | anti-ID HC-CDR1 |
| 58 | TSYWMH | anti-ID HC-CDR1 |
| 59 | NIYPGSGGTNYDEKFKR | anti-ID HC-CDR2 |
| 60 | YPGSGG | anti-ID HC-CDR2 |
| 61 | NIYPGSGGTN | anti-ID HC-CDR2 |
| 62 | WIGNIYPGSGGTN | anti-ID HC-CDR2 |
| 63 | DIQMTQTTSSLSASLGDRVTISCRASQDISNYLNWYQQKPDGTVKLL IYYTSRLHSGVPSRFSGSGSGTDYSLTISNLEQEDIATYFCQQGKTVP FTFGSGTKLEIK | anti-ID VL |
| 64 | QQGKTVPFT | anti-ID LC-CDR3 |
| 65 | QQGKTVPF | anti-ID LC-CDR3 |
| 66 | RASQDISNYLN | anti-ID LC-CDR1 |
| 67 | SNYLNWY | anti-ID LC-CDR1 |
| 68 | YTSRLHS | anti-ID LC-CDR2 |
| 69 | LLIYYTSRLH | anti-ID LC-CDR2 |
| 70 | GGGS | 3GS linker |
| 71 | GGGGSGGGGSGGGGS | Linker |
| 72 | GSTSGSGKPGSGEGSTKG | Linker |
| 73 | EVQLQQSGPELVKPGASVKMSCKASGYTFTDYYMKWVKQCHGKS LEWIGDINPNNGGTDYNQNFKGKATLTVDKSSSTAYMQLNSLTSED SAVYYCAREGNNYGSRDAMDYWGQGTSVTVSS | anti-ID B-1 VH |
| 74 | QVQLQQPGAELVRPGASVKLSCKTSGYSFTRYWMNWVKQRPGQG LEWIGMIHPSDSETRLNQKFKDKATLTVDNSSSTAYMQLSSPTSEDS AVYYCASIYYEEAWGQGTLVTVSA | anti-ID B-2 VH |
| 75 | EGNNYGSRDAMDY | anti-ID B-1 HC-CDR3 |
| 76 | IYYEEA | anti-ID B-2 HC-CDR3 |
| 77 | AREGNNYGSRDAMD | anti-ID B-1 HC-CDR3 |
| 78 | ASIYYEE | anti-ID B-2 HC-CDR3 |
| 79 | DYYMK | anti-ID B-1 HC-CDR1 |
| 80 | RYWMN | anti-ID B-2 HC-CDR1 |
| 81 | GYTFTDY | anti-ID B-1 HC-CDR1 |
| 82 | GYTFTDYYMK | anti-ID B-1 HC-CDR1 |
| 83 | TDYYMK | anti-ID B-1 HC-CDR1 |
| 84 | GYSFTRY | anti-ID B-2 HC-CDR1 |
| 85 | GYSFTRYWMN | anti-ID B-2 HC-CDR1 |
| 86 | TRYWMN | anti-ID B-2 HC-CDR1 |
| 87 | DINPNNGGTDYNQNFKG | anti-ID B-1 HC-CDR2 |
| 88 | MIHPSDSETRLNQKFKD | anti-ID B-2 HC-CDR2 |
| 89 | NPNNGG | anti-ID B-1 HC-CDR2 |
| 90 | DINPNNGGTD | anti-ID B-1 HC-CDR2 |
| 91 | WIGDINPNNGGTD | anti-ID B-1 HC-CDR2 |
| 92 | HPSDSE | anti-ID B-2 HC-CDR2 |
| 93 | MIHPSDSETR | anti-ID B-2 HC-CDR2 |
| 94 | WIGMIHPSDSETR | anti-ID B-2 HC-CDR2 |
| 95 | GYX₃FX₅X₆YX₈MX₁₀ | HC-CDR1 Consensus |
| | X₃ = T or S; | |
| | X₅ = T or S; | |
| | X₆ = D or R; | |
| | X₈ = Y or W; | |
| | X₁₀ = K or N | |
| 96 | WIGX₄IX₆PX₈X₉X₁₀X₁₁TX₁₃X₁₄NQX₁₇FKX₂₀ | HC-CDR2 Consensus |
| | X₄ = D or M; | |
| | X₆ = N or H; | |
| | X₈ = N or S; | |
| | X₉ = N or D; | |
| | X₁₀ = G or S; | |
| | X₁₁ = G or E; | |
| | X₁₃ = D or R; | |
| | X₁₄ = Y or L; | |
| | X₁₇ = N or K; | |
| | X₂₀ = G or D | |
| 97 | AX₂X₃X₄X₅X₆ X₇X₈ X₉ X₁₀X₁₁ X₁₂ X₁₃ X₁₄ X₁₅ | HC-CDR3 Consensus |
| | X₂ = R or S; | |
| | X₃ = E or I; | |
| | X₄ = G or Y; | |
| | X₅ = N or Y; | |
| | X₆ = N or E; | |
| | X₇ = Y or null; | |
| | X₈ = G or null; | |
| | X₉ = S or null; | |
| | X₁₀ = R or null; | |
| | X₁₁ = D or null; | |
| | X₁₂ = A or null; | |
| | X₁₃ = M or null; | |
| | X₁₄ = D or E; | |
| | X₁₅ = Y or A; | |
| 98 | QIVLTQSPALMSASPGEKVTMTCSASSGVIYMYWYQQKPRSSPKPW IYLTSNLASGVPARFSGSGSGTSYSLTISSMEAEDAATYYCQQWSSN PLTFGAGTKLELK | anti-ID B-1 VL |
| 99 | DIQMTQSPASLSASVGETVTITCRASGNIHNYLAWYQQKQGKSPQLL VYNAKTLADSVPSRFSGSGSGTQYSLKINSLQPEDFGSYYCQHFWST PYTFGGGTKLEIK | anti-ID B-2 VL |
| 100 | QQWSSNPLT | anti-ID B-1 LC-CDR3 |
| 101 | QQWSSNPL | anti-ID B-1 LC-CDR3 |
| 102 | QHFWSTPYT | anti-ID B-2 LC-CDR3 |
| 103 | QHFWSTPY | anti-ID B-2 LC-CDR3 |
| 104 | SASSGVIYMY | anti-ID B-1 LC-CDR1 |
| 105 | RASGNIHNYLA | anti-ID B-2 LC-CDR1 |
| 106 | IYMYWY | anti-ID B-1 LC-CDR1 |
| 107 | HNYLAWY | anti-ID B-2 LC-CDR1 |
| 108 | LTSNLAS | anti-ID B-1 LC-CDR2 |
| 109 | NAKTLAD | anti-ID B-2 LC-CDR2 |
| 110 | PWIYLTSNLA | anti-ID B-1 LC-CDR2 |
| 111 | LLVYNAKTLA | anti-ID B-2 LC-CDR2 |
| 112 | X₁AX₃X₄X₃X₆ X₇X₈ YX₁₀X₁₁WY | LC-CDR1 Consensus |
| | X₁ = S or R; | |
| | X₃ = S or R; | |
| | X₄ = S or G; | |
| | X₅ = G or N; | |
| | X₆ = V or I; | |
| | X₇ = I or H; | |
| | X₈ = N or null; | |
| | X₁₀ = M or L; | |
| | X₁₁ = Y or A | |
| 113 | X₁X₂X₃YX₅X₆ X₇X₈ LAX₁₁ | LC-CDR2 Consensus |
| | X₁ = P or L; | |
| | X₂ = W or L; | |
| | X₃ = I or V; | |
| | X₅ = L or N; | |
| | X₆ = T or A; | |
| | X₇ = S or K; | |
| | X₈ = N or T; | |
| | X₁₁ = S or D | |
| 114 | QX₂X₃X₄X₅X₆PX₈T | LC-CDR3 Consensus |
| | X₂ = Q or H; | |
| | X₃ =W or F; | |
| | X₄ = S or W; | |
| | X₅ = S or W; | |
| | X₆ = N or T; | |
| | X₈ = L or Y | |
| 115 | AKTTPPSVYPLAPGSAAQTNSMVTLGCLVKGYFPEPVTVTWNSGSL SSGVHTFPAVLQSDLYTLSSSVTVPSSTWPSETVTCNVAHPASSTKV DKKIVPRDCGCKPCICTVPEVSSVFIFPPKPKDVLTITLTPKVTCVVV DISKDDPEVQFSWFVDDVEVHTAQTQPREEQFNSTFRSVSELPIMHQ DWLNGKEFKCRVNSAAFPAPIEKTISKTKGRPKAPQVYTIPPPKEQM AKDKVSLTCMITDFFPEDITVEWQWNGQPAENYKNTQPIMDTDGSY FVYSKLNVQKSNWEAGNTFTCSVLHEGLHNHHTEKSLSHSPGK | anti-ID CH |
| 116 | QVQLQQPGSELVRPGGSVKLSCKASDYTFTSYWMHWVRQRPGQGL EWIGNIYPGSGGTNYDEKFKRKATLTVDTSSSTAYMQLRSLTSEDSA VYYCTREVTTVAYYYSMDYWGQGTSVTVSSAKTTPPSVYPLAPGS AAQTNSMVTLGCLVKGYFPEPVTVTWNSGSLSSGVHTFPAVLQSDL YTLSSSVTVPSSTWPSETVTCNVAHPASSTKVDKKIVPRDCGCKPCIC TVPEVSSVFIFPPKPKDVLTITLTPKVTCVVVDISKDDPEVQFSWFVD DVEVHTAQTQPREEQFNSTFRSVSELPIMHQDWLNGKEFKCRVNSA AFPAPIEKTISKTKGRPKAPQVYTIPPPKEQMAKDKVSLTCMITDFFP EDITVEWQWNGQPAENYKNTQPIMDTDGSYFVYSKLNVQKSNWEA GNTFTCSVLHEGLHNHHTEKSLSHSPGK | anti-ID heavy chain |
| 117 | MGWSSIILFLVATASGVHS | anti-ID HC signal sequence |
| 118 | RADAAPTVSIFPPSSEQLTSGGASVVCFLNNFYPKDINVKWKIDGSER QNGVLNSWTDQDSKDSTYSMSSTLTLTKDEYERHNNYTCEATHKT STSPIVKSFNRNEC | anti-ID CL |
| 119 | DIQMTQTTSSLSASLGDRVTISCRASQDISNYLNWYQQKPDGTVKLL IYYTSRLHSGVPSRFSGSGSGTDYSLTISNLEQEDIATYFCQQGKTVP FTFGSGTKLEIKRADAAPTVSIFPPSSEQLTSGGASVVCFLNNFYPKDI NVKWKIDGSERQNGVLNSWTDQDSKDSTYSMSSTLTLTKDEYERH NNYTCEATHKTSTSPIVKSFNRNEC | anti-ID light chain |
| 120 | MMSSAQFLGLLLLCFQGTRC | anti-ID LC signal sequence |
| 121 | AKTTAPSVYPLAPVCGDTTGSSVTLGCLVKGYFPEPVTLTWNSGSLS SGVHTFPAVLQSDLYTLSSSVTVTSSTWPSQSITCNVAHPASSTKVD | anti-ID B-1 CH |
| | KKIEPRGPTIKPCPPCKCPAPNLLGGPSVFIFPPKIKDVLMISLSPIVTC VVVDVSEDDPDVQISWFVNNVEVHTAQTQTHREDYNSTLRVVSAL PIQHQDWMSGKEFKCKVNNKDLPAPIERTISKPKGSVRAPQVYVLPP PEEEMTKKQVTLTCMVTDFMPEDIYVEWTNNGKTELNYKNTEPVL DSDGSYFMYSKLRVEKKNWVERNSYSCSVVHEGLHNHHTTKSFSR TPGK | |
| 122 | EVQLQQSGPELVKPGASVKMSCKASGYTFTDYYMKWVKQCHGKS LEWIGDINPNNGGTDYNQNFKGKATLTVDKSSSTAYMQLNSLTSED SAVYYCAREGNNYGSRDAMDYWGQGTSVTVSSAKTTAPSVYPLAP VCGDTTGSSVTLGCLVKGYFPEPVTLTWNSGSLSSGVHTFPAVLQSD LYTLSSSVTVTSSTWPSQSITCNVAHPASSTKVDKKIEPRGPTIKPCPP CKCPAPNLLGGPSVFIFPPKIKDVLMISLSPIVTCVVVDVSEDDPDVQI SWFVNNVEVHTAQTQTHREDYNSTLRVVSALPIQHQDWMSGKEFK CKVNNKDLPAPIERTISKPKGSVRAPQVYVLPPPEEEMTKKQVTLTC MVTDFMPEDIYVEWTNNGKTELNYKNTEPVLDSDGSYFMYSKLRV EKKNWVERNSYSCSVVHEGLHNHHTTKSFSRTPGK | anti-ID B-1 heavy chain |
| 123 | MGWSWIFLFLLSGTAGVLS | anti-ID B-1 HC signal sequence |
| 124 | RADAAPTVSIFPPSSEQLTSGGASVVCFLNNFYPKDINVKWKIDGSER QNGVLNSWTDQDSKDSTYSMSSTLTLTKDEYERHNSYTCEATHKTS TSPIVKSFNRNEC | anti-ID B-1 CL |
| 125 | QIVLTQSPALMSASPGEKVTMTCSASSGVIYMYWYQQKPRSSPKPW IYLTSNLASGVPARFSGSGSGTSYSLTISSMEAEDAATYYCQQWSSN PLTFGAGTKLELKRADAAPTVSIFPPSSEQLTSGGASVVCFLNNFYPK DINVKWKIDGSERQNGVLNSWTDQDSKDSTYSMSSTLTLTKDEYER HNSYTCEATHKTSTSPIVKSFNRNEC | anti-ID B-1 light chain |
| 126 | MDFQVQIFSFLLMSASVIMSRG | anti-ID B-1 LC signal sequence |
| 127 | AKTTPPSVYPLAPGSAAQTNSMVTLGCLVKGYFPEPVTVTWNSGSL SSGVHTFPAVLQSDLYTLSSSVTVPSSTWPSETVTCNVAHPASSTKV DKKIVPRDCGCKPCICTVPEVSSVFIFPPKPKDVLTITLTPKVTCVVV DISKDDPEVQFSWFVDDVEVHTAQTQPREEQFNSTFRSVSELPIMHQ DWLNGKEFKCRVNSAAFPAPIEKTISKTKGRPKAPQVYTIPPPKEQM AKDKVSLTCMITDFFPEDITVEWQWNGQPAENYKNTQPIMDTDGSY FVYSKLNVQKSNWEAGNTFTCSVLHEGLHNHHTEKSLSHSPGK | anti-ID B-2 CH |
| 128 | QVQLQQPGAELVRPGASVKLSCKTSGYSFTRYWMNWVKQRPGQG LEWIGMIHPSDSETRLNQKFKDKATLTVDNSSSTAYMQLSSPTSEDS AVYYCASIYYEEAWGQGTLVTVSAAKTTPPSVYPLAPGSAAQTNSM VTLGCLVKGYFPEPVTVTWNSGSLSSGVHTFPAVLQSDLYTLSSSVT VPSSTWPSETVTCNVAHPASSTKVDKKIVPRDCGCKPCICTVPEVSS VFIFPPKPKDVLTITLTPKVTCVVVDISKDDPEVQFSWFVDDVEVHT AQTQPREEQFNSTFRSVSELPIMHQDWLNGKEFKCRVNSAAFPAPIE KTISKTKGRPKAPQVYTIPPPKEQMAKDKVSLTCMITDFFPEDITVE WQWNGQPAENYKNTQPIMDTDGSYFVYSKLNVQKSNWEAGNTFT CSVLHEGLHNHHTEKSLSHSPGK | anti-ID B-2 heavy chain |
| 129 | MGWSSIILFLVATATGVHS | anti-ID B-2 HC signal sequence |
| 130 | DIQMTQSPASLSASVGETVTITCRASGNIHNYLAWYQQKQGKSPQLL VYNAKTLADSVPSRFSGSGSGTQYSLKINSLQPEDFGSYYCQHFWST PYTFGGGTKLEIK | anti-ID B-2 VL |
| 131 | DIQMTQSPASLSASVGETVTITCRASGNIHNYLAWYQQKQGKSPQLL VYNAKTLADSVPSRFSGSGSGTQYSLKINSLQPEDFGSYYCQHFWST | anti-ID B-2 light chain |
| | PYTFGGGTKLEIKRADAAPTVSIFPPSSEQLTSGGASVVCFLNNFYPK DINVKWKIDGSERQNGVLNSWTDQDSKDSTYSMSSTLTLTKDEYER HNSYTCEATHKTSTSPIVKSFNRNEC | |
| 132 | MSVLTQVLALLLLWLTGARC | anti-ID B-2 LC signal sequence |
| 133 | ESKYGPPCPPCP | spacer (IgG4hinge) (aa) Homo sapiens |
| 134 | GAATCTAAGTACGGACCGCCCTGCCCCCCTTGCCCT | spacer (IgG4hinge) (nt) Homo sapiens |
| 135 | ESKYGPPCPPCPGQPREPQVYTLPPSQEEMTKNQVSLTCLVKGFYPS DIAVEWESNGQPENNYKTTPPVLDSDGSFFLYSRLTVDKSRWQEGN VFSCSVMHEALHNHYTQKSLSLSLGK | Hinge-CH3 spacer Homo sapiens |
| 136 | ESKYGPPCPPCPAPEFLGGPSVFLFPPKPKDTLMISRTPEVTCVVVDV SQEDPEVQFNWYVDGVEVHNAKTKPREEQFNSTYRVVSVLTVLHQ DWLNGKEYKCKVSNKGLPSSIEKTISKAKGQPREPQVYTLPPSQEEM TKNQVSLTCLVKGFYPSDIAVEWESNGQPENNYKTTPPVLDSDGSFF LYSRLTVDKSRWQEGNVFSCSVMHEALHNHYTQKSLSLSLGK | Hinge-CH2-CH3 spacer Homo sapiens |
| 137 | RWPESPKAQASSVPTAQPQAEGSLAKATTAPATTRNTGRGGEEKKK EKEKEEQEERETKTPECPSHTQPLGVYLLTPAVQDLWLRDKATFTCF VVGSDLKDAHLTWEVAGKVPTGGVEEGLLERHSNGSQSQHSRLTLP RSLWNAGTSVTCTLNHPSLPPQRLMALREPAAQAPVKLSLNLLASS DPPEAASWLLCEVSGFSPPNILLMWLEDQREVNTSGFAPARPPPQPG STTFWAWSVLRVPAPPSPQPATYTCVVSHEDSRTLLNASRSLEVSYV TDH | IgD-hinge-Fc Homo sapiens |
| 138 | FWVLVVVGGVLACYSLLVTVAFIIFWV | CD28 (amino acids 153-179 of Accession No. P10747) Homo sapiens |
| 139 | IEVMYPPPYLDNEKSNGTIIHVKGKHLCPSPLFPGPSKP FWVLVVVGGVLACYSLLVTVAFIIFWV | CD28 (amino acids 114-179 of Accession No. P10747) Homo sapiens |
| 140 | RSKRSRLLHSDYMNMTPRRPGPTRKHYQPYAPPRDFAAYRS | CD28 (amino acids 180-220 of P10747) Homo sapiens |
| 141 | RSKRSRGGHSDYMNMTPRRPGPTRKHYQPYAPPRDFAAYRS | CD28 (LL to GG) Homo sapiens |
| 142 | KRGRKKLLYIFKQPFMRPVQTTQEEDGCSCRFPEEEEGGCEL | 4-1BB (amino acids 214-255 of Q07011.1) Homo sapiens |
| 143 | RVKFSRSADAPAYQQGQNQLYNELNLGRREEYDVLDKRRGRDPEM GGKPRRKNPQEGLYNELQKDKMAEAYSEIGMKGERRRGKGHDGL YQGLSTATKDTYDALHMQALPPR | CD3 zeta Homo sapiens |
| 144 | RVKFSRSAEPPAYQQGQNQLYNELNLGRREEYDVLDKRRGRDPEM GGKPRRKNPQEGLYNELQKDKMAEAYSEIGMKGERRRGKGHDGL YQGLSTATKDTYDALHMQALPPR | CD3 zeta Homo sapiens |
| 145 | RVKFSRSADAPAYKQGQNQLYNELNLGRREEYDVLDKRRGRDPEM | CD3 zeta Homo |
| | GGKPRRKNPQEGLYNELQKDKMAEAYSEIGMKGERRRGKGHDGL YQGLSTATKDTYDALHMQALPPR | sapiens |
| 146 | LEGGGEGRGSLLTCGDVEENPGPR | T2A artificial |
| 147 | MLLLVTSLLLCELPHPAFLLIPRKVCNGIGIGEFKDSLSINATNIKHFK NCTSISGDLHILPVAFRGDSFTHTPPLDPQELDILKTVKEITGFLLIQA WPENRTDLHAFENLEIIRGRTKQHGQFSLAVVSLNITSLGLRSLKEIS DGDVIISGNKNLCYANTINWKKLFGTSGQKTKIISNRGENSCKATGQ VCHALCSPEGCWGPEPRDCVSCRNVSRGRECVDKCNLLEGEPREFV ENSECIQCHPECLPQAMNITCTGRGPDNCIQCAHYIDGPHCVKTCPA GVMGENNTLVWKYADAGHVCHLCHPNCTYGCTGPGLEGCPTNGP KIPSIATGMVGALLLLLVVALGIGLFM | tEGFR artificial |
| 148 | RKVCNGIGIGEFKDSLSINATNIKHFKNCTSISGDLHILPVAFRGDSFT HTPPLDPQELDILKTVKEITGFLLIQAWPENRTDLHAFENLEIIRGRTK QHGQFSLAVVSLNITSLGLRSLKEISDGDVIISGNKNLCYANTINWKK LFGTSGQKTKIISNRGENSCKATGQVCHALCSPEGCWGPEPRDCVSC RNVSRGRECVDKCNLLEGEPREFVENSECIQCHPECLPQAMNITCTG RGPDNCIQCAHYIDGPHCVKTCPAGVMGENNTLVWKYADAGHVC HLCHPNCTYGCTGPGLEGCPTNGPKIPSIATGMVGALLLLLVVALGI GLFM | tEGFR artificial |
| 149 | EGRGSLLTCGDVEENPGP | T2A artificial |
| 150 | PLGLWA | MMP cleavable linker |
| 151 | GSGATNFSLLKQAGDVEENPGP | P2A |
| 152 | ATNFSLLKQAGDVEENPGP | P2A |
| 153 | QCTNYALLKLAGDVESNPGP | E2A |
| 154 | VKQTLNFDLLKLAGDVESNPGP | F2A |

The invention further provides the following non-limiting embodiments:
1. A method of expanding cells, comprising incubating an input composition, said input composition comprising cells expressing a chimeric antigen receptor comprising an extracellular antigen-binding domain that specifically binds or recognizes an antigen, with a plurality of particles that are or comprise beads, wherein the plurality of particles comprise a mean diameter of between or between about 2 µm and 5 µm and have attached a binding molecule that specifically binds to or recognizes the antigen-binding domain, wherein binding of the binding molecule to the antigen-binding domain induces expansion of the cells comprising the chimeric antigen receptor, thereby producing an output composition comprising expanded cells.
2. The method of embodiment 1, wherein the binding molecule does not bind or recognize a linker or spacer region of the chimeric antigen receptor, said linker or spacer region connecting the antigen-binding domain to the transmembrane domain of the antigen receptor .
3. The method of embodiment 1 or embodiment 2, wherein the binding molecule is an anti-idotypic antibody or antigen-binding fragment thereof that specifically binds to the antigen-binding domain.
4. The method of any of embodiments 1-3, wherein the antigen is CD19.
5. The method of embodiment 1 or embodiment 2, wherein the binding molecule comprises a recombinant antigen or a portion thereof recognized by the antigen-binding domain.
6. The method of embodiment 5, wherein the recombinant antigen is BCMA or is a portion thereof recognized by the antigen-binding domain.
7. A method of expanding cells, comprising incubating an input composition, said input composition comprising cells expressing a chimeric antigen receptor (CAR) comprising an antigen-binding domain that specifically binds or recognizes B cell maturation antigen (BCMA), with a plurality of particles having attached a binding molecule comprising the extracellular domain of BCMA or a portion of the extracellular domain recognized by the antigen-binding domain, wherein binding of the extracellular domain of BCMA or portion thereof to the antigen-binding domain induces expansion of the cells comprising the chimeric antigen receptor, thereby producing an output composition comprising expanded cells.
8. The method of any of embodiments 5-7, wherein the binding molecule is a fusion polypeptide comprising the recombinant antigen or the portion thereof linked to a moiety, optionally wherein the moiety facilitates attachment to the particle.
9. The method of embodiment 8, wherein the moiety is linked to the C-terminus of the recombinant antigen.
10. The method of embodiment 8 or embodiment 9, wherein the moiety is or comprises an Fc domain.
11. A method of expanding cells, comprising incubating an input composition, said input composition comprising cells expressing a chimeric antigen receptor (CAR) comprising an antigen-binding domain that specifically binds or recognizes CD19, with a plurality of particles, having attached a binding molecule that is an anti-idiotypic antibody or antigen-binding fragment thereof that specifically binds to the antigen-binding domain, wherein binding of the anti-idiotypic antibody or antigen-binding fragment thereof to the antigen-binding domain induces expansion of the cells comprising the chimeric antigen receptor, thereby producing an output composition comprising expanded cells.
12. The method of embodiments 3, 4 or embodiment 11, wherein the antigen-binding domain of the antigen receptor is or comprises antibody SJ25C1 or an antigen-binding fragment thereof.
13. The method of embodiments 3, 4 or embodiment 11, wherein the antigen-binding domain of the antigen receptor is or comprises antibody FMC63 or an antigen-binding fragment thereof.
14. The method of any of embodiments 7-13, wherein the particles are synthetic particles, insoluble particles, solid particles or are non-cellular particles.
15. The method of any of embodiments 7-14, wherein the plurality of particles comprises beads.
16. The method of any of embodiments 1-15, wherein the plurality of particles are from a composition having a concentration of the binding molecule of between or between about 0.5 µg/mL and 500 µg/mL, inclusive.
17. The method of any of embodiments 1-16, wherein, during the incubating, the ratio of total cells present in the input composition to the plurality of particles is from or from about 5:1 to 1:5, inclusive.
18. A method of expanding cells, comprising incubating an input composition, said input composition comprising cells expressing a chimeric antigen receptor comprising an extracellular antigen-binding domain that specifically binds or recognizes an antigen, with a plurality of particles that are or comprise beads having attached a binding molecule that specifically binds to or recognizes the antigen-binding domain, wherein:
   the plurality of particles are from a composition having a concentration of the binding molecule of between or between about 0.5 µg/mL and 500 µg/mL, inclusive, and, during the incubating, the ratio of total cells present in the input composition to the plurality of particles is from or from about 5:1 to 1:5, inclusive; and
   binding of the binding molecule to the antigen-binding domain induces expansion of the cells comprising the chimeric antigen receptor, thereby producing an output composition comprising expanded cells.
19. The method of embodiment 18, wherein the binding molecule does not bind or recognize a linker or spacer region of the chimeric antigen receptor, said linker or spacer region connecting the antigen-binding domain to the transmembrane domain of the antigen receptor .
20. The method of embodiment 18 or 19, wherein the binding molecule is an anti-idiotypic antibody or antigen-binding fragment thereof that specifically binds to the antigen-binding domain.
21. The method of embodiment 20, wherein the antigen is CD19.
22. The method of embodiment 18 or embodiment 19, wherein the binding molecule comprises a recombinant antigen or a portion thereof recognized by the antigen-binding domain.
23. The method of embodiment 22, wherein the recombinant antigen is BCMA or is a portion thereof recognized by the antigen-binding domain.
24. The method of any of embodiments 1-23, wherein:
   the plurality of particles are from a composition having a concentration of the binding molecule of between or between about 1 µg/mL and 200 µg/mL or 5 µg/mL and 100 µg/mL, each inclusive; or
   the plurality of particles are from a composition comprising a concentration of the binding molecule of at least or at least about 1 µg/mL, 5 µg/mL, 10 µg/mL, 25 µg/mL, 50 µg/mL, 100 µg/mL or 200 µg/mL.
25. The method of any of embodiments 1-24, wherein the plurality of particles are from a composition having a concentration of the binding molecule of or about 5 µg/mL, 10 µg/mL, 25 µg/mL, 50 µg/mL, 100 µg/mL or 200 µg/mL.
26. The method of any of embodiments 1-25, wherein, during the incubation, the ratio of total cells present in the input composition to the plurality of particles is from or from about 3:1 to 1:3 or 2:1 to 1:2, each inclusive.
27. The method of any of embodiments 1-26, wherein, during the incubation, the ratio of total cells present in the input composition to the plurality of particles is or is about 1:1.
28. The method of any of embodiments 15-27, wherein the plurality of beads comprise a mean diameter of between or between about 2 µm and 5 µm or a mean density of between or between about 1 g/cm³ and about 2 g/cm³.
29. The method of any of embodiments 1-6 and 28, wherein the mean diameter is of or of about 2.8 µm or 4.5 µm.
30. The method of any of embodiments 3, 4, 11-17, 20, 21 and 24-29, wherein the antigen-binding fragment is or comprises an scFv.
31. The method of any of embodiments 3, 4 and 11-30, wherein the anti-idiotypic antibody or antigen-binding fragment thereof comprises at least a portion of an immunoglobulin constant region, optionally an Fc region or a portion of the Fc comprising the CH2 and CH3 domains.
32. The method of any one of embodiments 3, 4, 11-17, 20, 21, and 24-31, wherein the anti-idiotypic antibody or antigen-binding fragment thereof is an intact antibody or full-length antibody.
33. The method of any of embodiments 1-32, wherein the binding molecule is attached to each of the plurality of particles at or near the C-terminal amino acid residue of the binding molecule.
34. The method of any of embodiments 1-33, wherein the plurality of particles is monodisperse.
35. The method of any of embodiments 1-34, wherein the binding molecule is covalently attached to the particle via a surface exposed functional group.
36. The method of embodiment 35, wherein the surface exposed functional group is an amino group, a carboxyl group, a thiol group, an aldehyde group, a chloromethyl group, an epoxy group, a hydroxyl group, a tosyl group or a hydrazine group.
37. The method of embodiment 35 or embodiment 36, wherein the surface exposed functional group is a tosyl group.
38. The method of any of embodiments 1-37, wherein the plurality of particles comprise particles comprising glass, silica, polyesters of hydroxy carboxylic acids, polyanhydrides of dicarboxylic acids, copolymers of hydroxy carboxylic acids, copolymers dicarboxylic acids, or metal.
39. The method of any of embodiments 1-38, wherein the particles comprise a surface comprising a polymer, a polysaccharide, a silica, a fatty acid, a carbon or a combination thereof.
40. The method of embodiment 39, wherein the polymer is polyethylene glycol, poly(lactic-co-glycolic acid), polyglutaraldehyde, polyurethane, polystyrene, and polyvinyl alcohol or combinations thereof.
41. The method of any of embodiments 1-40, wherein the plurality of particles comprise particles comprising a hydrophobic surface.
42. The method of any of embodiments 1-41, wherein the plurality of particles comprise particles comprising a polystyrene surface.
43. The method of any of embodiments 1-42, the plurality of particles comprises particles that are magnetic and/or comprise a magnetic core, a paramagnetic core or a superparamagnetic core.
44. The method of any of embodiments 1-43, wherein at least a portion of the incubation is performed in the presence of an agent that specifically binds to an additional molecule on the cell to provide an accessory signal and/or to block an inhibitory signal.
45. The method of embodiment 44, wherein the agent is provided together with the particles, optionally the agent is attached to each of the plurality of particles or a subset thereof.
46. The method of embodiment 44 or embodiment 45, wherein the agent is attached to the particles and the agent specifically binds to an additional molecule on the cell to provide an accessory signal and/or to block an inhibitory signal.
47. The method of any of embodiments 44-46, wherein the agent is a ligand or is an antibody or antigen-binding fragment thereof.
48. The method of any of embodiments 44-47, wherein the additional molecule:
   is a costimulatory molecule or is an activating co-receptor, optionally OX-40, ICOS, DAP10, B7-1, B7-2, CD28 or 4-1BB; or
   is an inhibitory receptor, optionally CTLA-4, PD-1, LAG-3, Tim-3, BTLA or TIGIT; or is a T cell surface protein, optionally CD2 or CD3.
49. The method of any of embodiments 44-48, wherein the additional molecule is CD2 or CD28.
50. The method of any of embodiments 44-49, wherein the particles comprise one or both of ananti-CD2 antibody and an anti-CD28 antibody.
51. The method of embodiment 50, wherein the binding molecule is an anti-CD19 anti-idiotype antibody.
52. The method of embodiment 50, wherein the binding molecule is a recombinant antigen that is recognized by the chimeric antigen receptor, optionally wherein the recombinant antigen is BCMA or is a portion thereof recognized by the antigen-binding domain.
53. The method of any of embodiments 45-52, wherein the agent is covalently attached to the particles.
54. The method of any of embodiments 45-53, wherein the ratio, optionally molar or weight ratio, of the binding molecule and the agent attached to the particles is or is about 1:1.
55. The method of any of embodiments 1-54, wherein the incubation is carried out for greater than or greater than about 2 hours, 4 hours, 8 hours, 12 hours, 24 hours, 2 days, 3 days, 4 days, 5 days, 6 days, 7 days, 8 days, 9 days, 10 days, 11 days or 12 days.
56. The method of any of embodiments 1-55, wherein the incubation is carried out for greater than or greater than about 24 hours, 2 days, 3 days, 4 days, 5 days, 6 days, 7 days, 8 days, 9 days, 10 days, 11 days or 12 days.
57. The method of any of embodiments 1-56, wherein the incubation is carried out for greater than or greater than 10 days, 11 days, 12 days, 13 days, 14 days, 15 days, or 16 days.
58. The method of any of embodiments 1-57, wherein the cells comprise immune cells or induced pluripotent stem cells (iPSC).
59. The method of any of embodiments 1-58, wherein the immune cell is a T cell or an NK cell.
60. The method of any of embodiments 1-59, wherein the cells comprise one or both of CD4+ T cells and CD8+ T cells.
61. The method of any of embodiments 1-60, wherein the ratio of the CD4+ cells to the CD8+ cells is between or between about 1:2 and 2:1, or 1:3 and 3:1, or is or is about 1:1.
62. The method of any of embodiments 1-61, wherein the cells are primary cells obtained from a subject, optionally a human subject.
63. The method of any of embodiments 1-62, wherein the input composition is produced by a method comprising contacting a composition of cells with a nucleic acid molecule encoding the chimeric antigen receptor under conditions to introduce the nucleic acid molecule into one or more cells in the composition.
64. A method of genetically engineering a cell, comprising:
   (a) contacting a composition of cells with a nucleic acid molecule encoding a chimeric antigen receptor under conditions to introduce the nucleic acid molecule into one or more cells in the composition, thereby producing an input composition; and
   (b) incubating cells of the input composition according to the method of any of embodiments 1-63.
65. The method of embodiment 63 or embodiment 64, wherein at least a portion of the contacting and incubating are carried out simultaneously.
66. The method of any of embodiments 63-65, wherein the contacting is carried out by transduction with a viral vector, optionally wherein the viral vector is a retroviral vector, a gamma-retroviral vector, or a lentiviral vector.
67. The method of any of embodiments 62-66, wherein the composition of cells comprises a plurality of T cells and, prior to the contacting, the method does not comprise stimulating or activating the T cells.
68. The method of any of embodiments 63-67, wherein the composition of cells comprises a plurality of T cells and, prior to the contacting, the method does not comprise incubating the composition in the presence of an agent or agents capable of inducing a signal through a TCR complex and/or incubation in the presence of an agent or agents capable of inducing proliferation of T cells, CD4+ T cells, and/or CD8+ T cells; and/or CD3-binding molecules, CD28-binding molecules, recombinant IL-2, recombinant IL-15, and recombinant IL-7 or a combination thereof, optionally wherein the method does not comprise stimulating the T cells in the presence of an anti-CD3 antibody and/or an anti-CD28 antibody.
69. The method of any of embodiments 1-68, wherein the surface expression of an activation marker or exhaustion marker of cells present in the output composition is less than surface expression of the marker in a composition of cells produced after a similar incubation but in the presence of a polyclonal stimulatory molecule capable of activating one or more intracellular signaling domains of one or more components of a TCR complex, optionally wherein the polyclonal stimulatory molecule comprises an anti-CD3 antibody or fragment and/or an anti-CD28 antibody or fragment.
70. The method of embodiment 69, wherein the exhaustion marker is an inhibitory receptor, optionally PD-1, CTLA-4, TIM-3, LAG-3, BTLA or TIGIT.
71. The method of embodiment 69, wherein the activation marker is HLA-DR, CD25, CD69, CD71, CD40L or 4-1BB.
72. The method of any of embodiments 1-71, wherein the number of cells in the output composition comprising the chimeric antigen receptor is increased or enriched by 1.2-fold, 2.0-fold, 3.0-fold, 4.0-fold, 5.0-fold, 6.0-fold, 7.0-fold, 8.0-fold, 9.0-fold, 10-fold or more compared to the number of the cells comprising the antigen receptor in the input composition.
73. The method of any of embodiments 1-72 that is performed in vitro or ex vivo.
74. The method of any of embodiments 1-73, wherein the CAR comprises an extracellular antigen-binding domain that specifically binds to the antigen, a transmembrane region and an intracellular signaling region comprising an ITAM.
75. The method of embodiment 74, wherein the intracellular signaling domain comprises an intracellular domain of a CD3-zeta (CD3ζ) chain.
76. The method of embodiment 74 or embodiment 75, wherein the CAR further comprises a costimulatory signaling region comprising a signaling domain of CD28 or 4-1BB.
77. The method of any of embodiments 1-76, further comprising removing the plurality of beads from the output composition.
78. A composition of cells produced by the method of any of embodiments 1-77.
79. A surface modified particle, comprising (i) a particle that is a bead comprising a diameter of between or between about 2 µm and 5 µm and (ii) and a binding molecule attached to the surface of the bead, wherein the binding molecule specifically binds to an extracellular antigen-binding domain of a chimeric antigen receptor.
80. The surface modified particle of embodiment 79, wherein the binding molecule does not bind or recognize a linker or spacer region of the recombinant antigen receptor, said linker or spacer region connecting the antigen-binding domain to the transmembrane domain of the antigen receptor.
81. The surface modified particle of embodiment 79 or embodiment 80, wherein the binding molecule comprises a recombinant antigen or a portion thereof recognized by the antigen-binding domain.
82. The surface modified particle of embodiment 81, wherein the recombinant antigen is BCMA, or is a portion thereof recognized by the antigen-binding domain.
83. A surface modified particle, comprising a particle and a binding molecule attached to the surface of the particle, wherein the binding molecule comprises an extracellular domain or a portion thereof of a recombinant antigen that is B cell maturation antigen (BCMA).
84. The surface modified particle of any of embodiments 81-83, wherein the binding molecule is a fusion polypeptide comprising the recombinant antigen or the portion thereof linked to a moiety, optionally wherein the moiety facilitates attachment to the particle.
85. The surface modified particle of embodiment 84, wherein the moiety is linked to the C-terminus of the recombinant antigen.
86. The surface modified particle of embodiment 84 or embodiment 85, wherein the moiety is or comprises an Fc domain.
87. The surface modified particle of embodiment 79 or embodiment 80, wherein the binding molecule comprises an anti-idiotypic antibody or antigen-binding fragment thereof.
88. The surface modified particle of any of embodiments 79, 80 and 87, wherein the antigen recognized by the antigen-binding domain is CD19.
89. The surface modified particle of embodiment 87 or embodiment 88, wherein the antigen-binding domain of the chimeric antigen receptor is or comprises antibody SJ25C1 or an antigen-binding fragment thereof.
90. The surface modified particle of embodiment 87 or embodiment 88, wherein the antigen-binding domain of the chimeric antigen receptor is or comprises antibody FMC63 or an antigen-binding fragment thereof.
91. The surface modified particle of embodiment 89 or embodiment 90, wherein the antigen-binding fragment is or comprises an scFv.
92. The surface modified particle of any of embodiments 87-91, wherein the anti-idiotypic antibody or antigen-binding fragment thereof comprises at least a portion of an immunoglobulin constant region, optionally an Fc region or a portion of the Fc comprising the CH2 and CH3 domains.
93. The surface modified particle of any one of embodiments 87-92, wherein the anti-idiotypic antibody or antigen-binding fragment thereof is an intact antibody or full-length antibody.
94. The surface modified particle of any of embodiments 79-93, wherein the binding molecule is attached to the particle at or near the C-terminal amino acid residue of the binding molecule.
95. The surface modified particle of any of embodiments 83-94, wherein the particle is a synthetic particle, insoluble particle, solid particle or a non-cellular particle.
96. The surface modified particle of any of embodiments 83-95, wherein the particle is a bead.
97. The surface modified particle of any of embodiments 79-96, wherein the particle has a diameter of between or between about 2 µm and 5 µm.
98. The surface modified particle of any of embodiments 79-97, wherein the particle has a diameter of or of about 2.8 µm or 4.5 µm.
99. The surface modified particle of any of embodiments 79-98, wherein the binding molecule is covalently attached to the particles.
100. The surface modified particle of any of embodiments 79-99, wherein the binding molecule is covalently attached to the particle via a surface exposed functional group.
101. The surface modified particle of embodiment 100, wherein the surface exposed functional group is an amino group, a carboxyl group, a thiol group, an aldehyde group, a chloromethyl group, an epoxy group, a hydroxyl group, a tosyl group or a hydrazine group.
102. The surface modified particle of embodiment 101, wherein the surface exposed functional group is a tosyl group.
103. The surface modified particle of any of embodiments 79-102, wherein the particle is or comprises glass, silica, polyesters of hydroxy carboxylic acids, polyanhydrides of dicarboxylic acids, copolymers of hydroxy carboxylic acids, copolymers dicarboxylic acids, or a metal.
104. The surface modified particle of any of embodiments 79-103, wherein the particle comprises a surface comprising a polymer, a polysaccharide, a silica, a fatty acid, a carbon or a combination thereof.
105. The surface modified particle of embodiment 104, wherein the polymer is polyethylene glycol, poly(lactic-co-glycolic acid), polyglutaraldehyde, polyurethane, polystyrene, and polyvinyl alcohol or combinations thereof.
106. The surface modified particle of any of embodiments 79-105, wherein the particle comprises a hydrophobic surface.
107. The surface modified particle of any of embodiments 79-106, wherein the particle comprises a polystyrene surface.
108. The surface modified particle of any of embodiments 79-107, the particle is magnetic and/or comprises a magnetic core, a paramagnetic core or a superparamagnetic core.
109. The surface modified particle of any of embodiments 79-108, wherein the particle comprises at least or about at least 10 copies, 10² copies, 10³ copies, 10⁴ copies, 10⁵ copies or 10⁶ copies of the binding molecule.
110. The surface modified particle of any of embodiments 79-109, wherein the particle further comprises at least one agent attached to the surface of the particle, wherein the at least one agent specifically binds to an additional molecule on a cell to provide an accessory signal and/or to block an inhibitory signal.
111. The surface modified particle of embodiment 110, wherein the at least one agent is a ligand or is an antibody or antigen-binding fragment thereof.
112. The surface modified particle of embodiment 110 or embodiment 111, wherein the additional molecule is a costimulatory molecule or an activating co-receptor, optionally OX-40, ICOS, DAP10, B7-1, B7-2, CD28 or 4-1BB.
113. The surface modified particle of embodiment 110 or embodiment 111, wherein the additional molecule is an inhibitory receptor, optionally CTLA-4, PD-1, LAG-3, Tim-3, BTLA or TIGIT.
114. The surface modified particle of embodiment 110 or embodiment 111, wherein the additional molecule is a T cell surface protein, optionally CD2 or CD3.
115. The surface modified particle of embodiment 110 or embodiment 111, wherein the additional molecule is CD2 or CD28.
116. The surface modified particle of embodiment 115, wherein the particle comprises one or both of an anti-CD2 antibody and an anti-CD28 antibody.
117. The surface modified particle of any of embodiments 110-116, wherein the at least one agent is covalently attached to the particle.
118. The surface modified particle of any of embodiments 110-117, wherein the ratio, optionally molar or weight ratio, of the binding molecule and the at least one agent comprised by the particle is or is about 1:1.
119. A composition, comprising a plurality of the surface modified particles of any of embodiments 79-118.
120. The composition of embodiment 119, wherein:
   the concentration of the binding molecule in the composition is between or between about 0.5 µg/mL and 500 µg/mL, 1 µg/mL and 200 µg/mL or 5 µg/mL and 100 µg/mL L; or
   the concentration of the binding molecule in the composition is at least or at least about 1 µg/mL, 5 µg/mL, 10 µg/mL, 25 µg/mL, 50 µg/mL, 100 µg/mL or 200 µg/mL..
121. The composition of embodiment 119 or embodiment 120, wherein the plurality of the surface modified particles is monodisperse.
122. A kit, comprising the surface modified particle of any of embodiments 79-118 or the composition of any of embodiments 119-121 and instructions for use.
123. The kit of embodiment 122, wherein instructions are for selecting or enriching, from a population of cells, cells expressing a chimeric antigen receptor comprising an antigen-binding domain specifically recognized by the binding molecule.
124. The kit of embodiment 122, wherein instructions are for stimulating or expanding, from a population of cells, cells expressing a chimeric antigen receptor comprising an antigen-binding domain specifically recognized by the binding molecule.
125. A method for expanding cells, comprising incubating a population of cells with the surface modified particle of any of embodiments 79-118 or the composition of any of embodiments 119-121.
126. A method of selecting or enriching cells, comprising contacting a population of cells with the surface modified particle of any of embodiments 79-118 or the composition of any of embodiments 119-121.
127. A long-term stimulation method for assessing a cell composition, the method comprising:
   incubating, for a period of time of at least 10 days, an input composition under conditions to stimulate a CAR-dependent activity in cells in the input composition, said input composition comprising T cells expressing a chimeric antigen receptor (CAR) comprising an extracellular antigen-binding domain that specifically binds or recognizes an antigen, thereby producing an output composition; and
   assessing one or more phenotype or activity of one or more cells of the output composition.
128. The method of embodiment 127, wherein the conditions to stimulate a CAR-dependent activity comprises the presence of a binding molecule that specifically binds to the antigen-binding domain of the CAR.
129. The method of embodiment 128, wherein the binding molecule is attached to a support.
130. The method of embodiment 129, wherein the support is a solid support.
131. The method of embodiment 130, wherein the solid support is the surface of a well of a microplate or a bead.
132. The method of embodiment 130 or embodiment 131, wherein the solid support is a microplate having the binding molecule attached to the microplate, and the incubation is carried out in the microplate.
133. The method of embodiment 130 or embodiment 131, wherein the solid support is a bead having attached the binding molecule, and the incubation is carried out in the presence of a plurality of the beads.
134. The method of any of embodiments 128-133, wherein the binding molecule is or comprises a recombinant antigen or a portion thereof recognized by the antigen-binding domain.
135. The method of embodiment 134, wherein the recombinant antigen or portion thereof is BCMA, or is a portion thereof recognized by the antigen-binding domain.
136. The method of any of embodiments 128-133, wherein the binding molecule is or comprises an anti-iditopytic antibody or antigen-binding fragment thereof that specifically binds to the antigen-binding domain.
137. The method of embodiment 136, wherein the antigen-binding domain of the antigen receptor is or comprises antibody SJ25C1 or an antigen-binding fragment thereof.
138. The method of embodiment 136, wherein the antigen-binding domain of the antigen receptor is or comprises antibody FMC63 or an antigen-binding fragment thereof.
139. The method of any of embodiments 127-138, wherein the method is carried out in vitro or ex vivo.
140. The method of any of embodiments 127-139, wherein the input composition is incubated in the presence of a media that does not comprise recombinant cytokines.
141. The method of any of embodiments 127-140, wherein the incubation is carried out continuously or is not interrupted for the period of time.
142. The method of any of embodiments 127-140, wherein during the incubation, cells are not replated, media is not changed and binding molecule is not added.
143. The method of any of embodiments 127-142, comprising assessing one or more phenotypes of activation, exhaustion or differentiation state of the one or more cells of the output composition.
144. The method of embodiment 143, wherein the phenotype is exhaustion and the assessing comprises measuring the expression, optionally surface expression, of one or more markers selected from CTLA-4, FOXP3, PD-1, TIGIT, LAB-3, 2B4, BTLA, TIM3, VISTA, or CD96.
145. The method of embodiment 143, wherein with the phenotype is activation and the assessing comprising measuring the expression, optionally surface expression, of one or more markers selected from CD25, CD26, CD27, CD28, CD30, CD71, CD154, CD40L, CD127, LAG3, or Ki67.
146. The method of embodiment 143, wherein the phenotype is differentiation state and the assessing comprises measuring one or more markers selected from (i) one or more of CD25, CD45RO, CD56, KLRG1, CD95 and/or (ii) one or of CD45RA, CD27, CD28, CD62L, and CCR7, optionally wherein the one or more markers are markers are positively or inversely associated with naive-like T cells.
147. The method of any of embodiments 127-146, comprising assessing one or more activities of the one or more cells of the output composition.
148. The method of any of embodiments 127-147, wherein the one or more activities comprises a CAR-dependent activity, optionally an antigen-stimulated activity.
149. The method of embodiment 147 or 148, wherein the one or more activities comprises cytolytic activity or cytokine production.
150. The method of any of embodiments 127-149, wherein the period of time is at least or at least about 11 days, 12 days, 13 days, 14 days, or 15 days.
151. The method of any of embodiments 127-149, wherein the period of time is or is about 11 days, 12 days, 13 days, 14 days or 15 days.
152. The method of any of embodiments 127-151, wherein the input composition comprises cells that have been exposed or contacted with a test agent or compound prior to the incubation, optionally wherein the exposing or contacting is carried out during one or more steps of a process for producing the input composition comprising the T cells expressing the CAR.
153. The method of any of embodiments 127-152, wherein the method is carried out on a plurality of input compositions, each of said input compositions of the plurality being produced by a different process.
154. The method of any of embodiments 127-153, further comprising comparing the phenotype or activity of the output composition to the phenotype or activity of a control composition, optionally wherein the control composition is a composition of T cells that have been incubated for the at least 10 days under the same conditions to stimulate the CAR-dependent activity, said composition of T cells having not been produced in the presence of the test agent or compound or having been produced by an alternative process compared to the input composition.
155. The method of any of embodiments 127-154, further comprising identifying an output composition that exhibits reduced exhaustion, reduced activation or decreased differentiation, optionally wherein the decreased differentiation comprises increased expression of one more naive-like T cell markers.

## Claims

1. A surface modified particle, comprising (i) a particle that is a bead comprising a diameter of between 2 µm and 5 µm and (ii) a binding molecule attached to the surface of the bead, wherein:
the binding molecule specifically binds to an extracellular antigen-binding domain of a chimeric antigen receptor; and
the binding molecule comprises a recombinant antigen that is BCMA or a portion thereof recognized by the antigen-binding domain.

2. The surface modified particle of claim 1, wherein the binding molecule does not bind or recognize a linker or spacer region of the recombinant antigen receptor, said linker or spacer region connecting the antigen-binding domain to the transmembrane domain of the antigen receptor.

3. The surface modified particle of claim 1 or 2, wherein the binding molecule is a fusion polypeptide comprising the recombinant antigen or the portion thereof linked to a moiety, optionally wherein the moiety facilitates attachment to the particle, further optionally wherein:
(i) the moiety is linked to the C-terminus of the recombinant antigen; and/or
(ii) the moiety is or comprises an Fc domain.

4. The surface modified particle of any of claims 1-3, wherein:
(i) the BCMA antigen is or comprises a polypeptide with an amino acid sequence with at least 70%, 75%, 80%, 85%, 86%, 87%, 88%, 89%, 90%, 91%, 92%, 93%, 94%, 95%, 96%, 97%, 98%, 99% or more sequence identity to SEQ ID NO: 1 or a fragment thereof containing at least 50, at least 55, at least 60, at least 65, at least 70, at least 75, at least 80, at least 85, at least 90, at least 95, at least 100, at least 110, at least 120, at least 130, at least 140, at least 150, at least 160, at least 170, or at least 180 contiguous amino acids of SEQ ID NO: 1; and/or
(ii) the BCMA antigen is or includes the sequence set forth in SEQ ID NO:1 or a portion thereof that is or contains an epitope recognized by the chimeric antigen receptor.

5. The surface modified particle of any of claims 1-4, wherein:
(i) the binding molecule is attached to the particle at or within 100 amino acids of the C-terminus of the binding molecule;
(ii) the binding molecule is covalently attached to the particles; and/or
(iii) the binding molecule is covalently attached to the particles via a surface exposed functional group,
optionally wherein the surface exposed functional group is selected from an amino group, a carboxyl group, a thiol group, an aldehyde group, a chloromethyl group, an epoxy group, a hydroxyl group, a tosyl group or a hydrazine group,
further optionally wherein the surface exposed functional group is a tosyl group.

6. The surface modified particle of any of claims 1-5, wherein:
(i) the particle has a diameter of 2.8 µm or 4.5 µm; and/or
(ii) the particle comprises at least 10 copies, 10² copies, 10³ copies, 10⁴ copies, 10⁵ copies or 10⁶ copies of the binding molecule.

7. The surface modified particle of any of claims 1-6, wherein the particle further comprises at least one agent attached to the surface of the particle,
wherein the at least one agent specifically binds to an additional molecule on a cell to provide an accessory signal and/or to block an inhibitory signal, optionally wherein:
(i) the at least one agent is a ligand or is an antibody or antigen-binding fragment thereof;
(ii) the additional molecule is:
(a) a costimulatory molecule or an activating co-receptor, optionally OX-40, ICOS, DAP10, B7-1, B7-2, CD28 or 4-1BB;
(b) an inhibitory receptor; optionally CTLA-4, PD-1, LAG-3, Tim-3, BTLA or TIGIT;
(c) T cell surface protein, optionally CD2 or CD3; or
(d) CD2 or CD28, optionally wherein the particle comprises one or both of an anti-CD2 antibody and an anti-CD28 antibody;
(iii) the at least one agent is covalently attached to the particle; and/or
(iv) the ratio of the binding molecule and the at least one agent attached to the particle is 1:1.

8. A composition, comprising a plurality of the surface modified particles of any of claims 1-7.

9. The composition of claim 8, wherein:
(i) the concentration of the binding molecule in the composition is between 0.5 µg/mL and 500 µg/mL, 1 µg/mL and 200 µg/mL or 5 µg/mL and 100 µg/mL L; or
(ii) the concentration of the binding molecule in the composition is at least 1 µg/mL, 5 µg/mL, 10 µg/mL, 25 µg/mL, 50 µg/mL, 100 µg/mL or 200 µg/mL.

10. A method for expanding cells, comprising incubating a population of cells with the surface modified particle of any of claims 1-7 or the composition of claim 8 or claim 9.

11. A method of expanding cells, comprising incubating an input composition, said input composition comprising cells expressing a chimeric antigen receptor comprising an extracellular antigen-binding domain that specifically binds or recognizes an antigen, with a plurality of particles that are or comprise beads, wherein the plurality of particles comprise a mean diameter of between 2 µm and 5 µm and have attached a binding molecule that specifically binds to or recognizes the antigen-binding domain, wherein binding of the binding molecule to the antigen-binding domain induces expansion of the cells comprising the chimeric antigen receptor, thereby producing an output composition comprising expanded cells;
and wherein the binding molecule comprises a recombinant antigen that is BCMA or a portion thereof recognized by the antigen-binding domain.

12. The method of claim 11, wherein:
(i) during the incubating, the ratio of total cells present in the input composition to the plurality of surface modified particles of the composition is:
(a) from 5:1 to 1:5, inclusive;
(b) from 3:1 to 1:3, or 2:1 to 1:2, each inclusive; and/or
(c) 1:1;
(ii) the plurality of beads comprise a mean diameter of between or between about 2 µm and 5 µm or a mean density of between or between about 1 g/cm³ and about 2 g/cm³;
(iii) the incubation is carried out for greater than:
(a) 2 hours, 4 hours, 8 hours, 12 hours, 24 hours, 2 days, 3 days, 4 days, 5 days, 6 days, 7 days, 8 days, 9 days, 10 days, 11 days, 12 days; and/or
(b) 10 days, 11 days, 12 days, 13 days, 14 days, 15 days, or 16 days;
(iv) the cells comprise immune cells or induced pluripotent stem cells (iPSC);
(v) the immune cell is a T cell or an NK cell;
(vi) the cells comprise one or both of CD4+ T cells and CD8+ T cells;
(vii) the ratio of the CD4+ cells to the CD8+ cells is between 1:2 and 2:1, or 1:3 and 3:1, or is 1:1; and/or
(viii) the cells are primary cells obtained from a subject, optionally a human subject.

13. A method of genetically engineering a cell, comprising:
(a) contacting a composition of cells with a nucleic acid molecule encoding a chimeric antigen receptor under conditions to introduce the nucleic acid molecule into one or more cells in the composition, thereby producing an input composition; and
(b) incubating cells of the input composition according to the method of any of claim 11 or claim 12.

14. The method of claim 13, wherein:
(i) at least a portion of the contacting and incubating are carried out simultaneously;
(ii) the contacting is carried out by transduction with a viral vector, optionally wherein the viral vector is a retroviral vector, a gamma-retroviral vector, or a lentiviral vector; and/or
(iii) the number of cells in the output composition comprising the chimeric antigen receptor is increased or enriched by 1.2-fold, 2.0-fold, 3.0-fold, 4.0-fold, 5.0-fold, 6.0-fold, 7.0-fold, 8.0-fold, 9.0-fold, 10-fold or more compared to the number of the cells comprising the antigen receptor in the input composition.

15. A method of selecting or enriching cells, comprising contacting a population of cells with the surface modified particle of any of claims 1-7 or the composition of claim 8 or claim 9.
